# EUROPEAN PATENT SPECIFICATION

(11) **EP 3 268 480 B1**
(45) Date of publication and mention of the grant of the patent: **19.05.2021**
(21) Application number: 16760977.5
(22) Date of filing: 09.03.2016
(51) Int. Cl.: C12N 15/85, C12N 15/09, C12N 5/10

(54) **TOOLS AND METHODS FOR USING CELL DIVISION LOCI TO CONTROL PROLIFERATION OF CELLS**
WERKZEUGE UND VERFAHREN ZUR VERWENDUNG VON ZELLTEILUNGS-LOCI ZUR KONTROLLE DER PROLIFERATION VON ZELLEN
OUTILS ET PROCÉDÉS DESTINÉS À UTILISER DES LOCUS DE DIVISION CELLULAIRE POUR MAÎTRISER LA PROLIFÉRATION DE CELLULES

(30) Priority: 09.03.2015 US 201562130258 P; 09.03.2015 US 201562130270 P
(43) Date of publication of application: 17.01.2018
(73) Proprietor: Sinai Health System, Toronto ON M5G 1X5 (CA)
(72) Inventor: NAGY, Andras, Toronto, Ontario M6G 4C2 (CA); MONETTI, Claudio, Toronto, Ontario M6P 4B8 (CA); LIANG, Qin, Toronto, Ontario M6E 3P6, (CA)
(74) Representative: Barker Brettell LLP
(86) International application number: PCT/CA2016/050256
(87) International publication number: WO 2016/141480

(56) References cited:
- WO-A2-2009/139921
- TIONG-TI LIM ET AL: "Lentiviral Vector Mediated Thymidine Kinase Expression in Pluripotent Stem Cells Enables Removal of Tumorigenic Cells", PLOS ONE, vol. 8, no. 7, 30 July 2013 (2013-07-30), page e70543, XP055396844, DOI: 10.1371/journal.pone.0070543
- JUNG JUYEON ET AL: "Ablation of tumor-derived stem cells transplanted to the central nervous system by genetic modification of embryonic stem cells with a suicide gene", HUMAN GENE THERAPY, LIEBERT, US, vol. 18, no. 12, 14 December 2007 (2007-12-14), pages 1182-1192, XP002559843, ISSN: 1043-0342, DOI: 10.1089/HUM.2007.078 [retrieved on 2007-11-20]
- WEIQIANG LI ET AL: "Safeguarding clinical translation of pluripotent stem cells with suicide genes", ORGANOGENESIS, vol. 9, no. 1, 1 January 2013 (2013-01-01) , pages 34-39, XP055461402, US ISSN: 1547-6278, DOI: 10.4161/org.24317
- LEE HAN ET AL: "Induced pluripotent stem cells in regenerative medicine: an argument for continued research on human embryonic stem cells", REGENERATIVE MEDICINE, vol. 4, no. 5, September 2009 (2009-09), pages 759-769, XP009505580,
- RONG Z ET AL.: 'A scalable approach to prevent teratoma formation of human embryonic stem cells' THE JOURNAL OF BIOLOGICAL CHEMISTRY vol. 287, no. 39, 04 August 2012, ISSN 0021-9258 pages 32338 - 32345, XP055311258
- HARA A ET AL.: 'Neuron-like differentiation and selective ablation of undifferentiated embryonic stem cells containing suicide gene with Oct-4 promoter''.' STEM CELLS AND DEVELOPMENT vol. 17, no. 4, 2008, ISSN 1547-3287 pages 619 - 628, XP055016439
- SARIN KY ET AL.: 'Conditional telomerase induction causes proliferation of hair follicle stem cells' NATURE vol. 436, 18 August 2005, ISSN 0028-0836 pages 1048 - 1052, XP055311269
- KYBA M: 'Enhanced hematopoietic differentiation of embryonic stem cells conditionally expressing Stat5' PNAS vol. 100, 30 September 2003, ISSN 0027-8424 pages 11904 - 11910, XP002274603
- WANG T ET AL.: 'Identification and characterization of essential genes in the human genome' SCIENCE vol. 350, no. 6264, 27 November 2015, ISSN 0036-8075 pages 1096 - 1101, XP055311281
- MALECKI M: 'Above all, do no harm': safeguarding pluripotent stem cell therapy against iatrogenic tumorigenesis' STEM CELL RESEARCH AND THERAPY vol. 5, no. 3, 03 June 2014, ISSN 1757-6512 XP021208900

### SEQUENCE LISTING

<110> Mount Sinai Hospital Nagy, Andras Monetti, Claudio Liang, Qin
<120> TOOLS AND METHODS FOR USING CELL DIVISION LOCI TO CONTROL PROLIFERATION OF CELLS
<130> 35941-2013
<150> US 62/130,258
   <151> 2015-03-09
<150> US 62/130,270
   <151> 2015-03-09
<160> 152
<170> PatentIn version 3.5
<210> 1
   <211> 562
   <212> DNA
   <213> Artificial Sequence
<220>
   <223> Synthetic DNA
<400> 1
   gtcgacggtt ctggcgtgaa acagactttg aattttgacc ttctcaagtt ggcgggagac      60
   gtggagtcca acccagggcc catggccagc tacccctgtc accagcacgc cagcgccttc     120
   gaccaggccg ccagaagcag gggccacagc aaccggcgga ccgccctgag acccaggcgg     180
   cagcaggaag ccaccgaagt ccggctggaa cagaagatgc ccaccctgtt aaccctagaa     240
   agatagtctg cgtaaaattg acgcatgcat tcttgaaata ttgctctctc tttctaaata     300
   gcgcgaatcc gtcgctgtgc atttaggaca tctcagtcgc cgcttggagc tcccgtgagg     360
   cgtgcttgtc aatgcggtaa gtgtcactga ttttgaacta taacgaccgc gtgagtcaaa     420
   atgacgcatg attatctttt acgtgacttt taagatttaa ctcatacgat aattatattg     480
   ttatttcatg ttctacttac gtgataactt attatatata tattttcttg ttatagatat     540
   cgtggcggcc gcattaccgc gg                                              562
<210> 2
   <211> 2031
   <212> DNA
   <213> Artificial Sequence
<220>
   <223> Synthetic DNA
<400> 2
   gtcgacatta ccgcgggttt tgttacttta tagaagaaat tttgagtttt tgtttttttt 60
   taataaataa ataaacataa ataaattgtt tgttgaattt attattagta tgtaagtgta      120
   aatataataa aacttaatat ctattcaaat taataaataa acctcgatat acagaccgat      180
   aaaacacatg cgtcaatttt acgcatgatt atctttaacg tacgtcacaa tatgattatc      240
   tttctagggt taagggtgta catcgacggc ccccacggca tgggcaagac caccaccacc      300
   cagctgctgg tggccctggg cagccgggac gacatcgtgt acgtgcccga gcccatgacc      360
   tactggcagg tgctgggcgc cagcgagacc atcgccaaca tctacaccac acagcacagg      420
   ctggaccagg gcgagatctc tgccggcgac gccgccgtgg tgatgaccag cgcccagatc      480
   acaatgggca tgccctacgc cgtgaccgac gccgtgctgg cccctcacgt gggcggcgag      540
   gccggctcta gccacgcccc tccccctgcc ctgaccctga tcttcgaccg gcaccccatc      600
   gcccacctgc tgtgctaccc tgccgccaga tacctgatgg gcagcatgac cccccaggcc      660
   gtgctggcct tcgtggccct gatccccccc accctgcccg gcaccaacat cgtgctggga      720
   gccctgcccg aggaccggca catcgaccgg ctggccaagc ggcagagacc cggcgagcgg      780
   ctggacctgg ccatgctggc cgccatccgg cgggtgtacg gcctgctggc caacaccgtg      840
   agatacctgc agggcggagg gtcttggtgg gaggactggg gccagctgtc cggcaccgcc      900
   gtgccacctc agggcgccga gccccagagc aatgccggcc ctcggcccca catcggcgac      960
   accctgttta ccctgttcag agcccccgag ctgctggccc ccaacggcga cctgtacaac     1020
   gtgttcgcct gggccctgga cgtgctggcc aagaggctgc ggcccatgca cgtgttcatc     1080
   ctggactacg accagagccc tgccggctgc agggacgccc tgctgcagct gaccagcggc     1140
   atggtgcaga cccacgtgac cacccccggc agcatcccca ccatctgcga cctggcccgg     1200
   accttcgccc gggagatggg cgaggccaac ggctccggag agggcagagg aagtctgcta     1260
   acatgcggtg acgtcgagga gaatcctggc ccactcgaga tggccacaac catggtgagc     1320
   aagggcgagg aggataacat ggccatcatc aaggagttca tgcgcttcaa ggtgcacatg     1380
   gagggctccg tgaacggcca cgagttcgag atcgagggcg agggcgaggg ccgcccctac     1440
   gagggcaccc agaccgccaa gctgaaggtg accaagggtg gccccctgcc cttcgcctgg     1500
   gacatcctgt cccctcagtt catgtacggc tccaaggcct acgtgaagca ccccgccgac     1560
   atccccgact acttgaagct gtccttcccc gagggcttca agtgggagcg cgtgatgaac     1620
   ttcgaggacg gcggcgtggt gaccgtgacc caggactcct ccctgcagga cggcgagttc     1680
   atctacaagg tgaagctgcg cggcaccaac ttcccctccg acggccccgt aatgcagaag    1740
   aagaccatgg gctgggaggc ctcctccgag cggatgtacc ccgaggacgg cgccctgaag     1800
   ggcgagatca agcagaggct gaagctgaag gacggcggcc actacgacgc tgaggtcaag     1860
   accacctaca aggccaagaa gcccgtgcag ctgcccggcg cctacaacgt caacatcaag     1920
   ttggacatca cctcccacaa cgaggactac accatcgtgg aacagtacga acgcgccgag     1980
   ggccgccact ccaccggcgg catggacgag ctgtacaagg ctagcaagct t 2031
<210> 3
   <211> 1848
   <212> DNA
   <213> Artificial Sequence
<220>
   <223> Plasmid DNA
<400> 3
   gcggccgcat ccaggcggcc gagatccgaa gttcctatac tatttgaaga ataggaactt 60
   cagatccccc gggctgcagg atctaccggg taggggaggc gcttttccca aggcagtctg 120
   gagcatgcgc tttagcagcc ccgctgggca cttggcgcta cacaagtggc ctctggcctc 180
   gcacacattc cacatccacc ggtaggcgcc aaccggctcc gttctttggt ggccccttcg 240
   cgccaccttc tactcctccc ctagtcagga agttcccccc cgccccgcag ctcgcgtcgt 300
   gcaggacgtg acaaatggaa gtagcacgtc tcactagtct cgtgcagatg gacagcaccg 360
   ctgagcaatg gaagcgggta ggcctttggg gcagcggcca atagcagctt tgctccttcg 420
   ctttctgggc tcagaggctg ggaaggggtg ggtccggggg cgggctcagg ggcgggctca 480
   ggggcggggc gggcgcccga aggtcctccg gaggcccggc attctgcacg cttcaaaagc 540
   gcacgtctgc cgcgctgttc tcctcttcct catctccggg cctttcgacc tgcagcaatt 600
   aatcatcggc atagtatatc ggcatagtat aatacgacaa ggtgaggaac taaaccatgg 660
   gatcggccat tgaacaagat ggattgcacg caggttctcc ggccgcttgg gtggagaggc 720
   tattcggcta tgactgggca caacagacaa tcggctgctc tgatgccgcc gtgttccggc 780
   tgtcagcgca ggggcgcccg gttctttttg tcaagaccga cctgtccggt gccctgaatg 840
   aactgcagga cgaggcagcg cggctatcgt ggctggccac gacgggcgtt ccttgcgcag 900
   ctgtgctcga cgttgtcact gaagcgggaa gggactggct gctattgggc gaagtgccgg 960
   ggcaggatct cctgtcatct caccttgctc ctgccgagaa agtatccatc atggctgatg 1020
   caatgcggcg gctgcatacg cttgatccgg ctacctgccc attcgaccac caagcgaaac 1080
   atcgcatcga gcgagcacgt actcggatgg aagccggtct tgtcgatcag gatgatctgg 1140
   acgaagagca tcaggggctc gcgccagccg aactgttcgc caggctcaag gcgcgcatgc 1200
   ccgacggcga tgatctcgtc gtgacccatg gcgatgcctg cttgccgaat atcatggtgg 1260
   aaaatggccg cttttctgga ttcatcgact gtggccggct gggtgtggcg gaccgctatc 1320
   aggacatagc gttggctacc cgtgatattg ctgaagagct tggcggcgaa tgggctgacc 1380
   gcttcctcgt gctttacggt atcgccgctc ccgattcgca gcgcatcgcc ttctatcgcc 1440
   ttcttgacga gttcttctga tcgagaaatt gatgatctat taaacaataa agatgtccac 1500
   atggaagttt ttcctgtcat actttgttaa gaagggtgag aacagagtac ctacattttg 1560
   aatggaagga ttggagctac gggggtgggg gtggggtggg attagataaa tgcctgctct 1620
   ttactgaagg ctctttacta ttgctttatg ataatgtttc atagttggat atcataattt 1680
   aaacaagcaa aaccaaatta agggccagct cattcctccc actcatgatc tatagatccg 1740
   gtcgacgaag ttcctatact atttgaagaa taggaacttc ggatctggcc ggcctaggga 1800
   taacagggta atggatcccc cgggctgcag gaattcgata tcaagctt 1848
<210> 4
   <211> 8003
   <212> DNA
   <213> Artificial Sequence
<220>
   <223> synthetic DNA
<400> 4
   ggtctacctt aagccaaggt agaaatactt ggttgaaaaa tgtttctgct gccaggcagt      60
   ggcagcacac accagcactt ggggggacag aggcaggcag atctcttgag tttaaggcca     120
   gcctagtcta tagagcaagt cctggaacac ccagggctac acagagaaac cttgtctcag     180
   aaaaccaaac aaaaagttct gttctggtag tgttggctaa ctgctttttt attgccactt     240
   cactcttgtt tgtgtggtct gtgaaaagga agacagaccc tttggcatct ttgtcagggg     300
   agattgatgg cctgggggga cctccaggaa ggggcttatt gtagttttag gggttctcgt     360
   ttctctgtgt tgaacttccg cagagctgtg cttcctcctg attggacctg ctgtgctcat     420
   tcagatcaat taaaaggcag gttttgctgt gtgagggaga ttattgtgaa agatttagga     480
   gccagcctac tctagctggg ggagggagca ctagggattg aaccccagtc tttggcccct     540
   gttgcttggg tgtcctgtca ctgagctgtg cccttggccc tgtcatcagg ctttgtcagt     600
   ggattcccat gtggttgtct gttttattat tttcctctgc aatggggctt gaaccatggg     660
   ccacacacac acactcacac actctgctcc ttgttttgag aacgtctcac tgacttgccc     720
   tggttggccc tgaggtcact ggcgtagata gactttggac ctgggactcc tacagcggcc     780
   ttcccagtag taggtctaca gcaccagact aggttcctct ggtatttctg aaagggcatt     840
   ggcttgtata atacaggaac attggcatct tgggaggtgt gagcctggag tctacagtgt     900
   cggtcccctg tgtgtcattt ggattttgag gcagggtctc ctgtagccct gactggcctc     960
   attctctggg tgctaagatt acacagatgt gtgccctgct ggggccctgg tcctgctcgt    1020
   tttgcctgat agggaagcta gccacccact gagcagcctt ctcagtcctg tcttgtttat    1080
   tcttttttat tttttcacag tttatgatat gactcgtctt taatggctgt aactgctgct    1140
   gacctcattg cattgttcat tgaggtcatt cactattttg ataaataaac tgtagttcta    1200
   aatcactaat catttgaatc ccctcctacc gctttggctt agatttcttt tcatttttct    1260
   tttttttctt tttttttggt ttttttgaga cagggtttct ctgtgtagct ctggctgtcc 1320
   tggaactcac tttgtagacc aggctggcct caaactctgc ctccagagtg ctggtattaa 1380
   agttgtatgc cagccaggta tatgccaccc aggtatatgc cagccaggat atggctgctt 1440
   tggtcctttg actttagaga tctgaaaagc aagaggtcta tggtacttac ccactaatga 1500
   gcagaaatca gaaataaaat aggttcaact tagtaatttg catatagcta attgtctgaa 1560
   gttcttggtg acacctgcac agcttcctaa aagacaaccg tgtgtggtct gtcctgtgcc 1620
   aggtagtgac gctgtggtac cgatctccag aagtgttgct gggctcggct cgttactcca 1680
   ctccggttga catctggagt atagggacca tatttgcaga actggccacc aagaagccgc 1740
   ttttccacgg cgactcagag attgaccagc tcttcaggat cttcaggtaa ttgtgtttta 1800
   tacctaagct gttaaaagcc tgtatggtta aaataataaa ggcaccgtgt atgtgatagg 1860
   acttttgctt cctttcagag ctctgggcac tcctaacaac gaagtgtggc cagaagtcga 1920
   gtccctgcag gactacaaga acacctttcc caagtggaag ccggggagcc tcgcatccca 1980
   cgtcaagaac ctggacgaga acggcttgga tttgctctca gtaagtgagc tgttctttat 2040
   ggttcgagcg tggtgccctt gggtgtcagt agtgacaggt aagggtgact ggatgtactc 2100
   agtcctggac ttgtcagcac gaagacacag cagctgagtc ggatgcccgg taatcctgta 2160
   gtgacagcga gctgcctttg aggtgtgaag tgcctggtgg tctgaggagc ctctggtgtt 2220
   ggactttgtc atttacttct catccggtag gagaggctgg actcacacac cttcttgctt 2280
   gagagtatgt tgatgagtta caatgactca ctcctctctg tttgctactg agccctcagt 2340
   tcatcggttt acactgttga ctgcttagga tactgagctg ttcaggcttt tgacagcata 2400
   actccattgc tcaggtctgg ccttgtttcc tcagaaaagc tagcaaagac taattaattc 2460
   tgattttctt cagttgtctt ggtgcacaat attagcaatg caaagaacaa aattactgtt 2520
   caggattaat aaaggtctcc tttaggaaat ttgctgtagg tagagcaggg tttaaaatct 2580
   gagtttgtag aaggaatcaa gtagacgatt cagttggtct ttccgctccc ttgcaatcta 2640
   taaacgatgc caccaaatgg ggctcagatt tgtccagacc cctacagctg tcacatagtt 2700
   tgttacatat tggtattggc taacttagaa gccttcccgt agcctgggtc caatctgctg 2760
   gtattatcta aagttattag ctctgtgtgt gtgtgtgtgt gtgtgtgtgt gtgtgtgtgt 2820
   gtgtgtgtgt tgctgggggc tatggacaga aggcagagct ttcttgaggt ggtgtaggaa 2880
   ggttggtcat tgactgttga ggcgcagccc tacttctcag tctttaaaca aaggacttca 2940
   cttaacagac atcagttctg ggcaccaatt tcctctgttg agccaacacc ctgcaatagc 3000
   taagcttgcc gtggccctgc tgcctgctga gcactgtggc tatcactgcc ctgtccccca 3060
   ctccagcggc agactgctga gttagcaggg cctgtgtcta tcgcaggacc ccagaagcaa 3120
   actgccccag ttggagacca ctggattatt gatgagactt ggcttcagtt tattttctcc 3180
   cacattgatg gattcgggca agtttcttgc tgtccttgaa agacttggtg tcttaagcca 3240
   aacagtgtgg cacatctttc tcatatgtct gtgcggactg acagacatat cctcaaggcc 3300
   ttcatattta tttgtttgcc tttttaaaaa tttacttatt tattttacat atgcaaatac 3360
   actgttgctg tcctcagaca cagtagaaga gggcatcgga tcccattaca gatggttgtg 3420
   agccaccatg tggttgctgg gagttgaact caggacttct ggaagagtag tcatcgctct 3480
   taattgctga gccatctctc cacctcctta atctattttc tctgaagata atatgcatga 3540
   tcagatttaa gatctggagt atttaaatta gtttacaata attagtccat ctattataac 3600
   tgtgtttata aaaatgctat ggggtttttc ctttgttggc ttgacgaagg ggttgtgtgg 3660
   acactatcat gaaacactat aactgattgc tactttttca gttgtgaata acagatggac 3720
   atttaatttc tagggtagat ataattagct agataatgtg aagatttatg ttttggtttt 3780
   aagctgtctg ggtgcttatc ttataattta taacaggtta ccaaggtgtc tgtgtgtgat 3840
   ttaagtatct tggagaggta aatagagggc aggcttctaa atttatttcc ctttttctct 3900
   ctagaaaatg ctagtctatg atcctgccaa acgaatctct ggcaaaatgg ccctgaagca 3960
   cccgtacttt gatgacttgg acaatcagat taagaagatg tagccctctg gatggatgtc 4020
   cctgtctgct ggtcgtaggg gaagatcgtg ttgtttaccg ttggctctct tcctgtcttg 4080
   tatagttttc tttgtttgta aactgtcatc tggacttttc ttaatttcct acgtataact 4140
   taattaacat gtaaatatta ttccatatga atttaaatat aattctgtat atgtgcagat 4200
   gtcactgtgg tggctgttaa ttactataac acaagtgtta attactacaa cataagactt 4260
   gagtctccct agacttccca gcagccattc ctgcagctcg gagcacagtt gaaggagctg 4320
   agctcaggcc tcgtgatgct ttcaagtgcc tccgtgttct ggatatatat gattcctggt 4380
   cagtttcttg ccatttggaa actacaaccc acctacggac agtgtttttc tacttgtgct 4440
   taagcagttg ggatgagaag gccaaagacc cgaggatgtc tagagtaatg acccccagat 4500
   ggaagtgcac caaagctggc tgggtttcac agctagagat caggggctgt ccagagcagg 4560
   acagcttaga acatttatga agactcccta tttttaggtt tgttgtaaag ctgttgtcta 4620
   gttggattcc tgtgctctgc atggtcagag gtaggttaga ggatttgcct tggcttctaa 4680
   atccaatttg aaaactgctt aaaatctcct gtcctctctt agcagtgtct aaaaatgtcc 4740
   ttgtccaaat atttagctga gattcctcac tttggaaaag gagccgtatc gctgtgctgc 4800
   ttagtgtaat tcttagaagc agcctgactt atctgctagc agtcaaaggg atgcctgaga 4860
   cgactgctcc tcttagaact aaaggctggg atgcttaagt ttgtctactg tttggaggat 4920
   ctcggtaaga ctgagcccct gttcctgtgg ccacctcagt ttaccagtac ctcagcctca 4980
   gcctcctgca tttgctggag tcagggaagg tccccagccc tgagccctga cgctcctgat 5040
   tgtagagact gtcagttgga ggtaaaacgt tcattgaagt agtcagcagc cacatgcatt 5100
   tagggcactc cagtgtcaga gaccatcctg gaggtttcta accctgccgc tggcagtcta 5160
   ctcccaagac agatcagtta gagtggtcag caaacaccaa ctgctgcaga aacctgtgtt 5220
   ggtgtggttc cctgctgctg ctgggaactg ggcccaagac tagagagctt ggggctgcgg 5280
   ttgatcatgg gttctgttcc tgcattacac cttgaaatcc aagccttcta atatctccct 5340
   tcggatcata agttgtgaat ttggtcctcc gcccccgcca ggttttctat acttgggttt 5400
   gtcttgctga cattttcaag agtcctgact aagacggtga ttagtgtgac atgacttgag 5460
   aactaccgat ttgaagcaca cttgaagtta acaaattctc tcatgattat acttttaact 5520
   ttttataaga ttgcttgagc tcacccagat ctctgttggg aagtaactgg gtaacaaaag 5580
   ccgttgcact ggtttgacag ctaacaactg ttggtacttt gtattcagaa ggaatgaggt 5640
   agcgattgaa tggctggggt gttgtttcca cagtttatac actaaaaatt tgggtagcca 5700
   ggaggtagtg gcgcgcaatc tttaatctca gcagaggcag tcctatctct tgagttggag 5760
   gccagcctgc ctgagttcca gaccagccag ggctacagga agaagtctta aaaaaatttt 5820
   ttttccctgt ggatgtaaac ccatgagaat gactgctgta tctatcactt tctgtataat 5880
   agaggtttct tccgccgcag cgctccattg gtctcagccc ctcatgttgg gggcgggaag 5940
   gaggggcaag cgtccccaag cacaggaagg cctgctgaag agttggcggc tttgagggtt 6000
   caaatcctgc ttgggtctga ggagtccccc aaacgatctt ggactgtaaa aacctgccag 6060
   gaacaagcgc taagggatgg ttctcaacct gtgggtcgcg accccctggg gagttggaca 6120
   gccctttcct gaggatcaga acaaacatcc tgcatatcag atattaacgt ggcatttcat 6180
   aactgcaaac ttacagttag gaagtagcag tgacaaagaa ttttatgggt ggggtggggt 6240
   gggggctcac caccatatta aagggttaca gcattaggag ggttgacaac agaaccatcg 6300
   cactgaggga aagtgagtta gcacgaagag atgagcattt cccagaacac agaataagac 6360
   acgtttaacc aacatcagtc tgtaacccca gcggactgtg acaagctctc acccatgccg 6420
   tggagctcag aactagcatt gcacgctttt accccatctt tggttgcctc actccctttg 6480
   agtagtaagt ccctcttcaa agtaatagcc tctaaacagc ctgctgcgca tgctctagtc 6540
   ctttgctttc ctccacgggt aggccacagg cttaagcagc agttggaaat ggaacttggg 6600
   ctttctggct cctggtttag aaaactcttg aaatggacaa gatttagtcc taggcaagtt 6660
   ctgtgtagtc agcaatggaa gcaaaggggg gtggggcaaa cattccagtc aggtggaatc 6720
   tgagcccttg agagtgtgag gcaggaggat cactaggcag gagttctcaa ccagcctaga 6780
   cgacagcttg gaacccatcg cactccacaa tgaggacccc tcagcttctg aagagaggag 6840
   agctgctagc tctgagaagg gtgtataaaa tgggcttttg gttcccagac atcagacttg 6900
   aagaggcaac cgagtaagcc agctatagac taagtaagat tgtcgtacct agcctactcc 6960
   ttgggagctc attcttcaga gtatgttgat ggatgaccta acgccatctc agatgctgga 7020
   gcattttgct ggaaaggctc tcagcactag cgaggctgcc tcaatgtaat cctgcctcag 7080
   cctgagaact gattatgagt taccgtttgg tactttatgt atttttctct cttcctagta 7140
   accaaggaca aacctgcttc cttccagcca cgtggcagac actagctgta ctaaagtatt 7200
   attacacacc ggtcccctgc tgccagaaca aagaactcag cctcgagaaa tttctcctat 7260
   tatatttggg atccagcatg tcccaagaca gggacgactc aataatttaa tgtgcctaat 7320
   ggcacggaat aaaaggatta ttcacaaaca gaaaaccgga cttaagcaga tgtttatttg 7380
   aacacaaata ccataaccct ccttctcatg atgttcccaa ttacacgttg caacattttc 7440
   acgaagaagc tctttctaaa acggccttct agggagcgaa taaacaggac tatatttggc 7500
   taacagctgc ttctgaaaac tgagatgtca gcaaagcaag ctgttgttta aagaaaacta 7560
   attacccagc taccttgggc ttggatgggg gtaaataacg ccctcccgtt tcttggcagt 7620
   gatttgtctg ggattttaat actgttaaga ttttatttct cttcctccac ttctcctttg 7680
   gatcaaatgg tttttaaagg attccttgtg ttcctgctgc caatggcgag tcctttgcgt 7740
   ttgccgcccc tgcttcgtca ccaactgcta ttttgtttag tgcaaagcag tgtgtttaag 7800
   gacacactaa actttagatg acccagtgac acagtgatcc gtatttgcaa gcgtctggtt 7860
   atgcagatgt cattgtgttg tttatcagca atgtcagcaa tgtctttttt tttttttttt 7920
   tatgcacagg ctttgaaact tattttggaa attcattcat tttgagggga ggcagtgagc 7980
   tgggagttat acaatctctc ttt 8003
<210> 5
   <211> 50
   <212> DNA
   <213> Artificial Sequence
<220>
   <223> Synthetic DNA
<400> 5
   ccctgaagca cccgtacttt gatgacttgg acaatcagat taagaagatg 50
<210> 6
   <211> 50
   <212> DNA
   <213> Artificial Sequence
<220>
   <223> Synthetic DNA
<400> 6
   tagccctctg gatggatgtc cctgtctgct ggtcgtaggg gaagatcgtg 50
<210> 7
   <211> 755
   <212> DNA
   <213> Artificial Sequence
<220>
   <223> synthetic DNA
<400> 7
   tgtggcacat ctttctcata tgtctgtgcg gactgacaga catatcctca aggccttcat      60
   atttatttgt ttgccttttt aaaaatttac ttatttattt tacatatgca aatacactgt     120
   tgctgtcctc agacacagta gaagagggca tcggatccca ttacagatgg ttgtgagcca     180
   ccatgtggtt gctgggagtt gaactcagga cttctggaag agtagtcatc gctcttaatt     240
   gctgagccat ctctccacct ccttaatcta ttttctctga agataatatg catgatcaga     300
   tttaagatct ggagtattta aattagttta caataattag tccatctatt ataactgtgt     360
   ttataaaaat gctatggggt ttttcctttg ttggcttgac gaaggggttg tgtggacact     420
   atcatgaaac actataactg attgctactt tttcagttgt gaataacaga tggacattta     480
   atttctaggg tagatataat tagctagata atgtgaagat ttatgttttg gttttaagct     540
   gtctgggtgc ttatcttata atttataaca ggttaccaag gtgtctgtgt gtgatttaag     600
   tatcttggag aggtaaatag agggcaggct tctaaattta tttccctttt tctctctaga     660
   aaatgctagt ctatgatcct gccaaacgaa tctctggcaa aatggccctg aagcacccgt     720
   actttgatga cttggacaat cagattaaga agatg                                755
<210> 8
   <211> 842
   <212> DNA
   <213> Artificial Sequence
<220>
   <223> synthetic DNA
<400> 8
   ccctctggat ggatgtccct gtctgctggt cgtaggggaa gatcgtgttg tttaccgttg 60
   gctctcttcc tgtcttgtat agttttcttt gtttgtaaac tgtcatctgg acttttctta 120
   atttcctacg tataacttaa ttaacatgta aatattattc catatgaatt taaatataat 180
   tctgtatatg tgcagatgtc actgtggtgg ctgttaatta ctataacaca agtgttaatt 240
   actacaacat aagacttgag tctccctaga cttcccagca gccattcctg cagctcggag 300
   cacagttgaa ggagctgagc tcaggcctcg tgatgctttc aagtgcctcc gtgttctgga 360
   tatatatgat tcctggtcag tttcttgcca tttggaaact acaacccacc tacggacagt 420
   gtttttctac ttgtgcttaa gcagttggga tgagaaggcc aaagacccga ggatgtctag 480
   agtaatgacc cccagatgga agtgcaccaa agctggctgg gtttcacagc tagagatcag 540
   gggctgtcca gagcaggaca gcttagaaca tttatgaaga ctccctattt ttaggtttgt 600
   tgtaaagctg ttgtctagtt ggattcctgt gctctgcatg gtcagaggta ggttagagga 660
   tttgccttgg cttctaaatc caatttgaaa actgcttaaa atctcctgtc ctctcttagc 720
   agtgtctaaa aatgtccttg tccaaatatt tagctgagat tcctcacttt ggaaaaggag 780
   ccgtatcgct gtgctgctta gtgtaattct tagaagcagc ctgacttatc tgctagcagt 840
   ca 842
<210> 9
   <211> 30
   <212> DNA
   <213> Artificial Sequence
<220>
   <223> oligonucleotide
<400> 9
   tgtggcacat ctttctcata tgtctgtgcg 30
<210> 10
   <211> 30
   <212> DNA
   <213> Artificial Sequence
<220>
   <223> oligonucleotide
<400> 10
   gaagcagcct gacttatctg ctagcagtca 30
<210> 11
   <211> 2034
   <212> DNA
   <213> Artificial Sequence
<220>
   <223> synthetic DNA
<400> 11
   ggttctggcg tgaaacagac tttgaatttt gaccttctca agttggcggg agacgtggag        60
   tccaacccag ggcccataac ttcgtataat gtatgctata cgaagttatt aggtctgaag       120
   aggagtttac gtccagccaa gctagcttgg ctgcaggtcg tcgacggtat cgataagctt       180
   gatatcgaat ttcgagggcc cctgcaggtc aattctaccg ggtaggggag gcgcttttcc       240
   caaggcagtc tggagcatgc gctttagcag ccccgctggc acttggcgct acacaagtgg       300
   cctctggcct cgcacacatt ccacatccac cggtagcgcc aaccggctcc gttctttggt       360
   ggccccttcg cgccaccttc tactcctccc ctagtcagga agttcccccc cgccccgcag       420
   ctcgcgtcgt gcaggacgtg acaaatggaa gtagcacgtc tcactagtct cgtgcagatg       480
   gacagcaccg ctgagcaatg gaagcgggta ggcctttggg gcagcggcca atagcagctt       540
   tgctccttcg ctttctgggc tcagaggctg ggaaggggtg ggtccggggg cgggctcagg 600
   ggcgggctca ggggcggggc gggcgcgaag gtcctcccga ggcccggcat tctcgcacgc 660
   ttcaaaagcg cacgtctgcc gcgctgttct cctcttcctc atctccgggc ctttcgacct 720
   gcagccaata tgggatcggc cattgaacaa gatggattgc acgcaggttc tccggccgct 780
   tgggtggaga ggctattcgg ctatgactgg gcacaacaga caatcggctg ctctgatgcc 840
   gccgtgttcc ggctgtcagc gcaggggcgc ccggttcttt ttgtcaagac cgacctgtcc 900
   ggtgccctga atgaactgca ggacgaggca gcgcggctat cgtggctggc cacgacgggc 960
   gttccttgcg cagctgtgct cgacgttgtc actgaagcgg gaagggactg gctgctattg 1020
   ggcgaagtgc cggggcagga tctcctgtca tctcaccttg ctcctgccga gaaagtatcc 1080
   atcatggctg atgcaatgcg gcggctgcat acgcttgatc cggctacctg cccattcgac 1140
   caccaagcga aacatcgcat cgagcgagca cgtactcgga tggaagccgg tcttgtcgat 1200
   caggatgatc tggacgaaga gcatcagggg ctcgcgccag ccgaactgtt cgccaggctc 1260
   aaggcgcgca tgcccgacgg cgatgatctc gtcgtgaccc atggcgatgc ctgcttgccg 1320
   aatatcatgg tggaaaatgg ccgcttttct ggattcatcg actgtggccg gctgggtgtg 1380
   gcggaccgct atcaggacat agcgttggct acccgtgata ttgctgaaga gcttggcggc 1440
   gaatgggctg accgcttcct cgtgctttac ggtatcgccg ctcccgattc gcagcgcatc 1500
   gccttctatc gccttcttga cgagttcttc tgaggggatc gatccgtcct gtaagtctgc 1560
   agaaattgat gatctattaa acaataaaga tgtccactaa aatggaagtt tttcctgtca 1620
   tactttgtta agaagggtga gaacagagta cctacatttt gaatggaagg attggagcta 1680
   cgggggtggg ggtggggtgg gattagataa atgcctgctc tttactgaag gctctttact 1740
   attgctttat gataatgttt catagttgga tatcataatt taaacaagca aaaccaaatt 1800
   aagggccagc tcattcctcc cactcatgat ctatagatct atagatctct cgtgggatca 1860
   ttgtttttct cttgattccc actttgtggt tctaagtact gtggtttcca aatgtgtcag 1920
   tttcatagcc tgaagaacga gatcagcagc ctctgttcca catacacttc attctcagta 1980
   ttgttttgcc aagttctaat tccatcagaa gctgactcta gagcttggcg cgcc 2034
<210> 12
   <211> 1971
   <212> DNA
   <213> Artificial Sequence
<220>
   <223> synthetic DNA
<400> 12
   ggcgcgccat aacttcgtat aatgtatgct atacgaagtt atctatggcc agctacccct 60
   gtcaccagca cgccagcgcc ttcgaccagg ccgccagaag caggggccac agcaaccggc 120
   ggaccgccct gagacccagg cggcagcagg aagccaccga agtccggctg gaacagaaga 180
   tgcccaccct gttaagggtg tacatcgacg gcccccacgg catgggcaag accaccacca 240
   cccagctgct ggtggccctg ggcagccggg acgacatcgt gtacgtgccc gagcccatga 300
   cctactggca ggtgctgggc gccagcgaga ccatcgccaa catctacacc acacagcaca 360
   ggctggacca gggcgagatc tctgccggcg acgccgccgt ggtgatgacc agcgcccaga 420
   tcacaatggg catgccctac gccgtgaccg acgccgtgct ggcccctcac gtgggcggcg 480
   aggccggctc tagccacgcc cctccccctg ccctgaccct gatcttcgac cggcacccca 540
   tcgcccacct gctgtgctac cctgccgcca gatacctgat gggcagcatg accccccagg 600
   ccgtgctggc cttcgtggcc ctgatccccc ccaccctgcc cggcaccaac atcgtgctgg 660
   gagccctgcc cgaggaccgg cacatcgacc ggctggccaa gcggcagaga cccggcgagc 720
   ggctggacct ggccatgctg gccgccatcc ggcgggtgta cggcctgctg gccaacaccg 780
   tgagatacct gcagggcgga gggtcttggt gggaggactg gggccagctg tccggcaccg 840
   ccgtgccacc tcagggcgcc gagccccaga gcaatgccgg ccctcggccc cacatcggcg 900
   acaccctgtt taccctgttc agagcccccg agctgctggc ccccaacggc gacctgtaca 960
   acgtgttcgc ctgggccctg gacgtgctgg ccaagaggct gcggcccatg cacgtgttca 1020
   tcctggacta cgaccagagc cctgccggct gcagggacgc cctgctgcag ctgaccagcg 1080
   gcatggtgca gacccacgtg accacccccg gcagcatccc caccatctgc gacctggccc 1140
   ggaccttcgc ccgggagatg ggcgaggcca acggctccgg agagggcaga ggaagtctgc 1200
   taacatgcgg tgacgtcgag gagaatcctg gcccactcga gatggccaca accatggtga 1260
   gcaagggcga ggaggataac atggccatca tcaaggagtt catgcgcttc aaggtgcaca 1320
   tggagggctc cgtgaacggc cacgagttcg agatcgaggg cgagggcgag ggccgcccct 1380
   acgagggcac ccagaccgcc aagctgaagg tgaccaaggg tggccccctg cccttcgcct 1440
   gggacatcct gtcccctcag ttcatgtacg gctccaaggc ctacgtgaag caccccgccg 1500
   acatccccga ctacttgaag ctgtccttcc ccgagggctt caagtgggag cgcgtgatga 1560
   acttcgagga cggcggcgtg gtgaccgtga cccaggactc ctccctgcag gacggcgagt 1620
   tcatctacaa ggtgaagctg cgcggcacca acttcccctc cgacggcccc gtaatgcaga 1680
   agaagaccat gggctgggag gcctcctccg agcggatgta ccccgaggac ggcgccctga 1740
   agggcgagat caagcagagg ctgaagctga aggacggcgg ccactacgac gctgaggtca 1800
   agaccaccta caaggccaag aagcccgtgc agctgcccgg cgcctacaac gtcaacatca 1860
   agttggacat cacctcccac aacgaggact acaccatcgt ggaacagtac gaacgcgccg 1920
   agggccgcca ctccaccggc ggcatggacg agctgtacaa gatctgaatt c 1971
<210> 13
   <211> 1726
   <212> DNA
   <213> Artificial Sequence
<220>
   <223> synthetic DNA
<400> 13
   gcggccgcat ctagagtcga ccagcttctg atggaattag aacttggcaa aacaatactg      60
   agaatgaagt gtatgtggaa cagaggctgc tgatctcgtt cttcaggcta tgaaactgac     120
   acatttggaa accacagtac ttagaaccac aaagtgggaa tcaagagaaa aacaatgatc     180
   ccacgagaga tctatagatc tatagatcat gagtgggagg aatgagctgg cccttaattt     240
   ggttttgctt gtttaaatta tgatatccaa ctatgaaaca ttatcataaa gcaatagtaa     300
   agagccttca gtaaagagca ggcatttatc taatcccacc ccacccccac ccccgtagct     360
   ccaatccttc cattcaaaat gtaggtactc tgttctcacc cttcttaaca aagtatgaca     420
   ggaaaaactt ccattttagt ggacatcttt attgtttaat agatcatcaa tttctgcatc     480
   ccggggatct gatatcatcg atgcatgggg tcgtgcgctc ctttcggtcg ggcgctgcgg     540
   gtcgtggggc gggcgtcagg caccgggctt gcgggtcatg caccaggtgc gcggtccttc     600
   gggcacctcg acgtcggcgg tgacggtgaa gccgagccgc tcgtagaagg ggaggttgcg     660
   gggcgcggag gtctccagga aggcgggcac cccggcgcgc tcggccgcct ccactccggg     720
   gagcacgacg gcgctgccca gacccttgcc ctggtggtcg ggcgagacgc cgacggtggc     780
   caggaaccac gcgggctcct tgggccggtg cggcgccagg aggccttcca tctgttgctg     840
   cgcggccagc cgggaaccgc tcaactcggc catgcgcggg ccgatctcgg cgaacaccgc     900
   ccccgcttcg acgctctccg gcgtggtcca gaccgccacc gcggcgccgt cgtccgcgac     960
   ccacaccttg ccgatgtcga gcccgacgcg cgtgaggaag agttcttgca gctcggtgac    1020
   ccgctcgatg tggcggtccg gatcgacggt gtggcgcgtg gcggggtagt cggcgaacgc    1080
   ggcggcgagg gtgcgtacgg ccctggggac gtcgtcgcgg gtggcgaggc gcaccgtggg    1140
   cttgtactcg gtcatggtaa gcttcagctg ctcgagatct agatggatgc aggtcgaaag    1200
   gcccggagat gaggaagagg agaacagcgc ggcagacgtg cgcttttgaa gcgtgcagaa    1260
   tgccgggcct ccggaggacc ttcgggcgcc cgccccgccc ctgagcccgc ccctgagccc    1320
   gcccccggac ccaccccttc ccagcctctg agcccagaaa gcgaaggagc aaagctgcta    1380
   ttggccgctg ccccaaaggc ctacccgctt ccattgctca gcggtgctgt ccatctgcac    1440
   gagactagtg agacgtgcta cttccatttg tcacgtcctg cacgacgcga gctgcggggc    1500
   gggggggaac ttcctgacta ggggaggagt agaaggtggc gcgaaggggc caccaaagaa    1560
   cggagccggt tggcgcctac cggtggatgt ggaatgtgtg cgaggccaga ggccacttgt 1620
   gtagcgccaa gtgcccagcg gggctgctaa agcgcatgct ccagactgcc ttgggaaaag 1680
   cgcctcccct acccggtaga atttcgaggt cgacgaattc ggatcc 1726
<210> 14
   <211> 847
   <212> DNA
   <213> Artificial Sequence
<220>
   <223> synthetic DNA
<400> 14
   gttacttccc ttcgagcctc aatgtcctca tttgtaaaat gacattaata cctactttta 60
   gctgtgggaa ttgagtacca tgatttatac aaagcagttt gtatggtgct ggttacatga 120
   gagttcagat ggtaactagt tagtaaaaaa tctctagtgt gcttgttgat tttattttat 180
   tttagtattt cttaaagatc aaatttaaca tcaatcctaa actttattta gctttttctg 240
   gcgcgtaaac taacatacta agttgtgtga ctataattca tttagtgact catttttagc 300
   tatttttata acacattgtg ctatgggggg ttttggaact tgctggaagc tacatcagaa 360
   actgccatag ttaattgcca tttcaagaat gttgtaaata actcaggtgg ccgtttaatt 420
   ctcaatgtaa atataattaa ctagacgtct ttcctatatt tgtgtctcag ttttaaagct 480
   atttctggat gcttgagtct taccgtaatt gataacaaaa agaggttatt gagaatatct 540
   atgatttaca gagtaagtta ttctagacct caagagtgaa atgtagggga ggagacattt 600
   gtgtgttaaa ctaatggaaa tgctcattta atagatattc actgaaagta ttagttttgg 660
   tttattgcta gaaaagttga ggttttatgg agatttttgt aaaaaatggt ttatttccta 720
   aataaatatc tctttttctt ttttctccca gaaaatgtta atctatgatc cagccaaacg 780
   aatttctggc aaaatggcac tgaatcatcc atattttaat gatttggaca atcagattaa 840
   gaagatg 847
<210> 15
   <211> 4382
   <212> DNA
   <213> Artificial Sequence
<220>
   <223> synthetic DNA
<400> 15
<210> 16
   <211> 931
   <212> DNA
   <213> Artificial Sequence
<220>
   <223> synthetic DNA
<400> 16
   ctttctgaca aaaagtttcc atatgttata tcaacagata gttgtgtttt tattgttaac 60
   tcttgtctat ttttgtctta tatatatttc tttgttatca aacttcagct gtacttcgtc 120
   ttctaatttc aaaaatataa cttaaaaatg taaatattct atatgaattt aaatataatt 180
   ctgtaaatgt gtgtaggtct cactgtaaca actatttgtt actataataa aactataata 240
   ttgatgtcag gaatcaggaa aaaatttgag ttggcttaaa tcatctcagt ccttatggca 300
   gttttatttt cctgtagttg gaactactaa aatttaggaa aatgctaagt tcaagtttcg 360
   taatgctttg aagtattttt atgctctgaa tgtttaaatg ttctcatcag tttcttgcca 420
   tgttgttaac tatacaacct ggctaaagat gaatattttt ctactggtat tttaattttt 480
   gacctaaatg tttaagcatt cggaatgaga aaactataca gatttgagaa atgatgctaa 540
   atttatagga gttttcagta acttaaaaag ctaacatgag agcatgccaa aatttgctaa 600
   gtcttacaaa gatcaagggc tgtccgcaac agggaagaac agttttgaaa atttatgaac 660
   tatcttattt ttaggtaggt tttgaaagct ttttgtctaa gtgaattctt atgccttggt 720
   cagagtaata actgaaggag ttgcttatct tggctttcga gtctgagttt aaaactacac 780
   attttgacat agtgtttatt agcagccatc taaaaaggct ctaatgtata tttaactaaa 840
   attactagct ttgggaatta aactgtttaa caaataatgc tgctcattgt gattcttacc 900
   tataagcagc ctaatttgaa ttatttgctg c 931
<210> 17
   <211> 1623
   <212> DNA
   <213> Artificial Sequence
<220>
   <223> synthetic DNA
<400> 17
   accggtaggc gccaaccggc tccgttcttt ggtggcccct tcgcgccacc ttctactcct      60
   cccctagtca ggaagttccc ccccgccccg cagctcgcgt cgtgcaggac gtgacaaatg     120
   gaagtagcac gtctcactag tctcgtgcag atggacagca ccgctgagca atggaagcgg     180
   gtaggccttt ggggcagcgg ccaatagcag ctttgctcct tcgctttctg ggctcagagg     240
   ctgggaaggg gtgggtccgg gggcgggctc aggggcgggc tcaggggcgg ggcgggcgcc     300
   cgaaggtcct ccggaggccc ggcattctgc acgcttcaaa agcgcacgtc tgccgcgctg     360
   ttctcctctt cctcatctcc gggcctttcg acctgcatcc atctagatct cgagcagctg     420
   aagcttacca tgaccgagta caagcccacg gtgcgcctcg ccacccgcga cgacgtcccc     480
   agggccgtac gcaccctcgc cgccgcgttc gccgactacc ccgccacgcg ccacaccgtc     540
   gatccggacc gccacatcga gcgggtcacc gagctgcaag aactcttcct cacgcgcgtc     600
   gggctcgaca tcggcaaggt gtgggtcgcg gacgacggcg ccgcggtggc ggtctggacc     660
   acgccggaga gcgtcgaagc gggggcggtg ttcgccgaga tcggcccgcg catggccgag     720
   ttgagcggtt cccggctggc cgcgcagcaa cagatggaag gcctcctggc gccgcaccgg     780
   cccaaggagc ccgcgtggtt cctggccacc gtcggcgtct cgcccgacca ccagggcaag     840
   ggtctgggca gcgccgtcgt gctccccgga gtggaggcgg ccgagcgcgc cggggtgccc     900
   gccttcctgg agacctccgc gccccgcaac ctccccttct acgagcggct cggcttcacc     960
   gtcaccgccg acgtcgaggt gcccgaagga ccgcgcacct ggtgcatgac ccgcaagccc    1020
   ggtgcctgac gcccgcccca cgacccgcag cgcccgaccg aaaggagcgc acgaccccat    1080
   gcatcgatga tatcagatcc ccgggatgca gaaattgatg atctattaaa caataaagat    1140
   gtccactaaa atggaagttt ttcctgtcat actttgttaa gaagggtgag aacagagtac    1200
   ctacattttg aatggaagga ttggagctac gggggtgggg gtggggtggg attagataaa    1260
   tgcctgctct ttactgaagg ctctttacta ttgctttatg ataatgtttc atagttggat    1320
   atcataattt aaacaagcaa aaccaaatta agggccagct cattcctccc actcatgatc    1380
   tatagatcta tagatctctc gtgggatcat tgtttttctc ttgattccca ctttgtggtt    1440
   ctaagtactg tggtttccaa atgtgtcagt ttcatagcct gaagaacgag atcagcagcc    1500
   tctgttccac atacacttca ttctcagtat tgttttgcca agttctaatt ccatcagaag    1560
   ctggtcgact ctagatgcgg cgaagttcct attcttcaaa tagtatagga acttcggccg    1620
   gcc                                                                  1623
<210> 18
   <211> 3990
   <212> DNA
   <213> Artificial Sequence
<220>
   <223> synthetic DNA
<400> 18
   ggatccggtt ctggcgtgaa acagactttg aattttgacc ttctcaagtt ggcgggagac 60
   gtggagtcca acccagggcc cataacttcg tataatgtat gctatacgaa gttattaggt 120
   ctgaagagga gtttacgtcc agccaagcta gcttggctgc aggtcgtcga cggtatcgat 180
   aagcttgata tcgagggccc ctgcaggtca attctaccgg gtaggggagg cgcttttccc 240
   aaggcagtct ggagcatgcg ctttagcagc cccgctgggc acttggcgct acacaagtgg 300
   cctctggcct cgcacacatt ccacatccac cggtaggcgc caaccggctc cgttctttgg 360
   tggccccttc gcgccacctt ctactcctcc cctagtcagg aagttccccc ccgccccgca 420
   gctcgcgtcg tgcaggacgt gacaaatgga agtagcacgt ctcactagtc tcgtgcagat 480
   ggacagcacc gctgagcaat ggaagcgggt aggcctttgg ggcagcggcc aatagcagct 540
   ttgctccttc gctttctggg ctcagaggct gggaaggggt gggtccgggg gcgggctcag 600
   gggcgggctc aggggcgggg cgggcgcccg aaggtcctcc cgaggcccgg cattctgcac 660
   gcttcaaaag cgcacgtctg ccgcgctgtt ctcctcttcc tcatctccgg gcctttcgac 720
   ctgcagccaa tatgggatcg gccattgaac aagatggatt gcacgcaggt tctccggccg 780
   cttgggtgga gaggctattc ggctatgact gggcacaaca gacaatcggc tgctctgatg 840
   ccgccgtgtt ccggctgtca gcgcaggggc gcccggttct ttttgtcaag accgacctgt 900
   ccggtgccct gaatgaactg caggacgagg cagcgcggct atcgtggctg gccacgacgg 960
   gcgttccttg cgcagctgtg ctcgacgttg tcactgaagc gggaagggac tggctgctat 1020
   tgggcgaagt gccggggcag gatctcctgt catctcacct tgctcctgcc gagaaagtat 1080
   ccatcatggc tgatgcaatg cggcggctgc atacgcttga tccggctacc tgcccattcg 1140
   accaccaagc gaaacatcgc atcgagcgag cacgtactcg gatggaagcc ggtcttgtcg 1200
   atcaggatga tctggacgaa gagcatcagg ggctcgcgcc agccgaactg ttcgccaggc 1260
   tcaaggcgcg catgcccgac ggcgatgatc tcgtcgtgac ccatggcgat gcctgcttgc 1320
   cgaatatcat ggtggaaaat ggccgctttt ctggattcat cgactgtggc cggctgggtg 1380
   tggcggaccg ctatcaggac atagcgttgg ctacccgtga tattgctgaa gagcttggcg 1440
   gcgaatgggc tgaccgcttc ctcgtgcttt acggtatcgc cgctcccgat tcgcagcgca 1500
   tcgccttcta tcgccttctt gacgagttct tctgagggga tcgatccgct gtaagtctgc 1560
   agaaattgat gatctattaa acaataaaga tgtccactaa aatggaagtt tttcctgtca 1620
   tactttgtta agaagggtga gaacagagta cctacatttt gaatggaagg attggagcta 1680
   cgggggtggg ggtggggtgg gattagataa atgcctgctc tttactgaag gctctttact 1740
   attgctttat gataatgttt catagttgga tatcataatt taaacaagca aaaccaaatt 1800
   aagggccagc tcattcctcc cactcatgat ctatagatct atagatctct cgtgggatca 1860
   ttgtttttct cttgattccc actttgtggt tctaagtact gtggtttcca aatgtgtcag 1920
   tttcatagcc tgaagaacga gatcagcagc ctctgttcca catacacttc attctcagta 1980
   ttgttttgcc aagttctaat tccatcagaa gctgactcta gagcttggcg cgccataact 2040
   tcgtataatg tatgctatac gaagttatct atggccagct acccctgtca ccagcacgcc 2100
   agcgccttcg accaggccgc cagaagcagg ggccacagca accggcggac cgccctgaga 2160
   cccaggcggc agcaggaagc caccgaagtc cggctggaac agaagatgcc caccctgtta 2220
   agggtgtaca tcgacggccc ccacggcatg ggcaagacca ccaccaccca gctgctggtg 2280
   gccctgggca gccgggacga catcgtgtac gtgcccgagc ccatgaccta ctggcaggtg 2340
   ctgggcgcca gcgagaccat cgccaacatc tacaccacac agcacaggct ggaccagggc 2400
   gagatctctg ccggcgacgc cgccgtggtg atgaccagcg cccagatcac aatgggcatg 2460
   ccctacgccg tgaccgacgc cgtgctggcc cctcacgtgg gcggcgaggc cggctctagc 2520
   cacgcccctc cccctgccct gaccctgatc ttcgaccggc accccatcgc ccacctgctg 2580
   tgctaccctg ccgccagata cctgatgggc agcatgaccc cccaggccgt gctggccttc 2640
   gtggccctga tcccccccac cctgcccggc accaacatcg tgctgggagc cctgcccgag 2700
   gaccggcaca tcgaccggct ggccaagcgg cagagacccg gcgagcggct ggacctggcc 2760
   atgctggccg ccatccggcg ggtgtacggc ctgctggcca acaccgtgag atacctgcag 2820
   ggcggagggt cttggtggga ggactggggc cagctgtccg gcaccgccgt gccacctcag 2880
   ggcgccgagc cccagagcaa tgccggccct cggccccaca tcggcgacac cctgtttacc 2940
   ctgttcagag cccccgagct gctggccccc aacggcgacc tgtacaacgt gttcgcctgg 3000
   gccctggacg tgctggccaa gaggctgcgg cccatgcacg tgttcatcct ggactacgac 3060
   cagagccctg ccggctgcag ggacgccctg ctgcagctga ccagcggcat ggtgcagacc 3120
   cacgtgacca cccccggcag catccccacc atctgcgacc tggcccggac cttcgcccgg 3180
   gagatgggcg aggccaacgg ctccggagag ggcagaggaa gtctgctaac atgcggtgac 3240
   gtcgaggaga atcctggccc actcgagatg gccacaacca tggtgagcaa gggcgaggag 3300
   gataacatgg ccatcatcaa ggagttcatg cgcttcaagg tgcacatgga gggctccgtg 3360
   aacggccacg agttcgagat cgagggcgag ggcgagggcc gcccctacga gggcacccag 3420
   accgccaagc tgaaggtgac caagggtggc cccctgccct tcgcctggga catcctgtcc 3480
   cctcagttca tgtacggctc caaggcctac gtgaagcacc ccgccgacat ccccgactac 3540
   ttgaagctgt ccttccccga gggcttcaag tgggagcgcg tgatgaactt cgaggacggc 3600
   ggcgtggtga ccgtgaccca ggactcctcc ctgcaggacg gcgagttcat ctacaaggtg 3660
   aagctgcgcg gcaccaactt cccctccgac ggccccgtaa tgcagaagaa gaccatgggc 3720
   tgggaggcct cctccgagcg gatgtacccc gaggacggcg ccctgaaggg cgagatcaag 3780
   cagaggctga agctgaagga cggcggccac tacgacgctg aggtcaagac cacctacaag 3840
   gccaagaagc ccgtgcagct gcccggcgcc tacaacgtca acatcaagtt ggacatcacc 3900
   tcccacaacg aggactacac catcgtggaa cagtacgaac gcgccgaggg ccgccactcc 3960
   accggcggca tggacgagct gtacaagtag 3990
<210> 19
   <211> 3860
   <212> DNA
   <213> Artificial Sequence
<220>
   <223> synthetic DNA
<400> 19
   ggatccggtt ctggcgtgaa acagactttg aattttgacc ttctcaagtt ggcgggagac      60
   gtggagtcca acccagggcc cataacttcg tataatgtat gctatacgaa gttattaggt     120
   ctgaagagga gtttacgtcc agccaagcta gcttggctgc aggtcgtcga cggtatcggc     180
   cgcatctaga gtcgaccagc ttctgatgga attagaactt ggcaaacaat actgagaatg     240
   aagtgtatgt ggaacagagg ctgctgatct cgttcttcag gctatgaaac tgacacattt     300
   ggaaaccaca gtacttagaa ccacaaagtg ggaatcaaga gaaaaacaat gatcccacga     360
   gagatctata gatctataga tcatgagtgg gaggaatgag ctggccctta atttggtttt     420
   gcttgtttaa attatgatat ccaactatga aacattatca taaagcaata gtaaagagcc     480
   ttcagtaaag agcaggcatt tatctaatcc caccccaccc ccacccccgt agctccaatc     540
   cttccattca aaatgtaggt actctgttct cacccttctt aacaaagtat gacaggaaaa     600
   acttccattt tagtggacat ctttattgtt taatagatca tcaatttctg catcccgggg     660
   atctgatatc atcgatgcat ggggtcgtgc gctcctttcg gtcgggcgct gcgggtcgtg     720
   gggcgggcgt caggcaccgg gcttgcgggt catgcaccag gtgcgcggtc cttcgggcac     780
   ctcgacgtcg gcggtgacgg tgaagccgag ccgctcgtag aaggggaggt tgcggggcgc     840
   ggaggtctcc aggaaggcgg gcaccccggc gcgctcggcc gcctccactc cggggagcac     900
   gacggcgctg cccagaccct tgccctggtg gtcgggcgag acgccgacgg tggccaggaa     960
   ccacgcgggc tccttgggcc ggtgcggcgc caggaggcct tccatctgtt gctgcgcggc    1020
   cagccgggaa ccgctcaact cggccatgcg cgggccgatc tcggcgaaca ccgcccccgc    1080
   ttcgacgctc tccggcgtgg tccagaccgc caccgcggcg ccgtcgtccg cgacccacac    1140
   cttgccgatg tcgagcccga cgcgcgtgag gaagagttct tgcagctcgg tgacccgctc 1200
   gatgtggcgg tccggatcga cggtgtggcg cgtggcgggg tagtcggcga acgcggcggc 1260
   gagggtgcgt acggccctgg ggacgtcgtc gcgggtggcg aggcgcaccg tgggcttgta 1320
   ctcggtcatg gtaagcttca gctgctcgag atctagatgg atgcaggtcg aaaggcccgg 1380
   agatgaggaa gaggagaaca gcgcggcaga cgtgcgcttt tgaagcgtgc agaatgccgg 1440
   gcctccggag gaccttcggg cgcccgcccc gcccctgagc ccgcccctga gcccgccccc 1500
   ggacccaccc cttcccagcc tctgagccca gaaagcgaag gagcaaagct gctattggcc 1560
   gctgccccaa aggcctaccc gcttccattg ctcagcggtg ctgtccatct gcacgagact 1620
   agtgagacgt gctacttcca tttgtcacgt cctgcacgac gcgagctgcg gggcgggggg 1680
   gaacttcctg actaggggag gagtagaagg tggcgcgaag gggccaccaa agaacggagc 1740
   cggttggcgc ctaccggtgg atgtggaatg tgtgcgaggc cagaggccac ttgtgtagcg 1800
   ccaagtgccc agcggggctg ctaaagcgca tgctccagac tgccttggga aaagcgcctc 1860
   ccctacccgg tagaatttcg aggtcgacga attcggatcg cgccataact tcgtataatg 1920
   tatgctatac gaagttatct atggccagct acccctgtca ccagcacgcc agcgccttcg 1980
   accaggccgc cagaagcagg ggccacagca accggcggac cgccctgaga cccaggcggc 2040
   agcaggaagc caccgaagtc cggctggaac agaagatgcc caccctgtta agggtgtaca 2100
   tcgacggccc ccacggcatg ggcaagacca ccaccaccca gctgctggtg gccctgggca 2160
   gccgggacga catcgtgtac gtgcccgagc ccatgaccta ctggcaggtg ctgggcgcca 2220
   gcgagaccat cgccaacatc tacaccacac agcacaggct ggaccagggc gagatctctg 2280
   ccggcgacgc cgccgtggtg atgaccagcg cccagatcac aatgggcatg ccctacgccg 2340
   tgaccgacgc cgtgctggcc cctcacgtgg gcggcgaggc cggctctagc cacgcccctc 2400
   cccctgccct gaccctgatc ttcgaccggc accccatcgc ccacctgctg tgctaccctg 2460
   ccgccagata cctgatgggc agcatgaccc cccaggccgt gctggccttc gtggccctga 2520
   tcccccccac cctgcccggc accaacatcg tgctgggagc cctgcccgag gaccggcaca 2580
   tcgaccggct ggccaagcgg cagagacccg gcgagcggct ggacctggcc atgctggccg 2640
   ccatccggcg ggtgtacggc ctgctggcca acaccgtgag atacctgcag ggcggagggt 2700
   cttggtggga ggactggggc cagctgtccg gcaccgccgt gccacctcag ggcgccgagc 2760
   cccagagcaa tgccggccct cggccccaca tcggcgacac cctgtttacc ctgttcagag 2820
   cccccgagct gctggccccc aacggcgacc tgtacaacgt gttcgcctgg gccctggacg 2880
   tgctggccaa gaggctgcgg cccatgcacg tgttcatcct ggactacgac cagagccctg 2940
   ccggctgcag ggacgccctg ctgcagctga ccagcggcat ggtgcagacc cacgtgacca 3000
   cccccggcag catccccacc atctgcgacc tggcccggac cttcgcccgg gagatgggcg 3060
   aggccaacgg ctccggagag ggcagaggaa gtctgctaac atgcggtgac gtcgaggaga     3120
   atcctggccc actcgagatg gccacaacca tggtgagcaa gggcgaggag gataacatgg     3180
   ccatcatcaa ggagttcatg cgcttcaagg tgcacatgga gggctccgtg aacggccacg     3240
   agttcgagat cgagggcgag ggcgagggcc gcccctacga gggcacccag accgccaagc     3300
   tgaaggtgac caagggtggc cccctgccct tcgcctggga catcctgtcc cctcagttca     3360
   tgtacggctc caaggcctac gtgaagcacc ccgccgacat ccccgactac ttgaagctgt     3420
   ccttccccga gggcttcaag tgggagcgcg tgatgaactt cgaggacggc ggcgtggtga     3480
   ccgtgaccca ggactcctcc ctgcaggacg gcgagttcat ctacaaggtg aagctgcgcg     3540
   gcaccaactt cccctccgac ggccccgtaa tgcagaagaa gaccatgggc tgggaggcct     3600
   cctccgagcg gatgtacccc gaggacggcg ccctgaaggg cgagatcaag cagaggctga     3660
   agctgaagga cggcggccac tacgacgctg aggtcaagac cacctacaag gccaagaagc     3720
   ccgtgcagct gcccggcgcc tacaacgtca acatcaagtt ggacatcacc tcccacaacg     3780
   aggactacac catcgtggaa cagtacgaac gcgccgaggg ccgccactcc accggcggca     3840
   tggacgagct gtacaagtag                                                  3860
<210> 20
   <211> 4848
   <212> DNA
   <213> Artificial Sequence
<220>
   <223> synthetic DNA
<400> 20
   tccaccggcg gcatggacga gctgtacaag tagctttctg acaaaaagtt tccatatgtt      60
   atgtcaacag atagttgtgt ttttattgtt aactcttgtc tatttttgtc ttatatatat     120
   ttctttgtta tcaaacttca gctgtacttc gtcttctaat ttcaaaaata taacttaaaa     180
   atgtaaatat tctatatgaa tttaaatata attctgtaaa tgtgtgtagg tctcactgta     240
   acaactattt gttactataa taaaactata atattgatgt caggaatcag gaaaaaattt     300
   gagttggctt aaatcatctc agtccttatg gcagttttat tttcctgtag ttggaactac     360
   taaaatttag gaaaatgcta agttcaagtt tcgtaatgct ttgaagtatt tttatgctct     420
   gaatgtttaa atgttctcat cagtttcttg ccatgttgtt aactatacaa cctggctaaa     480
   gatgaatatt tttctactgg tattttaatt tttgacctaa atgtttaagc attcggaatg     540
   agaaaactat acagatttga gaaatgatgc taaatttata ggagttttca gtaacttaaa     600
   aagctaacat gagagcatgc caaaatttgc taagtcttac aaagatcaag ggctgtccgc     660
   aacagggaag aacagttttg aaaatttatg aactatctta tttttaggta ggttttgaaa     720
   gctttttgtc taagtgaatt cttatgcctt ggtcagagta ataactgaag gaggtgctta 780
   tcttggcttt cgagtctgag tttaaaacta cacattttga catagtgttt attagcagcc 840
   atctaaaaag gctctaatgt atatttaact aaaattacta gctttgggaa ttaaactgtt 900
   taacaaataa tgctgctcat tgtgattctt acctataagc agcctaattt gaattatttg 960
   ctgcaatcga attcccgcgg ccgccatggc ggccgggagc atgcgacgtc gggcccaatt 1020
   cgccctatag tgagtcgtat tacaattcac tggccgtcgt tttacaacgt cgtgactggg 1080
   aaaaccctgg cgttacccaa cttaatcgcc ttgcagcaca tccccctttc gccagctggc 1140
   gtaatagcga agaggcccgc accgatcgcc cttcccaaca gttgcgcagc ctgaatggcg 1200
   aatggacgcg ccctgtagcg gcgcattaag cgcggcgggt gtggtggtta cgcgcagcgt 1260
   gaccgctaca cttgccagcg ccctagcgcc cgctcctttc gctttcttcc cttcctttct 1320
   cgccacgttc gccggctttc cccgtcaagc tctaaatcgg gggctccctt tagggttccg 1380
   atttagtgct ttacggcacc tcgaccccaa aaaacttgat tagggtgatg gttcacgtag 1440
   tgggccatcg ccctgataga cggtttttcg ccctttgacg ttggagtcca cgttctttaa 1500
   tagtggactc ttgttccaaa ctggaacaac actcaaccct atctcggtct attcttttga 1560
   tttataaggg attttgccga tttcggccta ttggttaaaa aatgagctga tttaacaaaa 1620
   atttaacgcg aattttaaca aaatattaac gcttacaatt tcctgatgcg gtattttctc 1680
   cttacgcatc tgtgcggtat ttcacaccgc atcaggtggc acttttcggg gaaatgtgcg 1740
   cggaacccct atttgtttat ttttctaaat acattcaaat atgtatccgc tcatgagaca 1800
   ataaccctga taaatgcttc aataatattg aaaaaggaag agtatgagta ttcaacattt 1860
   ccgtgtcgcc cttattccct tttttgcggc attttgcctt cctgtttttg ctcacccaga 1920
   aacgctggtg aaagtaaaag atgctgaaga tcagttgggt gcacgagtgg gttacatcga 1980
   actggatctc aacagcggta agatccttga gagttttcgc cccgaagaac gttttccaat 2040
   gatgagcact tttaaagttc tgctatgtgg cgcggtatta tcccgtattg acgccgggca 2100
   agagcaactc ggtcgccgca tacactattc tcagaatgac ttggttgagt actcaccagt 2160
   cacagaaaag catcttacgg atggcatgac agtaagagaa ttatgcagtg ctgccataac 2220
   catgagtgat aacactgcgg ccaacttact tctgacaacg atcggaggac cgaaggagct 2280
   aaccgctttt ttgcacaaca tgggggatca tgtaactcgc cttgatcgtt gggaaccgga 2340
   gctgaatgaa gccataccaa acgacgagcg tgacaccacg atgcctgtag caatggcaac 2400
   aacgttgcgc aaactattaa ctggcgaact acttactcta gcttcccggc aacaattaat 2460
   agactggatg gaggcggata aagttgcagg accacttctg cgctcggccc ttccggctgg 2520
   ctggtttatt gctgataaat ctggagccgg tgagcgtggg tctcgcggta tcattgcagc 2580
   actggggcca gatggtaagc cctcccgtat cgtagttatc tacacgacgg ggagtcaggc 2640
   aactatggat gaacgaaata gacagatcgc tgagataggt gcctcactga ttaagcattg 2700
   gtaactgtca gaccaagttt actcatatat actttagatt gatttaaaac ttcattttta 2760
   atttaaaagg atctaggtga agatcctttt tgataatctc atgaccaaaa tcccttaacg 2820
   tgagttttcg ttccactgag cgtcagaccc cgtagaaaag atcaaaggat cttcttgaga 2880
   tccttttttt ctgcgcgtaa tctgctgctt gcaaacaaaa aaaccaccgc taccagcggt 2940
   ggtttgtttg ccggatcaag agctaccaac tctttttccg aaggtaactg gcttcagcag 3000
   agcgcagata ccaaatactg ttcttctagt gtagccgtag ttaggccacc acttcaagaa 3060
   ctctgtagca ccgcctacat acctcgctct gctaatcctg ttaccagtgg ctgctgccag 3120
   tggcgataag tcgtgtctta ccgggttgga ctcaagacga tagttaccgg ataaggcgca 3180
   gcggtcgggc tgaacggggg gttcgtgcac acagcccagc ttggagcgaa cgacctacac 3240
   cgaactgaga tacctacagc gtgagctatg agaaagcgcc acgcttcccg aagggagaaa 3300
   ggcggacagg tatccggtaa gcggcagggt cggaacagga gagcgcacga gggagcttcc 3360
   agggggaaac gcctggtatc tttatagtcc tgtcgggttt cgccacctct gacttgagcg 3420
   tcgatttttg tgatgctcgt caggggggcg gagcctatgg aaaaacgcca gcaacgcggc 3480
   ctttttacgg ttcctggcct tttgctggcc ttttgctcac atgttctttc ctgcgttatc 3540
   ccctgattct gtggataacc gtattaccgc ctttgagtga gctgataccg ctcgccgcag 3600
   ccgaacgacc gagcgcagcg agtcagtgag cgaggaagcg gaagagcgcc caatacgcaa 3660
   accgcctctc cccgcgcgtt ggccgattca ttaatgcagc tggcacgaca ggtttcccga 3720
   ctggaaagcg ggcagtgagc gcaacgcaat taatgtgagt tagctcactc attaggcacc 3780
   ccaggcttta cactttatgc ttccggctcg tatgttgtgt ggaattgtga gcggataaca 3840
   atttcacaca ggaaacagct atgaccatga ttacgccaag ctatttaggt gacactatag 3900
   aatactcaag ctatgcatcc aacgcgttgg gagctctccc atatggtcga cctgcaggcg 3960
   gccgcgaatt cactagtgat tgttacttcc cttcgagcct caatgtcctc atttgtaaaa 4020
   tgacattaat acctactttt agctgtggga attgagtacc atgatttata caaagcagtt 4080
   tgtatggtgc tggttacatg agagttcaga tggtaactag ttagtaaaaa atctctagtg 4140
   tgcttgttga ttttatttta ttttagtatt tcttaaagat caaatttaac atcaatccta 4200
   aactttattt agctttttct ggcgtgtaaa ctaacatact aagttgtgtg actataattc 4260
   atttagtgac tcatttttag ctatttttat aacacattgt gctatggggg gttttggaac 4320
   ttgctggaag ctacatcaga aactgccata gttaattgcc atttcaagaa tgttgtaaat 4380
   aactcaggtg gccgtttaat tctcaatgta aatataatta actagacatc tttcctatat 4440
   ttgtgtctca gttttaaagc tatttctgga tgcttgagtc ttaccgtaat tgataacaaa 4500
   aagaggttat tgagaatatc tatgatttac agagtaagtt attctagacc tcaagagtga 4560
   aatgtagggg aggagacatt tgtgtgttaa actaatggaa atgctcattt aatagatatt 4620
   cactgaaagt attagttttg gtttattgct agaaaagttg aggttttatg gagatttttg 4680
   taaaaaatgg tttatttcct aaataaatat ctctttttct tttttctccc agaaaatgtt 4740
   aatctatgat ccagccaaac gaatttctgg caaaatggca ctgaatcatc catattttaa 4800
   tgatttggac aatcagatta agaagatggg atccggttct ggcgtgaa 4848
<210> 21
   <211> 8507
   <212> DNA
   <213> Artificial Sequence
<220>
   <223> synthetic DNA
<400> 21
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg taagaagatg tagccctcgt tttagagcta gaaatagcaa gttaaaataa 300
   ggctagtccg ttatcaactt gaaaaagtgg caccgagtcg gtgctttttt gttttagagc 360
   tagaaatagc aagttaaaat aaggctagtc cgtttttagc gcgtgcgcca attctgcaga 420
   caaatggctc tagaggtacc cgttacataa cttacggtaa atggcccgcc tggctgaccg 480
   cccaacgacc cccgcccatt gacgtcaata gtaacgccaa tagggacttt ccattgacgt 540
   caatgggtgg agtatttacg gtaaactgcc cacttggcag tacatcaagt gtatcatatg 600
   ccaagtacgc cccctattga cgtcaatgac ggtaaatggc ccgcctggca ttgtgcccag 660
   tacatgacct tatgggactt tcctacttgg cagtacatct acgtattagt catcgctatt 720
   accatggtcg aggtgagccc cacgttctgc ttcactctcc ccatctcccc cccctcccca 780
   cccccaattt tgtatttatt tattttttaa ttattttgtg cagcgatggg ggcggggggg 840
   gggggggggc gcgcgccagg cggggcgggg cggggcgagg ggcggggcgg ggcgaggcgg 900
   agaggtgcgg cggcagccaa tcagagcggc gcgctccgaa agtttccttt tatggcgagg 960
   cggcggcggc ggcggcccta taaaaagcga agcgcgcggc gggcgggagt cgctgcgacg 1020
   ctgccttcgc cccgtgcccc gctccgccgc cgcctcgcgc cgcccgcccc ggctctgact 1080
   gaccgcgtta ctcccacagg tgagcgggcg ggacggccct tctcctccgg gctgtaatta 1140
   gctgagcaag aggtaagggt ttaagggatg gttggttggt ggggtattaa tgtttaatta 1200
   cctggagcac ctgcctgaaa tcactttttt tcaggttgga ccggtgccac catggactat 1260
   aaggaccacg acggagacta caaggatcat gatattgatt acaaagacga tgacgataag 1320
   atggccccaa agaagaagcg gaaggtcggt atccacggag tcccagcagc cgacaagaag 1380
   tacagcatcg gcctggacat cggcaccaac tctgtgggct gggccgtgat caccgacgag 1440
   tacaaggtgc ccagcaagaa attcaaggtg ctgggcaaca ccgaccggca cagcatcaag 1500
   aagaacctga tcggagccct gctgttcgac agcggcgaaa cagccgaggc cacccggctg 1560
   aagagaaccg ccagaagaag atacaccaga cggaagaacc ggatctgcta tctgcaagag 1620
   atcttcagca acgagatggc caaggtggac gacagcttct tccacagact ggaagagtcc 1680
   ttcctggtgg aagaggataa gaagcacgag cggcacccca tcttcggcaa catcgtggac 1740
   gaggtggcct accacgagaa gtaccccacc atctaccacc tgagaaagaa actggtggac 1800
   agcaccgaca aggccgacct gcggctgatc tatctggccc tggcccacat gatcaagttc 1860
   cggggccact tcctgatcga gggcgacctg aaccccgaca acagcgacgt ggacaagctg 1920
   ttcatccagc tggtgcagac ctacaaccag ctgttcgagg aaaaccccat caacgccagc 1980
   ggcgtggacg ccaaggccat cctgtctgcc agactgagca agagcagacg gctggaaaat 2040
   ctgatcgccc agctgcccgg cgagaagaag aatggcctgt tcggaaacct gattgccctg 2100
   agcctgggcc tgacccccaa cttcaagagc aacttcgacc tggccgagga tgccaaactg 2160
   cagctgagca aggacaccta cgacgacgac ctggacaacc tgctggccca gatcggcgac 2220
   cagtacgccg acctgtttct ggccgccaag aacctgtccg acgccatcct gctgagcgac 2280
   atcctgagag tgaacaccga gatcaccaag gcccccctga gcgcctctat gatcaagaga 2340
   tacgacgagc accaccagga cctgaccctg ctgaaagctc tcgtgcggca gcagctgcct 2400
   gagaagtaca aagagatttt cttcgaccag agcaagaacg gctacgccgg ctacattgac 2460
   ggcggagcca gccaggaaga gttctacaag ttcatcaagc ccatcctgga aaagatggac 2520
   ggcaccgagg aactgctcgt gaagctgaac agagaggacc tgctgcggaa gcagcggacc 2580
   ttcgacaacg gcagcatccc ccaccagatc cacctgggag agctgcacgc cattctgcgg 2640
   cggcaggaag atttttaccc attcctgaag gacaaccggg aaaagatcga gaagatcctg 2700
   accttccgca tcccctacta cgtgggccct ctggccaggg gaaacagcag attcgcctgg 2760
   atgaccagaa agagcgagga aaccatcacc ccctggaact tcgaggaagt ggtggacaag 2820
   ggcgcttccg cccagagctt catcgagcgg atgaccaact tcgataagaa cctgcccaac 2880
   gagaaggtgc tgcccaagca cagcctgctg tacgagtact tcaccgtgta taacgagctg 2940
   accaaagtga aatacgtgac cgagggaatg agaaagcccg ccttcctgag cggcgagcag 3000
   aaaaaggcca tcgtggacct gctgttcaag accaaccgga aagtgaccgt gaagcagctg 3060
   aaagaggact acttcaagaa aatcgagtgc ttcgactccg tggaaatctc cggcgtggaa 3120
   gatcggttca acgcctccct gggcacatac cacgatctgc tgaaaattat caaggacaag 3180
   gacttcctgg acaatgagga aaacgaggac attctggaag atatcgtgct gaccctgaca 3240
   ctgtttgagg acagagagat gatcgaggaa cggctgaaaa cctatgccca cctgttcgac 3300
   gacaaagtga tgaagcagct gaagcggcgg agatacaccg gctggggcag gctgagccgg 3360
   aagctgatca acggcatccg ggacaagcag tccggcaaga caatcctgga tttcctgaag 3420
   tccgacggct tcgccaacag aaacttcatg cagctgatcc acgacgacag cctgaccttt 3480
   aaagaggaca tccagaaagc ccaggtgtcc ggccagggcg atagcctgca cgagcacatt 3540
   gccaatctgg ccggcagccc cgccattaag aagggcatcc tgcagacagt gaaggtggtg 3600
   gacgagctcg tgaaagtgat gggccggcac aagcccgaga acatcgtgat cgaaatggcc 3660
   agagagaacc agaccaccca gaagggacag aagaacagcc gcgagagaat gaagcggatc 3720
   gaagagggca tcaaagagct gggcagccag atcctgaaag aacaccccgt ggaaaacacc 3780
   cagctgcaga acgagaagct gtacctgtac tacctgcaga atgggcggga tatgtacgtg 3840
   gaccaggaac tggacatcaa ccggctgtcc gactacgatg tggaccatat cgtgcctcag 3900
   agctttctga aggacgactc catcgacaac aaggtgctga ccagaagcga caagaaccgg 3960
   ggcaagagcg acaacgtgcc ctccgaagag gtcgtgaaga agatgaagaa ctactggcgg 4020
   cagctgctga acgccaagct gattacccag agaaagttcg acaatctgac caaggccgag 4080
   agaggcggcc tgagcgaact ggataaggcc ggcttcatca agagacagct ggtggaaacc 4140
   cggcagatca caaagcacgt ggcacagatc ctggactccc ggatgaacac taagtacgac 4200
   gagaatgaca agctgatccg ggaagtgaaa gtgatcaccc tgaagtccaa gctggtgtcc 4260
   gatttccgga aggatttcca gttttacaaa gtgcgcgaga tcaacaacta ccaccacgcc 4320
   cacgacgcct acctgaacgc cgtcgtggga accgccctga tcaaaaagta ccctaagctg 4380
   gaaagcgagt tcgtgtacgg cgactacaag gtgtacgacg tgcggaagat gatcgccaag 4440
   agcgagcagg aaatcggcaa ggctaccgcc aagtacttct tctacagcaa catcatgaac 4500
   tttttcaaga ccgagattac cctggccaac ggcgagatcc ggaagcggcc tctgatcgag 4560
   acaaacggcg aaaccgggga gatcgtgtgg gataagggcc gggattttgc caccgtgcgg 4620
   aaagtgctga gcatgcccca agtgaatatc gtgaaaaaga ccgaggtgca gacaggcggc 4680
   ttcagcaaag agtctatcct gcccaagagg aacagcgata agctgatcgc cagaaagaag 4740
   gactgggacc ctaagaagta cggcggcttc gacagcccca ccgtggccta ttctgtgctg 4800
   gtggtggcca aagtggaaaa gggcaagtcc aagaaactga agagtgtgaa agagctgctg 4860
   gggatcacca tcatggaaag aagcagcttc gagaagaatc ccatcgactt tctggaagcc 4920
   aagggctaca aagaagtgaa aaaggacctg atcatcaagc tgcctaagta ctccctgttc 4980
   gagctggaaa acggccggaa gagaatgctg gcctctgccg gcgaactgca gaagggaaac 5040
   gaactggccc tgccctccaa atatgtgaac ttcctgtacc tggccagcca ctatgagaag 5100
   ctgaagggct cccccgagga taatgagcag aaacagctgt ttgtggaaca gcacaagcac 5160
   tacctggacg agatcatcga gcagatcagc gagttctcca agagagtgat cctggccgac 5220
   gctaatctgg acaaagtgct gtccgcctac aacaagcacc gggataagcc catcagagag 5280
   caggccgaga atatcatcca cctgtttacc ctgaccaatc tgggagcccc tgccgccttc 5340
   aagtactttg acaccaccat cgaccggaag aggtacacca gcaccaaaga ggtgctggac 5400
   gccaccctga tccaccagag catcaccggc ctgtacgaga cacggatcga cctgtctcag 5460
   ctgggaggcg acaaaaggcc ggcggccacg aaaaaggccg gccaggcaaa aaagaaaaag 5520
   taagaattcc tagagctcgc tgatcagcct cgactgtgcc ttctagttgc cagccatctg 5580
   ttgtttgccc ctcccccgtg ccttccttga ccctggaagg tgccactccc actgtccttt 5640
   cctaataaaa tgaggaaatt gcatcgcatt gtctgagtag gtgtcattct attctggggg 5700
   gtggggtggg gcaggacagc aagggggagg attgggaaga gaatagcagg catgctgggg 5760
   agcggccgca ggaaccccta gtgatggagt tggccactcc ctctctgcgc gctcgctcgc 5820
   tcactgaggc cgggcgacca aaggtcgccc gacgcccggg ctttgcccgg gcggcctcag 5880
   tgagcgagcg agcgcgcagc tgcctgcagg ggcgcctgat gcggtatttt ctccttacgc 5940
   atctgtgcgg tatttcacac cgcatacgtc aaagcaacca tagtacgcgc cctgtagcgg 6000
   cgcattaagc gcggcgggtg tggtggttac gcgcagcgtg accgctacac ttgccagcgc 6060
   cctagcgccc gctcctttcg ctttcttccc ttcctttctc gccacgttcg ccggctttcc 6120
   ccgtcaagct ctaaatcggg ggctcccttt agggttccga tttagtgctt tacggcacct 6180
   cgaccccaaa aaacttgatt tgggtgatgg ttcacgtagt gggccatcgc cctgatagac 6240
   ggtttttcgc cctttgacgt tggagtccac gttctttaat agtggactct tgttccaaac 6300
   tggaacaaca ctcaacccta tctcgggcta ttcttttgat ttataaggga ttttgccgat 6360
   ttcggcctat tggttaaaaa atgagctgat ttaacaaaaa tttaacgcga attttaacaa 6420
   aatattaacg tttacaattt tatggtgcac tctcagtaca atctgctctg atgccgcata 6480
   gttaagccag ccccgacacc cgccaacacc cgctgacgcg ccctgacggg cttgtctgct 6540
   cccggcatcc gcttacagac aagctgtgac cgtctccggg agctgcatgt gtcagaggtt 6600
   ttcaccgtca tcaccgaaac gcgcgagacg aaagggcctc gtgatacgcc tatttttata 6660
   ggttaatgtc atgataataa tggtttctta gacgtcaggt ggcacttttc ggggaaatgt 6720
   gcgcggaacc cctatttgtt tatttttcta aatacattca aatatgtatc cgctcatgag 6780
   acaataaccc tgataaatgc ttcaataata ttgaaaaagg aagagtatga gtattcaaca 6840
   tttccgtgtc gcccttattc ccttttttgc ggcattttgc cttcctgttt ttgctcaccc 6900
   agaaacgctg gtgaaagtaa aagatgctga agatcagttg ggtgcacgag tgggttacat 6960
   cgaactggat ctcaacagcg gtaagatcct tgagagtttt cgccccgaag aacgttttcc 7020
   aatgatgagc acttttaaag ttctgctatg tggcgcggta ttatcccgta ttgacgccgg 7080
   gcaagagcaa ctcggtcgcc gcatacacta ttctcagaat gacttggttg agtactcacc 7140
   agtcacagaa aagcatctta cggatggcat gacagtaaga gaattatgca gtgctgccat 7200
   aaccatgagt gataacactg cggccaactt acttctgaca acgatcggag gaccgaagga 7260
   gctaaccgct tttttgcaca acatggggga tcatgtaact cgccttgatc gttgggaacc 7320
   ggagctgaat gaagccatac caaacgacga gcgtgacacc acgatgcctg tagcaatggc 7380
   aacaacgttg cgcaaactat taactggcga actacttact ctagcttccc ggcaacaatt 7440
   aatagactgg atggaggcgg ataaagttgc aggaccactt ctgcgctcgg cccttccggc 7500
   tggctggttt attgctgata aatctggagc cggtgagcgt ggaagccgcg gtatcattgc 7560
   agcactgggg ccagatggta agccctcccg tatcgtagtt atctacacga cggggagtca 7620
   ggcaactatg gatgaacgaa atagacagat cgctgagata ggtgcctcac tgattaagca 7680
   ttggtaactg tcagaccaag tttactcata tatactttag attgatttaa aacttcattt 7740
   ttaatttaaa aggatctagg tgaagatcct ttttgataat ctcatgacca aaatccctta 7800
   acgtgagttt tcgttccact gagcgtcaga ccccgtagaa aagatcaaag gatcttcttg 7860
   agatcctttt tttctgcgcg taatctgctg cttgcaaaca aaaaaaccac cgctaccagc 7920
   ggtggtttgt ttgccggatc aagagctacc aactcttttt ccgaaggtaa ctggcttcag 7980
   cagagcgcag ataccaaata ctgtccttct agtgtagccg tagttaggcc accacttcaa 8040
   gaactctgta gcaccgccta catacctcgc tctgctaatc ctgttaccag tggctgctgc 8100
   cagtggcgat aagtcgtgtc ttaccgggtt ggactcaaga cgatagttac cggataaggc 8160
   gcagcggtcg ggctgaacgg ggggttcgtg cacacagccc agcttggagc gaacgaccta 8220
   caccgaactg agatacctac agcgtgagct atgagaaagc gccacgcttc ccgaagggag 8280
   aaaggcggac aggtatccgg taagcggcag ggtcggaaca ggagagcgca cgagggagct 8340
   tccaggggga aacgcctggt atctttatag tcctgtcggg tttcgccacc tctgacttga 8400
   gcgtcgattt ttgtgatgct cgtcaggggg gcggagccta tggaaaaacg ccagcaacgc 8460
   ggccttttta cggttcctgg ccttttgctg gccttttgct cacatgt 8507
<210> 22
   <211> 8507
   <212> DNA
   <213> Artificial Sequence
<220>
   <223> synthetic DNA
<400> 22
   catatacgat acaaggctgt tagagagata attggaatta atttgactgt aaacacaaag 60
   atattagtac aaaatacgtg acgtagaaag taataatttc ttgggtagtt tgcagtttta 120
   aaattatgtt ttaaaatgga ctatcatatg cttaccgtaa cttgaaagta tttcgatttc 180
   ttggctttat atatcttgtg gaaaggacga aacaccgaca gggacatcca tccaggtttt 240
   agagctagaa atagcaagtt aaaataaggc tagtccgtta tcaacttgaa aaagtggcac 300
   cgagtcggtg cttttttgtt ttagagctag aaatagcaag ttaaaataag gctagtccgt 360
   ttttagcgcg tgcgccaatt ctgcagacaa atggctctag aggtacccgt tacataactt 420
   acggtaaatg gcccgcctgg ctgaccgccc aacgaccccc gcccattgac gtcaatagta 480
   acgccaatag ggactttcca ttgacgtcaa tgggtggagt atttacggta aactgcccac 540
   ttggcagtac atcaagtgta tcatatgcca agtacgcccc ctattgacgt caatgacggt 600
   aaatggcccg cctggcattg tgcccagtac atgaccttat gggactttcc tacttggcag 660
   tacatctacg tattagtcat cgctattacc atggtcgagg tgagccccac gttctgcttc 720
   actctcccca tctccccccc ctccccaccc ccaattttgt atttatttat tttttaatta 780
   ttttgtgcag cgatgggggc gggggggggg ggggggcgcg cgccaggcgg ggcggggcgg 840
   ggcgaggggc ggggcggggc gaggcggaga ggtgcggcgg cagccaatca gagcggcgcg 900
   ctccgaaagt ttccttttat ggcgaggcgg cggcggcggc ggccctataa aaagcgaagc 960
   gcgcggcggg cgggagtcgc tgcgacgctg ccttcgcccc gtgccccgct ccgccgccgc 1020
   ctcgcgccgc ccgccccggc tctgactgac cgcgttactc ccacaggtga gcgggcggga 1080
   cggcccttct cctccgggct gtaattagct gagcaagagg taagggttta agggatggtt 1140
   ggttggtggg gtattaatgt ttaattacct ggagcacctg cctgaaatca ctttttttca 1200
   ggttggaccg gtgccaccat ggactataag gaccacgacg gagactacaa ggatcatgat 1260
   attgattaca aagacgatga cgataagatg gccccaaaga agaagcggaa ggtcggtatc 1320
   cacggagtcc cagcagccga caagaagtac agcatcggcc tggacatcgg caccaactct 1380
   gtgggctggg ccgtgatcac cgacgagtac aaggtgccca gcaagaaatt caaggtgctg 1440
   ggcaacaccg accggcacag catcaagaag aacctgatcg gagccctgct gttcgacagc 1500
   ggcgaaacag ccgaggccac ccggctgaag agaaccgcca gaagaagata caccagacgg 1560
   aagaaccgga tctgctatct gcaagagatc ttcagcaacg agatggccaa ggtggacgac 1620
   agcttcttcc acagactgga agagtccttc ctggtggaag aggataagaa gcacgagcgg 1680
   caccccatct tcggcaacat cgtggacgag gtggcctacc acgagaagta ccccaccatc 1740
   taccacctga gaaagaaact ggtggacagc accgacaagg ccgacctgcg gctgatctat 1800
   ctggccctgg cccacatgat caagttccgg ggccacttcc tgatcgaggg cgacctgaac 1860
   cccgacaaca gcgacgtgga caagctgttc atccagctgg tgcagaccta caaccagctg 1920
   ttcgaggaaa accccatcaa cgccagcggc gtggacgcca aggccatcct gtctgccaga 1980
   ctgagcaaga gcagacggct ggaaaatctg atcgcccagc tgcccggcga gaagaagaat 2040
   ggcctgttcg gaaacctgat tgccctgagc ctgggcctga cccccaactt caagagcaac 2100
   ttcgacctgg ccgaggatgc caaactgcag ctgagcaagg acacctacga cgacgacctg 2160
   gacaacctgc tggcccagat cggcgaccag tacgccgacc tgtttctggc cgccaagaac 2220
   ctgtccgacg ccatcctgct gagcgacatc ctgagagtga acaccgagat caccaaggcc 2280
   cccctgagcg cctctatgat caagagatac gacgagcacc accaggacct gaccctgctg 2340
   aaagctctcg tgcggcagca gctgcctgag aagtacaaag agattttctt cgaccagagc 2400
   aagaacggct acgccggcta cattgacggc ggagccagcc aggaagagtt ctacaagttc 2460
   atcaagccca tcctggaaaa gatggacggc accgaggaac tgctcgtgaa gctgaacaga 2520
   gaggacctgc tgcggaagca gcggaccttc gacaacggca gcatccccca ccagatccac 2580
   ctgggagagc tgcacgccat tctgcggcgg caggaagatt tttacccatt cctgaaggac 2640
   aaccgggaaa agatcgagaa gatcctgacc ttccgcatcc cctactacgt gggccctctg 2700
   gccaggggaa acagcagatt cgcctggatg accagaaaga gcgaggaaac catcaccccc 2760
   tggaacttcg aggaagtggt ggacaagggc gcttccgccc agagcttcat cgagcggatg 2820
   accaacttcg ataagaacct gcccaacgag aaggtgctgc ccaagcacag cctgctgtac 2880
   gagtacttca ccgtgtataa cgagctgacc aaagtgaaat acgtgaccga gggaatgaga 2940
   aagcccgcct tcctgagcgg cgagcagaaa aaggccatcg tggacctgct gttcaagacc 3000
   aaccggaaag tgaccgtgaa gcagctgaaa gaggactact tcaagaaaat cgagtgcttc 3060
   gactccgtgg aaatctccgg cgtggaagat cggttcaacg cctccctggg cacataccac 3120
   gatctgctga aaattatcaa ggacaaggac ttcctggaca atgaggaaaa cgaggacatt 3180
   ctggaagata tcgtgctgac cctgacactg tttgaggaca gagagatgat cgaggaacgg 3240
   ctgaaaacct atgcccacct gttcgacgac aaagtgatga agcagctgaa gcggcggaga 3300
   tacaccggct ggggcaggct gagccggaag ctgatcaacg gcatccggga caagcagtcc 3360
   ggcaagacaa tcctggattt cctgaagtcc gacggcttcg ccaacagaaa cttcatgcag 3420
   ctgatccacg acgacagcct gacctttaaa gaggacatcc agaaagccca ggtgtccggc 3480
   cagggcgata gcctgcacga gcacattgcc aatctggccg gcagccccgc cattaagaag 3540
   ggcatcctgc agacagtgaa ggtggtggac gagctcgtga aagtgatggg ccggcacaag 3600
   cccgagaaca tcgtgatcga aatggccaga gagaaccaga ccacccagaa gggacagaag 3660
   aacagccgcg agagaatgaa gcggatcgaa gagggcatca aagagctggg cagccagatc 3720
   ctgaaagaac accccgtgga aaacacccag ctgcagaacg agaagctgta cctgtactac 3780
   ctgcagaatg ggcgggatat gtacgtggac caggaactgg acatcaaccg gctgtccgac 3840
   tacgatgtgg accatatcgt gcctcagagc tttctgaagg acgactccat cgacaacaag 3900
   gtgctgacca gaagcgacaa gaaccggggc aagagcgaca acgtgccctc cgaagaggtc 3960
   gtgaagaaga tgaagaacta ctggcggcag ctgctgaacg ccaagctgat tacccagaga 4020
   aagttcgaca atctgaccaa ggccgagaga ggcggcctga gcgaactgga taaggccggc 4080
   ttcatcaaga gacagctggt ggaaacccgg cagatcacaa agcacgtggc acagatcctg 4140
   gactcccgga tgaacactaa gtacgacgag aatgacaagc tgatccggga agtgaaagtg 4200
   atcaccctga agtccaagct ggtgtccgat ttccggaagg atttccagtt ttacaaagtg 4260
   cgcgagatca acaactacca ccacgcccac gacgcctacc tgaacgccgt cgtgggaacc 4320
   gccctgatca aaaagtaccc taagctggaa agcgagttcg tgtacggcga ctacaaggtg 4380
   tacgacgtgc ggaagatgat cgccaagagc gagcaggaaa tcggcaaggc taccgccaag 4440
   tacttcttct acagcaacat catgaacttt ttcaagaccg agattaccct ggccaacggc 4500
   gagatccgga agcggcctct gatcgagaca aacggcgaaa ccggggagat cgtgtgggat 4560
   aagggccggg attttgccac cgtgcggaaa gtgctgagca tgccccaagt gaatatcgtg 4620
   aaaaagaccg aggtgcagac aggcggcttc agcaaagagt ctatcctgcc caagaggaac 4680
   agcgataagc tgatcgccag aaagaaggac tgggacccta agaagtacgg cggcttcgac 4740
   agccccaccg tggcctattc tgtgctggtg gtggccaaag tggaaaaggg caagtccaag 4800
   aaactgaaga gtgtgaaaga gctgctgggg atcaccatca tggaaagaag cagcttcgag 4860
   aagaatccca tcgactttct ggaagccaag ggctacaaag aagtgaaaaa ggacctgatc 4920
   atcaagctgc ctaagtactc cctgttcgag ctggaaaacg gccggaagag aatgctggcc 4980
   tctgccggcg aactgcagaa gggaaacgaa ctggccctgc cctccaaata tgtgaacttc 5040
   ctgtacctgg ccagccacta tgagaagctg aagggctccc ccgaggataa tgagcagaaa 5100
   cagctgtttg tggaacagca caagcactac ctggacgaga tcatcgagca gatcagcgag 5160
   ttctccaaga gagtgatcct ggccgacgct aatctggaca aagtgctgtc cgcctacaac 5220
   aagcaccggg ataagcccat cagagagcag gccgagaata tcatccacct gtttaccctg 5280
   accaatctgg gagcccctgc cgccttcaag tactttgaca ccaccatcga ccggaagagg 5340
   tacaccagca ccaaagaggt gctggacgcc accctgatcc accagagcat caccggcctg 5400
   tacgagacac ggatcgacct gtctcagctg ggaggcgaca aaaggccggc ggccacgaaa 5460
   aaggccggcc aggcaaaaaa gaaaaagtaa gaattcctag agctcgctga tcagcctcga 5520
   ctgtgccttc tagttgccag ccatctgttg tttgcccctc ccccgtgcct tccttgaccc 5580
   tggaaggtgc cactcccact gtcctttcct aataaaatga ggaaattgca tcgcattgtc 5640
   tgagtaggtg tcattctatt ctggggggtg gggtggggca ggacagcaag ggggaggatt 5700
   gggaagagaa tagcaggcat gctggggagc ggccgcagga acccctagtg atggagttgg 5760
   ccactccctc tctgcgcgct cgctcgctca ctgaggccgg gcgaccaaag gtcgcccgac 5820
   gcccgggctt tgcccgggcg gcctcagtga gcgagcgagc gcgcagctgc ctgcaggggc 5880
   gcctgatgcg gtattttctc cttacgcatc tgtgcggtat ttcacaccgc atacgtcaaa 5940
   gcaaccatag tacgcgccct gtagcggcgc attaagcgcg gcgggtgtgg tggttacgcg 6000
   cagcgtgacc gctacacttg ccagcgccct agcgcccgct cctttcgctt tcttcccttc 6060
   ctttctcgcc acgttcgccg gctttccccg tcaagctcta aatcgggggc tccctttagg 6120
   gttccgattt agtgctttac ggcacctcga ccccaaaaaa cttgatttgg gtgatggttc 6180
   acgtagtggg ccatcgccct gatagacggt ttttcgccct ttgacgttgg agtccacgtt 6240
   ctttaatagt ggactcttgt tccaaactgg aacaacactc aaccctatct cgggctattc 6300
   ttttgattta taagggattt tgccgatttc ggcctattgg ttaaaaaatg agctgattta 6360
   acaaaaattt aacgcgaatt ttaacaaaat attaacgttt acaattttat ggtgcactct 6420
   cagtacaatc tgctctgatg ccgcatagtt aagccagccc cgacacccgc caacacccgc 6480
   tgacgcgccc tgacgggctt gtctgctccc ggcatccgct tacagacaag ctgtgaccgt 6540
   ctccgggagc tgcatgtgtc agaggttttc accgtcatca ccgaaacgcg cgagacgaaa 6600
   gggcctcgtg atacgcctat ttttataggt taatgtcatg ataataatgg tttcttagac 6660
   gtcaggtggc acttttcggg gaaatgtgcg cggaacccct atttgtttat ttttctaaat 6720
   acattcaaat atgtatccgc tcatgagaca ataaccctga taaatgcttc aataatattg 6780
   aaaaaggaag agtatgagta ttcaacattt ccgtgtcgcc cttattccct tttttgcggc 6840
   attttgcctt cctgtttttg ctcacccaga aacgctggtg aaagtaaaag atgctgaaga 6900
   tcagttgggt gcacgagtgg gttacatcga actggatctc aacagcggta agatccttga 6960
   gagttttcgc cccgaagaac gttttccaat gatgagcact tttaaagttc tgctatgtgg 7020
   cgcggtatta tcccgtattg acgccgggca agagcaactc ggtcgccgca tacactattc 7080
   tcagaatgac ttggttgagt actcaccagt cacagaaaag catcttacgg atggcatgac 7140
   agtaagagaa ttatgcagtg ctgccataac catgagtgat aacactgcgg ccaacttact 7200
   tctgacaacg atcggaggac cgaaggagct aaccgctttt ttgcacaaca tgggggatca 7260
   tgtaactcgc cttgatcgtt gggaaccgga gctgaatgaa gccataccaa acgacgagcg 7320
   tgacaccacg atgcctgtag caatggcaac aacgttgcgc aaactattaa ctggcgaact 7380
   acttactcta gcttcccggc aacaattaat agactggatg gaggcggata aagttgcagg 7440
   accacttctg cgctcggccc ttccggctgg ctggtttatt gctgataaat ctggagccgg 7500
   tgagcgtgga agccgcggta tcattgcagc actggggcca gatggtaagc cctcccgtat 7560
   cgtagttatc tacacgacgg ggagtcaggc aactatggat gaacgaaata gacagatcgc     7620
   tgagataggt gcctcactga ttaagcattg gtaactgtca gaccaagttt actcatatat     7680
   actttagatt gatttaaaac ttcattttta atttaaaagg atctaggtga agatcctttt     7740
   tgataatctc atgaccaaaa tcccttaacg tgagttttcg ttccactgag cgtcagaccc     7800
   cgtagaaaag atcaaaggat cttcttgaga tccttttttt ctgcgcgtaa tctgctgctt     7860
   gcaaacaaaa aaaccaccgc taccagcggt ggtttgtttg ccggatcaag agctaccaac     7920
   tctttttccg aaggtaactg gcttcagcag agcgcagata ccaaatactg tccttctagt     7980
   gtagccgtag ttaggccacc acttcaagaa ctctgtagca ccgcctacat acctcgctct     8040
   gctaatcctg ttaccagtgg ctgctgccag tggcgataag tcgtgtctta ccgggttgga     8100
   ctcaagacga tagttaccgg ataaggcgca gcggtcgggc tgaacggggg gttcgtgcac     8160
   acagcccagc ttggagcgaa cgacctacac cgaactgaga tacctacagc gtgagctatg     8220
   agaaagcgcc acgcttcccg aagggagaaa ggcggacagg tatccggtaa gcggcagggt     8280
   cggaacagga gagcgcacga gggagcttcc agggggaaac gcctggtatc tttatagtcc     8340
   tgtcgggttt cgccacctct gacttgagcg tcgatttttg tgatgctcgt caggggggcg     8400
   gagcctatgg aaaaacgcca gcaacgcggc ctttttacgg ttcctggcct tttgctggcc     8460
   ttttgctcac atgtgagggc ctatttccca tgattccttc atatttg                   8507
<210> 23
   <211> 35
   <212> DNA
   <213> Mus musculus
<400> 23
   acagggacat ccatccagag ggctacatct tctta 35
<210> 24
   <211> 8509
   <212> DNA
   <213> Artificial Sequence
<220>
   <223> synthetic DNA
<400> 24
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccg ctatctgttg acataacata gttttagagc tagaaatagc aagttaaaat     300
   aaggctagtc cgttatcaac ttgaaaaagt ggcaccgagt cggtgctttt ttgttttaga     360
   gctagaaata gcaagttaaa ataaggctag tccgttttta gcgcgtgcgc caattctgca 420
   gacaaatggc tctagaggta cccgttacat aacttacggt aaatggcccg cctggctgac 480
   cgcccaacga cccccgccca ttgacgtcaa tagtaacgcc aatagggact ttccattgac 540
   gtcaatgggt ggagtattta cggtaaactg cccacttggc agtacatcaa gtgtatcata 600
   tgccaagtac gccccctatt gacgtcaatg acggtaaatg gcccgcctgg cattgtgccc 660
   agtacatgac cttatgggac tttcctactt ggcagtacat ctacgtatta gtcatcgcta 720
   ttaccatggt cgaggtgagc cccacgttct gcttcactct ccccatctcc cccccctccc 780
   cacccccaat tttgtattta tttatttttt aattattttg tgcagcgatg ggggcggggg 840
   gggggggggg gcgcgcgcca ggcggggcgg ggcggggcga ggggcggggc ggggcgaggc 900
   ggagaggtgc ggcggcagcc aatcagagcg gcgcgctccg aaagtttcct tttatggcga 960
   ggcggcggcg gcggcggccc tataaaaagc gaagcgcgcg gcgggcggga gtcgctgcga 1020
   cgctgccttc gccccgtgcc ccgctccgcc gccgcctcgc gccgcccgcc ccggctctga 1080
   ctgaccgcgt tactcccaca ggtgagcggg cgggacggcc cttctcctcc gggctgtaat 1140
   tagctgagca agaggtaagg gtttaaggga tggttggttg gtggggtatt aatgtttaat 1200
   tacctggagc acctgcctga aatcactttt tttcaggttg gaccggtgcc accatggact 1260
   ataaggacca cgacggagac tacaaggatc atgatattga ttacaaagac gatgacgata 1320
   agatggcccc aaagaagaag cggaaggtcg gtatccacgg agtcccagca gccgacaaga 1380
   agtacagcat cggcctggac atcggcacca actctgtggg ctgggccgtg atcaccgacg 1440
   agtacaaggt gcccagcaag aaattcaagg tgctgggcaa caccgaccgg cacagcatca 1500
   agaagaacct gatcggagcc ctgctgttcg acagcggcga aacagccgag gccacccggc 1560
   tgaagagaac cgccagaaga agatacacca gacggaagaa ccggatctgc tatctgcaag 1620
   agatcttcag caacgagatg gccaaggtgg acgacagctt cttccacaga ctggaagagt 1680
   ccttcctggt ggaagaggat aagaagcacg agcggcaccc catcttcggc aacatcgtgg 1740
   acgaggtggc ctaccacgag aagtacccca ccatctacca cctgagaaag aaactggtgg 1800
   acagcaccga caaggccgac ctgcggctga tctatctggc cctggcccac atgatcaagt 1860
   tccggggcca cttcctgatc gagggcgacc tgaaccccga caacagcgac gtggacaagc 1920
   tgttcatcca gctggtgcag acctacaacc agctgttcga ggaaaacccc atcaacgcca 1980
   gcggcgtgga cgccaaggcc atcctgtctg ccagactgag caagagcaga cggctggaaa 2040
   atctgatcgc ccagctgccc ggcgagaaga agaatggcct gttcggaaac ctgattgccc 2100
   tgagcctggg cctgaccccc aacttcaaga gcaacttcga cctggccgag gatgccaaac 2160
   tgcagctgag caaggacacc tacgacgacg acctggacaa cctgctggcc cagatcggcg 2220
   accagtacgc cgacctgttt ctggccgcca agaacctgtc cgacgccatc ctgctgagcg 2280
   acatcctgag agtgaacacc gagatcacca aggcccccct gagcgcctct atgatcaaga 2340
   gatacgacga gcaccaccag gacctgaccc tgctgaaagc tctcgtgcgg cagcagctgc 2400
   ctgagaagta caaagagatt ttcttcgacc agagcaagaa cggctacgcc ggctacattg 2460
   acggcggagc cagccaggaa gagttctaca agttcatcaa gcccatcctg gaaaagatgg 2520
   acggcaccga ggaactgctc gtgaagctga acagagagga cctgctgcgg aagcagcgga 2580
   ccttcgacaa cggcagcatc ccccaccaga tccacctggg agagctgcac gccattctgc 2640
   ggcggcagga agatttttac ccattcctga aggacaaccg ggaaaagatc gagaagatcc 2700
   tgaccttccg catcccctac tacgtgggcc ctctggccag gggaaacagc agattcgcct 2760
   ggatgaccag aaagagcgag gaaaccatca ccccctggaa cttcgaggaa gtggtggaca 2820
   agggcgcttc cgcccagagc ttcatcgagc ggatgaccaa cttcgataag aacctgccca 2880
   acgagaaggt gctgcccaag cacagcctgc tgtacgagta cttcaccgtg tataacgagc 2940
   tgaccaaagt gaaatacgtg accgagggaa tgagaaagcc cgccttcctg agcggcgagc 3000
   agaaaaaggc catcgtggac ctgctgttca agaccaaccg gaaagtgacc gtgaagcagc 3060
   tgaaagagga ctacttcaag aaaatcgagt gcttcgactc cgtggaaatc tccggcgtgg 3120
   aagatcggtt caacgcctcc ctgggcacat accacgatct gctgaaaatt atcaaggaca 3180
   aggacttcct ggacaatgag gaaaacgagg acattctgga agatatcgtg ctgaccctga 3240
   cactgtttga ggacagagag atgatcgagg aacggctgaa aacctatgcc cacctgttcg 3300
   acgacaaagt gatgaagcag ctgaagcggc ggagatacac cggctggggc aggctgagcc 3360
   ggaagctgat caacggcatc cgggacaagc agtccggcaa gacaatcctg gatttcctga 3420
   agtccgacgg cttcgccaac agaaacttca tgcagctgat ccacgacgac agcctgacct 3480
   ttaaagagga catccagaaa gcccaggtgt ccggccaggg cgatagcctg cacgagcaca 3540
   ttgccaatct ggccggcagc cccgccatta agaagggcat cctgcagaca gtgaaggtgg 3600
   tggacgagct cgtgaaagtg atgggccggc acaagcccga gaacatcgtg atcgaaatgg 3660
   ccagagagaa ccagaccacc cagaagggac agaagaacag ccgcgagaga atgaagcgga 3720
   tcgaagaggg catcaaagag ctgggcagcc agatcctgaa agaacacccc gtggaaaaca 3780
   cccagctgca gaacgagaag ctgtacctgt actacctgca gaatgggcgg gatatgtacg 3840
   tggaccagga actggacatc aaccggctgt ccgactacga tgtggaccat atcgtgcctc 3900
   agagctttct gaaggacgac tccatcgaca acaaggtgct gaccagaagc gacaagaacc 3960
   ggggcaagag cgacaacgtg ccctccgaag aggtcgtgaa gaagatgaag aactactggc 4020
   ggcagctgct gaacgccaag ctgattaccc agagaaagtt cgacaatctg accaaggccg 4080
   agagaggcgg cctgagcgaa ctggataagg ccggcttcat caagagacag ctggtggaaa 4140
   cccggcagat cacaaagcac gtggcacaga tcctggactc ccggatgaac actaagtacg 4200
   acgagaatga caagctgatc cgggaagtga aagtgatcac cctgaagtcc aagctggtgt 4260
   ccgatttccg gaaggatttc cagttttaca aagtgcgcga gatcaacaac taccaccacg 4320
   cccacgacgc ctacctgaac gccgtcgtgg gaaccgccct gatcaaaaag taccctaagc 4380
   tggaaagcga gttcgtgtac ggcgactaca aggtgtacga cgtgcggaag atgatcgcca 4440
   agagcgagca ggaaatcggc aaggctaccg ccaagtactt cttctacagc aacatcatga 4500
   actttttcaa gaccgagatt accctggcca acggcgagat ccggaagcgg cctctgatcg 4560
   agacaaacgg cgaaaccggg gagatcgtgt gggataaggg ccgggatttt gccaccgtgc 4620
   ggaaagtgct gagcatgccc caagtgaata tcgtgaaaaa gaccgaggtg cagacaggcg 4680
   gcttcagcaa agagtctatc ctgcccaaga ggaacagcga taagctgatc gccagaaaga 4740
   aggactggga ccctaagaag tacggcggct tcgacagccc caccgtggcc tattctgtgc 4800
   tggtggtggc caaagtggaa aagggcaagt ccaagaaact gaagagtgtg aaagagctgc 4860
   tggggatcac catcatggaa agaagcagct tcgagaagaa tcccatcgac tttctggaag 4920
   ccaagggcta caaagaagtg aaaaaggacc tgatcatcaa gctgcctaag tactccctgt 4980
   tcgagctgga aaacggccgg aagagaatgc tggcctctgc cggcgaactg cagaagggaa 5040
   acgaactggc cctgccctcc aaatatgtga acttcctgta cctggccagc cactatgaga 5100
   agctgaaggg ctcccccgag gataatgagc agaaacagct gtttgtggaa cagcacaagc 5160
   actacctgga cgagatcatc gagcagatca gcgagttctc caagagagtg atcctggccg 5220
   acgctaatct ggacaaagtg ctgtccgcct acaacaagca ccgggataag cccatcagag 5280
   agcaggccga gaatatcatc cacctgttta ccctgaccaa tctgggagcc cctgccgcct 5340
   tcaagtactt tgacaccacc atcgaccgga agaggtacac cagcaccaaa gaggtgctgg 5400
   acgccaccct gatccaccag agcatcaccg gcctgtacga gacacggatc gacctgtctc 5460
   agctgggagg cgacaaaagg ccggcggcca cgaaaaaggc cggccaggca aaaaagaaaa 5520
   agtaagaatt cctagagctc gctgatcagc ctcgactgtg ccttctagtt gccagccatc 5580
   tgttgtttgc ccctcccccg tgccttcctt gaccctggaa ggtgccactc ccactgtcct 5640
   ttcctaataa aatgaggaaa ttgcatcgca ttgtctgagt aggtgtcatt ctattctggg 5700
   gggtggggtg gggcaggaca gcaaggggga ggattgggaa gagaatagca ggcatgctgg 5760
   ggagcggccg caggaacccc tagtgatgga gttggccact ccctctctgc gcgctcgctc 5820
   gctcactgag gccgggcgac caaaggtcgc ccgacgcccg ggctttgccc gggcggcctc 5880
   agtgagcgag cgagcgcgca gctgcctgca ggggcgcctg atgcggtatt ttctccttac 5940
   gcatctgtgc ggtatttcac accgcatacg tcaaagcaac catagtacgc gccctgtagc 6000
   ggcgcattaa gcgcggcggg tgtggtggtt acgcgcagcg tgaccgctac acttgccagc 6060
   gccctagcgc ccgctccttt cgctttcttc ccttcctttc tcgccacgtt cgccggcttt 6120
   ccccgtcaag ctctaaatcg ggggctccct ttagggttcc gatttagtgc tttacggcac 6180
   ctcgacccca aaaaacttga tttgggtgat ggttcacgta gtgggccatc gccctgatag 6240
   acggtttttc gccctttgac gttggagtcc acgttcttta atagtggact cttgttccaa 6300
   actggaacaa cactcaaccc tatctcgggc tattcttttg atttataagg gattttgccg 6360
   atttcggcct attggttaaa aaatgagctg atttaacaaa aatttaacgc gaattttaac 6420
   aaaatattaa cgtttacaat tttatggtgc actctcagta caatctgctc tgatgccgca 6480
   tagttaagcc agccccgaca cccgccaaca cccgctgacg cgccctgacg ggcttgtctg 6540
   ctcccggcat ccgcttacag acaagctgtg accgtctccg ggagctgcat gtgtcagagg 6600
   ttttcaccgt catcaccgaa acgcgcgaga cgaaagggcc tcgtgatacg cctattttta 6660
   taggttaatg tcatgataat aatggtttct tagacgtcag gtggcacttt tcggggaaat 6720
   gtgcgcggaa cccctatttg tttatttttc taaatacatt caaatatgta tccgctcatg 6780
   agacaataac cctgataaat gcttcaataa tattgaaaaa ggaagagtat gagtattcaa 6840
   catttccgtg tcgcccttat tccctttttt gcggcatttt gccttcctgt ttttgctcac 6900
   ccagaaacgc tggtgaaagt aaaagatgct gaagatcagt tgggtgcacg agtgggttac 6960
   atcgaactgg atctcaacag cggtaagatc cttgagagtt ttcgccccga agaacgtttt 7020
   ccaatgatga gcacttttaa agttctgcta tgtggcgcgg tattatcccg tattgacgcc 7080
   gggcaagagc aactcggtcg ccgcatacac tattctcaga atgacttggt tgagtactca 7140
   ccagtcacag aaaagcatct tacggatggc atgacagtaa gagaattatg cagtgctgcc 7200
   ataaccatga gtgataacac tgcggccaac ttacttctga caacgatcgg aggaccgaag 7260
   gagctaaccg cttttttgca caacatgggg gatcatgtaa ctcgccttga tcgttgggaa 7320
   ccggagctga atgaagccat accaaacgac gagcgtgaca ccacgatgcc tgtagcaatg 7380
   gcaacaacgt tgcgcaaact attaactggc gaactactta ctctagcttc ccggcaacaa 7440
   ttaatagact ggatggaggc ggataaagtt gcaggaccac ttctgcgctc ggcccttccg 7500
   gctggctggt ttattgctga taaatctgga gccggtgagc gtggaagccg cggtatcatt 7560
   gcagcactgg ggccagatgg taagccctcc cgtatcgtag ttatctacac gacggggagt 7620
   caggcaacta tggatgaacg aaatagacag atcgctgaga taggtgcctc actgattaag 7680
   cattggtaac tgtcagacca agtttactca tatatacttt agattgattt aaaacttcat 7740
   ttttaattta aaaggatcta ggtgaagatc ctttttgata atctcatgac caaaatccct 7800
   taacgtgagt tttcgttcca ctgagcgtca gaccccgtag aaaagatcaa aggatcttct 7860
   tgagatcctt tttttctgcg cgtaatctgc tgcttgcaaa caaaaaaacc accgctacca 7920
   gcggtggttt gtttgccgga tcaagagcta ccaactcttt ttccgaaggt aactggcttc 7980
   agcagagcgc agataccaaa tactgtcctt ctagtgtagc cgtagttagg ccaccacttc 8040
   aagaactctg tagcaccgcc tacatacctc gctctgctaa tcctgttacc agtggctgct 8100
   gccagtggcg ataagtcgtg tcttaccggg ttggactcaa gacgatagtt accggataag 8160
   gcgcagcggt cgggctgaac ggggggttcg tgcacacagc ccagcttgga gcgaacgacc 8220
   tacaccgaac tgagatacct acagcgtgag ctatgagaaa gcgccacgct tcccgaaggg 8280
   agaaaggcgg acaggtatcc ggtaagcggc agggtcggaa caggagagcg cacgagggag 8340
   cttccagggg gaaacgcctg gtatctttat agtcctgtcg ggtttcgcca cctctgactt 8400
   gagcgtcgat ttttgtgatg ctcgtcaggg gggcggagcc tatggaaaaa cgccagcaac 8460
   gcggcctttt tacggttcct ggccttttgc tggccttttg ctcacatgt 8509
<210> 25
   <211> 9177
   <212> DNA
   <213> Artificial Sequence
<220>
   <223> synthetic DNA
<400> 25
   ctggcctttt gctcacatgt gagggcctat ttcccatgat tccttcatat ttgcatatac        60
   gatacaaggc tgttagagag ataattggaa ttaatttgac tgtaaacaca aagatattag       120
   tacaaaatac gtgacgtaga aagtaataat ttcttgggta gtttgcagtt ttaaaattat       180
   gttttaaaat ggactatcat atgcttaccg taacttgaaa gtatttcgat ttcttggctt       240
   tatatatctt gtggaaagga cgaaacaccg ctatctgttg acataacata gttttagagc       300
   tagaaatagc aagttaaaat aaggctagtc cgttatcaac ttgaaaaagt ggcaccgagt       360
   cggtgctttt ttgttttaga gctagaaata gcaagttaaa ataaggctag tccgttttta       420
   gcgcgtgcgc caattctgca gacaaatggc tctagaggta cccgttacat aacttacggt       480
   aaatggcccg cctggctgac cgcccaacga cccccgccca ttgacgtcaa tagtaacgcc       540
   aatagggact ttccattgac gtcaatgggt ggagtattta cggtaaactg cccacttggc       600
   agtacatcaa gtgtatcata tgccaagtac gccccctatt gacgtcaatg acggtaaatg       660
   gcccgcctgg cattgtgccc agtacatgac cttatgggac tttcctactt ggcagtacat       720
   ctacgtatta gtcatcgcta ttaccatggt cgaggtgagc cccacgttct gcttcactct       780
   ccccatctcc cccccctccc cacccccaat tttgtattta tttatttttt aattattttg        840
   tgcagcgatg ggggcggggg gggggggggg gcgcgcgcca ggcggggcgg ggcggggcga       900
   ggggcggggc ggggcgaggc ggagaggtgc ggcggcagcc aatcagagcg gcgcgctccg       960
   aaagtttcct tttatggcga ggcggcggcg gcggcggccc tataaaaagc gaagcgcgcg 1020
   gcgggcggga gtcgctgcga cgctgccttc gccccgtgcc ccgctccgcc gccgcctcgc 1080
   gccgcccgcc ccggctctga ctgaccgcgt tactcccaca ggtgagcggg cgggacggcc 1140
   cttctcctcc gggctgtaat tagctgagca agaggtaagg gtttaaggga tggttggttg 1200
   gtggggtatt aatgtttaat tacctggagc acctgcctga aatcactttt tttcaggttg 1260
   gaccggtgcc accatggact ataaggacca cgacggagac tacaaggatc atgatattga 1320
   ttacaaagac gatgacgata agatggcccc aaagaagaag cggaaggtcg gtatccacgg 1380
   agtcccagca gccgacaaga agtacagcat cggcctggac atcggcacca actctgtggg 1440
   ctgggccgtg atcaccgacg agtacaaggt gcccagcaag aaattcaagg tgctgggcaa 1500
   caccgaccgg cacagcatca agaagaacct gatcggagcc ctgctgttcg acagcggcga 1560
   aacagccgag gccacccggc tgaagagaac cgccagaaga agatacacca gacggaagaa 1620
   ccggatctgc tatctgcaag agatcttcag caacgagatg gccaaggtgg acgacagctt 1680
   cttccacaga ctggaagagt ccttcctggt ggaagaggat aagaagcacg agcggcaccc 1740
   catcttcggc aacatcgtgg acgaggtggc ctaccacgag aagtacccca ccatctacca 1800
   cctgagaaag aaactggtgg acagcaccga caaggccgac ctgcggctga tctatctggc 1860
   cctggcccac atgatcaagt tccggggcca cttcctgatc gagggcgacc tgaaccccga 1920
   caacagcgac gtggacaagc tgttcatcca gctggtgcag acctacaacc agctgttcga 1980
   ggaaaacccc atcaacgcca gcggcgtgga cgccaaggcc atcctgtctg ccagactgag 2040
   caagagcaga cggctggaaa atctgatcgc ccagctgccc ggcgagaaga agaatggcct 2100
   gttcggaaac ctgattgccc tgagcctggg cctgaccccc aacttcaaga gcaacttcga 2160
   cctggccgag gatgccaaac tgcagctgag caaggacacc tacgacgacg acctggacaa 2220
   cctgctggcc cagatcggcg accagtacgc cgacctgttt ctggccgcca agaacctgtc 2280
   cgacgccatc ctgctgagcg acatcctgag agtgaacacc gagatcacca aggcccccct 2340
   gagcgcctct atgatcaaga gatacgacga gcaccaccag gacctgaccc tgctgaaagc 2400
   tctcgtgcgg cagcagctgc ctgagaagta caaagagatt ttcttcgacc agagcaagaa 2460
   cggctacgcc ggctacattg acggcggagc cagccaggaa gagttctaca agttcatcaa 2520
   gcccatcctg gaaaagatgg acggcaccga ggaactgctc gtgaagctga acagagagga 2580
   cctgctgcgg aagcagcgga ccttcgacaa cggcagcatc ccccaccaga tccacctggg 2640
   agagctgcac gccattctgc ggcggcagga agatttttac ccattcctga aggacaaccg 2700
   ggaaaagatc gagaagatcc tgaccttccg catcccctac tacgtgggcc ctctggccag 2760
   gggaaacagc agattcgcct ggatgaccag aaagagcgag gaaaccatca ccccctggaa 2820
   cttcgaggaa gtggtggaca agggcgcttc cgcccagagc ttcatcgagc ggatgaccaa 2880
   cttcgataag aacctgccca acgagaaggt gctgcccaag cacagcctgc tgtacgagta 2940
   cttcaccgtg tataacgagc tgaccaaagt gaaatacgtg accgagggaa tgagaaagcc 3000
   cgccttcctg agcggcgagc agaaaaaggc catcgtggac ctgctgttca agaccaaccg 3060
   gaaagtgacc gtgaagcagc tgaaagagga ctacttcaag aaaatcgagt gcttcgactc 3120
   cgtggaaatc tccggcgtgg aagatcggtt caacgcctcc ctgggcacat accacgatct 3180
   gctgaaaatt atcaaggaca aggacttcct ggacaatgag gaaaacgagg acattctgga 3240
   agatatcgtg ctgaccctga cactgtttga ggacagagag atgatcgagg aacggctgaa 3300
   aacctatgcc cacctgttcg acgacaaagt gatgaagcag ctgaagcggc ggagatacac 3360
   cggctggggc aggctgagcc ggaagctgat caacggcatc cgggacaagc agtccggcaa 3420
   gacaatcctg gatttcctga agtccgacgg cttcgccaac agaaacttca tgcagctgat 3480
   ccacgacgac agcctgacct ttaaagagga catccagaaa gcccaggtgt ccggccaggg 3540
   cgatagcctg cacgagcaca ttgccaatct ggccggcagc cccgccatta agaagggcat 3600
   cctgcagaca gtgaaggtgg tggacgagct cgtgaaagtg atgggccggc acaagcccga 3660
   gaacatcgtg atcgaaatgg ccagagagaa ccagaccacc cagaagggac agaagaacag 3720
   ccgcgagaga atgaagcgga tcgaagaggg catcaaagag ctgggcagcc agatcctgaa 3780
   agaacacccc gtggaaaaca cccagctgca gaacgagaag ctgtacctgt actacctgca 3840
   gaatgggcgg gatatgtacg tggaccagga actggacatc aaccggctgt ccgactacga 3900
   tgtggaccat atcgtgcctc agagctttct gaaggacgac tccatcgaca acaaggtgct 3960
   gaccagaagc gacaagaacc ggggcaagag cgacaacgtg ccctccgaag aggtcgtgaa 4020
   gaagatgaag aactactggc ggcagctgct gaacgccaag ctgattaccc agagaaagtt 4080
   cgacaatctg accaaggccg agagaggcgg cctgagcgaa ctggataagg ccggcttcat 4140
   caagagacag ctggtggaaa cccggcagat cacaaagcac gtggcacaga tcctggactc 4200
   ccggatgaac actaagtacg acgagaatga caagctgatc cgggaagtga aagtgatcac 4260
   cctgaagtcc aagctggtgt ccgatttccg gaaggatttc cagttttaca aagtgcgcga 4320
   gatcaacaac taccaccacg cccacgacgc ctacctgaac gccgtcgtgg gaaccgccct 4380
   gatcaaaaag taccctaagc tggaaagcga gttcgtgtac ggcgactaca aggtgtacga 4440
   cgtgcggaag atgatcgcca agagcgagca ggaaatcggc aaggctaccg ccaagtactt 4500
   cttctacagc aacatcatga actttttcaa gaccgagatt accctggcca acggcgagat 4560
   ccggaagcgg cctctgatcg agacaaacgg cgaaaccggg gagatcgtgt gggataaggg 4620
   ccgggatttt gccaccgtgc ggaaagtgct gagcatgccc caagtgaata tcgtgaaaaa 4680
   gaccgaggtg cagacaggcg gcttcagcaa agagtctatc ctgcccaaga ggaacagcga 4740
   taagctgatc gccagaaaga aggactggga ccctaagaag tacggcggct tcgacagccc 4800
   caccgtggcc tattctgtgc tggtggtggc caaagtggaa aagggcaagt ccaagaaact 4860
   gaagagtgtg aaagagctgc tggggatcac catcatggaa agaagcagct tcgagaagaa 4920
   tcccatcgac tttctggaag ccaagggcta caaagaagtg aaaaaggacc tgatcatcaa 4980
   gctgcctaag tactccctgt tcgagctgga aaacggccgg aagagaatgc tggcctctgc 5040
   cggcgaactg cagaagggaa acgaactggc cctgccctcc aaatatgtga acttcctgta 5100
   cctggccagc cactatgaga agctgaaggg ctcccccgag gataatgagc agaaacagct 5160
   gtttgtggaa cagcacaagc actacctgga cgagatcatc gagcagatca gcgagttctc 5220
   caagagagtg atcctggccg acgctaatct ggacaaagtg ctgtccgcct acaacaagca 5280
   ccgggataag cccatcagag agcaggccga gaatatcatc cacctgttta ccctgaccaa 5340
   tctgggagcc cctgccgcct tcaagtactt tgacaccacc atcgaccgga agaggtacac 5400
   cagcaccaaa gaggtgctgg acgccaccct gatccaccag agcatcaccg gcctgtacga 5460
   gacacggatc gacctgtctc agctgggagg cgacaaaagg ccggcggcca cgaaaaaggc 5520
   cggccaggca aaaaagaaaa aggaattcgg cagtggagag ggcagaggaa gtctgctaac 5580
   atgcggtgac gtcgaggaga atcctggccc aatgaccgag tacaagccca cggtgcgcct 5640
   cgccacccgc gacgacgtcc ccagggccgt acgcaccctc gccgccgcgt tcgccgacta 5700
   ccccgccacg cgccacaccg tcgatccgga ccgccacatc gagcgggtca ccgagctgca 5760
   agaactcttc ctcacgcgcg tcgggctcga catcggcaag gtgtgggtcg cggacgacgg 5820
   cgccgcggtg gcggtctgga ccacgccgga gagcgtcgaa gcgggggcgg tgttcgccga 5880
   gatcggcccg cgcatggccg agttgagcgg ttcccggctg gccgcgcagc aacagatgga 5940
   aggcctcctg gcgccgcacc ggcccaagga gcccgcgtgg ttcctggcca ccgtcggagt 6000
   ctcgcccgac caccagggca agggtctggg cagcgccgtc gtgctccccg gagtggaggc 6060
   ggccgagcgc gccggggtgc ccgccttcct ggagacctcc gcgccccgca acctcccctt 6120
   ctacgagcgg ctcggcttca ccgtcaccgc cgacgtcgag gtgcccgaag gaccgcgcac 6180
   ctggtgcatg acccgcaagc ccggtgcctg agaattctaa ctagagctcg ctgatcagcc 6240
   tcgactgtgc cttctagttg ccagccatct gttgtttgcc cctcccccgt gccttccttg 6300
   accctggaag gtgccactcc cactgtcctt tcctaataaa atgaggaaat tgcatcgcat 6360
   tgtctgagta ggtgtcattc tattctgggg ggtggggtgg ggcaggacag caagggggag 6420
   gattgggaag agaatagcag gcatgctggg gagcggccgc aggaacccct agtgatggag 6480
   ttggccactc cctctctgcg cgctcgctcg ctcactgagg ccgggcgacc aaaggtcgcc 6540
   cgacgcccgg gctttgcccg ggcggcctca gtgagcgagc gagcgcgcag ctgcctgcag 6600
   gggcgcctga tgcggtattt tctccttacg catctgtgcg gtatttcaca ccgcatacgt 6660
   caaagcaacc atagtacgcg ccctgtagcg gcgcattaag cgcggcgggt gtggtggtta 6720
   cgcgcagcgt gaccgctaca cttgccagcg ccctagcgcc cgctcctttc gctttcttcc 6780
   cttcctttct cgccacgttc gccggctttc cccgtcaagc tctaaatcgg gggctccctt 6840
   tagggttccg atttagtgct ttacggcacc tcgaccccaa aaaacttgat ttgggtgatg 6900
   gttcacgtag tgggccatcg ccctgataga cggtttttcg ccctttgacg ttggagtcca 6960
   cgttctttaa tagtggactc ttgttccaaa ctggaacaac actcaaccct atctcgggct 7020
   attcttttga tttataaggg attttgccga tttcggccta ttggttaaaa aatgagctga 7080
   tttaacaaaa atttaacgcg aattttaaca aaatattaac gtttacaatt ttatggtgca 7140
   ctctcagtac aatctgctct gatgccgcat agttaagcca gccccgacac ccgccaacac 7200
   ccgctgacgc gccctgacgg gcttgtctgc tcccggcatc cgcttacaga caagctgtga 7260
   ccgtctccgg gagctgcatg tgtcagaggt tttcaccgtc atcaccgaaa cgcgcgagac 7320
   gaaagggcct cgtgatacgc ctatttttat aggttaatgt catgataata atggtttctt 7380
   agacgtcagg tggcactttt cggggaaatg tgcgcggaac ccctatttgt ttatttttct 7440
   aaatacattc aaatatgtat ccgctcatga gacaataacc ctgataaatg cttcaataat 7500
   attgaaaaag gaagagtatg agtattcaac atttccgtgt cgcccttatt cccttttttg 7560
   cggcattttg ccttcctgtt tttgctcacc cagaaacgct ggtgaaagta aaagatgctg 7620
   aagatcagtt gggtgcacga gtgggttaca tcgaactgga tctcaacagc ggtaagatcc 7680
   ttgagagttt tcgccccgaa gaacgttttc caatgatgag cacttttaaa gttctgctat 7740
   gtggcgcggt attatcccgt attgacgccg ggcaagagca actcggtcgc cgcatacact 7800
   attctcagaa tgacttggtt gagtactcac cagtcacaga aaagcatctt acggatggca 7860
   tgacagtaag agaattatgc agtgctgcca taaccatgag tgataacact gcggccaact 7920
   tacttctgac aacgatcgga ggaccgaagg agctaaccgc ttttttgcac aacatggggg 7980
   atcatgtaac tcgccttgat cgttgggaac cggagctgaa tgaagccata ccaaacgacg 8040
   agcgtgacac cacgatgcct gtagcaatgg caacaacgtt gcgcaaacta ttaactggcg 8100
   aactacttac tctagcttcc cggcaacaat taatagactg gatggaggcg gataaagttg 8160
   caggaccact tctgcgctcg gcccttccgg ctggctggtt tattgctgat aaatctggag 8220
   ccggtgagcg tggaagccgc ggtatcattg cagcactggg gccagatggt aagccctccc 8280
   gtatcgtagt tatctacacg acggggagtc aggcaactat ggatgaacga aatagacaga 8340
   tcgctgagat aggtgcctca ctgattaagc attggtaact gtcagaccaa gtttactcat 8400
   atatacttta gattgattta aaacttcatt tttaatttaa aaggatctag gtgaagatcc 8460
   tttttgataa tctcatgacc aaaatccctt aacgtgagtt ttcgttccac tgagcgtcag 8520
   accccgtaga aaagatcaaa ggatcttctt gagatccttt ttttctgcgc gtaatctgct 8580
   gcttgcaaac aaaaaaacca ccgctaccag cggtggtttg tttgccggat caagagctac 8640
   caactctttt tccgaaggta actggcttca gcagagcgca gataccaaat actgtccttc 8700
   tagtgtagcc gtagttaggc caccacttca agaactctgt agcaccgcct acatacctcg 8760
   ctctgctaat cctgttacca gtggctgctg ccagtggcga taagtcgtgt cttaccgggt 8820
   tggactcaag acgatagtta ccggataagg cgcagcggtc gggctgaacg gggggttcgt 8880
   gcacacagcc cagcttggag cgaacgacct acaccgaact gagataccta cagcgtgagc 8940
   tatgagaaag cgccacgctt cccgaaggga gaaaggcgga caggtatccg gtaagcggca 9000
   gggtcggaac aggagagcgc acgagggagc ttccaggggg aaacgcctgg tatctttata 9060
   gtcctgtcgg gtttcgccac ctctgacttg agcgtcgatt tttgtgatgc tcgtcagggg 9120
   ggcggagcct atggaaaaac gccagcaacg cggccttttt acggttcctg gcctttt 9177
<210> 26
   <211> 21
   <212> DNA
   <213> Homo sapiens
<400> 26
   ccatatgtta tatcaacaga t 21
<210> 27
   <211> 22
   <212> DNA
   <213> Artificial Sequence
<220>
   <223> oligonucleotide
<400> 27
   ggttggtcat tgactgttga gg 22
<210> 28
   <211> 20
   <212> DNA
   <213> Artificial Sequence
<220>
   <223> oligonucleotide
<400> 28
   gtgctggtga caggggtagc 20
<210> 29
   <211> 24
   <212> DNA
   <213> Artificial Sequence
<220>
   <223> oligonucleotide
<400> 29
   atggccacaa ccatggtgag caag 24
<210> 30
   <211> 25
   <212> DNA
   <213> Artificial Sequence
<220>
   <223> oligonucleotide
<400> 30
   cctcctgcat ttgctggagt caggg 25
<210> 31
   <211> 22
   <212> DNA
   <213> Artificial Sequence
<220>
   <223> oligonucleotide
<400> 31
   tgatggattc gggcaagttt ct 22
<210> 32
   <211> 20
   <212> DNA
   <213> Artificial Sequence
<220>
   <223> oligonucleotide
<400> 32
   acatcggcaa ggtgtgggtc 20
<210> 33
   <211> 25
   <212> DNA
   <213> Artificial Sequence
<220>
   <223> oligonucleotide
<400> 33
   accgagatcc tccaaacagt agaca 25
<210> 34
   <211> 14437
   <212> DNA
   <213> Artificial Sequence
<220>
   <223> synthetic DNA
<220>
   <221> misc_feature
   <222> (8819)..(8821)
   <223> n is a, c, g, or t
<400> 34
<210> 35
   <211> 7602
   <212> DNA
   <213> Artificial Sequence
<220>
   <223> synthetic DNA
<400> 35
   actcttcctt tttcaatgac cgctttggaa aaacaaagac tgtatttcct ggaaattaat        60
   gtttattcaa taaactgtgt attcagctat atcacatagt ggtgaggctg aaatgaggcg       120
   ggaagaggcg gttggggctt aattatatca atttgggtgg ccccacagcg cctccaaggc       180
   gccagtcctg ttttgacaag ttgcctctgg aagcctctac aatgcctctc ttctttttct       240
   ccagagtaag cggaggccag gggcccccgg cctctgctta atactaaaaa aaacagctgt 300
   tgtcatagta atgattgggt ggaaacattc caggcctggg tggagaggct ttttgcttcc 360
   tcttgcaaaa ccacactgac attccaggcc tgggtggaga ggctttttgc ttcctcttgc 420
   aaaaccacac tgccctctgg agggcagttg cctagcaact aactaaaaga ggatgtcgca 480
   cggccagctg cggtcagtta gtcacttcct gcttaactga cttgacattt tctattttaa 540
   gagtcgggag gaaaattact gtgttggagg ccctccgcca tcttctgaag ctgaatcgaa 600
   ttaacttgtt tattgcagct tataatggtt acaaataaag caatagcatc acaaatttca 660
   caaataaagc atttttttca ctgcattcta gttgtggttt gtccaaactc atcaatgtat 720
   cttatcatgt ctggatctga tatcatcgtc gacattgatt attgactagt tattaatagt 780
   aatcaattac ggggtcatta gttcatagcc catatatgga gttccgcgtt acataactta 840
   cggtaaatgg cccgcctggc tgaccgccca acgacccccg cccattgacg tcaataatga 900
   cgtatgttcc catagtaacg ccaataggga ctttccattg acgtcaatgg gtggagtatt 960
   tacggtaaac tgcccacttg gcagtacatc aagtgtatca tatgccaagt acgcccccta 1020
   ttgacgtcaa tgacggtaaa tggcccgcct ggcattatgc ccagtacatg accttatggg 1080
   actttcctac ttggcagtac atctacgtat tagtcatcgc tattaccatg gtcgaggtga 1140
   gccccacgtt ctgcttcact ctccccatct cccccccctc cccaccccca attttgtatt 1200
   tatttatttt ttaattattt tgtgcagcga tgggggcggg gggggggggg gggcgcgcgc 1260
   caggcggggc ggggcggggc gaggggcggg gcggggcgag gcggagaggt gcggcggcag 1320
   ccaatcagag cggcgcgctc cgaaagtttc cttttatggc gaggcggcgg cggcggcggc 1380
   cctataaaaa gcgaagcgcg cggcgggcgg gagtcgctgc gcgctgcctt cgccccgtgc 1440
   cccgctccgc gccgcctcgc gccgcccgcc ccggctctga ctgaccgcgt tactcccaca 1500
   ggtgagcggg cgggacggcc cttctcctcc gggctgtaat tagcgcttgg tttaatgacg 1560
   gcttgtttct tttctgtggc tgcgtgaaag ccttgagggg ctccgggagg gccctttgtg 1620
   cggggggagc ggctcggggg gtgcgtgcgt gtgtgtgtgc gtggggagcg ccgcgtgcgg 1680
   ctccgcgctg cccggcggct gtgagcgctg cgggcgcggc gcggggcttt gtgcgctccg 1740
   cagtgtgcgc gaggggagcg cggccggggg cggtgccccg cggtgcgggg ggggctgcga 1800
   ggggaacaaa ggctgcgtgc ggggtgtgtg cgtggggggg tgagcagggg gtgtgggcgc 1860
   gtcggtcggg ctgcaacccc ccctgcaccc ccctccccga gttgctgagc acggcccggc 1920
   ttcgggtgcg gggctccgta cggggcgtgg cgcggggctc gccgtgccgg gcggggggtg 1980
   gcggcaggtg ggggtgccgg gcggggcggg gccgcctcgg gccggggagg gctcggggga 2040
   ggggcgcggc ggcccccgga gcgccggcgg ctgtcgaggc gcggcgagcc gcagccattg 2100
   ccttttatgg taatcgtgcg agagggcgca gggacttcct ttgtcccaaa tctgtgcgga 2160
   gccgaaatct gggaggcgcc gccgcacccc ctctagcggg cgcggggcga agcggtgcgg 2220
   cgccggcagg aaggaaatgg gcggggaggg ccttcgtgcg tcgccgcgcc gccgtcccct 2280
   tctccctctc cagcctcggg gctgtccgcg gggggacggc tgccttcggg ggggacgggg 2340
   cagggcgggg ttcggcttct ggcgtgtgac cggcggctct agagcctctg ctaaccatgt 2400
   tcatgccttc ttctttttcc tacagctcct gggcaacgtg ctggttattg tgctgtctca 2460
   tcattttggc aaagaatttc gacctgcagc ccaagctgta ccccctcgaa actgacagga 2520
   gaaccaccat gggcccaaag aagaagagaa aggtttcgaa tttactgacc gtacaccaaa 2580
   atttgcctgc attaccggtc gatgcaacga gtgatgaggt tcgcaagaac ctgatggaca 2640
   tgttcaggga tcgccaggcg ttttctgagc atacctggaa aatgcttctg tccgtttgcc 2700
   ggtcgtgggc ggcatggtgc aagttgaata accggaaatg gtttcccgca gaacctgaag 2760
   atgttcgcga ttatcttcta tatcttcagg cgcgcggtct ggcagtaaaa actatccagc 2820
   aacatttggg ccagctaaac atgcttcatc gtcggtccgg gctgccacga ccaagtgaca 2880
   gcaatgctgt ttcactggtt atgcggcgga tccgaaaaga aaacgttgat gccggtgaac 2940
   gtgcaaaaca ggctctagcg ttcgaacgca ctgatttcga ccaggttcgt tcactcatgg 3000
   aaaatagcga tcgctgccag gatatacgta atctggcatt tctggggatt gcttataaca 3060
   ccctgttacg tatagccgaa attgccagga tcagggttaa agatatctca cgtactgacg 3120
   gtgggagaat gttaatccat attggcagaa cgaaaacgct ggttagcacc gcaggtgtag 3180
   agaaggcact tagcctgggg gtaactaaac tggtcgagcg atggatttcc gtctctggtg 3240
   tagctgatga tccgaataac tacctgtttt gccgggtcag aaaaaatggt gttgccgcgc 3300
   catctgccac cagccagcta tcaactcgcg ccctggaagg gatttttgaa gcaactcatc 3360
   gattgattta cggcgctaag gatgactctg gtcagagata cctggcctgg tctggacaca 3420
   gtgcccgtgt cggagccgcg cgagatatgg cccgcgctgg agtttcaata ccggagatca 3480
   tgcaagctgg tggctggacc aatgtaaata ttgtcatgaa ctatatccgt aacctggata 3540
   gtgaaacagg ggcaatggtg cgcctgctgg aagatggcga ttagccatta acgcgcgacc 3600
   agcttgatat cgaattaatt cactcctcag gtgcaggctg cctatcagaa ggtggtggct 3660
   ggtgtggcca atgccctggc tcacaaatac cactgaatta agaattccgc ccctctccct 3720
   cccccccccc taacgttact ggccgaagcc gcttggaata aggccggtgt gcgtttgtct 3780
   atatgttatt ttccaccata ttgccgtctt ttggcaatgt gagggcccgg aaacctggcc 3840
   ctgtcttctt gacgagcatt cctaggggtc tttcccctct cgccaaagga atgcaaggtc 3900
   tgttgaatgt cgtgaaggaa gcagttcctc tggaagcttc ttgaagacaa acaacgtctg 3960
   tagcgaccct ttgcaggcag cggaaccccc cacctggcga caggtgcctc tgcggccaaa 4020
   agccacgtgt ataagataca cctgcaaagg cggcacaacc ccagtgccac gttgtgagtt 4080
   ggatagttgt ggaaagagtc aaatggctct cctcaagcgt attcaacaag gggctgaagg 4140
   atgcccagaa ggtaccccat tgtatgggat ctgatctggg gcctcggtgc acatgcttta 4200
   catgtgttta gtcgaggtta aaaaacgtct aggccccccg aaccacgggg acgtggtttt 4260
   cctttgaaaa acacgatgat aatatggcca caaccatgac cgagtacaag cccacggtgc 4320
   gcctcgccac ccgcgacgac gtcccccggg ccgtacgcac cctcgccgcc gcgttcgccg 4380
   actaccccgc cacgcgccac accgtcgacc cggaccgcca catcgagcgg gtcaccgagc 4440
   tgcaagaact cttcctcacg cgcgtcgggc tcgacatcgg caaggtgtgg gtcgcggacg 4500
   acggcgccgc ggtggcggtc tggaccacgc cggagagcgt cgaagcgggg gcggtgttcg 4560
   ccgagatcgg cccgcgcatg gccgagttga gcggttcccg gctggccgcg cagcaacaga 4620
   tggaaggcct cctggcgccg caccggccca aggagcccgc gtggttcctg gccaccgtcg 4680
   gcgtctcgcc cgaccaccag ggcaagggtc tgggcagcgc cgtcgtgctc cccggagtgg 4740
   aggcggccga gcgcgccggg gtgcccgcct tcctggagac ctccgcgccc cgcaacctcc 4800
   ccttctacga gcggctcggc ttcaccgtca ccgccgacgt cgaggtgccc gaaggaccgc 4860
   gcacctggtg catgacccgc aagcccggtg cctgacgccc gccccacgac ccgcagcgcc 4920
   cgaccgaaag gagcgcacga ccccatgcat ggatcgatag cctcgactgt gccttctagt 4980
   tgccagccat ctgttgtttg cccctccccc gtgccttcct tgaccctgga aggtgccact 5040
   cccactgtcc tttcctaata aaatgaggaa attgcatcgc attgtctgag taggtgtcat 5100
   tctattctgg ggggtggggt ggggcaggac agcaaggggg aggattggga agacaatagc 5160
   aggcatgctg gggatgcggt gggctctatc ttctgaggcg gaaagaacct gcagcccaag 5220
   cttggcgtaa tcatggtcat agctgtttcc tgtgtgaaat tgttatccgc tcacaattcc 5280
   acacaacata cgagccggaa gcataaagtg taaagcctgg ggtgcctaat gagtgagcta 5340
   actcacatta attgcgttgc gctcactgcc cgctttccag tcgggaaacc tgtcgtgcca 5400
   gcggatccgc atctcaatta gtcagcaacc atagtcccgc ccctaactcc gcccatcccg 5460
   cccctaactc cgcccagttc cgcccattct ccgccccatg gctgactaat tttttttatt 5520
   tatgcagagg ccgaggccgc ctcggcctct gagctattcc agaagtagtg aggaggcttt 5580
   tttggaggcc taggcttttg caaaaagcta acttgtttat tgcagcttat aatggttaca 5640
   aataaagcaa tagcatcaca aatttcacaa ataaagcatt tttttcactg cattctagtt 5700
   gtggtttgtc caaactcatc aatgtatctt atcatgtctg gatccgctgc attaatgaat 5760
   cggccaacgc gcggggagag gcggtttgcg tattgggcgc tcttccgctt cctcgctcac 5820
   tgactcgctg cgctcggtcg ttcggctgcg gcgagcggta tcagctcact caaaggcggt 5880
   aatacggtta tccacagaat caggggataa cgcaggaaag aacatgtgag caaaaggcca 5940
   gcaaaaggcc aggaaccgta aaaaggccgc gttgctggcg tttttccata ggctccgccc 6000
   ccctgacgag catcacaaaa atcgacgctc aagtcagagg tggcgaaacc cgacaggact 6060
   ataaagatac caggcgtttc cccctggaag ctccctcgtg cgctctcctg ttccgaccct 6120
   gccgcttacc ggatacctgt ccgcctttct cccttcggga agcgtggcgc tttctcaatg 6180
   ctcacgctgt aggtatctca gttcggtgta ggtcgttcgc tccaagctgg gctgtgtgca 6240
   cgaacccccc gttcagcccg accgctgcgc cttatccggt aactatcgtc ttgagtccaa 6300
   cccggtaaga cacgacttat cgccactggc agcagccact ggtaacagga ttagcagagc 6360
   gaggtatgta ggcggtgcta cagagttctt gaagtggtgg cctaactacg gctacactag 6420
   aaggacagta tttggtatct gcgctctgct gaagccagtt accttcggaa aaagagttgg 6480
   tagctcttga tccggcaaac aaaccaccgc tggtagcggt ggtttttttg tttgcaagca 6540
   gcagattacg cgcagaaaaa aaggatctca agaagatcct ttgatctttt ctacggggtc 6600
   tgacgctcag tggaacgaaa actcacgtta agggattttg gtcatgagat tatcaaaaag 6660
   gatcttcacc tagatccttt taaattaaaa atgaagtttt aaatcaatct aaagtatata 6720
   tgagtaaact tggtctgaca gttaccaatg cttaatcagt gaggcaccta tctcagcgat 6780
   ctgtctattt cgttcatcca tagttgcctg actccccgtc gtgtagataa ctacgatacg 6840
   ggagggctta ccatctggcc ccagtgctgc aatgataccg cgagacccac gctcaccggc 6900
   tccagattta tcagcaataa accagccagc cggaagggcc gagcgcagaa gtggtcctgc 6960
   aactttatcc gcctccatcc agtctattaa ttgttgccgg gaagctagag taagtagttc 7020
   gccagttaat agtttgcgca acgttgttgc cattgctaca ggcatcgtgg tgtcacgctc 7080
   gtcgtttggt atggcttcat tcagctccgg ttcccaacga tcaaggcgag ttacatgatc 7140
   ccccatgttg tgcaaaaaag cggttagctc cttcggtcct ccgatcgttg tcagaagtaa 7200
   gttggccgca gtgttatcac tcatggttat ggcagcactg cataattctc ttactgtcat 7260
   gccatccgta agatgctttt ctgtgactgg tgagtactca accaagtcat tctgagaata 7320
   gtgtatgcgg cgaccgagtt gctcttgccc ggcgtcaata cgggataata ccgcgccaca 7380
   tagcagaact ttaaaagtgc tcatcattgg aaaacgttct tcggggcgaa aactctcaag 7440
   gatcttaccg ctgttgagat ccagttcgat gtaacccact cgtgcaccca actgatcttc 7500
   agcatctttt actttcacca gcgtttctgg gtgagcaaaa acaggaaggc aaaatgccgc 7560
   aaaaaaggga ataagggcga cacggaaatg ttgaatactc at 7602
<210> 36
   <211> 20
   <212> DNA
   <213> Artificial Sequence
<220>
   <223> oligonucleotide
<400> 36
   gtatctggcg gcagggtagc 20
<210> 37
   <211> 21
   <212> DNA
   <213> Artificial Sequence
<220>
   <223> oligonucleotide
<400> 37
   tttgttgctg gcattgttct g 21
<210> 38
   <211> 24
   <212> DNA
   <213> Artificial Sequence
<220>
   <223> oligonucleotide
<400> 38
   tggacgtaaa ctcctcttca gacc 24
<210> 39
   <211> 24
   <212> DNA
   <213> Artificial Sequence
<220>
   <223> oligonucleotide
<400> 39
   gaggactaca ccatcgtgga acag 24
<210> 40
   <211> 21
   <212> DNA
   <213> Artificial Sequence
<220>
   <223> oligonucleotide
<400> 40
   ggcttccacc aaagaccaca g 21
<210> 41
   <211> 2041
   <212> DNA
   <213> Artificial Sequence
<220>
   <223> synthetic DNA
<400> 41
   taccgttcgt atagcataca ttatacgaag ttataccatg tctagactgg acaagagcaa 60
   agtcataaac ggcgctctgg aattactcaa tggagtcggt atcgaaggcc tgacgacaag 120
   gaaactcgct caaaagctgg gagttgagca gcctaccctg tactggcacg tgaagaacaa 180
   gcgggccctg ctcgatgccc tgccaatcga gatgctggac aggcatcata cccacttctg 240
   ccccctggaa ggcgagtcat ggcaagactt tctgcggaac aacgccaagt cattccgctg 300
   tgctctcctc tcacatcgcg acggggctaa agtgcatctc ggcacccgcc caacagagaa 360
   acagtacgaa accctggaaa atcagctcgc gttcctgtgt cagcaaggct tctccctgga 420
   gaacgcactg tacgctctgt ccgccgtggg ccactttaca ctgggctgcg tattggagga 480
   acaggagcat caagtagcaa aagaggaaag agagacacct accaccgatt ctatgccccc 540
   acttctgaga caagcaattg agctgttcga ccggcaggga gccgaacctg ccttcctttt 600
   cggcctggaa ctaatcatat gtggcctgga gaaacagcta aagtgcgaaa gcggcgggcc 660
   ggccgacgcc cttgacgatt ttgacttaga catgctccca gccgatgccc ttgacgactt 720
   tgaccttgat atgctgcctg ctgacgctct tgacgatttt gaccttgaca tgctccccgg 780
   gtaactaagt aaggatccag acatgataag atacattgat gagtttggac aaaccacaac 840
   tagaatgcag tgaaaaaaat gctttatttg tgaaatttgt gatgctattg ctttatttgt 900
   aaccattata agctgcaata aacaagttaa caacaacaat tgcattcatt ttatgtttca 960
   ggttcagggg gaggtgtggg aggtttttta aagcaagtaa aacctctaca aatgtggtat 1020
   ggctgattat gatcctctag agtcgcagat ccagacatga taagatacat tgatgagttt 1080
   ggacaaacca caactagaat gcagtgaaaa aaatgcttta tttgtgaaat ttgtgatgct 1140
   attgctttat ttgtaaccat tataagctgc aataaacaag ttaacaacaa caattgcatt 1200
   cattttatgt ttcaggttca gggggaggtg tgggaggttt tttaaagcaa gtaaaacctc 1260
   tacaaatgtg gtatggctga ttatgatcct ctagagtcgc agatccagac atgataagat 1320
   acattgatga gtttggacaa accacaacta gaatgcagtg aaaaaaatgc tttatttgtg 1380
   aaatttgtga tgctattgct ttatttgtaa ccattataag ctgcaataaa caagttaaca 1440
   acaacaattg cattcatttt atgtttcagg ttcaggggga ggtgtgggag gttttttaaa 1500
   gcaagtaaaa cctctacaaa tgtggtatgg ctgattatga tcctctagag tcgcagatcc 1560
   tctagagtcg cagatctttt tccctctgcc aaaaattatg gggacatcat gaagcccctt 1620
   gagcatctga cttctggcta ataaaggaaa tttattttca ttgcaatagt gtgttggaat 1680
   tttttgtgtc tctcactcgg aaggacatat gggagggcaa atcatttaaa acatcagaat 1740
   gagtatttgg tttagagttt ggcaacatat gccatatgct ggctgccatg aacaaaggtg 1800
   gctataaaga ggtcatcagt atatgaaaca gccccctgct gtccattcct tattccatag 1860
   aaaagccttg acttgaggtt agattttttt tatattttgt tttgtgttat ttttttcttt 1920
   aacatcccta aaattttcct tacatgtttt actagccaga tttttcctcc tctcctgact 1980
   actcccagtc atagctgtcc ctcttctctt atgaagatcc ctcgacctgc agcccaagct 2040
   t 2041
<210> 42
   <211> 413
   <212> DNA
   <213> Artificial Sequence
<220>
   <223> synthetic DNA
<400> 42
   ccgcggtggc ggccgcacgt ctccctatca gtgatagaga agtcgacacg tctcgagctc 60
   cctatcagtg atagagaagg tacgtctaga acgtctccct atcagtgata gagaagtcga 120
   cacgtctcga gctccctatc agtgatagag aaggtacgtc tagaacgtct ccctatcagt 180
   gatagagaag tcgacacgtc tcgagctccc tatcagtgat agagaaggta cgtctagaac 240
   gtctccctat cagtgataga gaagtcgaca cgtctcgagc tccctatcag tgatagagaa 300
   ggtaccccct atataagcag agctcgttta gtgaaccgtc agatcgcctg gagacgccat 360
   ccacgctgtt ttgacctcca tagaagacac cgggaccgat ccagcctccg cgg 413
<210> 43
   <211> 1224
   <212> DNA
   <213> Artificial Sequence
<220>
   <223> synthetic DNA
<400> 43
   ggatcccaaa taccactgaa ttaagaattc cgcccctctc cctccccccc ccctaacgtt 60
   actggccgaa gccgcttgga ataaggccgg tgtgcgtttg tctatatgtt attttccacc 120
   atattgccgt cttttggcaa tgtgagggcc cggaaacctg gccctgtctt cttgacgagc 180
   attcctaggg gtctttcccc tctcgccaaa ggaatgcaag gtctgttgaa tgtcgtgaag 240
   gaagcagttc ctctggaagc ttcttgaaga caaacaacgt ctgtagcgac cctttgcagg 300
   cagcggaacc ccccacctgg cgacaggtgc ctctgcggcc aaaagccacg tgtataagat 360
   acacctgcaa aggcggcaca accccagtgc cacgttgtga gttggatagt tgtggaaaga 420
   gtcaaatggc tctcctcaag cgtattcaac aaggggctga aggatgccca gaaggtaccc 480
   cattgtatgg gatctgatct ggggcctcgg tgcacatgct ttacatgtgt ttagtcgagg 540
   ttaaaaaacg tctaggcccc ccgaaccacg gggacgtggt tttcctttga aaaacacgat 600
   gataatatgg ccacaaccat gaccgagtac aagcccacgg tgcgcctcgc cacccgcgac 660
   gacgtccccc gggccgtacg caccctcgcc gccgcgttcg ccgactaccc cgccacgcgc 720
   cacaccgtcg acccggaccg ccacatcgag cgggtcaccg agctgcaaga actcttcctc 780
   acgcgcgtcg ggctcgacat cggcaaggtg tgggtcgcgg acgacggcgc cgcggtggcg 840
   gtctggacca cgccggagag cgtcgaagcg ggggcggtgt tcgccgagat cggcccgcgc 900
   atggccgagt tgagcggttc ccggctggcc gcgcagcaac agatggaagg cctcctggcg 960
   ccgcaccggc ccaaggagcc cgcgtggttc ctggccaccg tcggcgtctc gcccgaccac 1020
   cagggcaagg gtctgggcag cgccgtcgtg ctccccggag tggaggcggc cgagcgcgcc 1080
   ggggtgcccg ccttcctgga gacctccgcg ccccgcaacc tccccttcta cgagcggctc 1140
   ggcttcaccg tcaccgccga cgtcgaggtg cccgaaggac cgcgcacctg gtgcatgacc 1200
   cgcaagcccg gtgcctgagg atcc 1224
<210> 44
   <211> 1044
   <212> DNA
   <213> Artificial Sequence
<220>
   <223> synthetic DNA
<400> 44
   actagttaat taagaaactg acctaaagac ttgcctccag ccccatccta tgggagcatt 60
   ttcttaactg aggttctctc ctctcaagat gacttcagct tgtgacaatg tgacctggcc 120
   taaagctagc cagcacagca caacaccttc cattctttct tctaaggtgc ttatggacat 180
   ttctttctct ttgagtacct tgagtgggat tgtctgtggc tgcttttggg aagcattcaa 240
   ttgaatgatt tttgtttttt gtttatttcc cccgagacag ggtttctctg tgtagccctg 300
   gctgtcctgg aactcacttt gtagacctcg aactcagaaa tccgcctgcc tctgcctccc 360
   aagtgctggg attaatggca tgtgccacca ctgcctggag aattgaatgc ttataaggta 420
   aacagatgct tttattaaaa acaacaacaa caactgttat tcctagtggt tggactttac 480
   agttgtactt tgtttccttt tgaaacagtg ttgcagcgaa cgaggccaca ggttggcctt 540
   gaacttgttc ttccctgtgc taggattaca tcgtgcacaa cttccttctc gcagactctt 600
   attattttaa gaaaaactaa aacagtacac cattttagta cggtagtagt gtgctgaaat 660
   tcagccctgg ggatcagggc cttttgcttt ttttggtaaa ttttgagaca tagtgtcact 720
   aagttgcttt agctagctgt gtatcagtct ccacagtggc cggcttgaca ggcctgcttg 780
   ctttgtgcat gtgaaggggc taagcccttg gctgtgtaga atatacaaac gcagtgtctg 840
   agaaggctca gaatggctca cttgcaagaa cagctaaacc ctaacaagac catctgacca 900
   tctgggatga ctgacgtttg ccggctacgc gggaggccct ggaaaagatg tcttccagat 960
   gatgcttaga ctgtttttga gttagtagca cggaatcaca gcactgtttc ccctcatttt 1020
   agggtccgtc gtaacctgac tagt 1044
<210> 45
   <211> 36
   <212> DNA
   <213> Artificial Sequence
<220>
   <223> oligonucleotide
<400> 45
   actagttaat taagaaactg acctaaagac ttgcct 36
<210> 46
   <211> 35
   <212> DNA
   <213> Artificial Sequence
<220>
   <223> oligonucleotide
<400> 46
   actagtcagg ttacgacgga ccctaaaatg agggg 35
<210> 47
   <211> 682
   <212> DNA
   <213> Artificial Sequence
<220>
   <223> synthetic DNA
<400> 47
   ccatggtacc gttcgtatag catacattat acgaagttat ttgagtaact atggaagact      60
   atatcaaaat agagaaaatt ggagaaggta agtggtttta gcagaatgtt ttttgaaaag     120
   aagataacaa tgctactgtg gaacacagaa acctttactg agtgcagtcg tagatgcatc     180
   tgtacgcgtg cgcatgagtg tgctgtgaga gttggtgttt gtggcctcct agacaggatc     240
   tgggatcact ctgagcacac ctgtagggga ttgtctttga ctctattaat tgacgtgggc     300
   aggaacctcc cccagtgtgg gtgccttcct tgcctgagat cctggactgt gtgactggag     360
   aagggtgtca agccacagtg aacctcactc tcccactgcc tgtgctcttg ctgctgctgc     420
   tgctggactc taaccccgat cctgagtgca aacaaaccct tccttgggct acttttgtct     480
   gggtgtttct cacagcacca gcaaaaggaa ggcacgcagc tgggcgtggt ggcacacacc     540
   tttaatccca gcacttggga ggcagaggca ggcggatttc tgagttcgag gccagcctgg     600
   tctacaaagt tccaggatag ccagggctat acagagaaac cctgtctcga aaaacagaaa     660
   agaaaaaaaa aagaaagcat gc                                               682
<210> 48
   <211> 51
   <212> DNA
   <213> Artificial Sequence
<220>
   <223> oligonucleotide
<400> 48
   gcatgctttc tttttttttt cttttctgtt tttcgagaca gggtttctct g 51
<210> 49
   <211> 72
   <212> DNA
   <213> Artificial Sequence
<220>
   <223> oligonucleotide
<400> 49
   ccatggtacc gttcgtatag catacattat acgaagttat ttgagtaact atggaagact      60
   atatcaaaat ag                                                           72
<210> 50
   <211> 24
   <212> DNA
   <213> Artificial Sequence
<220>
   <223> oligonucleotide
<400> 50
   caccgacgac ggaccctaaa atga 24
<210> 51
   <211> 24
   <212> DNA
   <213> Artificial Sequence
<220>
   <223> oligonucleotide
<400> 51
   aaactcattt tagggtccgt cgtc 24
<210> 52
   <211> 24
   <212> DNA
   <213> Artificial Sequence
<220>
   <223> oligonucleotide
<400> 52
   caccgatatc aaaatagaga aaat 24
<210> 53
   <211> 24
   <212> DNA
   <213> Artificial Sequence
<220>
   <223> oligonucleotide
<400> 53
   aaacattttc tctattttga tatc 24
<210> 54
   <211> 23
   <212> DNA
   <213> Artificial Sequence
<220>
   <223> oligonucleotide
<400> 54
   ccaggccaca tatgattagt tcc 23
<210> 55
   <211> 23
   <212> DNA
   <213> Artificial Sequence
<220>
   <223> oligonucleotide
<400> 55
   gtgagccaag aaattgtttc tcc 23
<210> 56
   <211> 22
   <212> DNA
   <213> Artificial Sequence
<220>
   <223> oligonucleotide
<400> 56
   ttgacctcca tagaagacac cg 22
<210> 57
   <211> 19
   <212> DNA
   <213> Artificial Sequence
<220>
   <223> oligonucleotide
<400> 57
   gctaagtccc ctgccttgg 19
<210> 58
   <211> 1978
   <212> DNA
   <213> Artificial Sequence
<220>
   <223> synthetic DNA
<400> 58
   ggatccggtt ctggcgtgaa acagactttg aattttgacc ttctcaagtt ggcgggagac 60
   gtggagtcca acccagggcc catggccagc tacccctgtc accagcacgc cagcgccttc 120
   gaccaggccg ccagaagcag gggccacagc aaccggcgga ccgccctgag acccaggcgg 180
   cagcaggaag ccaccgaagt ccggctggaa cagaagatgc ccaccctgtt aagggtgtac 240
   atcgacggcc cccacggcat gggcaagacc accaccaccc agctgctggt ggccctgggc 300
   agccgggacg acatcgtgta cgtgcccgag cccatgacct actggcaggt gctgggcgcc 360
   agcgagacca tcgccaacat ctacaccaca cagcacaggc tggaccaggg cgagatctct 420
   gccggcgacg ccgccgtggt gatgaccagc gcccagatca caatgggcat gccctacgcc 480
   gtgaccgacg ccgtgctggc ccctcacgtg ggcggcgagg ccggctctag ccacgcccct 540
   ccccctgccc tgaccctgat cttcgaccgg caccccatcg cccacctgct gtgctaccct 600
   gccgccagat acctgatggg cagcatgacc ccccaggccg tgctggcctt cgtggccctg 660
   atccccccca ccctgcccgg caccaacatc gtgctgggag ccctgcccga ggaccggcac 720
   atcgaccggc tggccaagcg gcagagaccc ggcgagcggc tggacctggc catgctggcc 780
   gccatccggc gggtgtacgg cctgctggcc aacaccgtga gatacctgca gggcggaggg 840
   tcttggtggg aggactgggg ccagctgtcc ggcaccgccg tgccacctca gggcgccgag 900
   ccccagagca atgccggccc tcggccccac atcggcgaca ccctgtttac cctgttcaga 960
   gcccccgagc tgctggcccc caacggcgac ctgtacaacg tgttcgcctg ggccctggac 1020
   gtgctggcca agaggctgcg gcccatgcac gtgttcatcc tggactacga ccagagccct 1080
   gccggctgca gggacgccct gctgcagctg accagcggca tggtgcagac ccacgtgacc 1140
   acccccggca gcatccccac catctgcgac ctggcccgga ccttcgcccg ggagatgggc 1200
   gaggccaacg gctccggaga gggcagagga agtctgctaa catgcggtga cgtcgaggag 1260
   aatcctggcc cactcgagat ggccacaacc atggtgagca agggcgagga ggataacatg 1320
   gccatcatca aggagttcat gcgcttcaag gtgcacatgg agggctccgt gaacggccac 1380
   gagttcgaga tcgagggcga gggcgagggc cgcccctacg agggcaccca gaccgccaag 1440
   ctgaaggtga ccaagggtgg ccccctgccc ttcgcctggg acatcctgtc ccctcagttc 1500
   atgtacggct ccaaggccta cgtgaagcac cccgccgaca tccccgacta cttgaagctg 1560
   tccttccccg agggcttcaa gtgggagcgc gtgatgaact tcgaggacgg cggcgtggtg 1620
   accgtgaccc aggactcctc cctgcaggac ggcgagttca tctacaaggt gaagctgcgc 1680
   ggcaccaact tcccctccga cggccccgta atgcagaaga agaccatggg ctgggaggcc 1740
   tcctccgagc ggatgtaccc cgaggacggc gccctgaagg gcgagatcaa gcagaggctg 1800
   aagctgaagg acggcggcca ctacgacgct gaggtcaaga ccacctacaa ggccaagaag 1860
   cccgtgcagc tgcccggcgc ctacaacgtc aacatcaagt tggacatcac ctcccacaac 1920
   gaggactaca ccatcgtgga acagtacgaa cgcgccgagg gccgccactc caccggcg 1978
<210> 59
   <211> 42
   <212> DNA
   <213> Artificial Sequence
<220>
   <223> oligonucleotide
<400> 59
   gctttctgac aaaaagtttc aatatgttat gtcaacagat ag 42
<210> 60
   <211> 42
   <212> DNA
   <213> Artificial Sequence
<220>
   <223> oligonucleotide
<400> 60
   ctatctgttg acataacata ttgaaacttt ttgtcagaaa gc 42
<210> 61
   <211> 758
   <212> DNA
   <213> Artificial Sequence
<220>
   <223> synthetic DNA
<400> 61
   ggagaatcct ggcccactcg aggtgagcaa gggcgaggag ctgttcaccg gggtggtgcc 60
   catcctggtc gagctggacg gcgacgtaaa cggccacaag ttcagcgtgt ccggcgaggg 120
   cgagggcgat gccacctacg gcaagctgac cctgaagttc atctgcacca ccggcaagct 180
   gcccgtgccc tggcccaccc tcgtgaccac cctgacctac ggcgtgcagt gcttcagccg 240
   ctaccccgac cacatgaagc agcacgactt cttcaagtcc gccatgcccg aaggctacgt 300
   ccaggagcgc accatcttct tcaaggacga cggcaactac aagacccgcg ccgaggtgaa 360
   gttcgagggc gacaccctgg tgaaccgcat cgagctgaag ggcatcgact tcaaggagga 420
   cggcaacatc ctggggcaca agctggagta caactacaac agccacaacg tctatatcat 480
   ggccgacaag cagaagaacg gcatcaaggt gaacttcaag atccgccaca acatcgagga 540
   cggcagcgtg cagctcgccg accactacca gcagaacacc cccatcggcg acggccccgt 600
   gctgctgccc gacaaccact acctgagcac ccagtccgcc ctgagcaaag accccaacga 660
   gaagcgcgat cacatggtcc tgctggagtt cgtgaccgcc gccgggatca ctctcggcat 720
   ggacgagctg tacaagtagc tttctgacaa aaagtttc 758
<210> 62
   <211> 6080
   <212> DNA
   <213> Artificial Sequence
<220>
   <223> synthetic DNA
<400> 62
   tagctttctg acaaaaagtt tcaatatgtt atgtcaacag atagttgtgt ttttattgtt      60
   aactcttgtc tatttttgtc ttatatatat ttctttgtta tcaaacttca gctgtacttc     120
   gtcttctaat ttcaaaaata taacttaaaa atgtaaatat tctatatgaa tttaaatata     180
   attctgtaaa tgtgtgtagg tctcactgta acaactattt gttactataa taaaactata     240
   atattgatgt caggaatcag gaaaaaattt gagttggctt aaatcatctc agtccttatg     300
   gcagttttat tttcctgtag ttggaactac taaaatttag gaaaatgcta agttcaagtt     360
   tcgtaatgct ttgaagtatt tttatgctct gaatgtttaa atgttctcat cagtttcttg     420
   ccatgttgtt aactatacaa cctggctaaa gatgaatatt tttctactgg tattttaatt     480
   tttgacctaa atgtttaagc attcggaatg agaaaactat acagatttga gaaatgatgc     540
   taaatttata ggagttttca gtaacttaaa aagctaacat gagagcatgc caaaatttgc     600
   taagtcttac aaagatcaag ggctgtccgc aacagggaag aacagttttg aaaatttatg     660
   aactatctta tttttaggta ggttttgaaa gctttttgtc taagtgaatt cttatgcctt     720
   ggtcagagta ataactgaag gaggtgctta tcttggcttt cgagtctgag tttaaaacta     780
   cacattttga catagtgttt attagcagcc atctaaaaag gctctaatgt atatttaact     840
   aaaattacta gctttgggaa ttaaactgtt taacaaataa tgctgctcat tgtgattctt     900
   acctataagc agcctaattt gaattatttg ctgcaatcga attcccgcgg ccgccatggc     960
   ggccgggagc atgcgacgtc gggcccaatt cgccctatag tgagtcgtat tacaattcac    1020
   tggccgtcgt tttacaacgt cgtgactggg aaaaccctgg cgttacccaa cttaatcgcc    1080
   ttgcagcaca tccccctttc gccagctggc gtaatagcga agaggcccgc accgatcgcc    1140
   cttcccaaca gttgcgcagc ctgaatggcg aatggacgcg ccctgtagcg gcgcattaag    1200
   cgcggcgggt gtggtggtta cgcgcagcgt gaccgctaca cttgccagcg ccctagcgcc    1260
   cgctcctttc gctttcttcc cttcctttct cgccacgttc gccggctttc cccgtcaagc    1320
   tctaaatcgg gggctccctt tagggttccg atttagtgct ttacggcacc tcgaccccaa    1380
   aaaacttgat tagggtgatg gttcacgtag tgggccatcg ccctgataga cggtttttcg    1440
   ccctttgacg ttggagtcca cgttctttaa tagtggactc ttgttccaaa ctggaacaac 1500
   actcaaccct atctcggtct attcttttga tttataaggg attttgccga tttcggccta 1560
   ttggttaaaa aatgagctga tttaacaaaa atttaacgcg aattttaaca aaatattaac 1620
   gcttacaatt tcctgatgcg gtattttctc cttacgcatc tgtgcggtat ttcacaccgc 1680
   atcaggtggc acttttcggg gaaatgtgcg cggaacccct atttgtttat ttttctaaat 1740
   acattcaaat atgtatccgc tcatgagaca ataaccctga taaatgcttc aataatattg 1800
   aaaaaggaag agtatgagta ttcaacattt ccgtgtcgcc cttattccct tttttgcggc 1860
   attttgcctt cctgtttttg ctcacccaga aacgctggtg aaagtaaaag atgctgaaga 1920
   tcagttgggt gcacgagtgg gttacatcga actggatctc aacagcggta agatccttga 1980
   gagttttcgc cccgaagaac gttttccaat gatgagcact tttaaagttc tgctatgtgg 2040
   cgcggtatta tcccgtattg acgccgggca agagcaactc ggtcgccgca tacactattc 2100
   tcagaatgac ttggttgagt actcaccagt cacagaaaag catcttacgg atggcatgac 2160
   agtaagagaa ttatgcagtg ctgccataac catgagtgat aacactgcgg ccaacttact 2220
   tctgacaacg atcggaggac cgaaggagct aaccgctttt ttgcacaaca tgggggatca 2280
   tgtaactcgc cttgatcgtt gggaaccgga gctgaatgaa gccataccaa acgacgagcg 2340
   tgacaccacg atgcctgtag caatggcaac aacgttgcgc aaactattaa ctggcgaact 2400
   acttactcta gcttcccggc aacaattaat agactggatg gaggcggata aagttgcagg 2460
   accacttctg cgctcggccc ttccggctgg ctggtttatt gctgataaat ctggagccgg 2520
   tgagcgtggg tctcgcggta tcattgcagc actggggcca gatggtaagc cctcccgtat 2580
   cgtagttatc tacacgacgg ggagtcaggc aactatggat gaacgaaata gacagatcgc 2640
   tgagataggt gcctcactga ttaagcattg gtaactgtca gaccaagttt actcatatat 2700
   actttagatt gatttaaaac ttcattttta atttaaaagg atctaggtga agatcctttt 2760
   tgataatctc atgaccaaaa tcccttaacg tgagttttcg ttccactgag cgtcagaccc 2820
   cgtagaaaag atcaaaggat cttcttgaga tccttttttt ctgcgcgtaa tctgctgctt 2880
   gcaaacaaaa aaaccaccgc taccagcggt ggtttgtttg ccggatcaag agctaccaac 2940
   tctttttccg aaggtaactg gcttcagcag agcgcagata ccaaatactg ttcttctagt 3000
   gtagccgtag ttaggccacc acttcaagaa ctctgtagca ccgcctacat acctcgctct 3060
   gctaatcctg ttaccagtgg ctgctgccag tggcgataag tcgtgtctta ccgggttgga 3120
   ctcaagacga tagttaccgg ataaggcgca gcggtcgggc tgaacggggg gttcgtgcac 3180
   acagcccagc ttggagcgaa cgacctacac cgaactgaga tacctacagc gtgagctatg 3240
   agaaagcgcc acgcttcccg aagggagaaa ggcggacagg tatccggtaa gcggcagggt 3300
   cggaacagga gagcgcacga gggagcttcc agggggaaac gcctggtatc tttatagtcc 3360
   tgtcgggttt cgccacctct gacttgagcg tcgatttttg tgatgctcgt caggggggcg 3420
   gagcctatgg aaaaacgcca gcaacgcggc ctttttacgg ttcctggcct tttgctggcc 3480
   ttttgctcac atgttctttc ctgcgttatc ccctgattct gtggataacc gtattaccgc 3540
   ctttgagtga gctgataccg ctcgccgcag ccgaacgacc gagcgcagcg agtcagtgag 3600
   cgaggaagcg gaagagcgcc caatacgcaa accgcctctc cccgcgcgtt ggccgattca 3660
   ttaatgcagc tggcacgaca ggtttcccga ctggaaagcg ggcagtgagc gcaacgcaat 3720
   taatgtgagt tagctcactc attaggcacc ccaggcttta cactttatgc ttccggctcg 3780
   tatgttgtgt ggaattgtga gcggataaca atttcacaca ggaaacagct atgaccatga 3840
   ttacgccaag ctatttaggt gacactatag aatactcaag ctatgcatcc aacgcgttgg 3900
   gagctctccc atatggtcga cctgcaggcg gccgcgaatt cactagtgat tgttacttcc 3960
   cttcgagcct caatgtcctc atttgtaaaa tgacattaat acctactttt agctgtggga 4020
   attgagtacc atgatttata caaagcagtt tgtatggtgc tggttacatg agagttcaga 4080
   tggtaactag ttagtaaaaa atctctagtg tgcttgttga ttttatttta ttttagtatt 4140
   tcttaaagat caaatttaac atcaatccta aactttattt agctttttct ggcgtgtaaa 4200
   ctaacatact aagttgtgtg actataattc atttagtgac tcatttttag ctatttttat 4260
   aacacattgt gctatggggg gttttggaac ttgctggaag ctacatcaga aactgccata 4320
   gttaattgcc atttcaagaa tgttgtaaat aactcaggtg gccgtttaat tctcaatgta 4380
   aatataatta actagacatc tttcctatat ttgtgtctca gttttaaagc tatttctgga 4440
   tgcttgagtc ttaccgtaat tgataacaaa aagaggttat tgagaatatc tatgatttac 4500
   agagtaagtt attctagacc tcaagagtga aatgtagggg aggagacatt tgtgtgttaa 4560
   actaatggaa atgctcattt aatagatatt cactgaaagt attagttttg gtttattgct 4620
   agaaaagttg aggttttatg gagatttttg taaaaaatgg tttatttcct aaataaatat 4680
   ctctttttct tttttctccc agaaaatgtt aatctatgat ccagccaaac gaatttctgg 4740
   caaaatggca ctgaatcatc catattttaa tgatttggac aatcagatta agaagatggg 4800
   atccggttct ggcgtgaaac agactttgaa ttttgacctt ctcaagttgg cgggagacgt 4860
   ggagtccaac ccagggccca tggccagcta cccctgtcac cagcacgcca gcgccttcga 4920
   ccaggccgcc agaagcaggg gccacagcaa ccggcggacc gccctgagac ccaggcggca 4980
   gcaggaagcc accgaagtcc ggctggaaca gaagatgccc accctgttaa gggtgtacat 5040
   cgacggcccc cacggcatgg gcaagaccac caccacccag ctgctggtgg ccctgggcag 5100
   ccgggacgac atcgtgtacg tgcccgagcc catgacctac tggcaggtgc tgggcgccag 5160
   cgagaccatc gccaacatct acaccacaca gcacaggctg gaccagggcg agatctctgc 5220
   cggcgacgcc gccgtggtga tgaccagcgc ccagatcaca atgggcatgc cctacgccgt 5280
   gaccgacgcc gtgctggccc ctcacgtggg cggcgaggcc ggctctagcc acgcccctcc 5340
   ccctgccctg accctgatct tcgaccggca ccccatcgcc cacctgctgt gctaccctgc 5400
   cgccagatac ctgatgggca gcatgacccc ccaggccgtg ctggccttcg tggccctgat 5460
   cccccccacc ctgcccggca ccaacatcgt gctgggagcc ctgcccgagg accggcacat 5520
   cgaccggctg gccaagcggc agagacccgg cgagcggctg gacctggcca tgctggccgc 5580
   catccggcgg gtgtacggcc tgctggccaa caccgtgaga tacctgcagg gcggagggtc 5640
   ttggtgggag gactggggcc agctgtccgg caccgccgtg ccacctcagg gcgccgagcc 5700
   ccagagcaat gccggccctc ggccccacat cggcgacacc ctgtttaccc tgttcagagc 5760
   ccccgagctg ctggccccca acggcgacct gtacaacgtg ttcgcctggg ccctggacgt 5820
   gctggccaag aggctgcggc ccatgcacgt gttcatcctg gactacgacc agagccctgc 5880
   cggctgcagg gacgccctgc tgcagctgac cagcggcatg gtgcagaccc acgtgaccac 5940
   ccccggcagc atccccacca tctgcgacct ggcccggacc ttcgcccggg agatgggcga 6000
   ggccaacggc tccggagagg gcagaggaag tctgctaaca tgcggtgacg tcgaggagaa 6060
   tcctggccca ctcgaggtga 6080
<210> 63
   <211> 58
   <212> DNA
   <213> Artificial Sequence
<220>
   <223> oligonucleotide
<400> 63
   taccgttcgt atagcataca ttatacgaag ttataccatg tctagactgg acaagagc 58
<210> 64
   <211> 31
   <212> DNA
   <213> Artificial Sequence
<220>
   <223> oligonucleotide
<400> 64
   gcagagggaa aaagatctgc gactctagag g 31
<210> 65
   <211> 23
   <212> DNA
   <213> Artificial Sequence
<220>
   <223> oligonucleotide
<400> 65
   acgagtgtac acacacgagg tag 23
<210> 66
   <211> 22
   <212> DNA
   <213> Artificial Sequence
<220>
   <223> oligonucleotide
<400> 66
   tgtcaaccgg agtggagtaa cg 22
<210> 67
   <211> 19
   <212> DNA
   <213> Artificial Sequence
<220>
   <223> oligonucleotide
<400> 67
   atggagggga atacagccc 19
<210> 68
   <211> 20
   <212> DNA
   <213> Artificial Sequence
<220>
   <223> oligonucleotide
<400> 68
   ttctttgcag ctccttcgtt 20
<210> 69
   <211> 982
   <212> DNA
   <213> Artificial Sequence
<220>
   <223> synthetic DNA
<400> 69
   aagatacatc tctgcttctt tctggattga tttttctttg aagatgaatg tgagaaatag 60
   aacttaaagg tctattctga gtgtgcttta taaatgattt tatcatcagt gatgggtgtt 120
   agaaaaatct aggtaattat gcctggagtt ctgacgtttc attggcaaac ttcagaggaa 180
   gtcttgaact tctggttgtc agcttttttt tttaacccat tcattgtgat tcaagtatac 240
   cgtttttctg cttagtcctt gacattgttt aggttataat tagaatttta tctgtggaat 300
   tgcacttttc attcttttgt tttacggtgc ttaagtgata tttcctactc ttggaaaaag 360
   taccttgaaa gtcttgagca tgtttgtctg tgacctcagc aagcagagga aaaagattaa 420
   aattgtcacg tgggcaactg taaacactta aaatgttttt gtcgttttaa acaaaatttt 480
   cctaatgaac agtctgaaca cattttagtt ctgtcctcat aactttaaaa aataatgttt 540
   tgaataaaat taggagtatc ataaagcggt ataaatttgt tgaaatattt gggtataact 600
   ttggcacacg caggtactac tttaaagtgt cacaaaaagg gaacataatt ctggtttttg 660
   tttggaaaga atagacaagt tgatacctga atttgagggt tattttgatt ggaagaagct 720
   aaatactaca ctagtataga atatacacgt ttccaatgtc tcaggagtca agaaggatgg     780
   aaagttattg taggagtcct tacccttgta aatattgttt gaatgtaatt aatatttatg     840
   gaatacttat gctgcagtag gcactgtgga aaatgcttta catatagtgt ttcattaatg 900
   cttaaaacta ctcgagttag tggcatgtaa ttaagtcagt ttttccttca ctttaggatc 960
   taccataccc attgactaac tc 982
<210> 70
   <211> 41
   <212> DNA
   <213> Artificial Sequence
<220>
   <223> oligonucleotide
<400> 70
   aaatcctgca ggaagataca tctctgcttc tttctggatt g 41
<210> 71
   <211> 45
   <212> DNA
   <213> Artificial Sequence
<220>
   <223> oligonucleotide
<400> 71
   aaatcctgca ggagttagtc aatgggtatg gtagatccta aagtg 45
<210> 72
   <211> 943
   <212> DNA
   <213> Artificial Sequence
<220>
   <223> synthetic DNA
<400> 72
   aagattatac caaaatagag aaaattggag aaggtgagtg gttttagtaa aataaatttt      60
   atggaatgat ttaacaatgc tacaacttct gtaatatgaa cacatttaaa tatttattaa     120
   aattcaacca ttaagttgtc ttgtagtacc agtgtgcatt aacatatgtt ctttacttaa     180
   taaacgcaaa tttgctcaca tttcaagaga attgaaatta ccgaatccct aatgaccgtt     240
   cgcctcaccc ccatagtctt acagtcttct gatactaaac acttatagta gtacttgtat     300
   tttgaagaca ctattgataa cttgcatgtg tgttgagggg cagaggttag ggctgcagaa     360
   ttatgctaac ttcataatat atttttagtt ttagtctctt tcaattgttt ttaggatgat     420
   aaatattttt atccagactt tatagactgg ttacaataat tgacttaatc taatttaaat     480
   aaaatttcta tttagttacc ctaagtataa atgacctgtc atatagacat gtttaagcta     540
   ttctaaaatt ataacttatg tatataatcc ttccctttct gtggtacttg aaggaatgtt     600
   tagatctata tatggcacaa atgaggggta tatcccttcg cctgttcctc gactccagca     660
   gaacattctc gattctccag gtacaaaata atttgctaat agaatccagt ttatataggt     720
   tctgccagaa gaaaattccc aactatattt ttaatgtgat caaagacccc acttgacaag     780
   aaatagtgaa aaacaagggg gaatactgga gggaatttag tggaatagga gtgacaagtt     840
   agtgtggtgt tcgattggag gtgtgtgtgt gtatacatat atatatatat ttttttttta     900
   aatcacttaa atacctcata cccattctac taaacaaatt ttg                      943
<210> 73
   <211> 38
   <212> DNA
   <213> Artificial Sequence
<220>
   <223> oligonucleotide
<400> 73
   ttaaccatgg aagattatac caaaatagag aaaattgg 38
<210> 74
   <211> 40
   <212> DNA
   <213> Artificial Sequence
<220>
   <223> oligonucleotide
<400> 74
   tttagcatgc caaaatttgt ttagtagaat gggtatgagg 40
<210> 75
   <211> 8604
   <212> DNA
   <213> Artificial Sequence
<220>
   <223> synthetic DNA
<400> 75
   tatagtgagt cgtattacaa ttcactggcc gtcgttttac aacgtcgtga ctgggaaaac 60
   cctggcgtta cccaacttaa tcgccttgca gcacatcccc ctttcgccag ctggcgtaat 120
   agcgaagagg cccgcaccga tcgcccttcc caacagttgc gcagcctgaa tggcgaatgg 180
   acgcgccctg tagcggcgca ttaagcgcgg cgggtgtggt ggttacgcgc agcgtgaccg 240
   ctacacttgc cagcgcccta gcgcccgctc ctttcgcttt cttcccttcc tttctcgcca 300
   cgttcgccgg ctttccccgt caagctctaa atcgggggct ccctttaggg ttccgattta 360
   gtgctttacg gcacctcgac cccaaaaaac ttgattaggg tgatggttca cgtagtgggc 420
   catcgccctg atagacggtt tttcgccctt tgacgttgga gtccacgttc tttaatagtg 480
   gactcttgtt ccaaactgga acaacactca accctatctc ggtctattct tttgatttat 540
   aagggatttt gccgatttcg gcctattggt taaaaaatga gctgatttaa caaaaattta 600
   acgcgaattt taacaaaata ttaacgctta caatttcctg atgcggtatt ttctccttac 660
   gcatctgtgc ggtatttcac accgcatcag gtggcacttt tcggggaaat gtgcgcggaa 720
   cccctatttg tttatttttc taaatacatt caaatatgta tccgctcatg agacaataac 780
   cctgataaat gcttcaataa tattgaaaaa ggaagagtat gagtattcaa catttccgtg 840
   tcgcccttat tccctttttt gcggcatttt gccttcctgt ttttgctcac ccagaaacgc 900
   tggtgaaagt aaaagatgct gaagatcagt tgggtgcacg agtgggttac atcgaactgg 960
   atctcaacag cggtaagatc cttgagagtt ttcgccccga agaacgtttt ccaatgatga 1020
   gcacttttaa agttctgcta tgtggcgcgg tattatcccg tattgacgcc gggcaagagc 1080
   aactcggtcg ccgcatacac tattctcaga atgacttggt tgagtactca ccagtcacag 1140
   aaaagcatct tacggatggc atgacagtaa gagaattatg cagtgctgcc ataaccatga 1200
   gtgataacac tgcggccaac ttacttctga caacgatcgg aggaccgaag gagctaaccg 1260
   cttttttgca caacatgggg gatcatgtaa ctcgccttga tcgttgggaa ccggagctga 1320
   atgaagccat accaaacgac gagcgtgaca ccacgatgcc tgtagcaatg gcaacaacgt 1380
   tgcgcaaact attaactggc gaactactta ctctagcttc ccggcaacaa ttaatagact 1440
   ggatggaggc ggataaagtt gcaggaccac ttctgcgctc ggcccttccg gctggctggt 1500
   ttattgctga taaatctgga gccggtgagc gtgggtctcg cggtatcatt gcagcactgg 1560
   ggccagatgg taagccctcc cgtatcgtag ttatctacac gacggggagt caggcaacta 1620
   tggatgaacg aaatagacag atcgctgaga taggtgcctc actgattaag cattggtaac 1680
   tgtcagacca agtttactca tatatacttt agattgattt aaaacttcat ttttaattta 1740
   aaaggatcta ggtgaagatc ctttttgata atctcatgac caaaatccct taacgtgagt 1800
   tttcgttcca ctgagcgtca gaccccgtag aaaagatcaa aggatcttct tgagatcctt 1860
   tttttctgcg cgtaatctgc tgcttgcaaa caaaaaaacc accgctacca gcggtggttt 1920
   gtttgccgga tcaagagcta ccaactcttt ttccgaaggt aactggcttc agcagagcgc 1980
   agataccaaa tactgttctt ctagtgtagc cgtagttagg ccaccacttc aagaactctg 2040
   tagcaccgcc tacatacctc gctctgctaa tcctgttacc agtggctgct gccagtggcg 2100
   ataagtcgtg tcttaccggg ttggactcaa gacgatagtt accggataag gcgcagcggt 2160
   cgggctgaac ggggggttcg tgcacacagc ccagcttgga gcgaacgacc tacaccgaac 2220
   tgagatacct acagcgtgag ctatgagaaa gcgccacgct tcccgaaggg agaaaggcgg 2280
   acaggtatcc ggtaagcggc agggtcggaa caggagagcg cacgagggag cttccagggg 2340
   gaaacgcctg gtatctttat agtcctgtcg ggtttcgcca cctctgactt gagcgtcgat 2400
   ttttgtgatg ctcgtcaggg gggcggagcc tatggaaaaa cgccagcaac gcggcctttt 2460
   tacggttcct ggccttttgc tggccttttg ctcacatgtt ctttcctgcg ttatcccctg 2520
   attctgtgga taaccgtatt accgcctttg agtgagctga taccgctcgc cgcagccgaa 2580
   cgaccgagcg cagcgagtca gtgagcgagg aagcggaaga gcgcccaata cgcaaaccgc 2640
   ctctccccgc gcgttggccg attcattaat gcagctggca cgacaggttt cccgactgga 2700
   aagcgggcag tgagcgcaac gcaattaatg tgagttagct cactcattag gcaccccagg 2760
   ctttacactt tatgcttccg gctcgtatgt tgtgtggaat tgtgagcgga taacaatttc 2820
   acacaggaaa cagctatgac catgattacg ccaagctatt taggtgacac tatagaatac 2880
   tcaagctatg catccaacgc gttgggagct ctcccatatg gtcgacctgc aggaagatac 2940
   atctctgctt ctttctggat tgatttttct ttgaagatga atgtgagaaa tagaacttaa 3000
   aggtctattc tgagtgtgct ttataaatga ttttatcatc agtgatgggt gttagaaaaa 3060
   tctaggtaat tatgcctgga gttctgacgt ttcattggca aacttcagag gaagtcttga 3120
   acttctggtt gtcagctttt ttttttaacc cattcattgt gattcaagta taccgttttt 3180
   ctgcttagtc cttgacattg tttaggttat aattagaatt ttatctgtgg aattgcactt 3240
   ttcattcttt tgttttacgg tgcttaagtg atatttccta ctcttggaaa aagtaccttg 3300
   aaagtcttga gcatgtttgt ctgtgacctc agcaagcaga ggaaaaagat taaaattgtc 3360
   acgtgggcaa ctgtaaacac ttaaaatgtt tttgtcgttt taaacaaaat tttcctaatg 3420
   aacagtctga acacatttta gttctgtcct cataacttta aaaaataatg ttttgaataa 3480
   aattaggagt atcataaagc ggtataaatt tgttgaaata tttgggtata actttggcac 3540
   acgcaggtac tactttaaag tgtcacaaaa agggaacata attctggttt ttgtttggaa 3600
   agaatagaca agttgatacc tgaatttgag ggttattttg attggaagaa gctaaatact 3660
   acactagtat agaatataca cgtttccaat gtctcaggag tcaagaagga tggaaagtta 3720
   ttgtaggagt ccttaccctt gtaaatattg tttgaatgta attaatattt atggaatact 3780
   tatgctgcag taggcactgt ggaaaatgct ttacatatag tgtttcatta atgcttaaaa 3840
   ctactcgagt tagtggcatg taattaagtc agtttttcct tcactttagg atctaccata 3900
   cccattgact aactcctgca ggcggccgcg aattcactag tgattaccgt tcgtatagca 3960
   tacattatac gaagttatac catgtctaga ctggacaaga gcaaagtcat aaacggcgct 4020
   ctggaattac tcaatggagt cggtatcgaa ggcctgacga caaggaaact cgctcaaaag 4080
   ctgggagttg agcagcctac cctgtactgg cacgtgaaga acaagcgggc cctgctcgat 4140
   gccctgccaa tcgagatgct ggacaggcat catacccact tctgccccct ggaaggcgag 4200
   tcatggcaag actttctgcg gaacaacgcc aagtcattcc gctgtgctct cctctcacat 4260
   cgcgacgggg ctaaagtgca tctcggcacc cgcccaacag agaaacagta cgaaaccctg 4320
   gaaaatcagc tcgcgttcct gtgtcagcaa ggcttctccc tggagaacgc actgtacgct 4380
   ctgtccgccg tgggccactt tacactgggc tgcgtattgg aggaacagga gcatcaagta 4440
   gcaaaagagg aaagagagac acctaccacc gattctatgc ccccacttct gagacaagca 4500
   attgagctgt tcgaccggca gggagccgaa cctgccttcc ttttcggcct ggaactaatc 4560
   atatgtggcc tggagaaaca gctaaagtgc gaaagcggcg ggccggccga cgcccttgac 4620
   gattttgact tagacatgct cccagccgat gcccttgacg actttgacct tgatatgctg 4680
   cctgctgacg ctcttgacga ttttgacctt gacatgctcc ccgggtaact aagtaaggat 4740
   cccaaatacc actgaattaa gaattccgcc cctctccctc ccccccccct aacgttactg 4800
   gccgaagccg cttggaataa ggccggtgtg cgtttgtcta tatgttattt tccaccatat 4860
   tgccgtcttt tggcaatgtg agggcccgga aacctggccc tgtcttcttg acgagcattc 4920
   ctaggggtct ttcccctctc gccaaaggaa tgcaaggtct gttgaatgtc gtgaaggaag 4980
   cagttcctct ggaagcttct tgaagacaaa caacgtctgt agcgaccctt tgcaggcagc 5040
   ggaacccccc acctggcgac aggtgcctct gcggccaaaa gccacgtgta taagatacac 5100
   ctgcaaaggc ggcacaaccc cagtgccacg ttgtgagttg gatagttgtg gaaagagtca 5160
   aatggctctc ctcaagcgta ttcaacaagg ggctgaagga tgcccagaag gtaccccatt 5220
   gtatgggatc tgatctgggg cctcggtgca catgctttac atgtgtttag tcgaggttaa 5280
   aaaacgtcta ggccccccga accacgggga cgtggttttc ctttgaaaaa cacgatgata 5340
   atatggccac aaccatgacc gagtacaagc ccacggtgcg cctcgccacc cgcgacgacg 5400
   tcccccgggc cgtacgcacc ctcgccgccg cgttcgccga ctaccccgcc acgcgccaca 5460
   ccgtcgaccc ggaccgccac atcgagcggg tcaccgagct gcaagaactc ttcctcacgc 5520
   gcgtcgggct cgacatcggc aaggtgtggg tcgcggacga cggcgccgcg gtggcggtct 5580
   ggaccacgcc ggagagcgtc gaagcggggg cggtgttcgc cgagatcggc ccgcgcatgg 5640
   ccgagttgag cggttcccgg ctggccgcgc agcaacagat ggaaggcctc ctggcgccgc 5700
   accggcccaa ggagcccgcg tggttcctgg ccaccgtcgg cgtctcgccc gaccaccagg 5760
   gcaagggtct gggcagcgcc gtcgtgctcc ccggagtgga ggcggccgag cgcgccgggg 5820
   tgcccgcctt cctggagacc tccgcgcccc gcaacctccc cttctacgag cggctcggct 5880
   tcaccgtcac cgccgacgtc gaggtgcccg aaggaccgcg cacctggtgc atgacccgca 5940
   agcccggtgc ctgaggatcc agacatgata agatacattg atgagtttgg acaaaccaca 6000
   actagaatgc agtgaaaaaa atgctttatt tgtgaaattt gtgatgctat tgctttattt 6060
   gtaaccatta taagctgcaa taaacaagtt aacaacaaca attgcattca ttttatgttt 6120
   caggttcagg gggaggtgtg ggaggttttt taaagcaagt aaaacctcta caaatgtggt 6180
   atggctgatt atgatcctct agagtcgcag atccagacat gataagatac attgatgagt 6240
   ttggacaaac cacaactaga atgcagtgaa aaaaatgctt tatttgtgaa atttgtgatg 6300
   ctattgcttt atttgtaacc attataagct gcaataaaca agttaacaac aacaattgca 6360
   ttcattttat gtttcaggtt cagggggagg tgtgggaggt tttttaaagc aagtaaaacc 6420
   tctacaaatg tggtatggct gattatgatc ctctagagtc gcagatccag acatgataag 6480
   atacattgat gagtttggac aaaccacaac tagaatgcag tgaaaaaaat gctttatttg 6540
   tgaaatttgt gatgctattg ctttatttgt aaccattata agctgcaata aacaagttaa 6600
   caacaacaat tgcattcatt ttatgtttca ggttcagggg gaggtgtggg aggtttttta 6660
   aagcaagtaa aacctctaca aatgtggtat ggctgattat gatcctctag agtcgcagat 6720
   cctctagagt cgcagatctt tttccctctg ccaaaaatta tggggacatc atgaagcccc 6780
   ttgagcatct gacttctggc taataaagga aatttatttt cattgcaata gtgtgttgga 6840
   attttttgtg tctctcactc ggaaggacat atgggagggc aaatcattta aaacatcaga 6900
   atgagtattt ggtttagagt ttggcaacat atgccatatg ctggctgcca tgaacaaagg 6960
   tggctataaa gaggtcatca gtatatgaaa cagccccctg ctgtccattc cttattccat 7020
   agaaaagcct tgacttgagg ttagattttt tttatatttt gttttgtgtt atttttttct 7080
   ttaacatccc taaaattttc cttacatgtt ttactagcca gatttttcct cctctcctga 7140
   ctactcccag tcatagctgt ccctcttctc ttatgaagat ccctcgacct gcagcccaag 7200
   cttatcgaat tcccgcggtg gcggccgcac gtctccctat cagtgataga gaagtcgaca 7260
   cgtctcgagc tccctatcag tgatagagaa ggtacgtcta gaacgtctcc ctatcagtga 7320
   tagagaagtc gacacgtctc gagctcccta tcagtgatag agaaggtacg tctagaacgt 7380
   ctccctatca gtgatagaga agtcgacacg tctcgagctc cctatcagtg atagagaagg 7440
   tacgtctaga acgtctccct atcagtgata gagaagtcga cacgtctcga gctccctatc 7500
   agtgatagag aaggtacccc ctatataagc agagctcgtt tagtgaaccg tcagatcgcc 7560
   tggagacgcc atccacgctg ttttgacctc catagaagac accgggaccg atccagcctc 7620
   cgcggccgcc atggaagatt ataccaaaat agagaaaatt ggagaaggtg agtggtttta 7680
   gtaaaataaa ttttatggaa tgatttaaca atgctacaac ttctgtaata tgaacacatt 7740
   taaatattta ttaaaattca accattaagt tgtcttgtag taccagtgtg cattaacata 7800
   tgttctttac ttaataaacg caaatttgct cacatttcaa gagaattgaa attaccgaat 7860
   ccctaatgac cgttcgcctc acccccatag tcttacagtc ttctgatact aaacacttat 7920
   agtagtactt gtattttgaa gacactattg ataacttgca tgtgtgttga ggggcagagg 7980
   ttagggctgc agaattatgc taacttcata atatattttt agttttagtc tctttcaatt 8040
   gtttttagga tgataaatat ttttatccag actttataga ctggttacaa taattgactt 8100
   aatctaattt aaataaaatt tctatttagt taccctaagt ataaatgacc tgtcatatag 8160
   acatgtttaa gctattctaa aattataact tatgtatata atccttccct ttctgtggta 8220
   cttgaaggaa tgtttagatc tatatatggc acaaatgagg ggtatatccc ttcgcctgtt 8280
   cctcgactcc agcagaacat tctcgattct ccaggtacaa aataatttgc taatagaatc 8340
   cagtttatat aggttctgcc agaagaaaat tcccaactat atttttaatg tgatcaaaga 8400
   ccccacttga caagaaatag tgaaaaacaa gggggaatac tggagggaat ttagtggaat 8460
   aggagtgaca agttagtgtg gtgttcgatt ggaggtgtgt gtgtgtatac atatatatat 8520
   atattttttt tttaaatcac ttaaatacct catacccatt ctactaaaca aattttggca 8580
   tgcgacgtcg ggcccaattc gccc 8604
<210> 76
   <211> 24
   <212> DNA
   <213> Artificial Sequence
<220>
   <223> oligonucleotide
<400> 76
   caccgatacc aaaatagaga aaat 24
<210> 77
   <211> 23
   <212> DNA
   <213> Artificial Sequence
<220>
   <223> oligonucleotide
<400> 77
   aaacattttc tctattttgg tac 23
<210> 78
   <211> 24
   <212> DNA
   <213> Artificial Sequence
<220>
   <223> oligonucleotide
<400> 78
   caccgcatag ttagtcaatg ggta 24
<210> 79
   <211> 24
   <212> DNA
   <213> Artificial Sequence
<220>
   <223> oligonucleotide
<400> 79
   aaactaccca ttgactaact atgc 24
<210> 80
   <211> 8436
   <212> DNA
   <213> Artificial Sequence
<220>
   <223> synthetic DNA
<400> 80
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg ataccaaaat agagaaaatg ttttagagct agaaatagca agttaaaata 300
   aggctagtcc gttatcaact tgaaaaagtg gcaccgagtc ggtgcttttt tgttttagag 360
   ctagaaatag caagttaaaa taaggctagt ccgtttttag cgcgtgcgcc aattctgcag 420
   acaaatggct ctagaggtac ccgttacata acttacggta aatggcccgc ctggctgacc 480
   gcccaacgac ccccgcccat tgacgtcaat agtaacgcca atagggactt tccattgacg 540
   tcaatgggtg gagtatttac ggtaaactgc ccacttggca gtacatcaag tgtatcatat 600
   gccaagtacg ccccctattg acgtcaatga cggtaaatgg cccgcctggc attgtgccca 660
   gtacatgacc ttatgggact ttcctacttg gcagtacatc tacgtattag tcatcgctat 720
   taccatggtc gaggtgagcc ccacgttctg cttcactctc cccatctccc ccccctcccc 780
   acccccaatt ttgtatttat ttatttttta attattttgt gcagcgatgg gggcgggggg 840
   gggggggggg cgcgcgccag gcggggcggg gcggggcgag gggcggggcg gggcgaggcg 900
   gagaggtgcg gcggcagcca atcagagcgg cgcgctccga aagtttcctt ttatggcgag 960
   gcggcggcgg cggcggccct ataaaaagcg aagcgcgcgg cgggcgggag tcgctgcgac 1020
   gctgccttcg ccccgtgccc cgctccgccg ccgcctcgcg ccgcccgccc cggctctgac 1080
   tgaccgcgtt actcccacag gtgagcgggc gggacggccc ttctcctccg ggctgtaatt 1140
   agctgagcaa gaggtaaggg tttaagggat ggttggttgg tggggtatta atgtttaatt 1200
   acctggagca cctgcctgaa atcacttttt ttcaggttgg accggtgcca ccatgtaccc 1260
   atacgatgtt ccagattacg cttcgccgaa gaaaaagcgc aaggtcgaag cgtccgacaa 1320
   gaagtacagc atcggcctgg ccatcggcac caactctgtg ggctgggccg tgatcaccga 1380
   cgagtacaag gtgcccagca agaaattcaa ggtgctgggc aacaccgacc ggcacagcat 1440
   caagaagaac ctgatcggag ccctgctgtt cgacagcggc gaaacagccg aggccacccg 1500
   gctgaagaga accgccagaa gaagatacac cagacggaag aaccggatct gctatctgca 1560
   agagatcttc agcaacgaga tggccaaggt ggacgacagc ttcttccaca gactggaaga 1620
   gtccttcctg gtggaagagg ataagaagca cgagcggcac cccatcttcg gcaacatcgt 1680
   ggacgaggtg gcctaccacg agaagtaccc caccatctac cacctgagaa agaaactggt 1740
   ggacagcacc gacaaggccg acctgcggct gatctatctg gccctggccc acatgatcaa 1800
   gttccggggc cacttcctga tcgagggcga cctgaacccc gacaacagcg acgtggacaa 1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta 7440
   ttgctgataa atctggagcc ggtgagcgtg gaagccgcgg tatcattgca gcactggggc 7500
   cagatggtaa gccctcccgt atcgtagtta tctacacgac ggggagtcag gcaactatgg 7560
   atgaacgaaa tagacagatc gctgagatag gtgcctcact gattaagcat tggtaactgt 7620
   cagaccaagt ttactcatat atactttaga ttgatttaaa acttcatttt taatttaaaa 7680
   ggatctaggt gaagatcctt tttgataatc tcatgaccaa aatcccttaa cgtgagtttt 7740
   cgttccactg agcgtcagac cccgtagaaa agatcaaagg atcttcttga gatccttttt 7800
   ttctgcgcgt aatctgctgc ttgcaaacaa aaaaaccacc gctaccagcg gtggtttgtt 7860
   tgccggatca agagctacca actctttttc cgaaggtaac tggcttcagc agagcgcaga 7920
   taccaaatac tgtccttcta gtgtagccgt agttaggcca ccacttcaag aactctgtag 7980
   caccgcctac atacctcgct ctgctaatcc tgttaccagt ggctgctgcc agtggcgata 8040
   agtcgtgtct taccgggttg gactcaagac gatagttacc ggataaggcg cagcggtcgg 8100
   gctgaacggg gggttcgtgc acacagccca gcttggagcg aacgacctac accgaactga 8160
   gatacctaca gcgtgagcta tgagaaagcg ccacgcttcc cgaagggaga aaggcggaca 8220
   ggtatccggt aagcggcagg gtcggaacag gagagcgcac gagggagctt ccagggggaa 8280
   acgcctggta tctttatagt cctgtcgggt ttcgccacct ctgacttgag cgtcgatttt 8340
   tgtgatgctc gtcagggggg cggagcctat ggaaaaacgc cagcaacgcg gcctttttac 8400
   ggttcctggc cttttgctgg ccttttgctc acatgt 8436
<210> 81
   <211> 8440
   <212> DNA
   <213> Artificial Sequence
<220>
   <223> synthetic DNA
<400> 81
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccc accgcatagt tagtcaatgg gtagttttag agctagaaat agcaagttaa 300
   aataaggcta gtccgttatc aacttgaaaa agtggcaccg agtcggtgct tttttgtttt 360
   agagctagaa atagcaagtt aaaataaggc tagtccgttt ttagcgcgtg cgccaattct 420
   gcagacaaat ggctctagag gtacccgtta cataacttac ggtaaatggc ccgcctggct 480
   gaccgcccaa cgacccccgc ccattgacgt caatagtaac gccaataggg actttccatt 540
   gacgtcaatg ggtggagtat ttacggtaaa ctgcccactt ggcagtacat caagtgtatc 600
   atatgccaag tacgccccct attgacgtca atgacggtaa atggcccgcc tggcattgtg 660
   cccagtacat gaccttatgg gactttccta cttggcagta catctacgta ttagtcatcg 720
   ctattaccat ggtcgaggtg agccccacgt tctgcttcac tctccccatc tcccccccct 780
   ccccaccccc aattttgtat ttatttattt tttaattatt ttgtgcagcg atgggggcgg 840
   gggggggggg ggggcgcgcg ccaggcgggg cggggcgggg cgaggggcgg ggcggggcga 900
   ggcggagagg tgcggcggca gccaatcaga gcggcgcgct ccgaaagttt ccttttatgg 960
   cgaggcggcg gcggcggcgg ccctataaaa agcgaagcgc gcggcgggcg ggagtcgctg 1020
   cgacgctgcc ttcgccccgt gccccgctcc gccgccgcct cgcgccgccc gccccggctc 1080
   tgactgaccg cgttactccc acaggtgagc gggcgggacg gcccttctcc tccgggctgt 1140
   aattagctga gcaagaggta agggtttaag ggatggttgg ttggtggggt attaatgttt 1200
   aattacctgg agcacctgcc tgaaatcact ttttttcagg ttggaccggt gccaccatgt 1260
   acccatacga tgttccagat tacgcttcgc cgaagaaaaa gcgcaaggtc gaagcgtccg 1320
   acaagaagta cagcatcggc ctggccatcg gcaccaactc tgtgggctgg gccgtgatca 1380
   ccgacgagta caaggtgccc agcaagaaat tcaaggtgct gggcaacacc gaccggcaca 1440
   gcatcaagaa gaacctgatc ggagccctgc tgttcgacag cggcgaaaca gccgaggcca 1500
   cccggctgaa gagaaccgcc agaagaagat acaccagacg gaagaaccgg atctgctatc 1560
   tgcaagagat cttcagcaac gagatggcca aggtggacga cagcttcttc cacagactgg 1620
   aagagtcctt cctggtggaa gaggataaga agcacgagcg gcaccccatc ttcggcaaca 1680
   tcgtggacga ggtggcctac cacgagaagt accccaccat ctaccacctg agaaagaaac 1740
   tggtggacag caccgacaag gccgacctgc ggctgatcta tctggccctg gcccacatga 1800
   tcaagttccg gggccacttc ctgatcgagg gcgacctgaa ccccgacaac agcgacgtgg 1860
   acaagctgtt catccagctg gtgcagacct acaaccagct gttcgaggaa aaccccatca 1920
   acgccagcgg cgtggacgcc aaggccatcc tgtctgccag actgagcaag agcagacggc 1980
   tggaaaatct gatcgcccag ctgcccggcg agaagaagaa tggcctgttc ggcaacctga 2040
   ttgccctgag cctgggcctg acccccaact tcaagagcaa cttcgacctg gccgaggatg 2100
   ccaaactgca gctgagcaag gacacctacg acgacgacct ggacaacctg ctggcccaga 2160
   tcggcgacca gtacgccgac ctgtttctgg ccgccaagaa cctgtccgac gccatcctgc 2220
   tgagcgacat cctgagagtg aacaccgaga tcaccaaggc ccccctgagc gcctctatga 2280
   tcaagagata cgacgagcac caccaggacc tgaccctgct gaaagctctc gtgcggcagc 2340
   agctgcctga gaagtacaaa gagattttct tcgaccagag caagaacggc tacgccggct 2400
   acattgacgg cggagccagc caggaagagt tctacaagtt catcaagccc atcctggaaa 2460
   agatggacgg caccgaggaa ctgctcgtga agctgaacag agaggacctg ctgcggaagc 2520
   agcggacctt cgacaacggc agcatccccc accagatcca cctgggagag ctgcacgcca 2580
   ttctgcggcg gcaggaagat ttttacccat tcctgaagga caaccgggaa aagatcgaga 2640
   agatcctgac cttccgcatc ccctactacg tgggccctct ggccagggga aacagcagat 2700
   tcgcctggat gaccagaaag agcgaggaaa ccatcacccc ctggaacttc gaggaagtgg 2760
   tggacaaggg cgcttccgcc cagagcttca tcgagcggat gaccaacttc gataagaacc 2820
   tgcccaacga gaaggtgctg cccaagcaca gcctgctgta cgagtacttc accgtgtata 2880
   acgagctgac caaagtgaaa tacgtgaccg agggaatgag aaagcccgcc ttcctgagcg 2940
   gcgagcagaa aaaggccatc gtggacctgc tgttcaagac caaccggaaa gtgaccgtga 3000
   agcagctgaa agaggactac ttcaagaaaa tcgagtgctt cgactccgtg gaaatctccg 3060
   gcgtggaaga tcggttcaac gcctccctgg gcacatacca cgatctgctg aaaattatca 3120
   aggacaagga cttcctggac aatgaggaaa acgaggacat tctggaagat atcgtgctga 3180
   ccctgacact gtttgaggac agagagatga tcgaggaacg gctgaaaacc tatgcccacc 3240
   tgttcgacga caaagtgatg aagcagctga agcggcggag atacaccggc tggggcaggc 3300
   tgagccggaa gctgatcaac ggcatccggg acaagcagtc cggcaagaca atcctggatt 3360
   tcctgaagtc cgacggcttc gccaacagaa acttcatgca gctgatccac gacgacagcc 3420
   tgacctttaa agaggacatc cagaaagccc aggtgtccgg ccagggcgat agcctgcacg 3480
   agcacattgc caatctggcc ggcagccccg ccattaagaa gggcatcctg cagacagtga 3540
   aggtggtgga cgagctcgtg aaagtgatgg gccggcacaa gcccgagaac atcgtgatcg 3600
   aaatggccag agagaaccag accacccaga agggacagaa gaacagccgc gagagaatga 3660
   agcggatcga agagggcatc aaagagctgg gcagccagat cctgaaagaa caccccgtgg 3720
   aaaacaccca gctgcagaac gagaagctgt acctgtacta cctgcagaat gggcgggata 3780
   tgtacgtgga ccaggaactg gacatcaacc ggctgtccga ctacgatgtg gaccatatcg 3840
   tgcctcagag ctttctgaag gacgactcca tcgacaacaa ggtgctgacc agaagcgaca 3900
   agaaccgggg caagagcgac aacgtgccct ccgaagaggt cgtgaagaag atgaagaact 3960
   actggcggca gctgctgaac gccaagctga ttacccagag aaagttcgac aatctgacca 4020
   aggccgagag aggcggcctg agcgaactgg ataaggccgg cttcatcaag agacagctgg 4080
   tggaaacccg gcagatcaca aagcacgtgg cacagatcct ggactcccgg atgaacacta 4140
   agtacgacga gaatgacaag ctgatccggg aagtgaaagt gatcaccctg aagtccaagc 4200
   tggtgtccga tttccggaag gatttccagt tttacaaagt gcgcgagatc aacaactacc 4260
   accacgccca cgacgcctac ctgaacgccg tcgtgggaac cgccctgatc aaaaagtacc 4320
   ctaagctgga aagcgagttc gtgtacggcg actacaaggt gtacgacgtg cggaagatga 4380
   tcgccaagag cgagcaggaa atcggcaagg ctaccgccaa gtacttcttc tacagcaaca 4440
   tcatgaactt tttcaagacc gagattaccc tggccaacgg cgagatccgg aagcggcctc 4500
   tgatcgagac aaacggcgaa accggggaga tcgtgtggga taagggccgg gattttgcca 4560
   ccgtgcggaa agtgctgagc atgccccaag tgaatatcgt gaaaaagacc gaggtgcaga 4620
   caggcggctt cagcaaagag tctatcctgc ccaagaggaa cagcgataag ctgatcgcca 4680
   gaaagaagga ctgggaccct aagaagtacg gcggcttcga cagccccacc gtggcctatt 4740
   ctgtgctggt ggtggccaaa gtggaaaagg gcaagtccaa gaaactgaag agtgtgaaag 4800
   agctgctggg gatcaccatc atggaaagaa gcagcttcga gaagaatccc atcgactttc 4860
   tggaagccaa gggctacaaa gaagtgaaaa aggacctgat catcaagctg cctaagtact 4920
   ccctgttcga gctggaaaac ggccggaaga gaatgctggc ctctgccggc gaactgcaga 4980
   agggaaacga actggccctg ccctccaaat atgtgaactt cctgtacctg gccagccact 5040
   atgagaagct gaagggctcc cccgaggata atgagcagaa acagctgttt gtggaacagc 5100
   acaagcacta cctggacgag atcatcgagc agatcagcga gttctccaag agagtgatcc 5160
   tggccgacgc taatctggac aaagtgctgt ccgcctacaa caagcaccgg gataagccca 5220
   tcagagagca ggccgagaat atcatccacc tgtttaccct gaccaatctg ggagcccctg 5280
   ccgccttcaa gtactttgac accaccatcg accggaagag gtacaccagc accaaagagg 5340
   tgctggacgc caccctgatc caccagagca tcaccggcct gtacgagaca cggatcgacc 5400
   tgtctcagct gggaggcgac agccccaaga agaagagaaa ggtggaggcc agctaagaat 5460
   tcctagagct cgctgatcag cctcgactgt gccttctagt tgccagccat ctgttgtttg 5520
   cccctccccc gtgccttcct tgaccctgga aggtgccact cccactgtcc tttcctaata 5580
   aaatgaggaa attgcatcgc attgtctgag taggtgtcat tctattctgg ggggtggggt 5640
   ggggcaggac agcaaggggg aggattggga agagaatagc aggcatgctg gggagcggcc 5700
   gcaggaaccc ctagtgatgg agttggccac tccctctctg cgcgctcgct cgctcactga 5760
   ggccgggcga ccaaaggtcg cccgacgccc gggctttgcc cgggcggcct cagtgagcga 5820
   gcgagcgcgc agctgcctgc aggggcgcct gatgcggtat tttctcctta cgcatctgtg 5880
   cggtatttca caccgcatac gtcaaagcaa ccatagtacg cgccctgtag cggcgcatta 5940
   agcgcggcgg gtgtggtggt tacgcgcagc gtgaccgcta cacttgccag cgccctagcg 6000
   cccgctcctt tcgctttctt cccttccttt ctcgccacgt tcgccggctt tccccgtcaa 6060
   gctctaaatc gggggctccc tttagggttc cgatttagtg ctttacggca cctcgacccc 6120
   aaaaaacttg atttgggtga tggttcacgt agtgggccat cgccctgata gacggttttt 6180
   cgccctttga cgttggagtc cacgttcttt aatagtggac tcttgttcca aactggaaca 6240
   acactcaacc ctatctcggg ctattctttt gatttataag ggattttgcc gatttcggcc 6300
   tattggttaa aaaatgagct gatttaacaa aaatttaacg cgaattttaa caaaatatta 6360
   acgtttacaa ttttatggtg cactctcagt acaatctgct ctgatgccgc atagttaagc 6420
   cagccccgac acccgccaac acccgctgac gcgccctgac gggcttgtct gctcccggca 6480
   tccgcttaca gacaagctgt gaccgtctcc gggagctgca tgtgtcagag gttttcaccg 6540
   tcatcaccga aacgcgcgag acgaaagggc ctcgtgatac gcctattttt ataggttaat 6600
   gtcatgataa taatggtttc ttagacgtca ggtggcactt ttcggggaaa tgtgcgcgga 6660
   acccctattt gtttattttt ctaaatacat tcaaatatgt atccgctcat gagacaataa 6720
   ccctgataaa tgcttcaata atattgaaaa aggaagagta tgagtattca acatttccgt 6780
   gtcgccctta ttcccttttt tgcggcattt tgccttcctg tttttgctca cccagaaacg 6840
   ctggtgaaag taaaagatgc tgaagatcag ttgggtgcac gagtgggtta catcgaactg 6900
   gatctcaaca gcggtaagat ccttgagagt tttcgccccg aagaacgttt tccaatgatg 6960
   agcactttta aagttctgct atgtggcgcg gtattatccc gtattgacgc cgggcaagag 7020
   caactcggtc gccgcataca ctattctcag aatgacttgg ttgagtactc accagtcaca 7080
   gaaaagcatc ttacggatgg catgacagta agagaattat gcagtgctgc cataaccatg 7140
   agtgataaca ctgcggccaa cttacttctg acaacgatcg gaggaccgaa ggagctaacc 7200
   gcttttttgc acaacatggg ggatcatgta actcgccttg atcgttggga accggagctg 7260
   aatgaagcca taccaaacga cgagcgtgac accacgatgc ctgtagcaat ggcaacaacg 7320
   ttgcgcaaac tattaactgg cgaactactt actctagctt cccggcaaca attaatagac 7380
   tggatggagg cggataaagt tgcaggacca cttctgcgct cggcccttcc ggctggctgg 7440
   tttattgctg ataaatctgg agccggtgag cgtggaagcc gcggtatcat tgcagcactg 7500
   gggccagatg gtaagccctc ccgtatcgta gttatctaca cgacggggag tcaggcaact 7560
   atggatgaac gaaatagaca gatcgctgag ataggtgcct cactgattaa gcattggtaa 7620
   ctgtcagacc aagtttactc atatatactt tagattgatt taaaacttca tttttaattt 7680
   aaaaggatct aggtgaagat cctttttgat aatctcatga ccaaaatccc ttaacgtgag 7740
   ttttcgttcc actgagcgtc agaccccgta gaaaagatca aaggatcttc ttgagatcct 7800
   ttttttctgc gcgtaatctg ctgcttgcaa acaaaaaaac caccgctacc agcggtggtt 7860
   tgtttgccgg atcaagagct accaactctt tttccgaagg taactggctt cagcagagcg 7920
   cagataccaa atactgtcct tctagtgtag ccgtagttag gccaccactt caagaactct 7980
   gtagcaccgc ctacatacct cgctctgcta atcctgttac cagtggctgc tgccagtggc 8040
   gataagtcgt gtcttaccgg gttggactca agacgatagt taccggataa ggcgcagcgg 8100
   tcgggctgaa cggggggttc gtgcacacag cccagcttgg agcgaacgac ctacaccgaa 8160
   ctgagatacc tacagcgtga gctatgagaa agcgccacgc ttcccgaagg gagaaaggcg 8220
   gacaggtatc cggtaagcgg cagggtcgga acaggagagc gcacgaggga gcttccaggg 8280
   ggaaacgcct ggtatcttta tagtcctgtc gggtttcgcc acctctgact tgagcgtcga 8340
   tttttgtgat gctcgtcagg ggggcggagc ctatggaaaa acgccagcaa cgcggccttt 8400
   ttacggttcc tggccttttg ctggcctttt gctcacatgt 8440
<210> 82
   <211> 8509
   <212> DNA
   <213> Artificial Sequence
<220>
   <223> synthetic DNA
<400> 82
   cgcctccctg ggcacatacc acgatctgct gaaaattatc aaggacaagg acttcctgga        60
   caatgaggaa aacgaggaca ttctggaaga tatcgtgctg accctgacac tgtttgagga       120
   cagagagatg atcgaggaac ggctgaaaac ctatgcccac ctgttcgacg acaaagtgat       180
   gaagcagctg aagcggcgga gatacaccgg ctggggcagg ctgagccgga agctgatcaa       240
   cggcatccgg gacaagcagt ccggcaagac aatcctggat ttcctgaagt ccgacggctt       300
   cgccaacaga aacttcatgc agctgatcca cgacgacagc ctgaccttta aagaggacat       360
   ccagaaagcc caggtgtccg gccagggcga tagcctgcac gagcacattg ccaatctggc       420
   cggcagcccc gccattaaga agggcatcct gcagacagtg aaggtggtgg acgagctcgt       480
   gaaagtgatg ggccggcaca agcccgagaa catcgtgatc gaaatggcca gagagaacca       540
   gaccacccag aagggacaga agaacagccg cgagagaatg aagcggatcg aagagggcat       600
   caaagagctg ggcagccaga tcctgaaaga acaccccgtg gaaaacaccc agctgcagaa 660
   cgagaagctg tacctgtact acctgcagaa tgggcgggat atgtacgtgg accaggaact 720
   ggacatcaac cggctgtccg actacgatgt ggaccatatc gtgcctcaga gctttctgaa 780
   ggacgactcc atcgacaaca aggtgctgac cagaagcgac aagaaccggg gcaagagcga 840
   caacgtgccc tccgaagagg tcgtgaagaa gatgaagaac tactggcggc agctgctgaa 900
   cgccaagctg attacccaga gaaagttcga caatctgacc aaggccgaga gaggcggcct 960
   gagcgaactg gataaggccg gcttcatcaa gagacagctg gtggaaaccc ggcagatcac 1020
   aaagcacgtg gcacagatcc tggactcccg gatgaacact aagtacgacg agaatgacaa 1080
   gctgatccgg gaagtgaaag tgatcaccct gaagtccaag ctggtgtccg atttccggaa 1140
   ggatttccag ttttacaaag tgcgcgagat caacaactac caccacgccc acgacgccta 1200
   cctgaacgcc gtcgtgggaa ccgccctgat caaaaagtac cctaagctgg aaagcgagtt 1260
   cgtgtacggc gactacaagg tgtacgacgt gcggaagatg atcgccaaga gcgagcagga 1320
   aatcggcaag gctaccgcca agtacttctt ctacagcaac atcatgaact ttttcaagac 1380
   cgagattacc ctggccaacg gcgagatccg gaagcggcct ctgatcgaga caaacggcga 1440
   aaccggggag atcgtgtggg ataagggccg ggattttgcc accgtgcgga aagtgctgag 1500
   catgccccaa gtgaatatcg tgaaaaagac cgaggtgcag acaggcggct tcagcaaaga 1560
   gtctatcctg cccaagagga acagcgataa gctgatcgcc agaaagaagg actgggaccc 1620
   taagaagtac ggcggcttcg acagccccac cgtggcctat tctgtgctgg tggtggccaa 1680
   agtggaaaag ggcaagtcca agaaactgaa gagtgtgaaa gagctgctgg ggatcaccat 1740
   catggaaaga agcagcttcg agaagaatcc catcgacttt ctggaagcca agggctacaa 1800
   agaagtgaaa aaggacctga tcatcaagct gcctaagtac tccctgttcg agctggaaaa 1860
   cggccggaag agaatgctgg cctctgccgg cgaactgcag aagggaaacg aactggccct 1920
   gccctccaaa tatgtgaact tcctgtacct ggccagccac tatgagaagc tgaagggctc 1980
   ccccgaggat aatgagcaga aacagctgtt tgtggaacag cacaagcact acctggacga 2040
   gatcatcgag cagatcagcg agttctccaa gagagtgatc ctggccgacg ctaatctgga 2100
   caaagtgctg tccgcctaca acaagcaccg ggataagccc atcagagagc aggccgagaa 2160
   tatcatccac ctgtttaccc tgaccaatct gggagcccct gccgccttca agtactttga 2220
   caccaccatc gaccggaaga ggtacaccag caccaaagag gtgctggacg ccaccctgat 2280
   ccaccagagc atcaccggcc tgtacgagac acggatcgac ctgtctcagc tgggaggcga 2340
   caaaaggccg gcggccacga aaaaggccgg ccaggcaaaa aagaaaaagt aagaattcct 2400
   agagctcgct gatcagcctc gactgtgcct tctagttgcc agccatctgt tgtttgcccc 2460
   tcccccgtgc cttccttgac cctggaaggt gccactccca ctgtcctttc ctaataaaat 2520
   gaggaaattg catcgcattg tctgagtagg tgtcattcta ttctgggggg tggggtgggg 2580
   caggacagca agggggagga ttgggaagag aatagcaggc atgctgggga gcggccgcag 2640
   gaacccctag tgatggagtt ggccactccc tctctgcgcg ctcgctcgct cactgaggcc 2700
   gggcgaccaa aggtcgcccg acgcccgggc tttgcccggg cggcctcagt gagcgagcga 2760
   gcgcgcagct gcctgcaggg gcgcctgatg cggtattttc tccttacgca tctgtgcggt 2820
   atttcacacc gcatacgtca aagcaaccat agtacgcgcc ctgtagcggc gcattaagcg 2880
   cggcgggtgt ggtggttacg cgcagcgtga ccgctacact tgccagcgcc ctagcgcccg 2940
   ctcctttcgc tttcttccct tcctttctcg ccacgttcgc cggctttccc cgtcaagctc 3000
   taaatcgggg gctcccttta gggttccgat ttagtgcttt acggcacctc gaccccaaaa 3060
   aacttgattt gggtgatggt tcacgtagtg ggccatcgcc ctgatagacg gtttttcgcc 3120
   ctttgacgtt ggagtccacg ttctttaata gtggactctt gttccaaact ggaacaacac 3180
   tcaaccctat ctcgggctat tcttttgatt tataagggat tttgccgatt tcggcctatt 3240
   ggttaaaaaa tgagctgatt taacaaaaat ttaacgcgaa ttttaacaaa atattaacgt 3300
   ttacaatttt atggtgcact ctcagtacaa tctgctctga tgccgcatag ttaagccagc 3360
   cccgacaccc gccaacaccc gctgacgcgc cctgacgggc ttgtctgctc ccggcatccg 3420
   cttacagaca agctgtgacc gtctccggga gctgcatgtg tcagaggttt tcaccgtcat 3480
   caccgaaacg cgcgagacga aagggcctcg tgatacgcct atttttatag gttaatgtca 3540
   tgataataat ggtttcttag acgtcaggtg gcacttttcg gggaaatgtg cgcggaaccc 3600
   ctatttgttt atttttctaa atacattcaa atatgtatcc gctcatgaga caataaccct 3660
   gataaatgct tcaataatat tgaaaaagga agagtatgag tattcaacat ttccgtgtcg 3720
   cccttattcc cttttttgcg gcattttgcc ttcctgtttt tgctcaccca gaaacgctgg 3780
   tgaaagtaaa agatgctgaa gatcagttgg gtgcacgagt gggttacatc gaactggatc 3840
   tcaacagcgg taagatcctt gagagttttc gccccgaaga acgttttcca atgatgagca 3900
   cttttaaagt tctgctatgt ggcgcggtat tatcccgtat tgacgccggg caagagcaac 3960
   tcggtcgccg catacactat tctcagaatg acttggttga gtactcacca gtcacagaaa 4020
   agcatcttac ggatggcatg acagtaagag aattatgcag tgctgccata accatgagtg 4080
   ataacactgc ggccaactta cttctgacaa cgatcggagg accgaaggag ctaaccgctt 4140
   ttttgcacaa catgggggat catgtaactc gccttgatcg ttgggaaccg gagctgaatg 4200
   aagccatacc aaacgacgag cgtgacacca cgatgcctgt agcaatggca acaacgttgc 4260
   gcaaactatt aactggcgaa ctacttactc tagcttcccg gcaacaatta atagactgga 4320
   tggaggcgga taaagttgca ggaccacttc tgcgctcggc ccttccggct ggctggttta 4380
   ttgctgataa atctggagcc ggtgagcgtg gaagccgcgg tatcattgca gcactggggc 4440
   cagatggtaa gccctcccgt atcgtagtta tctacacgac ggggagtcag gcaactatgg 4500
   atgaacgaaa tagacagatc gctgagatag gtgcctcact gattaagcat tggtaactgt 4560
   cagaccaagt ttactcatat atactttaga ttgatttaaa acttcatttt taatttaaaa 4620
   ggatctaggt gaagatcctt tttgataatc tcatgaccaa aatcccttaa cgtgagtttt 4680
   cgttccactg agcgtcagac cccgtagaaa agatcaaagg atcttcttga gatccttttt 4740
   ttctgcgcgt aatctgctgc ttgcaaacaa aaaaaccacc gctaccagcg gtggtttgtt 4800
   tgccggatca agagctacca actctttttc cgaaggtaac tggcttcagc agagcgcaga 4860
   taccaaatac tgtccttcta gtgtagccgt agttaggcca ccacttcaag aactctgtag 4920
   caccgcctac atacctcgct ctgctaatcc tgttaccagt ggctgctgcc agtggcgata 4980
   agtcgtgtct taccgggttg gactcaagac gatagttacc ggataaggcg cagcggtcgg 5040
   gctgaacggg gggttcgtgc acacagccca gcttggagcg aacgacctac accgaactga 5100
   gatacctaca gcgtgagcta tgagaaagcg ccacgcttcc cgaagggaga aaggcggaca 5160
   ggtatccggt aagcggcagg gtcggaacag gagagcgcac gagggagctt ccagggggaa 5220
   acgcctggta tctttatagt cctgtcgggt ttcgccacct ctgacttgag cgtcgatttt 5280
   tgtgatgctc gtcagggggg cggagcctat ggaaaaacgc cagcaacgcg gcctttttac 5340
   ggttcctggc cttttgctgg ccttttgctc acatgtgagg gcctatttcc catgattcct 5400
   tcatatttgc atatacgata caaggctgtt agagagataa ttggaattaa tttgactgta 5460
   aacacaaaga tattagtaca aaatacgtga cgtagaaagt aataatttct tgggtagttt 5520
   gcagttttaa aattatgttt taaaatggac tatcatatgc ttaccgtaac ttgaaagtat 5580
   ttcgatttct tggctttata tatcttgtgg aaaggacgaa acaccgccat agttagtcaa 5640
   tgggtagttt tagagctaga aatagcaagt taaaataagg ctagtccgtt atcaacttga 5700
   aaaagtggca ccgagtcggt gcttttttgt tttagagcta gaaatagcaa gttaaaataa 5760
   ggctagtccg tttttagcgc gtgcgccaat tctgcagaca aatggctcta gaggtacccg 5820
   ttacataact tacggtaaat ggcccgcctg gctgaccgcc caacgacccc cgcccattga 5880
   cgtcaatagt aacgccaata gggactttcc attgacgtca atgggtggag tatttacggt 5940
   aaactgccca cttggcagta catcaagtgt atcatatgcc aagtacgccc cctattgacg 6000
   tcaatgacgg taaatggccc gcctggcatt gtgcccagta catgacctta tgggactttc 6060
   ctacttggca gtacatctac gtattagtca tcgctattac catggtcgag gtgagcccca 6120
   cgttctgctt cactctcccc atctcccccc cctccccacc cccaattttg tatttattta 6180
   ttttttaatt attttgtgca gcgatggggg cggggggggg gggggggcgc gcgccaggcg 6240
   gggcggggcg gggcgagggg cggggcgggg cgaggcggag aggtgcggcg gcagccaatc 6300
   agagcggcgc gctccgaaag tttcctttta tggcgaggcg gcggcggcgg cggccctata 6360
   aaaagcgaag cgcgcggcgg gcgggagtcg ctgcgacgct gccttcgccc cgtgccccgc 6420
   tccgccgccg cctcgcgccg cccgccccgg ctctgactga ccgcgttact cccacaggtg 6480
   agcgggcggg acggcccttc tcctccgggc tgtaattagc tgagcaagag gtaagggttt 6540
   aagggatggt tggttggtgg ggtattaatg tttaattacc tggagcacct gcctgaaatc 6600
   actttttttc aggttggacc ggtgccacca tggactataa ggaccacgac ggagactaca 6660
   aggatcatga tattgattac aaagacgatg acgataagat ggccccaaag aagaagcgga 6720
   aggtcggtat ccacggagtc ccagcagccg acaagaagta cagcatcggc ctggacatcg 6780
   gcaccaactc tgtgggctgg gccgtgatca ccgacgagta caaggtgccc agcaagaaat 6840
   tcaaggtgct gggcaacacc gaccggcaca gcatcaagaa gaacctgatc ggagccctgc 6900
   tgttcgacag cggcgaaaca gccgaggcca cccggctgaa gagaaccgcc agaagaagat 6960
   acaccagacg gaagaaccgg atctgctatc tgcaagagat cttcagcaac gagatggcca 7020
   aggtggacga cagcttcttc cacagactgg aagagtcctt cctggtggaa gaggataaga 7080
   agcacgagcg gcaccccatc ttcggcaaca tcgtggacga ggtggcctac cacgagaagt 7140
   accccaccat ctaccacctg agaaagaaac tggtggacag caccgacaag gccgacctgc 7200
   ggctgatcta tctggccctg gcccacatga tcaagttccg gggccacttc ctgatcgagg 7260
   gcgacctgaa ccccgacaac agcgacgtgg acaagctgtt catccagctg gtgcagacct 7320
   acaaccagct gttcgaggaa aaccccatca acgccagcgg cgtggacgcc aaggccatcc 7380
   tgtctgccag actgagcaag agcagacggc tggaaaatct gatcgcccag ctgcccggcg 7440
   agaagaagaa tggcctgttc ggaaacctga ttgccctgag cctgggcctg acccccaact 7500
   tcaagagcaa cttcgacctg gccgaggatg ccaaactgca gctgagcaag gacacctacg 7560
   acgacgacct ggacaacctg ctggcccaga tcggcgacca gtacgccgac ctgtttctgg 7620
   ccgccaagaa cctgtccgac gccatcctgc tgagcgacat cctgagagtg aacaccgaga 7680
   tcaccaaggc ccccctgagc gcctctatga tcaagagata cgacgagcac caccaggacc 7740
   tgaccctgct gaaagctctc gtgcggcagc agctgcctga gaagtacaaa gagattttct 7800
   tcgaccagag caagaacggc tacgccggct acattgacgg cggagccagc caggaagagt 7860
   tctacaagtt catcaagccc atcctggaaa agatggacgg caccgaggaa ctgctcgtga 7920
   agctgaacag agaggacctg ctgcggaagc agcggacctt cgacaacggc agcatccccc 7980
   accagatcca cctgggagag ctgcacgcca ttctgcggcg gcaggaagat ttttacccat 8040
   tcctgaagga caaccgggaa aagatcgaga agatcctgac cttccgcatc ccctactacg 8100
   tgggccctct ggccagggga aacagcagat tcgcctggat gaccagaaag agcgaggaaa 8160
   ccatcacccc ctggaacttc gaggaagtgg tggacaaggg cgcttccgcc cagagcttca 8220
   tcgagcggat gaccaacttc gataagaacc tgcccaacga gaaggtgctg cccaagcaca 8280
   gcctgctgta cgagtacttc accgtgtata acgagctgac caaagtgaaa tacgtgaccg 8340
   agggaatgag aaagcccgcc ttcctgagcg gcgagcagaa aaaggccatc gtggacctgc 8400
   tgttcaagac caaccggaaa gtgaccgtga agcagctgaa agaggactac ttcaagaaaa 8460
   tcgagtgctt cgactccgtg gaaatctccg gcgtggaaga tcggttcaa 8509
<210> 83
   <211> 20
   <212> DNA
   <213> Artificial Sequence
<220>
   <223> oligonucleotide
<400> 83
   ggcccacctg gagctgttaa 20
<210> 84
   <211> 22
   <212> DNA
   <213> Artificial Sequence
<220>
   <223> oligonucleotide
<400> 84
   cgccgtttat gactttgctc tt 22
<210> 85
   <211> 20
   <212> DNA
   <213> Artificial Sequence
<220>
   <223> oligonucleotide
<400> 85
   agacgccatc cacgctgttt 20
<210> 86
   <211> 20
   <212> DNA
   <213> Artificial Sequence
<220>
   <223> oligonucleotide
<400> 86
   gccaaaactc ataggccaaa 20
<210> 87
   <211> 870
   <212> DNA
   <213> Artificial Sequence
<220>
   <223> oligonucleotide
<400> 87
   ccacagggag tgttagtgct gctcgcgatt ttgctggcat ctctatactc ttcgtgtgtg      60
   gcagcctaag accgcaagca gaagaacttg ccagaacttt gccagcatta actcagcgct     120
   tctcaacttc ctaaggttgc gacccttcaa catagcgtct catgttgtga tgccctgcta     180
   ccataattat tccattgcta cttcataact gtaatttttt tgctgctgtt atgagttgta     240
   aggtaaacta tctgatatgc gacccccaag agtcacgacc cacaggctga gaaccgctgc     300
   cttggccagc cttgagggag ggatggcgtt gtttcctcgg tcaggaaaaa aaaaggaaaa     360
   ctcatcgcag gattaagact ctgtaatctc aacagatctg aaataaagaa tgagcatccg     420
   ttttctagtt atattcagaa ggacggctta gtcaaccact ttgcacttcc aaaatgcctt     480
   ccctctcttc agagtgtctc tcagagcaca tggaccttcc tcattggtca gattttctgt     540
   cagtaccacc aacaattgct attggtccat tttgaagatt ctcccgcctt cttttaccta     600
   attggtttat tcgaaaaggc ggtcttttgt tcgaataaaa gacgatctac gattggttac     660
   tgcaaacaga gccaatgaga gcgagtcggt gattggttcc tctgatttgg gcgggctaga     720
   taaaggttca aatgaaacta gggaagctct cctaaccgac aagcgtctgt gaggggagcg     780
   tccaggtccc gcgtcgcgcg tcgcgggtcg cctagcgttt cccgctggtt ttgtcgcttt     840
   ccggattctc gagcctcttc aggaccgtca                                      870
<210> 88
   <211> 37
   <212> DNA
   <213> Artificial Sequence
<220>
   <223> synthetic DNA
<400> 88
   aattcctgca ggccacaggg agtgttagtg ctgctcg 37
<210> 89
   <211> 36
   <212> DNA
   <213> Artificial Sequence
<220>
   <223> synthetic DNA
<400> 89
   ttactagttg acggtcctga agaggctcga gaatcc 36
<210> 90
   <211> 818
   <212> DNA
   <213> Artificial Sequence
<220>
   <223> synthetic DNA
<400> 90
   agttgtcacc gctgcaggta cagctcgcgg atggggccgg gtagtagagc ctttacgtgg      60
   cctctgcgtt catggctgac ggcgagagtt gaggttccag ggccgagcgc tggccggcga     120
   ccggctggat ccggtcagcc ccatcttctg ggccttgagg ggtcgtggac tgggtgaagc     180
   tctttccgaa atgatatcta taatctaatc tgggttcttg agtgttgtgt ttttattgtt     240
   tgtgggagca ggttcaacta gtcctgcgat cggtctgtag tctaatggct tctgtgtgac     300
   ttccagccgg cttatactcg aattcagtgg ccctcttccc ttccctgggt gatttgaata     360
   actcatcacg gaaggtgcag agcatggcag ttcccattga ggagctggcc gaccgagtgg     420
   ctcaaaaaaa gtccagtttc gggatgtgaa tgtagccaag ggtttgcatt gctagcatcc     480
   caactgctac gggtggatac catctgtaat cggcacgagc ctatgtcggc actcactaaa     540
   tattttccca ttagatggaa gaagttgagt gtagtttatc gatgacataa aaaatattac     600
   tcaaggcaga aatgtccata cttgcagaac taaaatacct atttaaacgg gcatctgtag     660
   ccagacctag tggggcaggc ctgaaatggc agctgctcca gagactgagg caagaggatg     720
   ccttaagcta tttaatggga ctctcaaaaa cagcaacagg gggtgaaaaa aatggtctgg     780
   gaattttgct cagtggtcag agcatttgcc tagcatgt                              818
<210> 91
   <211> 27
   <212> DNA
   <213> Artificial Sequence
<220>
   <223> oligonucleotide
<400> 91
   ccgtcaccat ggagttgtca ccgctgc 27
<210> 92
   <211> 30
   <212> DNA
   <213> Artificial Sequence
<220>
   <223> oligonucleotide
<400> 92
   cccttgcatg cacatgctag gcaaatgctc 30
<210> 93
   <211> 8347
   <212> DNA
   <213> Artificial Sequence
<220>
   <223> synthetic DNA
<400> 93
   tatagtgagt cgtattacaa ttcactggcc gtcgttttac aacgtcgtga ctgggaaaac 60
   cctggcgtta cccaacttaa tcgccttgca gcacatcccc ctttcgccag ctggcgtaat 120
   agcgaagagg cccgcaccga tcgcccttcc caacagttgc gcagcctgaa tggcgaatgg 180
   acgcgccctg tagcggcgca ttaagcgcgg cgggtgtggt ggttacgcgc agcgtgaccg 240
   ctacacttgc cagcgcccta gcgcccgctc ctttcgcttt cttcccttcc tttctcgcca 300
   cgttcgccgg ctttccccgt caagctctaa atcgggggct ccctttaggg ttccgattta 360
   gtgctttacg gcacctcgac cccaaaaaac ttgattaggg tgatggttca cgtagtgggc 420
   catcgccctg atagacggtt tttcgccctt tgacgttgga gtccacgttc tttaatagtg 480
   gactcttgtt ccaaactgga acaacactca accctatctc ggtctattct tttgatttat 540
   aagggatttt gccgatttcg gcctattggt taaaaaatga gctgatttaa caaaaattta 600
   acgcgaattt taacaaaata ttaacgctta caatttcctg atgcggtatt ttctccttac 660
   gcatctgtgc ggtatttcac accgcatcag gtggcacttt tcggggaaat gtgcgcggaa 720
   cccctatttg tttatttttc taaatacatt caaatatgta tccgctcatg agacaataac 780
   cctgataaat gcttcaataa tattgaaaaa ggaagagtat gagtattcaa catttccgtg 840
   tcgcccttat tccctttttt gcggcatttt gccttcctgt ttttgctcac ccagaaacgc 900
   tggtgaaagt aaaagatgct gaagatcagt tgggtgcacg agtgggttac atcgaactgg 960
   atctcaacag cggtaagatc cttgagagtt ttcgccccga agaacgtttt ccaatgatga 1020
   gcacttttaa agttctgcta tgtggcgcgg tattatcccg tattgacgcc gggcaagagc 1080
   aactcggtcg ccgcatacac tattctcaga atgacttggt tgagtactca ccagtcacag 1140
   aaaagcatct tacggatggc atgacagtaa gagaattatg cagtgctgcc ataaccatga 1200
   gtgataacac tgcggccaac ttacttctga caacgatcgg aggaccgaag gagctaaccg 1260
   cttttttgca caacatgggg gatcatgtaa ctcgccttga tcgttgggaa ccggagctga 1320
   atgaagccat accaaacgac gagcgtgaca ccacgatgcc tgtagcaatg gcaacaacgt 1380
   tgcgcaaact attaactggc gaactactta ctctagcttc ccggcaacaa ttaatagact 1440
   ggatggaggc ggataaagtt gcaggaccac ttctgcgctc ggcccttccg gctggctggt 1500
   ttattgctga taaatctgga gccggtgagc gtgggtctcg cggtatcatt gcagcactgg 1560
   ggccagatgg taagccctcc cgtatcgtag ttatctacac gacggggagt caggcaacta 1620
   tggatgaacg aaatagacag atcgctgaga taggtgcctc actgattaag cattggtaac 1680
   tgtcagacca agtttactca tatatacttt agattgattt aaaacttcat ttttaattta 1740
   aaaggatcta ggtgaagatc ctttttgata atctcatgac caaaatccct taacgtgagt 1800
   tttcgttcca ctgagcgtca gaccccgtag aaaagatcaa aggatcttct tgagatcctt 1860
   tttttctgcg cgtaatctgc tgcttgcaaa caaaaaaacc accgctacca gcggtggttt 1920
   gtttgccgga tcaagagcta ccaactcttt ttccgaaggt aactggcttc agcagagcgc 1980
   agataccaaa tactgttctt ctagtgtagc cgtagttagg ccaccacttc aagaactctg 2040
   tagcaccgcc tacatacctc gctctgctaa tcctgttacc agtggctgct gccagtggcg 2100
   ataagtcgtg tcttaccggg ttggactcaa gacgatagtt accggataag gcgcagcggt 2160
   cgggctgaac ggggggttcg tgcacacagc ccagcttgga gcgaacgacc tacaccgaac 2220
   tgagatacct acagcgtgag ctatgagaaa gcgccacgct tcccgaaggg agaaaggcgg 2280
   acaggtatcc ggtaagcggc agggtcggaa caggagagcg cacgagggag cttccagggg 2340
   gaaacgcctg gtatctttat agtcctgtcg ggtttcgcca cctctgactt gagcgtcgat 2400
   ttttgtgatg ctcgtcaggg gggcggagcc tatggaaaaa cgccagcaac gcggcctttt 2460
   tacggttcct ggccttttgc tggccttttg ctcacatgtt ctttcctgcg ttatcccctg 2520
   attctgtgga taaccgtatt accgcctttg agtgagctga taccgctcgc cgcagccgaa 2580
   cgaccgagcg cagcgagtca gtgagcgagg aagcggaaga gcgcccaata cgcaaaccgc 2640
   ctctccccgc gcgttggccg attcattaat gcagctggca cgacaggttt cccgactgga 2700
   aagcgggcag tgagcgcaac gcaattaatg tgagttagct cactcattag gcaccccagg 2760
   ctttacactt tatgcttccg gctcgtatgt tgtgtggaat tgtgagcgga taacaatttc 2820
   acacaggaaa cagctatgac catgattacg ccaagctatt taggtgacac tatagaatac 2880
   tcaagctatg catccaacgc gttgggagct ctcccatatg gtcgacctgc aggccacagg 2940
   gagtgttagt gctgctcgcg attttgctgg catctctata ctcttcgtgt gtggcagcct 3000
   aagaccgcaa gcagaagaac ttgccagaac tttgccagca ttaactcagc gcttctcaac 3060
   ttcctaaggt tgcgaccctt caacatagcg tctcatgttg tgatgccctg ctaccataat 3120
   tattccattg ctacttcata actgtaattt ttttgctgct gttatgagtt gtaaggtaaa 3180
   ctatctgata tgcgaccccc aagagtcacg acccacaggc tgagaaccgc tgccttggcc 3240
   agccttgagg gagggatggc gttgtttcct cggtcaggaa aaaaaaagga aaactcatcg 3300
   caggattaag actctgtaat ctcaacagat ctgaaataaa gaatgagcat ccgttttcta 3360
   gttatattca gaaggacggc ttagtcaacc actttgcact tccaaaatgc cttccctctc 3420
   ttcagagtgt ctctcagagc acatggacct tcctcattgg tcagattttc tgtcagtacc 3480
   accaacaatt gctattggtc cattttgaag attctcccgc cttcttttac ctaattggtt 3540
   tattcgaaaa ggcggtcttt tgttcgaata aaagacgatc tacgattggt tactgcaaac 3600
   agagccaatg agagcgagtc ggtgattggt tcctctgatt tgggcgggct agataaaggt 3660
   tcaaatgaaa ctagggaagc tctcctaacc gacaagcgtc tgtgagggga gcgtccaggt 3720
   cccgcgtcgc gcgtcgcggg tcgcctagcg tttcccgctg gttttgtcgc tttccggatt 3780
   ctcgagcctc ttcaggaccg tcaactagtg attaccgttc gtatagcata cattatacga 3840
   agttatacca tgtctagact ggacaagagc aaagtcataa acggcgctct ggaattactc 3900
   aatggagtcg gtatcgaagg cctgacgaca aggaaactcg ctcaaaagct gggagttgag 3960
   cagcctaccc tgtactggca cgtgaagaac aagcgggccc tgctcgatgc cctgccaatc 4020
   gagatgctgg acaggcatca tacccacttc tgccccctgg aaggcgagtc atggcaagac 4080
   tttctgcgga acaacgccaa gtcattccgc tgtgctctcc tctcacatcg cgacggggct 4140
   aaagtgcatc tcggcacccg cccaacagag aaacagtacg aaaccctgga aaatcagctc 4200
   gcgttcctgt gtcagcaagg cttctccctg gagaacgcac tgtacgctct gtccgccgtg 4260
   ggccacttta cactgggctg cgtattggag gaacaggagc atcaagtagc aaaagaggaa 4320
   agagagacac ctaccaccga ttctatgccc ccacttctga gacaagcaat tgagctgttc 4380
   gaccggcagg gagccgaacc tgccttcctt ttcggcctgg aactaatcat atgtggcctg 4440
   gagaaacagc taaagtgcga aagcggcggg ccggccgacg cccttgacga ttttgactta 4500
   gacatgctcc cagccgatgc ccttgacgac tttgaccttg atatgctgcc tgctgacgct 4560
   cttgacgatt ttgaccttga catgctcccc gggtaactaa gtaaggatcc caaataccac 4620
   tgaattaaga attccgcccc tctccctccc ccccccctaa cgttactggc cgaagccgct 4680
   tggaataagg ccggtgtgcg tttgtctata tgttattttc caccatattg ccgtcttttg 4740
   gcaatgtgag ggcccggaaa cctggccctg tcttcttgac gagcattcct aggggtcttt 4800
   cccctctcgc caaaggaatg caaggtctgt tgaatgtcgt gaaggaagca gttcctctgg 4860
   aagcttcttg aagacaaaca acgtctgtag cgaccctttg caggcagcgg aaccccccac 4920
   ctggcgacag gtgcctctgc ggccaaaagc cacgtgtata agatacacct gcaaaggcgg 4980
   cacaacccca gtgccacgtt gtgagttgga tagttgtgga aagagtcaaa tggctctcct 5040
   caagcgtatt caacaagggg ctgaaggatg cccagaaggt accccattgt atgggatctg 5100
   atctggggcc tcggtgcaca tgctttacat gtgtttagtc gaggttaaaa aacgtctagg 5160
   ccccccgaac cacggggacg tggttttcct ttgaaaaaca cgatgataat atggccacaa 5220
   ccatgaccga gtacaagccc acggtgcgcc tcgccacccg cgacgacgtc ccccgggccg 5280
   tacgcaccct cgccgccgcg ttcgccgact accccgccac gcgccacacc gtcgacccgg 5340
   accgccacat cgagcgggtc accgagctgc aagaactctt cctcacgcgc gtcgggctcg 5400
   acatcggcaa ggtgtgggtc gcggacgacg gcgccgcggt ggcggtctgg accacgccgg 5460
   agagcgtcga agcgggggcg gtgttcgccg agatcggccc gcgcatggcc gagttgagcg 5520
   gttcccggct ggccgcgcag caacagatgg aaggcctcct ggcgccgcac cggcccaagg 5580
   agcccgcgtg gttcctggcc accgtcggcg tctcgcccga ccaccagggc aagggtctgg 5640
   gcagcgccgt cgtgctcccc ggagtggagg cggccgagcg cgccggggtg cccgccttcc 5700
   tggagacctc cgcgccccgc aacctcccct tctacgagcg gctcggcttc accgtcaccg 5760
   ccgacgtcga ggtgcccgaa ggaccgcgca cctggtgcat gacccgcaag cccggtgcct 5820
   gaggatccag acatgataag atacattgat gagtttggac aaaccacaac tagaatgcag 5880
   tgaaaaaaat gctttatttg tgaaatttgt gatgctattg ctttatttgt aaccattata 5940
   agctgcaata aacaagttaa caacaacaat tgcattcatt ttatgtttca ggttcagggg 6000
   gaggtgtggg aggtttttta aagcaagtaa aacctctaca aatgtggtat ggctgattat 6060
   gatcctctag agtcgcagat ccagacatga taagatacat tgatgagttt ggacaaacca 6120
   caactagaat gcagtgaaaa aaatgcttta tttgtgaaat ttgtgatgct attgctttat 6180
   ttgtaaccat tataagctgc aataaacaag ttaacaacaa caattgcatt cattttatgt 6240
   ttcaggttca gggggaggtg tgggaggttt tttaaagcaa gtaaaacctc tacaaatgtg 6300
   gtatggctga ttatgatcct ctagagtcgc agatccagac atgataagat acattgatga 6360
   gtttggacaa accacaacta gaatgcagtg aaaaaaatgc tttatttgtg aaatttgtga 6420
   tgctattgct ttatttgtaa ccattataag ctgcaataaa caagttaaca acaacaattg 6480
   cattcatttt atgtttcagg ttcaggggga ggtgtgggag gttttttaaa gcaagtaaaa 6540
   cctctacaaa tgtggtatgg ctgattatga tcctctagag tcgcagatcc tctagagtcg 6600
   cagatctttt tccctctgcc aaaaattatg gggacatcat gaagcccctt gagcatctga 6660
   cttctggcta ataaaggaaa tttattttca ttgcaatagt gtgttggaat tttttgtgtc 6720
   tctcactcgg aaggacatat gggagggcaa atcatttaaa acatcagaat gagtatttgg 6780
   tttagagttt ggcaacatat gccatatgct ggctgccatg aacaaaggtg gctataaaga 6840
   ggtcatcagt atatgaaaca gccccctgct gtccattcct tattccatag aaaagccttg 6900
   acttgaggtt agattttttt tatattttgt tttgtgttat ttttttcttt aacatcccta 6960
   aaattttcct tacatgtttt actagccaga tttttcctcc tctcctgact actcccagtc 7020
   atagctgtcc ctcttctctt atgaagatcc ctcgacctgc agcccaagct tatcgaattc 7080
   ccgcggtggc ggccgcacgt ctccctatca gtgatagaga agtcgacacg tctcgagctc 7140
   cctatcagtg atagagaagg tacgtctaga acgtctccct atcagtgata gagaagtcga 7200
   cacgtctcga gctccctatc agtgatagag aaggtacgtc tagaacgtct ccctatcagt 7260
   gatagagaag tcgacacgtc tcgagctccc tatcagtgat agagaaggta cgtctagaac 7320
   gtctccctat cagtgataga gaagtcgaca cgtctcgagc tccctatcag tgatagagaa 7380
   ggtaccccct atataagcag agctcgttta gtgaaccgtc agatcgcctg gagacgccat 7440
   ccacgctgtt ttgacctcca tagaagacac cgggaccgat ccagcctccg cggccgccat 7500
   ggagttgtca ccgctgcagg tacagctcgc ggatggggcc gggtagtaga gcctttacgt 7560
   ggcctctgcg ttcatggctg acggcgagag ttgaggttcc agggccgagc gctggccggc 7620
   gaccggctgg atccggtcag ccccatcttc tgggccttga ggggtcgtgg actgggtgaa 7680
   gctctttccg aaatgatatc tataatctaa tctgggttct tgagtgttgt gtttttattg 7740
   tttgtgggag caggttcaac tagtcctgcg atcggtctgt agtctaatgg cttctgtgtg 7800
   acttccagcc ggcttatact cgaattcagt ggccctcttc ccttccctgg gtgatttgaa 7860
   taactcatca cggaaggtgc agagcatggc agttcccatt gaggagctgg ccgaccgagt 7920
   ggctcaaaaa aagtccagtt tcgggatgtg aatgtagcca agggtttgca ttgctagcat 7980
   cccaactgct acgggtggat accatctgta atcggcacga gcctatgtcg gcactcacta 8040
   aatattttcc cattagatgg aagaagttga gtgtagttta tcgatgacat aaaaaatatt 8100
   actcaaggca gaaatgtcca tacttgcaga actaaaatac ctatttaaac gggcatctgt 8160
   agccagacct agtggggcag gcctgaaatg gcagctgctc cagagactga ggcaagagga 8220
   tgccttaagc tatttaatgg gactctcaaa aacagcaaca gggggtgaaa aaaatggtct 8280
   gggaattttg ctcagtggtc agagcatttg cctagcatgt gcatgcgacg tcgggcccaa 8340
   ttcgccc 8347
<210> 94
   <211> 24
   <212> DNA
   <213> Artificial Sequence
<220>
   <223> oligonucleotide
<400> 94
   caccgcatgg tgacggtcct gaag 24
<210> 95
   <211> 24
   <212> DNA
   <213> Artificial Sequence
<220>
   <223> oligonucleotide
<400> 95
   aaaccttcag gaccgtcacc atgc 24
<210> 96
   <211> 24
   <212> DNA
   <213> Artificial Sequence
<220>
   <223> oligonucleotide
<400> 96
   caccgccgct gcaggtacag ctcg 24
<210> 97
   <211> 24
   <212> DNA
   <213> Artificial Sequence
<220>
   <223> oligonucleotide
<400> 97
   aaaccgagct gtacctgcag cggc 24
<210> 98
   <211> 8436
   <212> DNA
   <213> Artificial Sequence
<220>
   <223> synthetic DNA
<400> 98
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccg ccgctgcagg tacagctcgg ttttagagct agaaatagca agttaaaata     300
   aggctagtcc gttatcaact tgaaaaagtg gcaccgagtc ggtgcttttt tgttttagag     360
   ctagaaatag caagttaaaa taaggctagt ccgtttttag cgcgtgcgcc aattctgcag     420
   acaaatggct ctagaggtac ccgttacata acttacggta aatggcccgc ctggctgacc     480
   gcccaacgac ccccgcccat tgacgtcaat agtaacgcca atagggactt tccattgacg     540
   tcaatgggtg gagtatttac ggtaaactgc ccacttggca gtacatcaag tgtatcatat     600
   gccaagtacg ccccctattg acgtcaatga cggtaaatgg cccgcctggc attgtgccca     660
   gtacatgacc ttatgggact ttcctacttg gcagtacatc tacgtattag tcatcgctat     720
   taccatggtc gaggtgagcc ccacgttctg cttcactctc cccatctccc ccccctcccc     780
   acccccaatt ttgtatttat ttatttttta attattttgt gcagcgatgg gggcgggggg     840
   gggggggggg cgcgcgccag gcggggcggg gcggggcgag gggcggggcg gggcgaggcg     900
   gagaggtgcg gcggcagcca atcagagcgg cgcgctccga aagtttcctt ttatggcgag     960
   gcggcggcgg cggcggccct ataaaaagcg aagcgcgcgg cgggcgggag tcgctgcgac    1020
   gctgccttcg ccccgtgccc cgctccgccg ccgcctcgcg ccgcccgccc cggctctgac 1080
   tgaccgcgtt actcccacag gtgagcgggc gggacggccc ttctcctccg ggctgtaatt 1140
   agctgagcaa gaggtaaggg tttaagggat ggttggttgg tggggtatta atgtttaatt 1200
   acctggagca cctgcctgaa atcacttttt ttcaggttgg accggtgcca ccatgtaccc 1260
   atacgatgtt ccagattacg cttcgccgaa gaaaaagcgc aaggtcgaag cgtccgacaa 1320
   gaagtacagc atcggcctgg ccatcggcac caactctgtg ggctgggccg tgatcaccga 1380
   cgagtacaag gtgcccagca agaaattcaa ggtgctgggc aacaccgacc ggcacagcat 1440
   caagaagaac ctgatcggag ccctgctgtt cgacagcggc gaaacagccg aggccacccg 1500
   gctgaagaga accgccagaa gaagatacac cagacggaag aaccggatct gctatctgca 1560
   agagatcttc agcaacgaga tggccaaggt ggacgacagc ttcttccaca gactggaaga 1620
   gtccttcctg gtggaagagg ataagaagca cgagcggcac cccatcttcg gcaacatcgt 1680
   ggacgaggtg gcctaccacg agaagtaccc caccatctac cacctgagaa agaaactggt 1740
   ggacagcacc gacaaggccg acctgcggct gatctatctg gccctggccc acatgatcaa 1800
   gttccggggc cacttcctga tcgagggcga cctgaacccc gacaacagcg acgtggacaa 1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta 7440
   ttgctgataa atctggagcc ggtgagcgtg gaagccgcgg tatcattgca gcactggggc 7500
   cagatggtaa gccctcccgt atcgtagtta tctacacgac ggggagtcag gcaactatgg 7560
   atgaacgaaa tagacagatc gctgagatag gtgcctcact gattaagcat tggtaactgt 7620
   cagaccaagt ttactcatat atactttaga ttgatttaaa acttcatttt taatttaaaa 7680
   ggatctaggt gaagatcctt tttgataatc tcatgaccaa aatcccttaa cgtgagtttt 7740
   cgttccactg agcgtcagac cccgtagaaa agatcaaagg atcttcttga gatccttttt 7800
   ttctgcgcgt aatctgctgc ttgcaaacaa aaaaaccacc gctaccagcg gtggtttgtt 7860
   tgccggatca agagctacca actctttttc cgaaggtaac tggcttcagc agagcgcaga 7920
   taccaaatac tgtccttcta gtgtagccgt agttaggcca ccacttcaag aactctgtag 7980
   caccgcctac atacctcgct ctgctaatcc tgttaccagt ggctgctgcc agtggcgata 8040
   agtcgtgtct taccgggttg gactcaagac gatagttacc ggataaggcg cagcggtcgg 8100
   gctgaacggg gggttcgtgc acacagccca gcttggagcg aacgacctac accgaactga 8160
   gatacctaca gcgtgagcta tgagaaagcg ccacgcttcc cgaagggaga aaggcggaca 8220
   ggtatccggt aagcggcagg gtcggaacag gagagcgcac gagggagctt ccagggggaa 8280
   acgcctggta tctttatagt cctgtcgggt ttcgccacct ctgacttgag cgtcgatttt 8340
   tgtgatgctc gtcagggggg cggagcctat ggaaaaacgc cagcaacgcg gcctttttac 8400
   ggttcctggc cttttgctgg ccttttgctc acatgt 8436
<210> 99
   <211> 8436
   <212> DNA
   <213> Artificial Sequence
<220>
   <223> synthetic DNA
<400> 99
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg catggtgacg gtcctgaagg ttttagagct agaaatagca agttaaaata 300
   aggctagtcc gttatcaact tgaaaaagtg gcaccgagtc ggtgcttttt tgttttagag 360
   ctagaaatag caagttaaaa taaggctagt ccgtttttag cgcgtgcgcc aattctgcag 420
   acaaatggct ctagaggtac ccgttacata acttacggta aatggcccgc ctggctgacc 480
   gcccaacgac ccccgcccat tgacgtcaat agtaacgcca atagggactt tccattgacg 540
   tcaatgggtg gagtatttac ggtaaactgc ccacttggca gtacatcaag tgtatcatat 600
   gccaagtacg ccccctattg acgtcaatga cggtaaatgg cccgcctggc attgtgccca 660
   gtacatgacc ttatgggact ttcctacttg gcagtacatc tacgtattag tcatcgctat 720
   taccatggtc gaggtgagcc ccacgttctg cttcactctc cccatctccc ccccctcccc 780
   acccccaatt ttgtatttat ttatttttta attattttgt gcagcgatgg gggcgggggg 840
   gggggggggg cgcgcgccag gcggggcggg gcggggcgag gggcggggcg gggcgaggcg 900
   gagaggtgcg gcggcagcca atcagagcgg cgcgctccga aagtttcctt ttatggcgag 960
   gcggcggcgg cggcggccct ataaaaagcg aagcgcgcgg cgggcgggag tcgctgcgac 1020
   gctgccttcg ccccgtgccc cgctccgccg ccgcctcgcg ccgcccgccc cggctctgac 1080
   tgaccgcgtt actcccacag gtgagcgggc gggacggccc ttctcctccg ggctgtaatt 1140
   agctgagcaa gaggtaaggg tttaagggat ggttggttgg tggggtatta atgtttaatt 1200
   acctggagca cctgcctgaa atcacttttt ttcaggttgg accggtgcca ccatgtaccc 1260
   atacgatgtt ccagattacg cttcgccgaa gaaaaagcgc aaggtcgaag cgtccgacaa 1320
   gaagtacagc atcggcctgg ccatcggcac caactctgtg ggctgggccg tgatcaccga 1380
   cgagtacaag gtgcccagca agaaattcaa ggtgctgggc aacaccgacc ggcacagcat 1440
   caagaagaac ctgatcggag ccctgctgtt cgacagcggc gaaacagccg aggccacccg 1500
   gctgaagaga accgccagaa gaagatacac cagacggaag aaccggatct gctatctgca 1560
   agagatcttc agcaacgaga tggccaaggt ggacgacagc ttcttccaca gactggaaga 1620
   gtccttcctg gtggaagagg ataagaagca cgagcggcac cccatcttcg gcaacatcgt 1680
   ggacgaggtg gcctaccacg agaagtaccc caccatctac cacctgagaa agaaactggt 1740
   ggacagcacc gacaaggccg acctgcggct gatctatctg gccctggccc acatgatcaa 1800
   gttccggggc cacttcctga tcgagggcga cctgaacccc gacaacagcg acgtggacaa 1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta     7440
   ttgctgataa atctggagcc ggtgagcgtg gaagccgcgg tatcattgca gcactggggc     7500
   cagatggtaa gccctcccgt atcgtagtta tctacacgac ggggagtcag gcaactatgg     7560
   atgaacgaaa tagacagatc gctgagatag gtgcctcact gattaagcat tggtaactgt     7620
   cagaccaagt ttactcatat atactttaga ttgatttaaa acttcatttt taatttaaaa     7680
   ggatctaggt gaagatcctt tttgataatc tcatgaccaa aatcccttaa cgtgagtttt     7740
   cgttccactg agcgtcagac cccgtagaaa agatcaaagg atcttcttga gatccttttt     7800
   ttctgcgcgt aatctgctgc ttgcaaacaa aaaaaccacc gctaccagcg gtggtttgtt     7860
   tgccggatca agagctacca actctttttc cgaaggtaac tggcttcagc agagcgcaga     7920
   taccaaatac tgtccttcta gtgtagccgt agttaggcca ccacttcaag aactctgtag     7980
   caccgcctac atacctcgct ctgctaatcc tgttaccagt ggctgctgcc agtggcgata     8040
   agtcgtgtct taccgggttg gactcaagac gatagttacc ggataaggcg cagcggtcgg     8100
   gctgaacggg gggttcgtgc acacagccca gcttggagcg aacgacctac accgaactga     8160
   gatacctaca gcgtgagcta tgagaaagcg ccacgcttcc cgaagggaga aaggcggaca     8220
   ggtatccggt aagcggcagg gtcggaacag gagagcgcac gagggagctt ccagggggaa     8280
   acgcctggta tctttatagt cctgtcgggt ttcgccacct ctgacttgag cgtcgatttt     8340
   tgtgatgctc gtcagggggg cggagcctat ggaaaaacgc cagcaacgcg gcctttttac     8400
   ggttcctggc cttttgctgg ccttttgctc acatgt                              8436
<210> 100
   <211> 874
   <212> DNA
   <213> Artificial Sequence
<220>
   <223> synthetic DNA
<400> 100
   catttcagat tcgaaccatg acacatttcc atagcagctg tgccatttta caccacccac      60
   cccaatgttc caccaggctt ctatgaaacc actgattata taagtatttt cctgattaga     120
   gtacacgttc atgatagatc atttagaaaa ctcaaaaaag caaaatgacg aaattctctt     180
   tattctaatt acggagccat gacctgaatt catattactc ccgggagaaa aggaaaagcc     240
   ggtttttttc tcaggcaatt ctgtcattgt gtttgatagc atggtccaat aatagtgctg     300
   gcgcctgccc tgcctggacc ttgtcctcag attcctccgt cctggctgtc cccacgaccc     360
   tatcttctga aaaggtcatg cagttaacca ggcttctgaa tcatatttgc tggttgcttt     420
   caggagtgtc acgggtgctg tgtggagctg tcacattctt gggcatctgt ttccgtattt     480
   ttaaagccac ctcaacgttt gatagaaaag cataaaactt ttgaggaagt gagctctaca     540
   aacccaccac ccacgtaaga actgagccgt cctgcccaga gggcatctga aaaagagatc     600
   aacaggaaaa gggtggggaa atcctgcctt ccttcccgtc ttcaagtacc cattggcttc     660
   agacctttat tctcattggt ttatctggat gtcgctccac gctggctttc cagaaccagc     720
   cactgagagt cacaaagttg ggatttaaaa gatgcggtgg gaggggcggg tgattttttg    780
   aactgctggc gaccgagttc tggcggtcct gacgcgcacc ttgggcttgc tcgcgttcgg     840
   tttcggcagc aggaccaaga aggcttaggg aagg                                  874
<210> 101
   <211> 42
   <212> DNA
   <213> Artificial Sequence
<220>
   <223> oligonucleotide
<400> 101
   aattcctgca ggcatttcag attcgaacca tgacacattt cc 42
<210> 102
   <211> 37
   <212> DNA
   <213> Artificial Sequence
<220>
   <223> oligonucleotide
<400> 102
   ttactagtcc ttccctaagc cttcttggtc ctgctgc 37
<210> 103
   <211> 825
   <212> DNA
   <213> Artificial Sequence
<220>
   <223> synthetic DNA
<400> 103
   gcccgcgtcg caaaccgcag accccaagga ggagaccctc cagcccggcg cctggaccct      60
   cgcgacagag ctccagtgta ggtaagcgag cctgccctgc acctccgagg ccggtgcggt     120
   catcctgtgg ggccgggcct gtagagggac ggttcctggt cacggcttgc cgctaacctc     180
   gcgtaggggt aatggttttt ttgtttattt gtttgtttgt ttgcttgttt gttcctgacc     240
   ggtttacagc agggagaaaa acgaaatgca gacaagacaa ggagttggag caaagacaaa     300
   ctcattctga cggacacggg gtcttttttt gcatttattt actttgcatg tattttgcat     360
   ttattttgtg ttcgtgctct ggcgacggcc agaagagagc gtcggaaccc cggggccgga     420
   gttacagttg tgagctgccc ttttttttag agtgctagga agagaactcg gcccccctgg      480
   aagagcagcc tgggctatta acagctgaac cagctttcta actcctactc tttattattt     540
   atttgcagca ctagagatgg aacccaggac tccgtgtatg ttaggcaagg gttctactac     600
   agaactatat ccccaaccct ctccccgacc cttttttttt taagacggga actcggtatg     660
   tatcggtgtg tagccttggc ttgctgacct gaaacgtgct atgtagacta ggctagccta     720
   gagctcacag atctctgcct gtgttgggga tggttgtatg cagtgtgtat tcaggaacta     780
   tttctgagat ctggttacaa tccagacact gcctcaatgt gatgt                    825
<210> 104
   <211> 23
   <212> DNA
   <213> Artificial Sequence
<220>
   <223> oligonucleotide
<400> 104
   agccatgggc ccgcgtcgca aac 23
<210> 105
   <211> 34
   <212> DNA
   <213> Artificial Sequence
<220>
   <223> oligonucleotide
<400> 105
   aattgcatgc acatcacatt gaggcagtgt ctgg 34
<210> 106
   <211> 8358
   <212> DNA
   <213> Artificial Sequence
<220>
   <223> synthetic DNA
<400> 106
   tatagtgagt cgtattacaa ttcactggcc gtcgttttac aacgtcgtga ctgggaaaac      60
   cctggcgtta cccaacttaa tcgccttgca gcacatcccc ctttcgccag ctggcgtaat     120
   agcgaagagg cccgcaccga tcgcccttcc caacagttgc gcagcctgaa tggcgaatgg     180
   acgcgccctg tagcggcgca ttaagcgcgg cgggtgtggt ggttacgcgc agcgtgaccg     240
   ctacacttgc cagcgcccta gcgcccgctc ctttcgcttt cttcccttcc tttctcgcca     300
   cgttcgccgg ctttccccgt caagctctaa atcgggggct ccctttaggg ttccgattta     360
   gtgctttacg gcacctcgac cccaaaaaac ttgattaggg tgatggttca cgtagtgggc     420
   catcgccctg atagacggtt tttcgccctt tgacgttgga gtccacgttc tttaatagtg     480
   gactcttgtt ccaaactgga acaacactca accctatctc ggtctattct tttgatttat 540
   aagggatttt gccgatttcg gcctattggt taaaaaatga gctgatttaa caaaaattta 600
   acgcgaattt taacaaaata ttaacgctta caatttcctg atgcggtatt ttctccttac 660
   gcatctgtgc ggtatttcac accgcatcag gtggcacttt tcggggaaat gtgcgcggaa 720
   cccctatttg tttatttttc taaatacatt caaatatgta tccgctcatg agacaataac 780
   cctgataaat gcttcaataa tattgaaaaa ggaagagtat gagtattcaa catttccgtg 840
   tcgcccttat tccctttttt gcggcatttt gccttcctgt ttttgctcac ccagaaacgc 900
   tggtgaaagt aaaagatgct gaagatcagt tgggtgcacg agtgggttac atcgaactgg 960
   atctcaacag cggtaagatc cttgagagtt ttcgccccga agaacgtttt ccaatgatga 1020
   gcacttttaa agttctgcta tgtggcgcgg tattatcccg tattgacgcc gggcaagagc 1080
   aactcggtcg ccgcatacac tattctcaga atgacttggt tgagtactca ccagtcacag 1140
   aaaagcatct tacggatggc atgacagtaa gagaattatg cagtgctgcc ataaccatga 1200
   gtgataacac tgcggccaac ttacttctga caacgatcgg aggaccgaag gagctaaccg 1260
   cttttttgca caacatgggg gatcatgtaa ctcgccttga tcgttgggaa ccggagctga 1320
   atgaagccat accaaacgac gagcgtgaca ccacgatgcc tgtagcaatg gcaacaacgt 1380
   tgcgcaaact attaactggc gaactactta ctctagcttc ccggcaacaa ttaatagact 1440
   ggatggaggc ggataaagtt gcaggaccac ttctgcgctc ggcccttccg gctggctggt 1500
   ttattgctga taaatctgga gccggtgagc gtgggtctcg cggtatcatt gcagcactgg 1560
   ggccagatgg taagccctcc cgtatcgtag ttatctacac gacggggagt caggcaacta 1620
   tggatgaacg aaatagacag atcgctgaga taggtgcctc actgattaag cattggtaac 1680
   tgtcagacca agtttactca tatatacttt agattgattt aaaacttcat ttttaattta 1740
   aaaggatcta ggtgaagatc ctttttgata atctcatgac caaaatccct taacgtgagt 1800
   tttcgttcca ctgagcgtca gaccccgtag aaaagatcaa aggatcttct tgagatcctt 1860
   tttttctgcg cgtaatctgc tgcttgcaaa caaaaaaacc accgctacca gcggtggttt 1920
   gtttgccgga tcaagagcta ccaactcttt ttccgaaggt aactggcttc agcagagcgc 1980
   agataccaaa tactgttctt ctagtgtagc cgtagttagg ccaccacttc aagaactctg 2040
   tagcaccgcc tacatacctc gctctgctaa tcctgttacc agtggctgct gccagtggcg 2100
   ataagtcgtg tcttaccggg ttggactcaa gacgatagtt accggataag gcgcagcggt 2160
   cgggctgaac ggggggttcg tgcacacagc ccagcttgga gcgaacgacc tacaccgaac 2220
   tgagatacct acagcgtgag ctatgagaaa gcgccacgct tcccgaaggg agaaaggcgg 2280
   acaggtatcc ggtaagcggc agggtcggaa caggagagcg cacgagggag cttccagggg 2340
   gaaacgcctg gtatctttat agtcctgtcg ggtttcgcca cctctgactt gagcgtcgat 2400
   ttttgtgatg ctcgtcaggg gggcggagcc tatggaaaaa cgccagcaac gcggcctttt 2460
   tacggttcct ggccttttgc tggccttttg ctcacatgtt ctttcctgcg ttatcccctg 2520
   attctgtgga taaccgtatt accgcctttg agtgagctga taccgctcgc cgcagccgaa 2580
   cgaccgagcg cagcgagtca gtgagcgagg aagcggaaga gcgcccaata cgcaaaccgc 2640
   ctctccccgc gcgttggccg attcattaat gcagctggca cgacaggttt cccgactgga 2700
   aagcgggcag tgagcgcaac gcaattaatg tgagttagct cactcattag gcaccccagg 2760
   ctttacactt tatgcttccg gctcgtatgt tgtgtggaat tgtgagcgga taacaatttc 2820
   acacaggaaa cagctatgac catgattacg ccaagctatt taggtgacac tatagaatac 2880
   tcaagctatg catccaacgc gttgggagct ctcccatatg gtcgacctgc aggcatttca 2940
   gattcgaacc atgacacatt tccatagcag ctgtgccatt ttacaccacc caccccaatg 3000
   ttccaccagg cttctatgaa accactgatt atataagtat tttcctgatt agagtacacg 3060
   ttcatgatag atcatttaga aaactcaaaa aagcaaaatg acgaaattct ctttattcta 3120
   attacggagc catgacctga attcatatta ctcccgggag aaaaggaaaa gccggttttt 3180
   ttctcaggca attctgtcat tgtgtttgat agcatggtcc aataatagtg ctggcgcctg 3240
   ccctgcctgg accttgtcct cagattcctc cgtcctggct gtccccacga ccctatcttc 3300
   tgaaaaggtc atgcagttaa ccaggcttct gaatcatatt tgctggttgc tttcaggagt 3360
   gtcacgggtg ctgtgtggag ctgtcacatt cttgggcatc tgtttccgta tttttaaagc 3420
   cacctcaacg tttgatagaa aagcataaaa cttttgagga agtgagctct acaaacccac 3480
   cacccacgta agaactgagc cgtcctgccc agagggcatc tgaaaaagag atcaacagga 3540
   aaagggtggg gaaatcctgc cttccttccc gtcttcaagt acccattggc ttcagacctt 3600
   tattctcatt ggtttatctg gatgtcgctc cacgctggct ttccagaacc agccactgag 3660
   agtcacaaag ttgggattta aaagatgcgg tgggaggggc gggtgatttt ttgaactgct 3720
   ggcgaccgag ttctggcggt cctgacgcgc accttgggct tgctcgcgtt cggtttcggc 3780
   agcaggacca agaaggctta gggaaggact agtgattacc gttcgtatag catacattat 3840
   acgaagttat accatgtcta gactggacaa gagcaaagtc ataaacggcg ctctggaatt 3900
   actcaatgga gtcggtatcg aaggcctgac gacaaggaaa ctcgctcaaa agctgggagt 3960
   tgagcagcct accctgtact ggcacgtgaa gaacaagcgg gccctgctcg atgccctgcc 4020
   aatcgagatg ctggacaggc atcataccca cttctgcccc ctggaaggcg agtcatggca 4080
   agactttctg cggaacaacg ccaagtcatt ccgctgtgct ctcctctcac atcgcgacgg 4140
   ggctaaagtg catctcggca cccgcccaac agagaaacag tacgaaaccc tggaaaatca 4200
   gctcgcgttc ctgtgtcagc aaggcttctc cctggagaac gcactgtacg ctctgtccgc 4260
   cgtgggccac tttacactgg gctgcgtatt ggaggaacag gagcatcaag tagcaaaaga 4320
   ggaaagagag acacctacca ccgattctat gcccccactt ctgagacaag caattgagct 4380
   gttcgaccgg cagggagccg aacctgcctt ccttttcggc ctggaactaa tcatatgtgg 4440
   cctggagaaa cagctaaagt gcgaaagcgg cgggccggcc gacgcccttg acgattttga 4500
   cttagacatg ctcccagccg atgcccttga cgactttgac cttgatatgc tgcctgctga 4560
   cgctcttgac gattttgacc ttgacatgct ccccgggtaa ctaagtaagg atcccaaata 4620
   ccactgaatt aagaattccg cccctctccc tccccccccc ctaacgttac tggccgaagc 4680
   cgcttggaat aaggccggtg tgcgtttgtc tatatgttat tttccaccat attgccgtct 4740
   tttggcaatg tgagggcccg gaaacctggc cctgtcttct tgacgagcat tcctaggggt 4800
   ctttcccctc tcgccaaagg aatgcaaggt ctgttgaatg tcgtgaagga agcagttcct 4860
   ctggaagctt cttgaagaca aacaacgtct gtagcgaccc tttgcaggca gcggaacccc 4920
   ccacctggcg acaggtgcct ctgcggccaa aagccacgtg tataagatac acctgcaaag 4980
   gcggcacaac cccagtgcca cgttgtgagt tggatagttg tggaaagagt caaatggctc 5040
   tcctcaagcg tattcaacaa ggggctgaag gatgcccaga aggtacccca ttgtatggga 5100
   tctgatctgg ggcctcggtg cacatgcttt acatgtgttt agtcgaggtt aaaaaacgtc 5160
   taggcccccc gaaccacggg gacgtggttt tcctttgaaa aacacgatga taatatggcc 5220
   acaaccatga ccgagtacaa gcccacggtg cgcctcgcca cccgcgacga cgtcccccgg 5280
   gccgtacgca ccctcgccgc cgcgttcgcc gactaccccg ccacgcgcca caccgtcgac 5340
   ccggaccgcc acatcgagcg ggtcaccgag ctgcaagaac tcttcctcac gcgcgtcggg 5400
   ctcgacatcg gcaaggtgtg ggtcgcggac gacggcgccg cggtggcggt ctggaccacg 5460
   ccggagagcg tcgaagcggg ggcggtgttc gccgagatcg gcccgcgcat ggccgagttg 5520
   agcggttccc ggctggccgc gcagcaacag atggaaggcc tcctggcgcc gcaccggccc 5580
   aaggagcccg cgtggttcct ggccaccgtc ggcgtctcgc ccgaccacca gggcaagggt 5640
   ctgggcagcg ccgtcgtgct ccccggagtg gaggcggccg agcgcgccgg ggtgcccgcc 5700
   ttcctggaga cctccgcgcc ccgcaacctc cccttctacg agcggctcgg cttcaccgtc 5760
   accgccgacg tcgaggtgcc cgaaggaccg cgcacctggt gcatgacccg caagcccggt 5820
   gcctgaggat ccagacatga taagatacat tgatgagttt ggacaaacca caactagaat 5880
   gcagtgaaaa aaatgcttta tttgtgaaat ttgtgatgct attgctttat ttgtaaccat 5940
   tataagctgc aataaacaag ttaacaacaa caattgcatt cattttatgt ttcaggttca 6000
   gggggaggtg tgggaggttt tttaaagcaa gtaaaacctc tacaaatgtg gtatggctga 6060
   ttatgatcct ctagagtcgc agatccagac atgataagat acattgatga gtttggacaa 6120
   accacaacta gaatgcagtg aaaaaaatgc tttatttgtg aaatttgtga tgctattgct 6180
   ttatttgtaa ccattataag ctgcaataaa caagttaaca acaacaattg cattcatttt 6240
   atgtttcagg ttcaggggga ggtgtgggag gttttttaaa gcaagtaaaa cctctacaaa 6300
   tgtggtatgg ctgattatga tcctctagag tcgcagatcc agacatgata agatacattg 6360
   atgagtttgg acaaaccaca actagaatgc agtgaaaaaa atgctttatt tgtgaaattt 6420
   gtgatgctat tgctttattt gtaaccatta taagctgcaa taaacaagtt aacaacaaca 6480
   attgcattca ttttatgttt caggttcagg gggaggtgtg ggaggttttt taaagcaagt 6540
   aaaacctcta caaatgtggt atggctgatt atgatcctct agagtcgcag atcctctaga 6600
   gtcgcagatc tttttccctc tgccaaaaat tatggggaca tcatgaagcc ccttgagcat 6660
   ctgacttctg gctaataaag gaaatttatt ttcattgcaa tagtgtgttg gaattttttg 6720
   tgtctctcac tcggaaggac atatgggagg gcaaatcatt taaaacatca gaatgagtat 6780
   ttggtttaga gtttggcaac atatgccata tgctggctgc catgaacaaa ggtggctata 6840
   aagaggtcat cagtatatga aacagccccc tgctgtccat tccttattcc atagaaaagc 6900
   cttgacttga ggttagattt tttttatatt ttgttttgtg ttattttttt ctttaacatc 6960
   cctaaaattt tccttacatg ttttactagc cagatttttc ctcctctcct gactactccc 7020
   agtcatagct gtccctcttc tcttatgaag atccctcgac ctgcagccca agcttatcga 7080
   attcccgcgg tggcggccgc acgtctccct atcagtgata gagaagtcga cacgtctcga 7140
   gctccctatc agtgatagag aaggtacgtc tagaacgtct ccctatcagt gatagagaag 7200
   tcgacacgtc tcgagctccc tatcagtgat agagaaggta cgtctagaac gtctccctat 7260
   cagtgataga gaagtcgaca cgtctcgagc tccctatcag tgatagagaa ggtacgtcta 7320
   gaacgtctcc ctatcagtga tagagaagtc gacacgtctc gagctcccta tcagtgatag 7380
   agaaggtacc ccctatataa gcagagctcg tttagtgaac cgtcagatcg cctggagacg 7440
   ccatccacgc tgttttgacc tccatagaag acaccgggac cgatccagcc tccgcggccg 7500
   ccatgggccc gcgtcgcaaa ccgcagaccc caaggaggag accctccagc ccggcgcctg 7560
   gaccctcgcg acagagctcc agtgtaggta agcgagcctg ccctgcacct ccgaggccgg 7620
   tgcggtcatc ctgtggggcc gggcctgtag agggacggtt cctggtcacg gcttgccgct 7680
   aacctcgcgt aggggtaatg gtttttttgt ttatttgttt gtttgtttgc ttgtttgttc 7740
   ctgaccggtt tacagcaggg agaaaaacga aatgcagaca agacaaggag ttggagcaaa 7800
   gacaaactca ttctgacgga cacggggtct ttttttgcat ttatttactt tgcatgtatt 7860
   ttgcatttat tttgtgttcg tgctctggcg acggccagaa gagagcgtcg gaaccccggg 7920
   gccggagtta cagttgtgag ctgccctttt ttttagagtg ctaggaagag aactcggccc 7980
   ccctggaaga gcagcctggg ctattaacag ctgaaccagc tttctaactc ctactcttta 8040
   ttatttattt gcagcactag agatggaacc caggactccg tgtatgttag gcaagggttc 8100
   tactacagaa ctatatcccc aaccctctcc ccgacccttt tttttttaag acgggaactc 8160
   ggtatgtatc ggtgtgtagc cttggcttgc tgacctgaaa cgtgctatgt agactaggct 8220
   agcctagagc tcacagatct ctgcctgtgt tggggatggt tgtatgcagt gtgtattcag 8280
   gaactatttc tgagatctgg ttacaatcca gacactgcct caatgtgatg tgcatgcgac 8340
   gtcgggccca attcgccc 8358
<210> 107
   <211> 24
   <212> DNA
   <213> Artificial Sequence
<220>
   <223> oligonucleotide
<400> 107
   caccgtcgca aaccgcagac ccca 24
<210> 108
   <211> 24
   <212> DNA
   <213> Artificial Sequence
<220>
   <223> oligonucleotide
<400> 108
   aaactggggt ctgcggtttg cgac 24
<210> 109
   <211> 24
   <212> DNA
   <213> Artificial Sequence
<220>
   <223> oligonucleotide
<400> 109
   caccgtggct ggcctcgagg ggcc 24
<210> 110
   <211> 24
   <212> DNA
   <213> Artificial Sequence
<220>
   <223> oligonucleotide
<400> 110
   aaacggcccc tcgaggccag ccac 24
<210> 111
   <211> 8436
   <212> DNA
   <213> Artificial Sequence
<220>
   <223> synthetic DNA
<400> 111
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg tcgcaaaccg cagaccccag ttttagagct agaaatagca agttaaaata 300
   aggctagtcc gttatcaact tgaaaaagtg gcaccgagtc ggtgcttttt tgttttagag 360
   ctagaaatag caagttaaaa taaggctagt ccgtttttag cgcgtgcgcc aattctgcag 420
   acaaatggct ctagaggtac ccgttacata acttacggta aatggcccgc ctggctgacc 480
   gcccaacgac ccccgcccat tgacgtcaat agtaacgcca atagggactt tccattgacg 540
   tcaatgggtg gagtatttac ggtaaactgc ccacttggca gtacatcaag tgtatcatat 600
   gccaagtacg ccccctattg acgtcaatga cggtaaatgg cccgcctggc attgtgccca 660
   gtacatgacc ttatgggact ttcctacttg gcagtacatc tacgtattag tcatcgctat 720
   taccatggtc gaggtgagcc ccacgttctg cttcactctc cccatctccc ccccctcccc 780
   acccccaatt ttgtatttat ttatttttta attattttgt gcagcgatgg gggcgggggg 840
   gggggggggg cgcgcgccag gcggggcggg gcggggcgag gggcggggcg gggcgaggcg 900
   gagaggtgcg gcggcagcca atcagagcgg cgcgctccga aagtttcctt ttatggcgag 960
   gcggcggcgg cggcggccct ataaaaagcg aagcgcgcgg cgggcgggag tcgctgcgac 1020
   gctgccttcg ccccgtgccc cgctccgccg ccgcctcgcg ccgcccgccc cggctctgac 1080
   tgaccgcgtt actcccacag gtgagcgggc gggacggccc ttctcctccg ggctgtaatt 1140
   agctgagcaa gaggtaaggg tttaagggat ggttggttgg tggggtatta atgtttaatt 1200
   acctggagca cctgcctgaa atcacttttt ttcaggttgg accggtgcca ccatgtaccc 1260
   atacgatgtt ccagattacg cttcgccgaa gaaaaagcgc aaggtcgaag cgtccgacaa 1320
   gaagtacagc atcggcctgg ccatcggcac caactctgtg ggctgggccg tgatcaccga 1380
   cgagtacaag gtgcccagca agaaattcaa ggtgctgggc aacaccgacc ggcacagcat 1440
   caagaagaac ctgatcggag ccctgctgtt cgacagcggc gaaacagccg aggccacccg 1500
   gctgaagaga accgccagaa gaagatacac cagacggaag aaccggatct gctatctgca 1560
   agagatcttc agcaacgaga tggccaaggt ggacgacagc ttcttccaca gactggaaga 1620
   gtccttcctg gtggaagagg ataagaagca cgagcggcac cccatcttcg gcaacatcgt 1680
   ggacgaggtg gcctaccacg agaagtaccc caccatctac cacctgagaa agaaactggt 1740
   ggacagcacc gacaaggccg acctgcggct gatctatctg gccctggccc acatgatcaa 1800
   gttccggggc cacttcctga tcgagggcga cctgaacccc gacaacagcg acgtggacaa 1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta 7440
   ttgctgataa atctggagcc ggtgagcgtg gaagccgcgg tatcattgca gcactggggc 7500
   cagatggtaa gccctcccgt atcgtagtta tctacacgac ggggagtcag gcaactatgg 7560
   atgaacgaaa tagacagatc gctgagatag gtgcctcact gattaagcat tggtaactgt 7620
   cagaccaagt ttactcatat atactttaga ttgatttaaa acttcatttt taatttaaaa 7680
   ggatctaggt gaagatcctt tttgataatc tcatgaccaa aatcccttaa cgtgagtttt 7740
   cgttccactg agcgtcagac cccgtagaaa agatcaaagg atcttcttga gatccttttt 7800
   ttctgcgcgt aatctgctgc ttgcaaacaa aaaaaccacc gctaccagcg gtggtttgtt 7860
   tgccggatca agagctacca actctttttc cgaaggtaac tggcttcagc agagcgcaga 7920
   taccaaatac tgtccttcta gtgtagccgt agttaggcca ccacttcaag aactctgtag 7980
   caccgcctac atacctcgct ctgctaatcc tgttaccagt ggctgctgcc agtggcgata 8040
   agtcgtgtct taccgggttg gactcaagac gatagttacc ggataaggcg cagcggtcgg 8100
   gctgaacggg gggttcgtgc acacagccca gcttggagcg aacgacctac accgaactga 8160
   gatacctaca gcgtgagcta tgagaaagcg ccacgcttcc cgaagggaga aaggcggaca 8220
   ggtatccggt aagcggcagg gtcggaacag gagagcgcac gagggagctt ccagggggaa 8280
   acgcctggta tctttatagt cctgtcgggt ttcgccacct ctgacttgag cgtcgatttt 8340
   tgtgatgctc gtcagggggg cggagcctat ggaaaaacgc cagcaacgcg gcctttttac 8400
   ggttcctggc cttttgctgg ccttttgctc acatgt 8436
<210> 112
   <211> 8436
   <212> DNA
   <213> Artificial Sequence
<220>
   <223> synthetic DNA
<400> 112
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccg tggctggcct cgaggggccg ttttagagct agaaatagca agttaaaata     300
   aggctagtcc gttatcaact tgaaaaagtg gcaccgagtc ggtgcttttt tgttttagag     360
   ctagaaatag caagttaaaa taaggctagt ccgtttttag cgcgtgcgcc aattctgcag     420
   acaaatggct ctagaggtac ccgttacata acttacggta aatggcccgc ctggctgacc     480
   gcccaacgac ccccgcccat tgacgtcaat agtaacgcca atagggactt tccattgacg 540
   tcaatgggtg gagtatttac ggtaaactgc ccacttggca gtacatcaag tgtatcatat 600
   gccaagtacg ccccctattg acgtcaatga cggtaaatgg cccgcctggc attgtgccca 660
   gtacatgacc ttatgggact ttcctacttg gcagtacatc tacgtattag tcatcgctat 720
   taccatggtc gaggtgagcc ccacgttctg cttcactctc cccatctccc ccccctcccc 780
   acccccaatt ttgtatttat ttatttttta attattttgt gcagcgatgg gggcgggggg 840
   gggggggggg cgcgcgccag gcggggcggg gcggggcgag gggcggggcg gggcgaggcg 900
   gagaggtgcg gcggcagcca atcagagcgg cgcgctccga aagtttcctt ttatggcgag 960
   gcggcggcgg cggcggccct ataaaaagcg aagcgcgcgg cgggcgggag tcgctgcgac 1020
   gctgccttcg ccccgtgccc cgctccgccg ccgcctcgcg ccgcccgccc cggctctgac 1080
   tgaccgcgtt actcccacag gtgagcgggc gggacggccc ttctcctccg ggctgtaatt 1140
   agctgagcaa gaggtaaggg tttaagggat ggttggttgg tggggtatta atgtttaatt 1200
   acctggagca cctgcctgaa atcacttttt ttcaggttgg accggtgcca ccatgtaccc 1260
   atacgatgtt ccagattacg cttcgccgaa gaaaaagcgc aaggtcgaag cgtccgacaa 1320
   gaagtacagc atcggcctgg ccatcggcac caactctgtg ggctgggccg tgatcaccga 1380
   cgagtacaag gtgcccagca agaaattcaa ggtgctgggc aacaccgacc ggcacagcat 1440
   caagaagaac ctgatcggag ccctgctgtt cgacagcggc gaaacagccg aggccacccg 1500
   gctgaagaga accgccagaa gaagatacac cagacggaag aaccggatct gctatctgca 1560
   agagatcttc agcaacgaga tggccaaggt ggacgacagc ttcttccaca gactggaaga 1620
   gtccttcctg gtggaagagg ataagaagca cgagcggcac cccatcttcg gcaacatcgt 1680
   ggacgaggtg gcctaccacg agaagtaccc caccatctac cacctgagaa agaaactggt 1740
   ggacagcacc gacaaggccg acctgcggct gatctatctg gccctggccc acatgatcaa 1800
   gttccggggc cacttcctga tcgagggcga cctgaacccc gacaacagcg acgtggacaa 1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta 7440
   ttgctgataa atctggagcc ggtgagcgtg gaagccgcgg tatcattgca gcactggggc 7500
   cagatggtaa gccctcccgt atcgtagtta tctacacgac ggggagtcag gcaactatgg 7560
   atgaacgaaa tagacagatc gctgagatag gtgcctcact gattaagcat tggtaactgt 7620
   cagaccaagt ttactcatat atactttaga ttgatttaaa acttcatttt taatttaaaa 7680
   ggatctaggt gaagatcctt tttgataatc tcatgaccaa aatcccttaa cgtgagtttt 7740
   cgttccactg agcgtcagac cccgtagaaa agatcaaagg atcttcttga gatccttttt 7800
   ttctgcgcgt aatctgctgc ttgcaaacaa aaaaaccacc gctaccagcg gtggtttgtt 7860
   tgccggatca agagctacca actctttttc cgaaggtaac tggcttcagc agagcgcaga 7920
   taccaaatac tgtccttcta gtgtagccgt agttaggcca ccacttcaag aactctgtag 7980
   caccgcctac atacctcgct ctgctaatcc tgttaccagt ggctgctgcc agtggcgata 8040
   agtcgtgtct taccgggttg gactcaagac gatagttacc ggataaggcg cagcggtcgg 8100
   gctgaacggg gggttcgtgc acacagccca gcttggagcg aacgacctac accgaactga 8160
   gatacctaca gcgtgagcta tgagaaagcg ccacgcttcc cgaagggaga aaggcggaca 8220
   ggtatccggt aagcggcagg gtcggaacag gagagcgcac gagggagctt ccagggggaa 8280
   acgcctggta tctttatagt cctgtcgggt ttcgccacct ctgacttgag cgtcgatttt 8340
   tgtgatgctc gtcagggggg cggagcctat ggaaaaacgc cagcaacgcg gcctttttac 8400
   ggttcctggc cttttgctgg ccttttgctc acatgt 8436
<210> 113
   <211> 29
   <212> DNA
   <213> Artificial Sequence
<220>
   <223> oligonucleotide
<400> 113
   tctggaactc actatgtagg tctactggc 29
<210> 114
   <211> 22
   <212> DNA
   <213> Artificial Sequence
<220>
   <223> oligonucleotide
<400> 114
   ttgacctcca tagaagacac cg 22
<210> 115
   <211> 30
   <212> DNA
   <213> Artificial Sequence
<220>
   <223> oligonucleotide
<400> 115
   ctcttcttct cacctggtct cttcaggacc 30
<210> 116
   <211> 24
   <212> DNA
   <213> Artificial Sequence
<220>
   <223> oligonucleotide
<400> 116
   acctcctctc cttacatgct ggtc 24
<210> 117
   <211> 23
   <212> DNA
   <213> Artificial Sequence
<220>
   <223> oligonucleotide
<400> 117
   tacattccac tggcagctta ggg 23
<210> 118
   <211> 617
   <212> DNA
   <213> Artificial Sequence
<220>
   <223> synthetic DNA
<400> 118
   ggagctccgg cgctgcccca gatctggcag ctgtacctca agaactaccg catcgccacc        60
   ttcaagaact ggcccttcct ggaggactgc gcctgcaccc cagagcgagt gagtcccagc       120
   ttccagcgac tccaccctgg gcccgacggt gccttagttg tgtgacccgg gatcagaacc       180
   cgctatcccc accctcgctt cgtactgtta gccaggctgc tttgctctgc tctgtcctcc       240
   acgaggccac cccccccccc cgctctcccc accctctgcc tgaaccactg ttagcctccg       300
   ggcctcttcc cactgtcctc agttctcggc ccggaacgat ctctgggaat catgggataa       360
   ggtcagactg ttgatcccgc tcacgagctg tcattttcag atggcggagg ctggcttcat       420
   ccactgccct accgagaacg agcctgattt ggcccagtgt tttttctgct ttaaggaatt       480
   ggaaggctgg gaacccgatg acaacccgat gtaagtccca caggctactc tcggtgggtt       540
   tcctggtgtt ccccttccat tgagagcctt ttgggttgaa ctggagttgg gacaaaccat       600
   ttgtatttag tagaaga                                                      617
<210> 119
   <211> 31
   <212> DNA
   <213> Artificial Sequence
<220>
   <223> oligonucleotide
<400> 119
   caccatggga gctccggcgc tgccccagat c 31
<210> 120
   <211> 34
   <212> DNA
   <213> Artificial Sequence
<220>
   <223> oligonucleotide
<400> 120
   taactggaac acttgagcaa gaagcatgct cttc 34
<210> 121
   <211> 775
   <212> DNA
   <213> Artificial Sequence
<220>
   <223> synthetic DNA
<400> 121
   cagtgtgaag ctctttagaa gccactgcaa ccacaggccg cccgacagga acagagacac      60
   tgaaaacggg cccgcagcaa ggcaggctca gcagccaaca gtcacaccca ggaagcagta     120
   tttttcttct gctcctggac tctcttgcgg tgtatggctg cttccctttg gtctgagcca     180
   ggccgatggt ctcagaaata gacacccatt gactttcttt tccagcgctg ggacatacag     240
   accccgcctc catcccaggg tgtctatagg aaggatggcg gctgctgcag ggaggagggt     300
   ctcctgtctt cctaagggcg cccctccacc agcctgtggg tgggtccgag gcacttccat     360
   tccgatatct agctggccaa atcctgcaaa ccttgaggca ggaagaacct gcagagcaca     420
   tgggacttgc agcggacatg ctttaaagag gtgccccagg cccgtccacc gccctcggcc     480
   accctccgtg tcctctgggg agcagctgcg gaagattcga gtcagaatag caagaaggaa     540
   ccgcagcaga aggtacaact cccagcatgc cctgcgcccg ccacgcccac aaggccaggc     600
   gcagatgggc gtggggcggg actttcccgg ctcgcctcgc gccgtccact cccagaaggc     660
   agcgggcgag ggcgtggggc cggggctctc ccggcatgct ctgcggcgcg cctccgcccg     720
   cgcgatttga atcctgcgtt tgagtcgtct tggcggaggt tgtggtgacg ccatc          775
<210> 122
   <211> 39
   <212> DNA
   <213> Artificial Sequence
<220>
   <223> oligonucleotide
<400> 122
   aattcctgca ggcagtgtga agctctttag aagccactg 39
<210> 123
   <211> 35
   <212> DNA
   <213> Artificial Sequence
<220>
   <223> oligonucleotide
<400> 123
   ttactagtga tggcgtcacc acaacctccg ccaag 35
<210> 124
   <211> 8050
   <212> DNA
   <213> Artificial Sequence
<220>
   <223> synthetic DNA
<400> 124
   tatagtgagt cgtattacaa ttcactggcc gtcgttttac aacgtcgtga ctgggaaaac 60
   cctggcgtta cccaacttaa tcgccttgca gcacatcccc ctttcgccag ctggcgtaat 120
   agcgaagagg cccgcaccga tcgcccttcc caacagttgc gcagcctgaa tggcgaatgg 180
   acgcgccctg tagcggcgca ttaagcgcgg cgggtgtggt ggttacgcgc agcgtgaccg 240
   ctacacttgc cagcgcccta gcgcccgctc ctttcgcttt cttcccttcc tttctcgcca 300
   cgttcgccgg ctttccccgt caagctctaa atcgggggct ccctttaggg ttccgattta 360
   gtgctttacg gcacctcgac cccaaaaaac ttgattaggg tgatggttca cgtagtgggc 420
   catcgccctg atagacggtt tttcgccctt tgacgttgga gtccacgttc tttaatagtg 480
   gactcttgtt ccaaactgga acaacactca accctatctc ggtctattct tttgatttat 540
   aagggatttt gccgatttcg gcctattggt taaaaaatga gctgatttaa caaaaattta 600
   acgcgaattt taacaaaata ttaacgctta caatttcctg atgcggtatt ttctccttac 660
   gcatctgtgc ggtatttcac accgcatcag gtggcacttt tcggggaaat gtgcgcggaa 720
   cccctatttg tttatttttc taaatacatt caaatatgta tccgctcatg agacaataac 780
   cctgataaat gcttcaataa tattgaaaaa ggaagagtat gagtattcaa catttccgtg 840
   tcgcccttat tccctttttt gcggcatttt gccttcctgt ttttgctcac ccagaaacgc 900
   tggtgaaagt aaaagatgct gaagatcagt tgggtgcacg agtgggttac atcgaactgg 960
   atctcaacag cggtaagatc cttgagagtt ttcgccccga agaacgtttt ccaatgatga 1020
   gcacttttaa agttctgcta tgtggcgcgg tattatcccg tattgacgcc gggcaagagc 1080
   aactcggtcg ccgcatacac tattctcaga atgacttggt tgagtactca ccagtcacag 1140
   aaaagcatct tacggatggc atgacagtaa gagaattatg cagtgctgcc ataaccatga 1200
   gtgataacac tgcggccaac ttacttctga caacgatcgg aggaccgaag gagctaaccg 1260
   cttttttgca caacatgggg gatcatgtaa ctcgccttga tcgttgggaa ccggagctga 1320
   atgaagccat accaaacgac gagcgtgaca ccacgatgcc tgtagcaatg gcaacaacgt 1380
   tgcgcaaact attaactggc gaactactta ctctagcttc ccggcaacaa ttaatagact 1440
   ggatggaggc ggataaagtt gcaggaccac ttctgcgctc ggcccttccg gctggctggt 1500
   ttattgctga taaatctgga gccggtgagc gtgggtctcg cggtatcatt gcagcactgg 1560
   ggccagatgg taagccctcc cgtatcgtag ttatctacac gacggggagt caggcaacta 1620
   tggatgaacg aaatagacag atcgctgaga taggtgcctc actgattaag cattggtaac 1680
   tgtcagacca agtttactca tatatacttt agattgattt aaaacttcat ttttaattta 1740
   aaaggatcta ggtgaagatc ctttttgata atctcatgac caaaatccct taacgtgagt 1800
   tttcgttcca ctgagcgtca gaccccgtag aaaagatcaa aggatcttct tgagatcctt 1860
   tttttctgcg cgtaatctgc tgcttgcaaa caaaaaaacc accgctacca gcggtggttt 1920
   gtttgccgga tcaagagcta ccaactcttt ttccgaaggt aactggcttc agcagagcgc 1980
   agataccaaa tactgttctt ctagtgtagc cgtagttagg ccaccacttc aagaactctg 2040
   tagcaccgcc tacatacctc gctctgctaa tcctgttacc agtggctgct gccagtggcg 2100
   ataagtcgtg tcttaccggg ttggactcaa gacgatagtt accggataag gcgcagcggt 2160
   cgggctgaac ggggggttcg tgcacacagc ccagcttgga gcgaacgacc tacaccgaac 2220
   tgagatacct acagcgtgag ctatgagaaa gcgccacgct tcccgaaggg agaaaggcgg 2280
   acaggtatcc ggtaagcggc agggtcggaa caggagagcg cacgagggag cttccagggg 2340
   gaaacgcctg gtatctttat agtcctgtcg ggtttcgcca cctctgactt gagcgtcgat 2400
   ttttgtgatg ctcgtcaggg gggcggagcc tatggaaaaa cgccagcaac gcggcctttt 2460
   tacggttcct ggccttttgc tggccttttg ctcacatgtt ctttcctgcg ttatcccctg 2520
   attctgtgga taaccgtatt accgcctttg agtgagctga taccgctcgc cgcagccgaa 2580
   cgaccgagcg cagcgagtca gtgagcgagg aagcggaaga gcgcccaata cgcaaaccgc 2640
   ctctccccgc gcgttggccg attcattaat gcagctggca cgacaggttt cccgactgga 2700
   aagcgggcag tgagcgcaac gcaattaatg tgagttagct cactcattag gcaccccagg 2760
   ctttacactt tatgcttccg gctcgtatgt tgtgtggaat tgtgagcgga taacaatttc 2820
   acacaggaaa cagctatgac catgattacg ccaagctatt taggtgacac tatagaatac 2880
   tcaagctatg catccaacgc gttgggagct ctcccatatg gtcgacctgc aggcagtgtg 2940
   aagctcttta gaagccactg caaccacagg ccgcccgaca ggaacagaga cactgaaaac 3000
   gggcccgcag caaggcaggc tcagcagcca acagtcacac ccaggaagca gtatttttct 3060
   tctgctcctg gactctcttg cggtgtatgg ctgcttccct ttggtctgag ccaggccgat 3120
   ggtctcagaa atagacaccc attgactttc ttttccagcg ctgggacata cagaccccgc 3180
   ctccatccca gggtgtctat aggaaggatg gcggctgctg cagggaggag ggtctcctgt 3240
   cttcctaagg gcgcccctcc accagcctgt gggtgggtcc gaggcacttc cattccgata 3300
   tctagctggc caaatcctgc aaaccttgag gcaggaagaa cctgcagagc acatgggact 3360
   tgcagcggac atgctttaaa gaggtgcccc aggcccgtcc accgccctcg gccaccctcc 3420
   gtgtcctctg gggagcagct gcggaagatt cgagtcagaa tagcaagaag gaaccgcagc 3480
   agaaggtaca actcccagca tgccctgcgc ccgccacgcc cacaaggcca ggcgcagatg 3540
   ggcgtggggc gggactttcc cggctcgcct cgcgccgtcc actcccagaa ggcagcgggc 3600
   gagggcgtgg ggccggggct ctcccggcat gctctgcggc gcgcctccgc ccgcgcgatt 3660
   tgaatcctgc gtttgagtcg tcttggcgga ggttgtggtg acgccatcac tagtgattac 3720
   cgttcgtata gcatacatta tacgaagtta taccatgtct agactggaca agagcaaagt 3780
   cataaacggc gctctggaat tactcaatgg agtcggtatc gaaggcctga cgacaaggaa 3840
   actcgctcaa aagctgggag ttgagcagcc taccctgtac tggcacgtga agaacaagcg 3900
   ggccctgctc gatgccctgc caatcgagat gctggacagg catcataccc acttctgccc 3960
   cctggaaggc gagtcatggc aagactttct gcggaacaac gccaagtcat tccgctgtgc 4020
   tctcctctca catcgcgacg gggctaaagt gcatctcggc acccgcccaa cagagaaaca 4080
   gtacgaaacc ctggaaaatc agctcgcgtt cctgtgtcag caaggcttct ccctggagaa 4140
   cgcactgtac gctctgtccg ccgtgggcca ctttacactg ggctgcgtat tggaggaaca 4200
   ggagcatcaa gtagcaaaag aggaaagaga gacacctacc accgattcta tgcccccact 4260
   tctgagacaa gcaattgagc tgttcgaccg gcagggagcc gaacctgcct tccttttcgg 4320
   cctggaacta atcatatgtg gcctggagaa acagctaaag tgcgaaagcg gcgggccggc 4380
   cgacgccctt gacgattttg acttagacat gctcccagcc gatgcccttg acgactttga 4440
   ccttgatatg ctgcctgctg acgctcttga cgattttgac cttgacatgc tccccgggta 4500
   actaagtaag gatcccaaat accactgaat taagaattcc gcccctctcc ctcccccccc 4560
   cctaacgtta ctggccgaag ccgcttggaa taaggccggt gtgcgtttgt ctatatgtta 4620
   ttttccacca tattgccgtc ttttggcaat gtgagggccc ggaaacctgg ccctgtcttc 4680
   ttgacgagca ttcctagggg tctttcccct ctcgccaaag gaatgcaagg tctgttgaat 4740
   gtcgtgaagg aagcagttcc tctggaagct tcttgaagac aaacaacgtc tgtagcgacc 4800
   ctttgcaggc agcggaaccc cccacctggc gacaggtgcc tctgcggcca aaagccacgt 4860
   gtataagata cacctgcaaa ggcggcacaa ccccagtgcc acgttgtgag ttggatagtt 4920
   gtggaaagag tcaaatggct ctcctcaagc gtattcaaca aggggctgaa ggatgcccag 4980
   aaggtacccc attgtatggg atctgatctg gggcctcggt gcacatgctt tacatgtgtt 5040
   tagtcgaggt taaaaaacgt ctaggccccc cgaaccacgg ggacgtggtt ttcctttgaa 5100
   aaacacgatg ataatatggc cacaaccatg accgagtaca agcccacggt gcgcctcgcc 5160
   acccgcgacg acgtcccccg ggccgtacgc accctcgccg ccgcgttcgc cgactacccc 5220
   gccacgcgcc acaccgtcga cccggaccgc cacatcgagc gggtcaccga gctgcaagaa 5280
   ctcttcctca cgcgcgtcgg gctcgacatc ggcaaggtgt gggtcgcgga cgacggcgcc 5340
   gcggtggcgg tctggaccac gccggagagc gtcgaagcgg gggcggtgtt cgccgagatc 5400
   ggcccgcgca tggccgagtt gagcggttcc cggctggccg cgcagcaaca gatggaaggc 5460
   ctcctggcgc cgcaccggcc caaggagccc gcgtggttcc tggccaccgt cggcgtctcg 5520
   cccgaccacc agggcaaggg tctgggcagc gccgtcgtgc tccccggagt ggaggcggcc 5580
   gagcgcgccg gggtgcccgc cttcctggag acctccgcgc cccgcaacct ccccttctac 5640
   gagcggctcg gcttcaccgt caccgccgac gtcgaggtgc ccgaaggacc gcgcacctgg 5700
   tgcatgaccc gcaagcccgg tgcctgagga tccagacatg ataagataca ttgatgagtt 5760
   tggacaaacc acaactagaa tgcagtgaaa aaaatgcttt atttgtgaaa tttgtgatgc 5820
   tattgcttta tttgtaacca ttataagctg caataaacaa gttaacaaca acaattgcat 5880
   tcattttatg tttcaggttc agggggaggt gtgggaggtt ttttaaagca agtaaaacct 5940
   ctacaaatgt ggtatggctg attatgatcc tctagagtcg cagatccaga catgataaga 6000
   tacattgatg agtttggaca aaccacaact agaatgcagt gaaaaaaatg ctttatttgt 6060
   gaaatttgtg atgctattgc tttatttgta accattataa gctgcaataa acaagttaac 6120
   aacaacaatt gcattcattt tatgtttcag gttcaggggg aggtgtggga ggttttttaa 6180
   agcaagtaaa acctctacaa atgtggtatg gctgattatg atcctctaga gtcgcagatc 6240
   cagacatgat aagatacatt gatgagtttg gacaaaccac aactagaatg cagtgaaaaa 6300
   aatgctttat ttgtgaaatt tgtgatgcta ttgctttatt tgtaaccatt ataagctgca 6360
   ataaacaagt taacaacaac aattgcattc attttatgtt tcaggttcag ggggaggtgt 6420
   gggaggtttt ttaaagcaag taaaacctct acaaatgtgg tatggctgat tatgatcctc 6480
   tagagtcgca gatcctctag agtcgcagat ctttttccct ctgccaaaaa ttatggggac 6540
   atcatgaagc cccttgagca tctgacttct ggctaataaa ggaaatttat tttcattgca 6600
   atagtgtgtt ggaatttttt gtgtctctca ctcggaagga catatgggag ggcaaatcat 6660
   ttaaaacatc agaatgagta tttggtttag agtttggcaa catatgccat atgctggctg 6720
   ccatgaacaa aggtggctat aaagaggtca tcagtatatg aaacagcccc ctgctgtcca 6780
   ttccttattc catagaaaag ccttgacttg aggttagatt ttttttatat tttgttttgt 6840
   gttatttttt tctttaacat ccctaaaatt ttccttacat gttttactag ccagattttt 6900
   cctcctctcc tgactactcc cagtcatagc tgtccctctt ctcttatgaa gatccctcga 6960
   cctgcagccc aagcttatcg aattcccgcg gtggcggccg cacgtctccc tatcagtgat 7020
   agagaagtcg acacgtctcg agctccctat cagtgataga gaaggtacgt ctagaacgtc 7080
   tccctatcag tgatagagaa gtcgacacgt ctcgagctcc ctatcagtga tagagaaggt 7140
   acgtctagaa cgtctcccta tcagtgatag agaagtcgac acgtctcgag ctccctatca 7200
   gtgatagaga aggtacgtct agaacgtctc cctatcagtg atagagaagt cgacacgtct 7260
   cgagctccct atcagtgata gagaaggtac cccctatata agcagagctc gtttagtgaa 7320
   ccgtcagatc gcctggagac gccatccacg ctgttttgac ctccatagaa gacaccggga 7380
   ccgatccagc ctccgcggcc gccatgggag ctccggcgct gccccagatc tggcagctgt 7440
   acctcaagaa ctaccgcatc gccaccttca agaactggcc cttcctggag gactgcgcct 7500
   gcaccccaga gcgagtgagt cccagcttcc agcgactcca ccctgggccc gacggtgcct 7560
   tagttgtgtg acccgggatc agaacccgct atccccaccc tcgcttcgta ctgttagcca 7620
   ggctgctttg ctctgctctg tcctccacga ggccaccccc cccccccgct ctccccaccc 7680
   tctgcctgaa ccactgttag cctccgggcc tcttcccact gtcctcagtt ctcggcccgg 7740
   aacgatctct gggaatcatg ggataaggtc agactgttga tcccgctcac gagctgtcat 7800
   tttcagatgg cggaggctgg cttcatccac tgccctaccg agaacgagcc tgatttggcc 7860
   cagtgttttt tctgctttaa ggaattggaa ggctgggaac ccgatgacaa cccgatgtaa 7920
   gtcccacagg ctactctcgg tgggtttcct ggtgttcccc ttccattgag agccttttgg 7980
   gttgaactgg agttgggaca aaccatttgt atttagtaga agagcatgcg acgtcgggcc 8040
   caattcgccc 8050
<210> 125
   <211> 24
   <212> DNA
   <213> Artificial Sequence
<220>
   <223> oligonucleotide
<400> 125
   caccggacgc catcatggga gctc 24
<210> 126
   <211> 24
   <212> DNA
   <213> Artificial Sequence
<220>
   <223> oligonucleotide
<400> 126
   aaacgagctc ccatgatggc gtcc 24
<210> 127
   <211> 24
   <212> DNA
   <213> Artificial Sequence
<220>
   <223> oligonucleotide
<400> 127
   caccggccaa gacgactcaa acgc 24
<210> 128
   <211> 24
   <212> DNA
   <213> Artificial Sequence
<220>
   <223> oligonucleotide
<400> 128
   aaacgcgttt gagtcgtctt ggcc 24
<210> 129
   <211> 8436
   <212> DNA
   <213> Artificial Sequence
<220>
   <223> synthetic DNA
<400> 129
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccg gacgccatca tgggagctcg ttttagagct agaaatagca agttaaaata     300
   aggctagtcc gttatcaact tgaaaaagtg gcaccgagtc ggtgcttttt tgttttagag     360
   ctagaaatag caagttaaaa taaggctagt ccgtttttag cgcgtgcgcc aattctgcag     420
   acaaatggct ctagaggtac ccgttacata acttacggta aatggcccgc ctggctgacc     480
   gcccaacgac ccccgcccat tgacgtcaat agtaacgcca atagggactt tccattgacg     540
   tcaatgggtg gagtatttac ggtaaactgc ccacttggca gtacatcaag tgtatcatat     600
   gccaagtacg ccccctattg acgtcaatga cggtaaatgg cccgcctggc attgtgccca     660
   gtacatgacc ttatgggact ttcctacttg gcagtacatc tacgtattag tcatcgctat     720
   taccatggtc gaggtgagcc ccacgttctg cttcactctc cccatctccc ccccctcccc     780
   acccccaatt ttgtatttat ttatttttta attattttgt gcagcgatgg gggcgggggg     840
   gggggggggg cgcgcgccag gcggggcggg gcggggcgag gggcggggcg gggcgaggcg     900
   gagaggtgcg gcggcagcca atcagagcgg cgcgctccga aagtttcctt ttatggcgag     960
   gcggcggcgg cggcggccct ataaaaagcg aagcgcgcgg cgggcgggag tcgctgcgac    1020
   gctgccttcg ccccgtgccc cgctccgccg ccgcctcgcg ccgcccgccc cggctctgac    1080
   tgaccgcgtt actcccacag gtgagcgggc gggacggccc ttctcctccg ggctgtaatt    1140
   agctgagcaa gaggtaaggg tttaagggat ggttggttgg tggggtatta atgtttaatt    1200
   acctggagca cctgcctgaa atcacttttt ttcaggttgg accggtgcca ccatgtaccc 1260
   atacgatgtt ccagattacg cttcgccgaa gaaaaagcgc aaggtcgaag cgtccgacaa 1320
   gaagtacagc atcggcctgg ccatcggcac caactctgtg ggctgggccg tgatcaccga 1380
   cgagtacaag gtgcccagca agaaattcaa ggtgctgggc aacaccgacc ggcacagcat 1440
   caagaagaac ctgatcggag ccctgctgtt cgacagcggc gaaacagccg aggccacccg 1500
   gctgaagaga accgccagaa gaagatacac cagacggaag aaccggatct gctatctgca 1560
   agagatcttc agcaacgaga tggccaaggt ggacgacagc ttcttccaca gactggaaga 1620
   gtccttcctg gtggaagagg ataagaagca cgagcggcac cccatcttcg gcaacatcgt 1680
   ggacgaggtg gcctaccacg agaagtaccc caccatctac cacctgagaa agaaactggt 1740
   ggacagcacc gacaaggccg acctgcggct gatctatctg gccctggccc acatgatcaa 1800
   gttccggggc cacttcctga tcgagggcga cctgaacccc gacaacagcg acgtggacaa 1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta 7440
   ttgctgataa atctggagcc ggtgagcgtg gaagccgcgg tatcattgca gcactggggc 7500
   cagatggtaa gccctcccgt atcgtagtta tctacacgac ggggagtcag gcaactatgg 7560
   atgaacgaaa tagacagatc gctgagatag gtgcctcact gattaagcat tggtaactgt 7620
   cagaccaagt ttactcatat atactttaga ttgatttaaa acttcatttt taatttaaaa 7680
   ggatctaggt gaagatcctt tttgataatc tcatgaccaa aatcccttaa cgtgagtttt 7740
   cgttccactg agcgtcagac cccgtagaaa agatcaaagg atcttcttga gatccttttt 7800
   ttctgcgcgt aatctgctgc ttgcaaacaa aaaaaccacc gctaccagcg gtggtttgtt 7860
   tgccggatca agagctacca actctttttc cgaaggtaac tggcttcagc agagcgcaga 7920
   taccaaatac tgtccttcta gtgtagccgt agttaggcca ccacttcaag aactctgtag 7980
   caccgcctac atacctcgct ctgctaatcc tgttaccagt ggctgctgcc agtggcgata 8040
   agtcgtgtct taccgggttg gactcaagac gatagttacc ggataaggcg cagcggtcgg 8100
   gctgaacggg gggttcgtgc acacagccca gcttggagcg aacgacctac accgaactga 8160
   gatacctaca gcgtgagcta tgagaaagcg ccacgcttcc cgaagggaga aaggcggaca 8220
   ggtatccggt aagcggcagg gtcggaacag gagagcgcac gagggagctt ccagggggaa 8280
   acgcctggta tctttatagt cctgtcgggt ttcgccacct ctgacttgag cgtcgatttt 8340
   tgtgatgctc gtcagggggg cggagcctat ggaaaaacgc cagcaacgcg gcctttttac 8400
   ggttcctggc cttttgctgg ccttttgctc acatgt 8436
<210> 130
   <211> 8436
   <212> DNA
   <213> Artificial Sequence
<220>
   <223> synthetic DNA
<400> 130
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccg gccaagacga ctcaaacgcg ttttagagct agaaatagca agttaaaata     300
   aggctagtcc gttatcaact tgaaaaagtg gcaccgagtc ggtgcttttt tgttttagag     360
   ctagaaatag caagttaaaa taaggctagt ccgtttttag cgcgtgcgcc aattctgcag     420
   acaaatggct ctagaggtac ccgttacata acttacggta aatggcccgc ctggctgacc     480
   gcccaacgac ccccgcccat tgacgtcaat agtaacgcca atagggactt tccattgacg     540
   tcaatgggtg gagtatttac ggtaaactgc ccacttggca gtacatcaag tgtatcatat     600
   gccaagtacg ccccctattg acgtcaatga cggtaaatgg cccgcctggc attgtgccca     660
   gtacatgacc ttatgggact ttcctacttg gcagtacatc tacgtattag tcatcgctat     720
   taccatggtc gaggtgagcc ccacgttctg cttcactctc cccatctccc ccccctcccc     780
   acccccaatt ttgtatttat ttatttttta attattttgt gcagcgatgg gggcgggggg     840
   gggggggggg cgcgcgccag gcggggcggg gcggggcgag gggcggggcg gggcgaggcg     900
   gagaggtgcg gcggcagcca atcagagcgg cgcgctccga aagtttcctt ttatggcgag     960
   gcggcggcgg cggcggccct ataaaaagcg aagcgcgcgg cgggcgggag tcgctgcgac    1020
   gctgccttcg ccccgtgccc cgctccgccg ccgcctcgcg ccgcccgccc cggctctgac    1080
   tgaccgcgtt actcccacag gtgagcgggc gggacggccc ttctcctccg ggctgtaatt    1140
   agctgagcaa gaggtaaggg tttaagggat ggttggttgg tggggtatta atgtttaatt    1200
   acctggagca cctgcctgaa atcacttttt ttcaggttgg accggtgcca ccatgtaccc    1260
   atacgatgtt ccagattacg cttcgccgaa gaaaaagcgc aaggtcgaag cgtccgacaa    1320
   gaagtacagc atcggcctgg ccatcggcac caactctgtg ggctgggccg tgatcaccga    1380
   cgagtacaag gtgcccagca agaaattcaa ggtgctgggc aacaccgacc ggcacagcat    1440
   caagaagaac ctgatcggag ccctgctgtt cgacagcggc gaaacagccg aggccacccg    1500
   gctgaagaga accgccagaa gaagatacac cagacggaag aaccggatct gctatctgca    1560
   agagatcttc agcaacgaga tggccaaggt ggacgacagc ttcttccaca gactggaaga    1620
   gtccttcctg gtggaagagg ataagaagca cgagcggcac cccatcttcg gcaacatcgt    1680
   ggacgaggtg gcctaccacg agaagtaccc caccatctac cacctgagaa agaaactggt    1740
   ggacagcacc gacaaggccg acctgcggct gatctatctg gccctggccc acatgatcaa    1800
   gttccggggc cacttcctga tcgagggcga cctgaacccc gacaacagcg acgtggacaa    1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta 7440
   ttgctgataa atctggagcc ggtgagcgtg gaagccgcgg tatcattgca gcactggggc 7500
   cagatggtaa gccctcccgt atcgtagtta tctacacgac ggggagtcag gcaactatgg 7560
   atgaacgaaa tagacagatc gctgagatag gtgcctcact gattaagcat tggtaactgt 7620
   cagaccaagt ttactcatat atactttaga ttgatttaaa acttcatttt taatttaaaa 7680
   ggatctaggt gaagatcctt tttgataatc tcatgaccaa aatcccttaa cgtgagtttt 7740
   cgttccactg agcgtcagac cccgtagaaa agatcaaagg atcttcttga gatccttttt 7800
   ttctgcgcgt aatctgctgc ttgcaaacaa aaaaaccacc gctaccagcg gtggtttgtt 7860
   tgccggatca agagctacca actctttttc cgaaggtaac tggcttcagc agagcgcaga 7920
   taccaaatac tgtccttcta gtgtagccgt agttaggcca ccacttcaag aactctgtag 7980
   caccgcctac atacctcgct ctgctaatcc tgttaccagt ggctgctgcc agtggcgata 8040
   agtcgtgtct taccgggttg gactcaagac gatagttacc ggataaggcg cagcggtcgg 8100
   gctgaacggg gggttcgtgc acacagccca gcttggagcg aacgacctac accgaactga 8160
   gatacctaca gcgtgagcta tgagaaagcg ccacgcttcc cgaagggaga aaggcggaca 8220
   ggtatccggt aagcggcagg gtcggaacag gagagcgcac gagggagctt ccagggggaa 8280
   acgcctggta tctttatagt cctgtcgggt ttcgccacct ctgacttgag cgtcgatttt 8340
   tgtgatgctc gtcagggggg cggagcctat ggaaaaacgc cagcaacgcg gcctttttac 8400
   ggttcctggc cttttgctgg ccttttgctc acatgt 8436
<210> 131
   <211> 24
   <212> DNA
   <213> Artificial Sequence
<220>
   <223> oligonucleotide
<400> 131
   cactcgtttg caacgccaac ctgg 24
<210> 132
   <211> 22
   <212> DNA
   <213> Artificial Sequence
<220>
   <223> oligonucleotide
<400> 132
   gaacccgatg acaacccgat ag 22
<210> 133
   <211> 24
   <212> DNA
   <213> Artificial Sequence
<220>
   <223> oligonucleotide
<400> 133
   tccatctgct tcttgacagt gagg 24
<210> 134
   <211> 817
   <212> DNA
   <213> Artificial Sequence
<220>
   <223> synthetic DNA
<400> 134
   gattaaaggc gtgcaccact actgtctgcc ttttgttttg gttttatcat aattgttgca      60
   tttttgcccg caggaggatc aggaattcaa ggacaacctc tacagccaac ccagttgagg     120
   gccagcctgt tctatcgaga tcctgtctcc ggacaaaaaa agtttcatga gagcgtggtg     180
   tgttggcaca gtcttttagt ccgagcactg acgcagtaga ttaaggcaga tcttcatgat     240
   ttggtgcggt cctgggctgc atccaaagac cttgtttcca aaacaaacaa acgaaaaagt     300
   gtaataaaaa tacaagcaag gttttgcaga aataaatctg tggcctcaag ggtccctttc     360
   tccagctttt cccagaggtg tcaaattaca taacagtctc aaacccagga aattgaactt     420
   tggcacaacc cactgaccgg tccgaatttc aaagtctttt tcctattgac ctacaaggtt     480
   ttcaagaatc atgttgtaag caactgtgtt ctgaggaatc tatgtttaaa aacccatccg     540
   tggatcttgg cccagggtcc agagactgag ctagccacgc cccggccgcg ccgcagccac     600
   tcccacggca gttcaagtgt taagtcccaa agaccgcgct ctgtgcatgc gcagacccgt     660
   ccacagctgg ctcctagcca acccggccgg acgagcaccc ggcgccgtca cgtgacgcac     720
   ccaaccggcg tcgacctata aaaggccggg cgttgacgtc agcggtctct tccgccgcag     780
   ccgccgccat cgtcggcgcg cttccctgtt cacctct                              817
<210> 135
   <211> 37
   <212> DNA
   <213> Artificial Sequence
<220>
   <223> oligonucleotide
<400> 135
   tttaacctgc agggattaaa ggcgtgcacc actactg 37
<210> 136
   <211> 36
   <212> DNA
   <213> Artificial Sequence
<220>
   <223> oligonucleotide
<400> 136
   tatataacta gtagaggtga acagggaagc gcgccg 36
<210> 137
   <211> 826
   <212> DNA
   <213> Artificial Sequence
<220>
   <223> synthetic DNA
<400> 137
   gactctgaga atccgtcgcc atccgccacc atggtgagtc tcctcggctc cgctagactc 60
   ggggaccgag acgaatctct ggggcagcgg gacgtggctg tagcgggacg ctgagaggga 120
   cgggaggaag agacatggct gccctggccc gggcggcagg acgtggtcgg gccgcggcgc 180
   catatctgcg cgtccctgag ggccttggga gtgtcaactg ccgaggtcgg ggtgttttct 240
   tgaagtcctt caactccccg cggccgccgg ggtgactgcg ggaggggttg tgcttggtga 300
   tgtggcagcg ggcaaagcgc cgtccccgcg cccctggtga cgggcggagg gtgtcctcgg 360
   gaggtgacag cctgtagggc tggcttcctt ggacacctcc agtgggctga acgccttccg 420
   ggccctttcc ggtagccccc gtctctgttt tctatctgag ttcacacgtg agcaccggtc 480
   cccataatct aagaaagtgg ctcactgggc ctagtggcgc attgtggcct ttgatccggg 540
   ctttgacctt ggcgcacagc acccagtggt ttggggaaga ggtgtgtgta gcagaggagg 600
   ttttttcgtg ctttggtccc aatcaatccg gcatctttgc agtgccgagg tggccgtgca 660
   ccttggcttt gaattcttgt gctgaggtta tgtgacttga gcctcaagat agggtcttct 720
   agcacaggct tgctcttaag tgtcgcagtt gtcggtttcg gcgtttgttt agagctgtgg 780
   acacatctgt gaacttttga tgcttatttc agaggtcctg ggttgt 826
<210> 138
   <211> 36
   <212> DNA
   <213> Artificial Sequence
<220>
   <223> oligonucleotide
<400> 138
   ttatagcatg cgactctgag aatccgtcgc catccg 36
<210> 139
   <211> 36
   <212> DNA
   <213> Artificial Sequence
<220>
   <223> oligonucleotide
<400> 139
   ttatagcatg cacaacccag gacctctgaa ataagc 36
<210> 140
   <211> 8317
   <212> DNA
   <213> Artificial Sequence
<220>
   <223> synthetic DNA
<400> 140
   tatagtgagt cgtattacaa ttcactggcc gtcgttttac aacgtcgtga ctgggaaaac      60
   cctggcgtta cccaacttaa tcgccttgca gcacatcccc ctttcgccag ctggcgtaat     120
   agcgaagagg cccgcaccga tcgcccttcc caacagttgc gcagcctgaa tggcgaatgg     180
   acgcgccctg tagcggcgca ttaagcgcgg cgggtgtggt ggttacgcgc agcgtgaccg     240
   ctacacttgc cagcgcccta gcgcccgctc ctttcgcttt cttcccttcc tttctcgcca     300
   cgttcgccgg ctttccccgt caagctctaa atcgggggct ccctttaggg ttccgattta     360
   gtgctttacg gcacctcgac cccaaaaaac ttgattaggg tgatggttca cgtagtgggc     420
   catcgccctg atagacggtt tttcgccctt tgacgttgga gtccacgttc tttaatagtg     480
   gactcttgtt ccaaactgga acaacactca accctatctc ggtctattct tttgatttat     540
   aagggatttt gccgatttcg gcctattggt taaaaaatga gctgatttaa caaaaattta     600
   acgcgaattt taacaaaata ttaacgctta caatttcctg atgcggtatt ttctccttac     660
   gcatctgtgc ggtatttcac accgcatcag gtggcacttt tcggggaaat gtgcgcggaa     720
   cccctatttg tttatttttc taaatacatt caaatatgta tccgctcatg agacaataac     780
   cctgataaat gcttcaataa tattgaaaaa ggaagagtat gagtattcaa catttccgtg     840
   tcgcccttat tccctttttt gcggcatttt gccttcctgt ttttgctcac ccagaaacgc     900
   tggtgaaagt aaaagatgct gaagatcagt tgggtgcacg agtgggttac atcgaactgg     960
   atctcaacag cggtaagatc cttgagagtt ttcgccccga agaacgtttt ccaatgatga    1020
   gcacttttaa agttctgcta tgtggcgcgg tattatcccg tattgacgcc gggcaagagc    1080
   aactcggtcg ccgcatacac tattctcaga atgacttggt tgagtactca ccagtcacag    1140
   aaaagcatct tacggatggc atgacagtaa gagaattatg cagtgctgcc ataaccatga    1200
   gtgataacac tgcggccaac ttacttctga caacgatcgg aggaccgaag gagctaaccg    1260
   cttttttgca caacatgggg gatcatgtaa ctcgccttga tcgttgggaa ccggagctga    1320
   atgaagccat accaaacgac gagcgtgaca ccacgatgcc tgtagcaatg gcaacaacgt    1380
   tgcgcaaact attaactggc gaactactta ctctagcttc ccggcaacaa ttaatagact    1440
   ggatggaggc ggataaagtt gcaggaccac ttctgcgctc ggcccttccg gctggctggt    1500
   ttattgctga taaatctgga gccggtgagc gtgggtctcg cggtatcatt gcagcactgg    1560
   ggccagatgg taagccctcc cgtatcgtag ttatctacac gacggggagt caggcaacta 1620
   tggatgaacg aaatagacag atcgctgaga taggtgcctc actgattaag cattggtaac 1680
   tgtcagacca agtttactca tatatacttt agattgattt aaaacttcat ttttaattta 1740
   aaaggatcta ggtgaagatc ctttttgata atctcatgac caaaatccct taacgtgagt 1800
   tttcgttcca ctgagcgtca gaccccgtag aaaagatcaa aggatcttct tgagatcctt 1860
   tttttctgcg cgtaatctgc tgcttgcaaa caaaaaaacc accgctacca gcggtggttt 1920
   gtttgccgga tcaagagcta ccaactcttt ttccgaaggt aactggcttc agcagagcgc 1980
   agataccaaa tactgttctt ctagtgtagc cgtagttagg ccaccacttc aagaactctg 2040
   tagcaccgcc tacatacctc gctctgctaa tcctgttacc agtggctgct gccagtggcg 2100
   ataagtcgtg tcttaccggg ttggactcaa gacgatagtt accggataag gcgcagcggt 2160
   cgggctgaac ggggggttcg tgcacacagc ccagcttgga gcgaacgacc tacaccgaac 2220
   tgagatacct acagcgtgag ctatgagaaa gcgccacgct tcccgaaggg agaaaggcgg 2280
   acaggtatcc ggtaagcggc agggtcggaa caggagagcg cacgagggag cttccagggg 2340
   gaaacgcctg gtatctttat agtcctgtcg ggtttcgcca cctctgactt gagcgtcgat 2400
   ttttgtgatg ctcgtcaggg gggcggagcc tatggaaaaa cgccagcaac gcggcctttt 2460
   tacggttcct ggccttttgc tggccttttg ctcacatgtt ctttcctgcg ttatcccctg 2520
   attctgtgga taaccgtatt accgcctttg agtgagctga taccgctcgc cgcagccgaa 2580
   cgaccgagcg cagcgagtca gtgagcgagg aagcggaaga gcgcccaata cgcaaaccgc 2640
   ctctccccgc gcgttggccg attcattaat gcagctggca cgacaggttt cccgactgga 2700
   aagcgggcag tgagcgcaac gcaattaatg tgagttagct cactcattag gcaccccagg 2760
   ctttacactt tatgcttccg gctcgtatgt tgtgtggaat tgtgagcgga taacaatttc 2820
   acacaggaaa cagctatgac catgattacg ccaagctatt taggtgacac tatagaatac 2880
   tcaagctatg catccaacgc gttgggagct ctcccatatg gtcgacctgc agggattaaa 2940
   ggcgtgcacc actactgtct gccttttgtt ttggttttat cataattgtt gcatttttgc 3000
   ccgcaggagg atcaggaatt caaggacaac ctctacagcc aacccagttg agggccagcc 3060
   tgttctatcg agatcctgtc tccggacaaa aaaagtttca tgagagcgtg gtgtgttggc 3120
   acagtctttt agtccgagca ctgacgcagt agattaaggc agatcttcat gatttggtgc 3180
   ggtcctgggc tgcatccaaa gaccttgttt ccaaaacaaa caaacgaaaa agtgtaataa 3240
   aaatacaagc aaggttttgc agaaataaat ctgtggcctc aagggtccct ttctccagct 3300
   tttcccagag gtgtcaaatt acataacagt ctcaaaccca ggaaattgaa ctttggcaca 3360
   acccactgac cggtccgaat ttcaaagtct ttttcctatt gacctacaag gttttcaaga 3420
   atcatgttgt aagcaactgt gttctgagga atctatgttt aaaaacccat ccgtggatct 3480
   tggcccaggg tccagagact gagctagcca cgccccggcc gcgccgcagc cactcccacg 3540
   gcagttcaag tgttaagtcc caaagaccgc gctctgtgca tgcgcagacc cgtccacagc 3600
   tggctcctag ccaacccggc cggacgagca cccggcgccg tcacgtgacg cacccaaccg 3660
   gcgtcgacct ataaaaggcc gggcgttgac gtcagcggtc tcttccgccg cagccgccgc 3720
   catcgtcggc gcgcttccct gttcacctct actagtgatt accgttcgta tagcatacat 3780
   tatacgaagt tataccatgt ctagactgga caagagcaaa gtcataaacg gcgctctgga 3840
   attactcaat ggagtcggta tcgaaggcct gacgacaagg aaactcgctc aaaagctggg 3900
   agttgagcag cctaccctgt actggcacgt gaagaacaag cgggccctgc tcgatgccct 3960
   gccaatcgag atgctggaca ggcatcatac ccacttctgc cccctggaag gcgagtcatg 4020
   gcaagacttt ctgcggaaca acgccaagtc attccgctgt gctctcctct cacatcgcga 4080
   cggggctaaa gtgcatctcg gcacccgccc aacagagaaa cagtacgaaa ccctggaaaa 4140
   tcagctcgcg ttcctgtgtc agcaaggctt ctccctggag aacgcactgt acgctctgtc 4200
   cgccgtgggc cactttacac tgggctgcgt attggaggaa caggagcatc aagtagcaaa 4260
   agaggaaaga gagacaccta ccaccgattc tatgccccca cttctgagac aagcaattga 4320
   gctgttcgac cggcagggag ccgaacctgc cttccttttc ggcctggaac taatcatatg 4380
   tggcctggag aaacagctaa agtgcgaaag cggcgggccg gccgacgccc ttgacgattt 4440
   tgacttagac atgctcccag ccgatgccct tgacgacttt gaccttgata tgctgcctgc 4500
   tgacgctctt gacgattttg accttgacat gctccccggg taactaagta aggatcccaa 4560
   ataccactga attaagaatt ccgcccctct ccctcccccc cccctaacgt tactggccga 4620
   agccgcttgg aataaggccg gtgtgcgttt gtctatatgt tattttccac catattgccg 4680
   tcttttggca atgtgagggc ccggaaacct ggccctgtct tcttgacgag cattcctagg 4740
   ggtctttccc ctctcgccaa aggaatgcaa ggtctgttga atgtcgtgaa ggaagcagtt 4800
   cctctggaag cttcttgaag acaaacaacg tctgtagcga ccctttgcag gcagcggaac 4860
   cccccacctg gcgacaggtg cctctgcggc caaaagccac gtgtataaga tacacctgca 4920
   aaggcggcac aaccccagtg ccacgttgtg agttggatag ttgtggaaag agtcaaatgg 4980
   ctctcctcaa gcgtattcaa caaggggctg aaggatgccc agaaggtacc ccattgtatg 5040
   ggatctgatc tggggcctcg gtgcacatgc tttacatgtg tttagtcgag gttaaaaaac 5100
   gtctaggccc cccgaaccac ggggacgtgg ttttcctttg aaaaacacga tgataatatg 5160
   gccacaacca tgaccgagta caagcccacg gtgcgcctcg ccacccgcga cgacgtcccc 5220
   cgggccgtac gcaccctcgc cgccgcgttc gccgactacc ccgccacgcg ccacaccgtc 5280
   gacccggacc gccacatcga gcgggtcacc gagctgcaag aactcttcct cacgcgcgtc 5340
   gggctcgaca tcggcaaggt gtgggtcgcg gacgacggcg ccgcggtggc ggtctggacc 5400
   acgccggaga gcgtcgaagc gggggcggtg ttcgccgaga tcggcccgcg catggccgag 5460
   ttgagcggtt cccggctggc cgcgcagcaa cagatggaag gcctcctggc gccgcaccgg 5520
   cccaaggagc ccgcgtggtt cctggccacc gtcggcgtct cgcccgacca ccagggcaag 5580
   ggtctgggca gcgccgtcgt gctccccgga gtggaggcgg ccgagcgcgc cggggtgccc 5640
   gccttcctgg agacctccgc gccccgcaac ctccccttct acgagcggct cggcttcacc 5700
   gtcaccgccg acgtcgaggt gcccgaagga ccgcgcacct ggtgcatgac ccgcaagccc 5760
   ggtgcctgag gatccagaca tgataagata cattgatgag tttggacaaa ccacaactag 5820
   aatgcagtga aaaaaatgct ttatttgtga aatttgtgat gctattgctt tatttgtaac 5880
   cattataagc tgcaataaac aagttaacaa caacaattgc attcatttta tgtttcaggt 5940
   tcagggggag gtgtgggagg ttttttaaag caagtaaaac ctctacaaat gtggtatggc 6000
   tgattatgat cctctagagt cgcagatcca gacatgataa gatacattga tgagtttgga 6060
   caaaccacaa ctagaatgca gtgaaaaaaa tgctttattt gtgaaatttg tgatgctatt 6120
   gctttatttg taaccattat aagctgcaat aaacaagtta acaacaacaa ttgcattcat 6180
   tttatgtttc aggttcaggg ggaggtgtgg gaggtttttt aaagcaagta aaacctctac 6240
   aaatgtggta tggctgatta tgatcctcta gagtcgcaga tccagacatg ataagataca 6300
   ttgatgagtt tggacaaacc acaactagaa tgcagtgaaa aaaatgcttt atttgtgaaa 6360
   tttgtgatgc tattgcttta tttgtaacca ttataagctg caataaacaa gttaacaaca 6420
   acaattgcat tcattttatg tttcaggttc agggggaggt gtgggaggtt ttttaaagca 6480
   agtaaaacct ctacaaatgt ggtatggctg attatgatcc tctagagtcg cagatcctct 6540
   agagtcgcag atctttttcc ctctgccaaa aattatgggg acatcatgaa gccccttgag 6600
   catctgactt ctggctaata aaggaaattt attttcattg caatagtgtg ttggaatttt 6660
   ttgtgtctct cactcggaag gacatatggg agggcaaatc atttaaaaca tcagaatgag 6720
   tatttggttt agagtttggc aacatatgcc atatgctggc tgccatgaac aaaggtggct 6780
   ataaagaggt catcagtata tgaaacagcc ccctgctgtc cattccttat tccatagaaa 6840
   agccttgact tgaggttaga ttttttttat attttgtttt gtgttatttt tttctttaac 6900
   atccctaaaa ttttccttac atgttttact agccagattt ttcctcctct cctgactact 6960
   cccagtcata gctgtccctc ttctcttatg aagatccctc gacctgcagc ccaagcttat 7020
   cgaattcccg cggtggcggc cgcacgtctc cctatcagtg atagagaagt cgacacgtct 7080
   cgagctccct atcagtgata gagaaggtac gtctagaacg tctccctatc agtgatagag 7140
   aagtcgacac gtctcgagct ccctatcagt gatagagaag gtacgtctag aacgtctccc 7200
   tatcagtgat agagaagtcg acacgtctcg agctccctat cagtgataga gaaggtacgt 7260
   ctagaacgtc tccctatcag tgatagagaa gtcgacacgt ctcgagctcc ctatcagtga 7320
   tagagaaggt accccctata taagcagagc tcgtttagtg aaccgtcaga tcgcctggag 7380
   acgccatcca cgctgttttg acctccatag aagacaccgg gaccgatcca gcctccgcgg 7440
   ccgccatggc ggccgggagc atgcgactct gagaatccgt cgccatccgc caccatggtg 7500
   agtctcctcg gctccgctag actcggggac cgagacgaat ctctggggca gcgggacgtg 7560
   gctgtagcgg gacgctgaga gggacgggag gaagagacat ggctgccctg gcccgggcgg 7620
   caggacgtgg tcgggccgcg gcgccatatc tgcgcgtccc tgagggcctt gggagtgtca 7680
   actgccgagg tcggggtgtt ttcttgaagt ccttcaactc cccgcggccg ccggggtgac 7740
   tgcgggaggg gttgtgcttg gtgatgtggc agcgggcaaa gcgccgtccc cgcgcccctg 7800
   gtgacgggcg gagggtgtcc tcgggaggtg acagcctgta gggctggctt ccttggacac 7860
   ctccagtggg ctgaacgcct tccgggccct ttccggtagc ccccgtctct gttttctatc 7920
   tgagttcaca cgtgagcacc ggtccccata atctaagaaa gtggctcact gggcctagtg 7980
   gcgcattgtg gcctttgatc cgggctttga ccttggcgca cagcacccag tggtttgggg 8040
   aagaggtgtg tgtagcagag gaggtttttt cgtgctttgg tcccaatcaa tccggcatct 8100
   ttgcagtgcc gaggtggccg tgcaccttgg ctttgaattc ttgtgctgag gttatgtgac 8160
   ttgagcctca agatagggtc ttctagcaca ggcttgctct taagtgtcgc agttgtcggt 8220
   ttcggcgttt gtttagagct gtggacacat ctgtgaactt ttgatgctta tttcagaggt 8280
   cctgggttgt gcatgcgacg tcgggcccaa ttcgccc 8317
<210> 141
   <211> 23
   <212> DNA
   <213> Artificial Sequence
<220>
   <223> oligonucleotide
<400> 141
   caccgtcggc tccgctagac tcg 23
<210> 142
   <211> 23
   <212> DNA
   <213> Artificial Sequence
<220>
   <223> oligonucleotide
<400> 142
   aaaccgagtc tagcggagcc gac 23
<210> 143
   <211> 23
   <212> DNA
   <213> Artificial Sequence
<220>
   <223> oligonucleotide
<400> 143
   caccgactca ccatggtggc gga 23
<210> 144
   <211> 23
   <212> DNA
   <213> Artificial Sequence
<220>
   <223> oligonucleotide
<400> 144
   aaactccgcc accatggtga gtc 23
<210> 145
   <211> 8435
   <212> DNA
   <213> Artificial Sequence
<220>
   <223> synthetic DNA
<400> 145
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccg tcggctccgc tagactcggt tttagagcta gaaatagcaa gttaaaataa     300
   ggctagtccg ttatcaactt gaaaaagtgg caccgagtcg gtgctttttt gttttagagc     360
   tagaaatagc aagttaaaat aaggctagtc cgtttttagc gcgtgcgcca attctgcaga     420
   caaatggctc tagaggtacc cgttacataa cttacggtaa atggcccgcc tggctgaccg     480
   cccaacgacc cccgcccatt gacgtcaata gtaacgccaa tagggacttt ccattgacgt     540
   caatgggtgg agtatttacg gtaaactgcc cacttggcag tacatcaagt gtatcatatg     600
   ccaagtacgc cccctattga cgtcaatgac ggtaaatggc ccgcctggca ttgtgcccag     660
   tacatgacct tatgggactt tcctacttgg cagtacatct acgtattagt catcgctatt     720
   accatggtcg aggtgagccc cacgttctgc ttcactctcc ccatctcccc cccctcccca     780
   cccccaattt tgtatttatt tattttttaa ttattttgtg cagcgatggg ggcggggggg     840
   gggggggggc gcgcgccagg cggggcgggg cggggcgagg ggcggggcgg ggcgaggcgg     900
   agaggtgcgg cggcagccaa tcagagcggc gcgctccgaa agtttccttt tatggcgagg 960
   cggcggcggc ggcggcccta taaaaagcga agcgcgcggc gggcgggagt cgctgcgacg 1020
   ctgccttcgc cccgtgcccc gctccgccgc cgcctcgcgc cgcccgcccc ggctctgact 1080
   gaccgcgtta ctcccacagg tgagcgggcg ggacggccct tctcctccgg gctgtaatta 1140
   gctgagcaag aggtaagggt ttaagggatg gttggttggt ggggtattaa tgtttaatta 1200
   cctggagcac ctgcctgaaa tcactttttt tcaggttgga ccggtgccac catgtaccca 1260
   tacgatgttc cagattacgc ttcgccgaag aaaaagcgca aggtcgaagc gtccgacaag 1320
   aagtacagca tcggcctggc catcggcacc aactctgtgg gctgggccgt gatcaccgac 1380
   gagtacaagg tgcccagcaa gaaattcaag gtgctgggca acaccgaccg gcacagcatc 1440
   aagaagaacc tgatcggagc cctgctgttc gacagcggcg aaacagccga ggccacccgg 1500
   ctgaagagaa ccgccagaag aagatacacc agacggaaga accggatctg ctatctgcaa 1560
   gagatcttca gcaacgagat ggccaaggtg gacgacagct tcttccacag actggaagag 1620
   tccttcctgg tggaagagga taagaagcac gagcggcacc ccatcttcgg caacatcgtg 1680
   gacgaggtgg cctaccacga gaagtacccc accatctacc acctgagaaa gaaactggtg 1740
   gacagcaccg acaaggccga cctgcggctg atctatctgg ccctggccca catgatcaag 1800
   ttccggggcc acttcctgat cgagggcgac ctgaaccccg acaacagcga cgtggacaag 1860
   ctgttcatcc agctggtgca gacctacaac cagctgttcg aggaaaaccc catcaacgcc 1920
   agcggcgtgg acgccaaggc catcctgtct gccagactga gcaagagcag acggctggaa 1980
   aatctgatcg cccagctgcc cggcgagaag aagaatggcc tgttcggcaa cctgattgcc 2040
   ctgagcctgg gcctgacccc caacttcaag agcaacttcg acctggccga ggatgccaaa 2100
   ctgcagctga gcaaggacac ctacgacgac gacctggaca acctgctggc ccagatcggc 2160
   gaccagtacg ccgacctgtt tctggccgcc aagaacctgt ccgacgccat cctgctgagc 2220
   gacatcctga gagtgaacac cgagatcacc aaggcccccc tgagcgcctc tatgatcaag 2280
   agatacgacg agcaccacca ggacctgacc ctgctgaaag ctctcgtgcg gcagcagctg 2340
   cctgagaagt acaaagagat tttcttcgac cagagcaaga acggctacgc cggctacatt 2400
   gacggcggag ccagccagga agagttctac aagttcatca agcccatcct ggaaaagatg 2460
   gacggcaccg aggaactgct cgtgaagctg aacagagagg acctgctgcg gaagcagcgg 2520
   accttcgaca acggcagcat cccccaccag atccacctgg gagagctgca cgccattctg 2580
   cggcggcagg aagattttta cccattcctg aaggacaacc gggaaaagat cgagaagatc 2640
   ctgaccttcc gcatccccta ctacgtgggc cctctggcca ggggaaacag cagattcgcc 2700
   tggatgacca gaaagagcga ggaaaccatc accccctgga acttcgagga agtggtggac 2760
   aagggcgctt ccgcccagag cttcatcgag cggatgacca acttcgataa gaacctgccc 2820
   aacgagaagg tgctgcccaa gcacagcctg ctgtacgagt acttcaccgt gtataacgag 2880
   ctgaccaaag tgaaatacgt gaccgaggga atgagaaagc ccgccttcct gagcggcgag 2940
   cagaaaaagg ccatcgtgga cctgctgttc aagaccaacc ggaaagtgac cgtgaagcag 3000
   ctgaaagagg actacttcaa gaaaatcgag tgcttcgact ccgtggaaat ctccggcgtg 3060
   gaagatcggt tcaacgcctc cctgggcaca taccacgatc tgctgaaaat tatcaaggac 3120
   aaggacttcc tggacaatga ggaaaacgag gacattctgg aagatatcgt gctgaccctg 3180
   acactgtttg aggacagaga gatgatcgag gaacggctga aaacctatgc ccacctgttc 3240
   gacgacaaag tgatgaagca gctgaagcgg cggagataca ccggctgggg caggctgagc 3300
   cggaagctga tcaacggcat ccgggacaag cagtccggca agacaatcct ggatttcctg 3360
   aagtccgacg gcttcgccaa cagaaacttc atgcagctga tccacgacga cagcctgacc 3420
   tttaaagagg acatccagaa agcccaggtg tccggccagg gcgatagcct gcacgagcac 3480
   attgccaatc tggccggcag ccccgccatt aagaagggca tcctgcagac agtgaaggtg 3540
   gtggacgagc tcgtgaaagt gatgggccgg cacaagcccg agaacatcgt gatcgaaatg 3600
   gccagagaga accagaccac ccagaaggga cagaagaaca gccgcgagag aatgaagcgg 3660
   atcgaagagg gcatcaaaga gctgggcagc cagatcctga aagaacaccc cgtggaaaac 3720
   acccagctgc agaacgagaa gctgtacctg tactacctgc agaatgggcg ggatatgtac 3780
   gtggaccagg aactggacat caaccggctg tccgactacg atgtggacca tatcgtgcct 3840
   cagagctttc tgaaggacga ctccatcgac aacaaggtgc tgaccagaag cgacaagaac 3900
   cggggcaaga gcgacaacgt gccctccgaa gaggtcgtga agaagatgaa gaactactgg 3960
   cggcagctgc tgaacgccaa gctgattacc cagagaaagt tcgacaatct gaccaaggcc 4020
   gagagaggcg gcctgagcga actggataag gccggcttca tcaagagaca gctggtggaa 4080
   acccggcaga tcacaaagca cgtggcacag atcctggact cccggatgaa cactaagtac 4140
   gacgagaatg acaagctgat ccgggaagtg aaagtgatca ccctgaagtc caagctggtg 4200
   tccgatttcc ggaaggattt ccagttttac aaagtgcgcg agatcaacaa ctaccaccac 4260
   gcccacgacg cctacctgaa cgccgtcgtg ggaaccgccc tgatcaaaaa gtaccctaag 4320
   ctggaaagcg agttcgtgta cggcgactac aaggtgtacg acgtgcggaa gatgatcgcc 4380
   aagagcgagc aggaaatcgg caaggctacc gccaagtact tcttctacag caacatcatg 4440
   aactttttca agaccgagat taccctggcc aacggcgaga tccggaagcg gcctctgatc 4500
   gagacaaacg gcgaaaccgg ggagatcgtg tgggataagg gccgggattt tgccaccgtg 4560
   cggaaagtgc tgagcatgcc ccaagtgaat atcgtgaaaa agaccgaggt gcagacaggc 4620
   ggcttcagca aagagtctat cctgcccaag aggaacagcg ataagctgat cgccagaaag 4680
   aaggactggg accctaagaa gtacggcggc ttcgacagcc ccaccgtggc ctattctgtg 4740
   ctggtggtgg ccaaagtgga aaagggcaag tccaagaaac tgaagagtgt gaaagagctg 4800
   ctggggatca ccatcatgga aagaagcagc ttcgagaaga atcccatcga ctttctggaa 4860
   gccaagggct acaaagaagt gaaaaaggac ctgatcatca agctgcctaa gtactccctg 4920
   ttcgagctgg aaaacggccg gaagagaatg ctggcctctg ccggcgaact gcagaaggga 4980
   aacgaactgg ccctgccctc caaatatgtg aacttcctgt acctggccag ccactatgag 5040
   aagctgaagg gctcccccga ggataatgag cagaaacagc tgtttgtgga acagcacaag 5100
   cactacctgg acgagatcat cgagcagatc agcgagttct ccaagagagt gatcctggcc 5160
   gacgctaatc tggacaaagt gctgtccgcc tacaacaagc accgggataa gcccatcaga 5220
   gagcaggccg agaatatcat ccacctgttt accctgacca atctgggagc ccctgccgcc 5280
   ttcaagtact ttgacaccac catcgaccgg aagaggtaca ccagcaccaa agaggtgctg 5340
   gacgccaccc tgatccacca gagcatcacc ggcctgtacg agacacggat cgacctgtct 5400
   cagctgggag gcgacagccc caagaagaag agaaaggtgg aggccagcta agaattccta 5460
   gagctcgctg atcagcctcg actgtgcctt ctagttgcca gccatctgtt gtttgcccct 5520
   cccccgtgcc ttccttgacc ctggaaggtg ccactcccac tgtcctttcc taataaaatg 5580
   aggaaattgc atcgcattgt ctgagtaggt gtcattctat tctggggggt ggggtggggc 5640
   aggacagcaa gggggaggat tgggaagaga atagcaggca tgctggggag cggccgcagg 5700
   aacccctagt gatggagttg gccactccct ctctgcgcgc tcgctcgctc actgaggccg 5760
   ggcgaccaaa ggtcgcccga cgcccgggct ttgcccgggc ggcctcagtg agcgagcgag 5820
   cgcgcagctg cctgcagggg cgcctgatgc ggtattttct ccttacgcat ctgtgcggta 5880
   tttcacaccg catacgtcaa agcaaccata gtacgcgccc tgtagcggcg cattaagcgc 5940
   ggcgggtgtg gtggttacgc gcagcgtgac cgctacactt gccagcgccc tagcgcccgc 6000
   tcctttcgct ttcttccctt cctttctcgc cacgttcgcc ggctttcccc gtcaagctct 6060
   aaatcggggg ctccctttag ggttccgatt tagtgcttta cggcacctcg accccaaaaa 6120
   acttgatttg ggtgatggtt cacgtagtgg gccatcgccc tgatagacgg tttttcgccc 6180
   tttgacgttg gagtccacgt tctttaatag tggactcttg ttccaaactg gaacaacact 6240
   caaccctatc tcgggctatt cttttgattt ataagggatt ttgccgattt cggcctattg 6300
   gttaaaaaat gagctgattt aacaaaaatt taacgcgaat tttaacaaaa tattaacgtt 6360
   tacaatttta tggtgcactc tcagtacaat ctgctctgat gccgcatagt taagccagcc 6420
   ccgacacccg ccaacacccg ctgacgcgcc ctgacgggct tgtctgctcc cggcatccgc 6480
   ttacagacaa gctgtgaccg tctccgggag ctgcatgtgt cagaggtttt caccgtcatc 6540
   accgaaacgc gcgagacgaa agggcctcgt gatacgccta tttttatagg ttaatgtcat 6600
   gataataatg gtttcttaga cgtcaggtgg cacttttcgg ggaaatgtgc gcggaacccc 6660
   tatttgttta tttttctaaa tacattcaaa tatgtatccg ctcatgagac aataaccctg 6720
   ataaatgctt caataatatt gaaaaaggaa gagtatgagt attcaacatt tccgtgtcgc 6780
   ccttattccc ttttttgcgg cattttgcct tcctgttttt gctcacccag aaacgctggt 6840
   gaaagtaaaa gatgctgaag atcagttggg tgcacgagtg ggttacatcg aactggatct 6900
   caacagcggt aagatccttg agagttttcg ccccgaagaa cgttttccaa tgatgagcac 6960
   ttttaaagtt ctgctatgtg gcgcggtatt atcccgtatt gacgccgggc aagagcaact 7020
   cggtcgccgc atacactatt ctcagaatga cttggttgag tactcaccag tcacagaaaa 7080
   gcatcttacg gatggcatga cagtaagaga attatgcagt gctgccataa ccatgagtga 7140
   taacactgcg gccaacttac ttctgacaac gatcggagga ccgaaggagc taaccgcttt 7200
   tttgcacaac atgggggatc atgtaactcg ccttgatcgt tgggaaccgg agctgaatga 7260
   agccatacca aacgacgagc gtgacaccac gatgcctgta gcaatggcaa caacgttgcg 7320
   caaactatta actggcgaac tacttactct agcttcccgg caacaattaa tagactggat 7380
   ggaggcggat aaagttgcag gaccacttct gcgctcggcc cttccggctg gctggtttat 7440
   tgctgataaa tctggagccg gtgagcgtgg aagccgcggt atcattgcag cactggggcc 7500
   agatggtaag ccctcccgta tcgtagttat ctacacgacg gggagtcagg caactatgga 7560
   tgaacgaaat agacagatcg ctgagatagg tgcctcactg attaagcatt ggtaactgtc 7620
   agaccaagtt tactcatata tactttagat tgatttaaaa cttcattttt aatttaaaag 7680
   gatctaggtg aagatccttt ttgataatct catgaccaaa atcccttaac gtgagttttc 7740
   gttccactga gcgtcagacc ccgtagaaaa gatcaaagga tcttcttgag atcctttttt 7800
   tctgcgcgta atctgctgct tgcaaacaaa aaaaccaccg ctaccagcgg tggtttgttt 7860
   gccggatcaa gagctaccaa ctctttttcc gaaggtaact ggcttcagca gagcgcagat 7920
   accaaatact gtccttctag tgtagccgta gttaggccac cacttcaaga actctgtagc 7980
   accgcctaca tacctcgctc tgctaatcct gttaccagtg gctgctgcca gtggcgataa 8040
   gtcgtgtctt accgggttgg actcaagacg atagttaccg gataaggcgc agcggtcggg 8100
   ctgaacgggg ggttcgtgca cacagcccag cttggagcga acgacctaca ccgaactgag 8160
   atacctacag cgtgagctat gagaaagcgc cacgcttccc gaagggagaa aggcggacag 8220
   gtatccggta agcggcaggg tcggaacagg agagcgcacg agggagcttc cagggggaaa 8280
   cgcctggtat ctttatagtc ctgtcgggtt tcgccacctc tgacttgagc gtcgattttt 8340
   gtgatgctcg tcaggggggc ggagcctatg gaaaaacgcc agcaacgcgg cctttttacg 8400
   gttcctggcc ttttgctggc cttttgctca catgt 8435
<210> 146
   <211> 8435
   <212> DNA
   <213> Artificial Sequence
<220>
   <223> synthetic DNA
<400> 146
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg actcaccatg gtggcggagt tttagagcta gaaatagcaa gttaaaataa 300
   ggctagtccg ttatcaactt gaaaaagtgg caccgagtcg gtgctttttt gttttagagc 360
   tagaaatagc aagttaaaat aaggctagtc cgtttttagc gcgtgcgcca attctgcaga 420
   caaatggctc tagaggtacc cgttacataa cttacggtaa atggcccgcc tggctgaccg 480
   cccaacgacc cccgcccatt gacgtcaata gtaacgccaa tagggacttt ccattgacgt 540
   caatgggtgg agtatttacg gtaaactgcc cacttggcag tacatcaagt gtatcatatg 600
   ccaagtacgc cccctattga cgtcaatgac ggtaaatggc ccgcctggca ttgtgcccag 660
   tacatgacct tatgggactt tcctacttgg cagtacatct acgtattagt catcgctatt 720
   accatggtcg aggtgagccc cacgttctgc ttcactctcc ccatctcccc cccctcccca 780
   cccccaattt tgtatttatt tattttttaa ttattttgtg cagcgatggg ggcggggggg 840
   gggggggggc gcgcgccagg cggggcgggg cggggcgagg ggcggggcgg ggcgaggcgg 900
   agaggtgcgg cggcagccaa tcagagcggc gcgctccgaa agtttccttt tatggcgagg 960
   cggcggcggc ggcggcccta taaaaagcga agcgcgcggc gggcgggagt cgctgcgacg 1020
   ctgccttcgc cccgtgcccc gctccgccgc cgcctcgcgc cgcccgcccc ggctctgact 1080
   gaccgcgtta ctcccacagg tgagcgggcg ggacggccct tctcctccgg gctgtaatta 1140
   gctgagcaag aggtaagggt ttaagggatg gttggttggt ggggtattaa tgtttaatta 1200
   cctggagcac ctgcctgaaa tcactttttt tcaggttgga ccggtgccac catgtaccca 1260
   tacgatgttc cagattacgc ttcgccgaag aaaaagcgca aggtcgaagc gtccgacaag 1320
   aagtacagca tcggcctggc catcggcacc aactctgtgg gctgggccgt gatcaccgac 1380
   gagtacaagg tgcccagcaa gaaattcaag gtgctgggca acaccgaccg gcacagcatc 1440
   aagaagaacc tgatcggagc cctgctgttc gacagcggcg aaacagccga ggccacccgg 1500
   ctgaagagaa ccgccagaag aagatacacc agacggaaga accggatctg ctatctgcaa 1560
   gagatcttca gcaacgagat ggccaaggtg gacgacagct tcttccacag actggaagag 1620
   tccttcctgg tggaagagga taagaagcac gagcggcacc ccatcttcgg caacatcgtg 1680
   gacgaggtgg cctaccacga gaagtacccc accatctacc acctgagaaa gaaactggtg 1740
   gacagcaccg acaaggccga cctgcggctg atctatctgg ccctggccca catgatcaag 1800
   ttccggggcc acttcctgat cgagggcgac ctgaaccccg acaacagcga cgtggacaag 1860
   ctgttcatcc agctggtgca gacctacaac cagctgttcg aggaaaaccc catcaacgcc 1920
   agcggcgtgg acgccaaggc catcctgtct gccagactga gcaagagcag acggctggaa 1980
   aatctgatcg cccagctgcc cggcgagaag aagaatggcc tgttcggcaa cctgattgcc 2040
   ctgagcctgg gcctgacccc caacttcaag agcaacttcg acctggccga ggatgccaaa 2100
   ctgcagctga gcaaggacac ctacgacgac gacctggaca acctgctggc ccagatcggc 2160
   gaccagtacg ccgacctgtt tctggccgcc aagaacctgt ccgacgccat cctgctgagc 2220
   gacatcctga gagtgaacac cgagatcacc aaggcccccc tgagcgcctc tatgatcaag 2280
   agatacgacg agcaccacca ggacctgacc ctgctgaaag ctctcgtgcg gcagcagctg 2340
   cctgagaagt acaaagagat tttcttcgac cagagcaaga acggctacgc cggctacatt 2400
   gacggcggag ccagccagga agagttctac aagttcatca agcccatcct ggaaaagatg 2460
   gacggcaccg aggaactgct cgtgaagctg aacagagagg acctgctgcg gaagcagcgg 2520
   accttcgaca acggcagcat cccccaccag atccacctgg gagagctgca cgccattctg 2580
   cggcggcagg aagattttta cccattcctg aaggacaacc gggaaaagat cgagaagatc 2640
   ctgaccttcc gcatccccta ctacgtgggc cctctggcca ggggaaacag cagattcgcc 2700
   tggatgacca gaaagagcga ggaaaccatc accccctgga acttcgagga agtggtggac 2760
   aagggcgctt ccgcccagag cttcatcgag cggatgacca acttcgataa gaacctgccc 2820
   aacgagaagg tgctgcccaa gcacagcctg ctgtacgagt acttcaccgt gtataacgag 2880
   ctgaccaaag tgaaatacgt gaccgaggga atgagaaagc ccgccttcct gagcggcgag 2940
   cagaaaaagg ccatcgtgga cctgctgttc aagaccaacc ggaaagtgac cgtgaagcag 3000
   ctgaaagagg actacttcaa gaaaatcgag tgcttcgact ccgtggaaat ctccggcgtg 3060
   gaagatcggt tcaacgcctc cctgggcaca taccacgatc tgctgaaaat tatcaaggac 3120
   aaggacttcc tggacaatga ggaaaacgag gacattctgg aagatatcgt gctgaccctg 3180
   acactgtttg aggacagaga gatgatcgag gaacggctga aaacctatgc ccacctgttc 3240
   gacgacaaag tgatgaagca gctgaagcgg cggagataca ccggctgggg caggctgagc 3300
   cggaagctga tcaacggcat ccgggacaag cagtccggca agacaatcct ggatttcctg 3360
   aagtccgacg gcttcgccaa cagaaacttc atgcagctga tccacgacga cagcctgacc 3420
   tttaaagagg acatccagaa agcccaggtg tccggccagg gcgatagcct gcacgagcac 3480
   attgccaatc tggccggcag ccccgccatt aagaagggca tcctgcagac agtgaaggtg 3540
   gtggacgagc tcgtgaaagt gatgggccgg cacaagcccg agaacatcgt gatcgaaatg 3600
   gccagagaga accagaccac ccagaaggga cagaagaaca gccgcgagag aatgaagcgg 3660
   atcgaagagg gcatcaaaga gctgggcagc cagatcctga aagaacaccc cgtggaaaac 3720
   acccagctgc agaacgagaa gctgtacctg tactacctgc agaatgggcg ggatatgtac 3780
   gtggaccagg aactggacat caaccggctg tccgactacg atgtggacca tatcgtgcct 3840
   cagagctttc tgaaggacga ctccatcgac aacaaggtgc tgaccagaag cgacaagaac 3900
   cggggcaaga gcgacaacgt gccctccgaa gaggtcgtga agaagatgaa gaactactgg 3960
   cggcagctgc tgaacgccaa gctgattacc cagagaaagt tcgacaatct gaccaaggcc 4020
   gagagaggcg gcctgagcga actggataag gccggcttca tcaagagaca gctggtggaa 4080
   acccggcaga tcacaaagca cgtggcacag atcctggact cccggatgaa cactaagtac 4140
   gacgagaatg acaagctgat ccgggaagtg aaagtgatca ccctgaagtc caagctggtg 4200
   tccgatttcc ggaaggattt ccagttttac aaagtgcgcg agatcaacaa ctaccaccac 4260
   gcccacgacg cctacctgaa cgccgtcgtg ggaaccgccc tgatcaaaaa gtaccctaag 4320
   ctggaaagcg agttcgtgta cggcgactac aaggtgtacg acgtgcggaa gatgatcgcc 4380
   aagagcgagc aggaaatcgg caaggctacc gccaagtact tcttctacag caacatcatg 4440
   aactttttca agaccgagat taccctggcc aacggcgaga tccggaagcg gcctctgatc 4500
   gagacaaacg gcgaaaccgg ggagatcgtg tgggataagg gccgggattt tgccaccgtg 4560
   cggaaagtgc tgagcatgcc ccaagtgaat atcgtgaaaa agaccgaggt gcagacaggc 4620
   ggcttcagca aagagtctat cctgcccaag aggaacagcg ataagctgat cgccagaaag 4680
   aaggactggg accctaagaa gtacggcggc ttcgacagcc ccaccgtggc ctattctgtg 4740
   ctggtggtgg ccaaagtgga aaagggcaag tccaagaaac tgaagagtgt gaaagagctg 4800
   ctggggatca ccatcatgga aagaagcagc ttcgagaaga atcccatcga ctttctggaa 4860
   gccaagggct acaaagaagt gaaaaaggac ctgatcatca agctgcctaa gtactccctg 4920
   ttcgagctgg aaaacggccg gaagagaatg ctggcctctg ccggcgaact gcagaaggga 4980
   aacgaactgg ccctgccctc caaatatgtg aacttcctgt acctggccag ccactatgag 5040
   aagctgaagg gctcccccga ggataatgag cagaaacagc tgtttgtgga acagcacaag 5100
   cactacctgg acgagatcat cgagcagatc agcgagttct ccaagagagt gatcctggcc 5160
   gacgctaatc tggacaaagt gctgtccgcc tacaacaagc accgggataa gcccatcaga 5220
   gagcaggccg agaatatcat ccacctgttt accctgacca atctgggagc ccctgccgcc 5280
   ttcaagtact ttgacaccac catcgaccgg aagaggtaca ccagcaccaa agaggtgctg 5340
   gacgccaccc tgatccacca gagcatcacc ggcctgtacg agacacggat cgacctgtct 5400
   cagctgggag gcgacagccc caagaagaag agaaaggtgg aggccagcta agaattccta 5460
   gagctcgctg atcagcctcg actgtgcctt ctagttgcca gccatctgtt gtttgcccct 5520
   cccccgtgcc ttccttgacc ctggaaggtg ccactcccac tgtcctttcc taataaaatg 5580
   aggaaattgc atcgcattgt ctgagtaggt gtcattctat tctggggggt ggggtggggc 5640
   aggacagcaa gggggaggat tgggaagaga atagcaggca tgctggggag cggccgcagg 5700
   aacccctagt gatggagttg gccactccct ctctgcgcgc tcgctcgctc actgaggccg 5760
   ggcgaccaaa ggtcgcccga cgcccgggct ttgcccgggc ggcctcagtg agcgagcgag 5820
   cgcgcagctg cctgcagggg cgcctgatgc ggtattttct ccttacgcat ctgtgcggta 5880
   tttcacaccg catacgtcaa agcaaccata gtacgcgccc tgtagcggcg cattaagcgc 5940
   ggcgggtgtg gtggttacgc gcagcgtgac cgctacactt gccagcgccc tagcgcccgc 6000
   tcctttcgct ttcttccctt cctttctcgc cacgttcgcc ggctttcccc gtcaagctct 6060
   aaatcggggg ctccctttag ggttccgatt tagtgcttta cggcacctcg accccaaaaa 6120
   acttgatttg ggtgatggtt cacgtagtgg gccatcgccc tgatagacgg tttttcgccc 6180
   tttgacgttg gagtccacgt tctttaatag tggactcttg ttccaaactg gaacaacact 6240
   caaccctatc tcgggctatt cttttgattt ataagggatt ttgccgattt cggcctattg 6300
   gttaaaaaat gagctgattt aacaaaaatt taacgcgaat tttaacaaaa tattaacgtt 6360
   tacaatttta tggtgcactc tcagtacaat ctgctctgat gccgcatagt taagccagcc 6420
   ccgacacccg ccaacacccg ctgacgcgcc ctgacgggct tgtctgctcc cggcatccgc 6480
   ttacagacaa gctgtgaccg tctccgggag ctgcatgtgt cagaggtttt caccgtcatc 6540
   accgaaacgc gcgagacgaa agggcctcgt gatacgccta tttttatagg ttaatgtcat 6600
   gataataatg gtttcttaga cgtcaggtgg cacttttcgg ggaaatgtgc gcggaacccc 6660
   tatttgttta tttttctaaa tacattcaaa tatgtatccg ctcatgagac aataaccctg 6720
   ataaatgctt caataatatt gaaaaaggaa gagtatgagt attcaacatt tccgtgtcgc 6780
   ccttattccc ttttttgcgg cattttgcct tcctgttttt gctcacccag aaacgctggt 6840
   gaaagtaaaa gatgctgaag atcagttggg tgcacgagtg ggttacatcg aactggatct 6900
   caacagcggt aagatccttg agagttttcg ccccgaagaa cgttttccaa tgatgagcac 6960
   ttttaaagtt ctgctatgtg gcgcggtatt atcccgtatt gacgccgggc aagagcaact 7020
   cggtcgccgc atacactatt ctcagaatga cttggttgag tactcaccag tcacagaaaa 7080
   gcatcttacg gatggcatga cagtaagaga attatgcagt gctgccataa ccatgagtga 7140
   taacactgcg gccaacttac ttctgacaac gatcggagga ccgaaggagc taaccgcttt 7200
   tttgcacaac atgggggatc atgtaactcg ccttgatcgt tgggaaccgg agctgaatga 7260
   agccatacca aacgacgagc gtgacaccac gatgcctgta gcaatggcaa caacgttgcg 7320
   caaactatta actggcgaac tacttactct agcttcccgg caacaattaa tagactggat 7380
   ggaggcggat aaagttgcag gaccacttct gcgctcggcc cttccggctg gctggtttat 7440
   tgctgataaa tctggagccg gtgagcgtgg aagccgcggt atcattgcag cactggggcc 7500
   agatggtaag ccctcccgta tcgtagttat ctacacgacg gggagtcagg caactatgga 7560
   tgaacgaaat agacagatcg ctgagatagg tgcctcactg attaagcatt ggtaactgtc 7620
   agaccaagtt tactcatata tactttagat tgatttaaaa cttcattttt aatttaaaag 7680
   gatctaggtg aagatccttt ttgataatct catgaccaaa atcccttaac gtgagttttc 7740
   gttccactga gcgtcagacc ccgtagaaaa gatcaaagga tcttcttgag atcctttttt 7800
   tctgcgcgta atctgctgct tgcaaacaaa aaaaccaccg ctaccagcgg tggtttgttt 7860
   gccggatcaa gagctaccaa ctctttttcc gaaggtaact ggcttcagca gagcgcagat 7920
   accaaatact gtccttctag tgtagccgta gttaggccac cacttcaaga actctgtagc 7980
   accgcctaca tacctcgctc tgctaatcct gttaccagtg gctgctgcca gtggcgataa 8040
   gtcgtgtctt accgggttgg actcaagacg atagttaccg gataaggcgc agcggtcggg 8100
   ctgaacgggg ggttcgtgca cacagcccag cttggagcga acgacctaca ccgaactgag 8160
   atacctacag cgtgagctat gagaaagcgc cacgcttccc gaagggagaa aggcggacag 8220
   gtatccggta agcggcaggg tcggaacagg agagcgcacg agggagcttc caggggggaa 8280
   cgcctggtat ctttatagtc ctgtcgggtt tcgccacctc tgacttgagc gtcgattttt 8340
   gtgatgctcg tcaggggggc ggagcctatg gaaaaacgcc agcaacgcgg cctttttacg 8400
   gttcctggcc ttttgctggc cttttgctca catgt 8435
<210> 147
   <211> 30
   <212> DNA
   <213> Artificial Sequence
<220>
   <223> oligonucleotide
<400> 147
   agaccaggtt agccttgaac aaagatctcc 30
<210> 148
   <211> 6681
   <212> DNA
   <213> Artificial Sequence
<220>
   <223> synthetic DNA
<400> 148
   tatagtgagt cgtattacaa ttcactggcc gtcgttttac aacgtcgtga ctgggaaaac 60
   cctggcgtta cccaacttaa tcgccttgca gcacatcccc ctttcgccag ctggcgtaat 120
   agcgaagagg cccgcaccga tcgcccttcc caacagttgc gcagcctgaa tggcgaatgg 180
   acgcgccctg tagcggcgca ttaagcgcgg cgggtgtggt ggttacgcgc agcgtgaccg 240
   ctacacttgc cagcgcccta gcgcccgctc ctttcgcttt cttcccttcc tttctcgcca 300
   cgttcgccgg ctttccccgt caagctctaa atcgggggct ccctttaggg ttccgattta 360
   gtgctttacg gcacctcgac cccaaaaaac ttgattaggg tgatggttca cgtagtgggc 420
   catcgccctg atagacggtt tttcgccctt tgacgttgga gtccacgttc tttaatagtg 480
   gactcttgtt ccaaactgga acaacactca accctatctc ggtctattct tttgatttat 540
   aagggatttt gccgatttcg gcctattggt taaaaaatga gctgatttaa caaaaattta 600
   acgcgaattt taacaaaata ttaacgctta caatttcctg atgcggtatt ttctccttac 660
   gcatctgtgc ggtatttcac accgcatcag gtggcacttt tcggggaaat gtgcgcggaa 720
   cccctatttg tttatttttc taaatacatt caaatatgta tccgctcatg agacaataac 780
   cctgataaat gcttcaataa tattgaaaaa ggaagagtat gagtattcaa catttccgtg 840
   tcgcccttat tccctttttt gcggcatttt gccttcctgt ttttgctcac ccagaaacgc 900
   tggtgaaagt aaaagatgct gaagatcagt tgggtgcacg agtgggttac atcgaactgg 960
   atctcaacag cggtaagatc cttgagagtt ttcgccccga agaacgtttt ccaatgatga 1020
   gcacttttaa agttctgcta tgtggcgcgg tattatcccg tattgacgcc gggcaagagc 1080
   aactcggtcg ccgcatacac tattctcaga atgacttggt tgagtactca ccagtcacag 1140
   aaaagcatct tacggatggc atgacagtaa gagaattatg cagtgctgcc ataaccatga 1200
   gtgataacac tgcggccaac ttacttctga caacgatcgg aggaccgaag gagctaaccg 1260
   cttttttgca caacatgggg gatcatgtaa ctcgccttga tcgttgggaa ccggagctga 1320
   atgaagccat accaaacgac gagcgtgaca ccacgatgcc tgtagcaatg gcaacaacgt 1380
   tgcgcaaact attaactggc gaactactta ctctagcttc ccggcaacaa ttaatagact 1440
   ggatggaggc ggataaagtt gcaggaccac ttctgcgctc ggcccttccg gctggctggt 1500
   ttattgctga taaatctgga gccggtgagc gtgggtctcg cggtatcatt gcagcactgg 1560
   ggccagatgg taagccctcc cgtatcgtag ttatctacac gacggggagt caggcaacta 1620
   tggatgaacg aaatagacag atcgctgaga taggtgcctc actgattaag cattggtaac 1680
   tgtcagacca agtttactca tatatacttt agattgattt aaaacttcat ttttaattta 1740
   aaaggatcta ggtgaagatc ctttttgata atctcatgac caaaatccct taacgtgagt 1800
   tttcgttcca ctgagcgtca gaccccgtag aaaagatcaa aggatcttct tgagatcctt 1860
   tttttctgcg cgtaatctgc tgcttgcaaa caaaaaaacc accgctacca gcggtggttt 1920
   gtttgccgga tcaagagcta ccaactcttt ttccgaaggt aactggcttc agcagagcgc 1980
   agataccaaa tactgttctt ctagtgtagc cgtagttagg ccaccacttc aagaactctg 2040
   tagcaccgcc tacatacctc gctctgctaa tcctgttacc agtggctgct gccagtggcg 2100
   ataagtcgtg tcttaccggg ttggactcaa gacgatagtt accggataag gcgcagcggt 2160
   cgggctgaac ggggggttcg tgcacacagc ccagcttgga gcgaacgacc tacaccgaac 2220
   tgagatacct acagcgtgag ctatgagaaa gcgccacgct tcccgaaggg agaaaggcgg 2280
   acaggtatcc ggtaagcggc agggtcggaa caggagagcg cacgagggag cttccagggg 2340
   gaaacgcctg gtatctttat agtcctgtcg ggtttcgcca cctctgactt gagcgtcgat 2400
   ttttgtgatg ctcgtcaggg gggcggagcc tatggaaaaa cgccagcaac gcggcctttt 2460
   tacggttcct ggccttttgc tggccttttg ctcacatgtt ctttcctgcg ttatcccctg 2520
   attctgtgga taaccgtatt accgcctttg agtgagctga taccgctcgc cgcagccgaa 2580
   cgaccgagcg cagcgagtca gtgagcgagg aagcggaaga gcgcccaata cgcaaaccgc 2640
   ctctccccgc gcgttggccg attcattaat gcagctggca cgacaggttt cccgactgga 2700
   aagcgggcag tgagcgcaac gcaattaatg tgagttagct cactcattag gcaccccagg 2760
   ctttacactt tatgcttccg gctcgtatgt tgtgtggaat tgtgagcgga taacaatttc 2820
   acacaggaaa cagctatgac catgattacg ccaagctatt taggtgacac tatagaatac 2880
   tcaagctatg catccaacgc gttgggagct ctcccatatg gtcgacctgc aggcggccgc 2940
   gaattcacta gtgattaccg ttcgtatagc atacattata cgaagttata ccatgtctag 3000
   actggacaag agcaaagtca taaacggcgc tctggaatta ctcaatggag tcggtatcga 3060
   aggcctgacg acaaggaaac tcgctcaaaa gctgggagtt gagcagccta ccctgtactg 3120
   gcacgtgaag aacaagcggg ccctgctcga tgccctgcca atcgagatgc tggacaggca 3180
   tcatacccac ttctgccccc tggaaggcga gtcatggcaa gactttctgc ggaacaacgc 3240
   caagtcattc cgctgtgctc tcctctcaca tcgcgacggg gctaaagtgc atctcggcac 3300
   ccgcccaaca gagaaacagt acgaaaccct ggaaaatcag ctcgcgttcc tgtgtcagca 3360
   aggcttctcc ctggagaacg cactgtacgc tctgtccgcc gtgggccact ttacactggg 3420
   ctgcgtattg gaggaacagg agcatcaagt agcaaaagag gaaagagaga cacctaccac 3480
   cgattctatg cccccacttc tgagacaagc aattgagctg ttcgaccggc agggagccga 3540
   acctgccttc cttttcggcc tggaactaat catatgtggc ctggagaaac agctaaagtg 3600
   cgaaagcggc gggccggccg acgcccttga cgattttgac ttagacatgc tcccagccga 3660
   tgcccttgac gactttgacc ttgatatgct gcctgctgac gctcttgacg attttgacct 3720
   tgacatgctc cccgggtaac taagtaagga tcccaaatac cactgaatta agaattccgc 3780
   ccctctccct cccccccccc taacgttact ggccgaagcc gcttggaata aggccggtgt 3840
   gcgtttgtct atatgttatt ttccaccata ttgccgtctt ttggcaatgt gagggcccgg 3900
   aaacctggcc ctgtcttctt gacgagcatt cctaggggtc tttcccctct cgccaaagga 3960
   atgcaaggtc tgttgaatgt cgtgaaggaa gcagttcctc tggaagcttc ttgaagacaa 4020
   acaacgtctg tagcgaccct ttgcaggcag cggaaccccc cacctggcga caggtgcctc 4080
   tgcggccaaa agccacgtgt ataagataca cctgcaaagg cggcacaacc ccagtgccac 4140
   gttgtgagtt ggatagttgt ggaaagagtc aaatggctct cctcaagcgt attcaacaag 4200
   gggctgaagg atgcccagaa ggtaccccat tgtatgggat ctgatctggg gcctcggtgc 4260
   acatgcttta catgtgttta gtcgaggtta aaaaacgtct aggccccccg aaccacgggg 4320
   acgtggtttt cctttgaaaa acacgatgat aatatggcca caaccatgac cgagtacaag 4380
   cccacggtgc gcctcgccac ccgcgacgac gtcccccggg ccgtacgcac cctcgccgcc 4440
   gcgttcgccg actaccccgc cacgcgccac accgtcgacc cggaccgcca catcgagcgg 4500
   gtcaccgagc tgcaagaact cttcctcacg cgcgtcgggc tcgacatcgg caaggtgtgg 4560
   gtcgcggacg acggcgccgc ggtggcggtc tggaccacgc cggagagcgt cgaagcgggg 4620
   gcggtgttcg ccgagatcgg cccgcgcatg gccgagttga gcggttcccg gctggccgcg 4680
   cagcaacaga tggaaggcct cctggcgccg caccggccca aggagcccgc gtggttcctg 4740
   gccaccgtcg gcgtctcgcc cgaccaccag ggcaagggtc tgggcagcgc cgtcgtgctc 4800
   cccggagtgg aggcggccga gcgcgccggg gtgcccgcct tcctggagac ctccgcgccc 4860
   cgcaacctcc ccttctacga gcggctcggc ttcaccgtca ccgccgacgt cgaggtgccc 4920
   gaaggaccgc gcacctggtg catgacccgc aagcccggtg cctgaggatc cagacatgat 4980
   aagatacatt gatgagtttg gacaaaccac aactagaatg cagtgaaaaa aatgctttat 5040
   ttgtgaaatt tgtgatgcta ttgctttatt tgtaaccatt ataagctgca ataaacaagt 5100
   taacaacaac aattgcattc attttatgtt tcaggttcag ggggaggtgt gggaggtttt 5160
   ttaaagcaag taaaacctct acaaatgtgg tatggctgat tatgatcctc tagagtcgca 5220
   gatccagaca tgataagata cattgatgag tttggacaaa ccacaactag aatgcagtga 5280
   aaaaaatgct ttatttgtga aatttgtgat gctattgctt tatttgtaac cattataagc 5340
   tgcaataaac aagttaacaa caacaattgc attcatttta tgtttcaggt tcagggggag 5400
   gtgtgggagg ttttttaaag caagtaaaac ctctacaaat gtggtatggc tgattatgat 5460
   cctctagagt cgcagatcca gacatgataa gatacattga tgagtttgga caaaccacaa 5520
   ctagaatgca gtgaaaaaaa tgctttattt gtgaaatttg tgatgctatt gctttatttg 5580
   taaccattat aagctgcaat aaacaagtta acaacaacaa ttgcattcat tttatgtttc 5640
   aggttcaggg ggaggtgtgg gaggtttttt aaagcaagta aaacctctac aaatgtggta 5700
   tggctgatta tgatcctcta gagtcgcaga tcctctagag tcgcagatct ttttccctct 5760
   gccaaaaatt atggggacat catgaagccc cttgagcatc tgacttctgg ctaataaagg 5820
   aaatttattt tcattgcaat agtgtgttgg aattttttgt gtctctcact cggaaggaca 5880
   tatgggaggg caaatcattt aaaacatcag aatgagtatt tggtttagag tttggcaaca 5940
   tatgccatat gctggctgcc atgaacaaag gtggctataa agaggtcatc agtatatgaa 6000
   acagccccct gctgtccatt ccttattcca tagaaaagcc ttgacttgag gttagatttt 6060
   ttttatattt tgttttgtgt tatttttttc tttaacatcc ctaaaatttt ccttacatgt 6120
   tttactagcc agatttttcc tcctctcctg actactccca gtcatagctg tccctcttct 6180
   cttatgaaga tccctcgacc tgcagcccaa gcttatcgaa ttcccgcggt ggcggccgca 6240
   cgtctcccta tcagtgatag agaagtcgac acgtctcgag ctccctatca gtgatagaga 6300
   aggtacgtct agaacgtctc cctatcagtg atagagaagt cgacacgtct cgagctccct 6360
   atcagtgata gagaaggtac gtctagaacg tctccctatc agtgatagag aagtcgacac 6420
   gtctcgagct ccctatcagt gatagagaag gtacgtctag aacgtctccc tatcagtgat 6480
   agagaagtcg acacgtctcg agctccctat cagtgataga gaaggtaccc cctatataag 6540
   cagagctcgt ttagtgaacc gtcagatcgc ctggagacgc catccacgct gttttgacct 6600
   ccatagaaga caccgggacc gatccagcct ccgcggccgc catggcggcc gggagcatgc 6660
   gacgtcgggc ccaattcgcc c 6681
<210> 149
   <211> 8434
   <212> DNA
   <213> Artificial Sequence
<220>
   <223> synthetic DNA
<400> 149
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccg ggtcttcgag aagacctgtt ttagagctag aaatagcaag ttaaaataag     300
   gctagtccgt tatcaacttg aaaaagtggc accgagtcgg tgcttttttg ttttagagct     360
   agaaatagca agttaaaata aggctagtcc gtttttagcg cgtgcgccaa ttctgcagac     420
   aaatggctct agaggtaccc gttacataac ttacggtaaa tggcccgcct ggctgaccgc     480
   ccaacgaccc ccgcccattg acgtcaatag taacgccaat agggactttc cattgacgtc     540
   aatgggtgga gtatttacgg taaactgccc acttggcagt acatcaagtg tatcatatgc     600
   caagtacgcc ccctattgac gtcaatgacg gtaaatggcc cgcctggcat tgtgcccagt 660
   acatgacctt atgggacttt cctacttggc agtacatcta cgtattagtc atcgctatta 720
   ccatggtcga ggtgagcccc acgttctgct tcactctccc catctccccc ccctccccac 780
   ccccaatttt gtatttattt attttttaat tattttgtgc agcgatgggg gcgggggggg 840
   ggggggggcg cgcgccaggc ggggcggggc ggggcgaggg gcggggcggg gcgaggcgga 900
   gaggtgcggc ggcagccaat cagagcggcg cgctccgaaa gtttcctttt atggcgaggc 960
   ggcggcggcg gcggccctat aaaaagcgaa gcgcgcggcg ggcgggagtc gctgcgacgc 1020
   tgccttcgcc ccgtgccccg ctccgccgcc gcctcgcgcc gcccgccccg gctctgactg 1080
   accgcgttac tcccacaggt gagcgggcgg gacggccctt ctcctccggg ctgtaattag 1140
   ctgagcaaga ggtaagggtt taagggatgg ttggttggtg gggtattaat gtttaattac 1200
   ctggagcacc tgcctgaaat cacttttttt caggttggac cggtgccacc atgtacccat 1260
   acgatgttcc agattacgct tcgccgaaga aaaagcgcaa ggtcgaagcg tccgacaaga 1320
   agtacagcat cggcctggcc atcggcacca actctgtggg ctgggccgtg atcaccgacg 1380
   agtacaaggt gcccagcaag aaattcaagg tgctgggcaa caccgaccgg cacagcatca 1440
   agaagaacct gatcggagcc ctgctgttcg acagcggcga aacagccgag gccacccggc 1500
   tgaagagaac cgccagaaga agatacacca gacggaagaa ccggatctgc tatctgcaag 1560
   agatcttcag caacgagatg gccaaggtgg acgacagctt cttccacaga ctggaagagt 1620
   ccttcctggt ggaagaggat aagaagcacg agcggcaccc catcttcggc aacatcgtgg 1680
   acgaggtggc ctaccacgag aagtacccca ccatctacca cctgagaaag aaactggtgg 1740
   acagcaccga caaggccgac ctgcggctga tctatctggc cctggcccac atgatcaagt 1800
   tccggggcca cttcctgatc gagggcgacc tgaaccccga caacagcgac gtggacaagc 1860
   tgttcatcca gctggtgcag acctacaacc agctgttcga ggaaaacccc atcaacgcca 1920
   gcggcgtgga cgccaaggcc atcctgtctg ccagactgag caagagcaga cggctggaaa 1980
   atctgatcgc ccagctgccc ggcgagaaga agaatggcct gttcggcaac ctgattgccc 2040
   tgagcctggg cctgaccccc aacttcaaga gcaacttcga cctggccgag gatgccaaac 2100
   tgcagctgag caaggacacc tacgacgacg acctggacaa cctgctggcc cagatcggcg 2160
   accagtacgc cgacctgttt ctggccgcca agaacctgtc cgacgccatc ctgctgagcg 2220
   acatcctgag agtgaacacc gagatcacca aggcccccct gagcgcctct atgatcaaga 2280
   gatacgacga gcaccaccag gacctgaccc tgctgaaagc tctcgtgcgg cagcagctgc 2340
   ctgagaagta caaagagatt ttcttcgacc agagcaagaa cggctacgcc ggctacattg 2400
   acggcggagc cagccaggaa gagttctaca agttcatcaa gcccatcctg gaaaagatgg 2460
   acggcaccga ggaactgctc gtgaagctga acagagagga cctgctgcgg aagcagcgga 2520
   ccttcgacaa cggcagcatc ccccaccaga tccacctggg agagctgcac gccattctgc 2580
   ggcggcagga agatttttac ccattcctga aggacaaccg ggaaaagatc gagaagatcc 2640
   tgaccttccg catcccctac tacgtgggcc ctctggccag gggaaacagc agattcgcct 2700
   ggatgaccag aaagagcgag gaaaccatca ccccctggaa cttcgaggaa gtggtggaca 2760
   agggcgcttc cgcccagagc ttcatcgagc ggatgaccaa cttcgataag aacctgccca 2820
   acgagaaggt gctgcccaag cacagcctgc tgtacgagta cttcaccgtg tataacgagc 2880
   tgaccaaagt gaaatacgtg accgagggaa tgagaaagcc cgccttcctg agcggcgagc 2940
   agaaaaaggc catcgtggac ctgctgttca agaccaaccg gaaagtgacc gtgaagcagc 3000
   tgaaagagga ctacttcaag aaaatcgagt gcttcgactc cgtggaaatc tccggcgtgg 3060
   aagatcggtt caacgcctcc ctgggcacat accacgatct gctgaaaatt atcaaggaca 3120
   aggacttcct ggacaatgag gaaaacgagg acattctgga agatatcgtg ctgaccctga 3180
   cactgtttga ggacagagag atgatcgagg aacggctgaa aacctatgcc cacctgttcg 3240
   acgacaaagt gatgaagcag ctgaagcggc ggagatacac cggctggggc aggctgagcc 3300
   ggaagctgat caacggcatc cgggacaagc agtccggcaa gacaatcctg gatttcctga 3360
   agtccgacgg cttcgccaac agaaacttca tgcagctgat ccacgacgac agcctgacct 3420
   ttaaagagga catccagaaa gcccaggtgt ccggccaggg cgatagcctg cacgagcaca 3480
   ttgccaatct ggccggcagc cccgccatta agaagggcat cctgcagaca gtgaaggtgg 3540
   tggacgagct cgtgaaagtg atgggccggc acaagcccga gaacatcgtg atcgaaatgg 3600
   ccagagagaa ccagaccacc cagaagggac agaagaacag ccgcgagaga atgaagcgga 3660
   tcgaagaggg catcaaagag ctgggcagcc agatcctgaa agaacacccc gtggaaaaca 3720
   cccagctgca gaacgagaag ctgtacctgt actacctgca gaatgggcgg gatatgtacg 3780
   tggaccagga actggacatc aaccggctgt ccgactacga tgtggaccat atcgtgcctc 3840
   agagctttct gaaggacgac tccatcgaca acaaggtgct gaccagaagc gacaagaacc 3900
   ggggcaagag cgacaacgtg ccctccgaag aggtcgtgaa gaagatgaag aactactggc 3960
   ggcagctgct gaacgccaag ctgattaccc agagaaagtt cgacaatctg accaaggccg 4020
   agagaggcgg cctgagcgaa ctggataagg ccggcttcat caagagacag ctggtggaaa 4080
   cccggcagat cacaaagcac gtggcacaga tcctggactc ccggatgaac actaagtacg 4140
   acgagaatga caagctgatc cgggaagtga aagtgatcac cctgaagtcc aagctggtgt 4200
   ccgatttccg gaaggatttc cagttttaca aagtgcgcga gatcaacaac taccaccacg 4260
   cccacgacgc ctacctgaac gccgtcgtgg gaaccgccct gatcaaaaag taccctaagc 4320
   tggaaagcga gttcgtgtac ggcgactaca aggtgtacga cgtgcggaag atgatcgcca 4380
   agagcgagca ggaaatcggc aaggctaccg ccaagtactt cttctacagc aacatcatga 4440
   actttttcaa gaccgagatt accctggcca acggcgagat ccggaagcgg cctctgatcg 4500
   agacaaacgg cgaaaccggg gagatcgtgt gggataaggg ccgggatttt gccaccgtgc 4560
   ggaaagtgct gagcatgccc caagtgaata tcgtgaaaaa gaccgaggtg cagacaggcg 4620
   gcttcagcaa agagtctatc ctgcccaaga ggaacagcga taagctgatc gccagaaaga 4680
   aggactggga ccctaagaag tacggcggct tcgacagccc caccgtggcc tattctgtgc 4740
   tggtggtggc caaagtggaa aagggcaagt ccaagaaact gaagagtgtg aaagagctgc 4800
   tggggatcac catcatggaa agaagcagct tcgagaagaa tcccatcgac tttctggaag 4860
   ccaagggcta caaagaagtg aaaaaggacc tgatcatcaa gctgcctaag tactccctgt 4920
   tcgagctgga aaacggccgg aagagaatgc tggcctctgc cggcgaactg cagaagggaa 4980
   acgaactggc cctgccctcc aaatatgtga acttcctgta cctggccagc cactatgaga 5040
   agctgaaggg ctcccccgag gataatgagc agaaacagct gtttgtggaa cagcacaagc 5100
   actacctgga cgagatcatc gagcagatca gcgagttctc caagagagtg atcctggccg 5160
   acgctaatct ggacaaagtg ctgtccgcct acaacaagca ccgggataag cccatcagag 5220
   agcaggccga gaatatcatc cacctgttta ccctgaccaa tctgggagcc cctgccgcct 5280
   tcaagtactt tgacaccacc atcgaccgga agaggtacac cagcaccaaa gaggtgctgg 5340
   acgccaccct gatccaccag agcatcaccg gcctgtacga gacacggatc gacctgtctc 5400
   agctgggagg cgacagcccc aagaagaaga gaaaggtgga ggccagctaa gaattcctag 5460
   agctcgctga tcagcctcga ctgtgccttc tagttgccag ccatctgttg tttgcccctc 5520
   ccccgtgcct tccttgaccc tggaaggtgc cactcccact gtcctttcct aataaaatga 5580
   ggaaattgca tcgcattgtc tgagtaggtg tcattctatt ctggggggtg gggtggggca 5640
   ggacagcaag ggggaggatt gggaagagaa tagcaggcat gctggggagc ggccgcagga 5700
   acccctagtg atggagttgg ccactccctc tctgcgcgct cgctcgctca ctgaggccgg 5760
   gcgaccaaag gtcgcccgac gcccgggctt tgcccgggcg gcctcagtga gcgagcgagc 5820
   gcgcagctgc ctgcaggggc gcctgatgcg gtattttctc cttacgcatc tgtgcggtat 5880
   ttcacaccgc atacgtcaaa gcaaccatag tacgcgccct gtagcggcgc attaagcgcg 5940
   gcgggtgtgg tggttacgcg cagcgtgacc gctacacttg ccagcgccct agcgcccgct 6000
   cctttcgctt tcttcccttc ctttctcgcc acgttcgccg gctttccccg tcaagctcta 6060
   aatcgggggc tccctttagg gttccgattt agtgctttac ggcacctcga ccccaaaaaa 6120
   cttgatttgg gtgatggttc acgtagtggg ccatcgccct gatagacggt ttttcgccct 6180
   ttgacgttgg agtccacgtt ctttaatagt ggactcttgt tccaaactgg aacaacactc 6240
   aaccctatct cgggctattc ttttgattta taagggattt tgccgatttc ggcctattgg 6300
   ttaaaaaatg agctgattta acaaaaattt aacgcgaatt ttaacaaaat attaacgttt 6360
   acaattttat ggtgcactct cagtacaatc tgctctgatg ccgcatagtt aagccagccc 6420
   cgacacccgc caacacccgc tgacgcgccc tgacgggctt gtctgctccc ggcatccgct 6480
   tacagacaag ctgtgaccgt ctccgggagc tgcatgtgtc agaggttttc accgtcatca 6540
   ccgaaacgcg cgagacgaaa gggcctcgtg atacgcctat ttttataggt taatgtcatg 6600
   ataataatgg tttcttagac gtcaggtggc acttttcggg gaaatgtgcg cggaacccct 6660
   atttgtttat ttttctaaat acattcaaat atgtatccgc tcatgagaca ataaccctga 6720
   taaatgcttc aataatattg aaaaaggaag agtatgagta ttcaacattt ccgtgtcgcc 6780
   cttattccct tttttgcggc attttgcctt cctgtttttg ctcacccaga aacgctggtg 6840
   aaagtaaaag atgctgaaga tcagttgggt gcacgagtgg gttacatcga actggatctc 6900
   aacagcggta agatccttga gagttttcgc cccgaagaac gttttccaat gatgagcact 6960
   tttaaagttc tgctatgtgg cgcggtatta tcccgtattg acgccgggca agagcaactc 7020
   ggtcgccgca tacactattc tcagaatgac ttggttgagt actcaccagt cacagaaaag 7080
   catcttacgg atggcatgac agtaagagaa ttatgcagtg ctgccataac catgagtgat 7140
   aacactgcgg ccaacttact tctgacaacg atcggaggac cgaaggagct aaccgctttt 7200
   ttgcacaaca tgggggatca tgtaactcgc cttgatcgtt gggaaccgga gctgaatgaa 7260
   gccataccaa acgacgagcg tgacaccacg atgcctgtag caatggcaac aacgttgcgc 7320
   aaactattaa ctggcgaact acttactcta gcttcccggc aacaattaat agactggatg 7380
   gaggcggata aagttgcagg accacttctg cgctcggccc ttccggctgg ctggtttatt 7440
   gctgataaat ctggagccgg tgagcgtgga agccgcggta tcattgcagc actggggcca 7500
   gatggtaagc cctcccgtat cgtagttatc tacacgacgg ggagtcaggc aactatggat 7560
   gaacgaaata gacagatcgc tgagataggt gcctcactga ttaagcattg gtaactgtca 7620
   gaccaagttt actcatatat actttagatt gatttaaaac ttcattttta atttaaaagg 7680
   atctaggtga agatcctttt tgataatctc atgaccaaaa tcccttaacg tgagttttcg 7740
   ttccactgag cgtcagaccc cgtagaaaag atcaaaggat cttcttgaga tccttttttt 7800
   ctgcgcgtaa tctgctgctt gcaaacaaaa aaaccaccgc taccagcggt ggtttgtttg 7860
   ccggatcaag agctaccaac tctttttccg aaggtaactg gcttcagcag agcgcagata 7920
   ccaaatactg tccttctagt gtagccgtag ttaggccacc acttcaagaa ctctgtagca 7980
   ccgcctacat acctcgctct gctaatcctg ttaccagtgg ctgctgccag tggcgataag 8040
   tcgtgtctta ccgggttgga ctcaagacga tagttaccgg ataaggcgca gcggtcgggc 8100
   tgaacggggg gttcgtgcac acagcccagc ttggagcgaa cgacctacac cgaactgaga 8160
   tacctacagc gtgagctatg agaaagcgcc acgcttcccg aagggagaaa ggcggacagg      8220
   tatccggtaa gcggcagggt cggaacagga gagcgcacga gggagcttcc agggggaaac      8280
   gcctggtatc tttatagtcc tgtcgggttt cgccacctct gacttgagcg tcgatttttg      8340
   tgatgctcgt caggggggcg gagcctatgg aaaaacgcca gcaacgcggc ctttttacgg      8400
   ttcctggcct tttgctggcc ttttgctcac atgt                                  8434
<210> 150
   <211> 8506
   <212> DNA
   <213> Artificial Sequence
<220>
   <223> synthetic DNA
<400> 150
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg ggtcttcgag aagacctgtt ttagagctag aaatagcaag ttaaaataag 300
   gctagtccgt tatcaacttg aaaaagtggc accgagtcgg tgcttttttg ttttagagct 360
   agaaatagca agttaaaata aggctagtcc gtttttagcg cgtgcgccaa ttctgcagac 420
   aaatggctct agaggtaccc gttacataac ttacggtaaa tggcccgcct ggctgaccgc 480
   ccaacgaccc ccgcccattg acgtcaatag taacgccaat agggactttc cattgacgtc 540
   aatgggtgga gtatttacgg taaactgccc acttggcagt acatcaagtg tatcatatgc 600
   caagtacgcc ccctattgac gtcaatgacg gtaaatggcc cgcctggcat tgtgcccagt 660
   acatgacctt atgggacttt cctacttggc agtacatcta cgtattagtc atcgctatta 720
   ccatggtcga ggtgagcccc acgttctgct tcactctccc catctccccc ccctccccac 780
   ccccaatttt gtatttattt attttttaat tattttgtgc agcgatgggg gcgggggggg 840
   ggggggggcg cgcgccaggc ggggcggggc ggggcgaggg gcggggcggg gcgaggcgga 900
   gaggtgcggc ggcagccaat cagagcggcg cgctccgaaa gtttcctttt atggcgaggc 960
   ggcggcggcg gcggccctat aaaaagcgaa gcgcgcggcg ggcgggagtc gctgcgacgc 1020
   tgccttcgcc ccgtgccccg ctccgccgcc gcctcgcgcc gcccgccccg gctctgactg 1080
   accgcgttac tcccacaggt gagcgggcgg gacggccctt ctcctccggg ctgtaattag 1140
   ctgagcaaga ggtaagggtt taagggatgg ttggttggtg gggtattaat gtttaattac 1200
   ctggagcacc tgcctgaaat cacttttttt caggttggac cggtgccacc atggactata 1260
   aggaccacga cggagactac aaggatcatg atattgatta caaagacgat gacgataaga 1320
   tggccccaaa gaagaagcgg aaggtcggta tccacggagt cccagcagcc gacaagaagt 1380
   acagcatcgg cctggacatc ggcaccaact ctgtgggctg ggccgtgatc accgacgagt 1440
   acaaggtgcc cagcaagaaa ttcaaggtgc tgggcaacac cgaccggcac agcatcaaga 1500
   agaacctgat cggagccctg ctgttcgaca gcggcgaaac agccgaggcc acccggctga 1560
   agagaaccgc cagaagaaga tacaccagac ggaagaaccg gatctgctat ctgcaagaga 1620
   tcttcagcaa cgagatggcc aaggtggacg acagcttctt ccacagactg gaagagtcct 1680
   tcctggtgga agaggataag aagcacgagc ggcaccccat cttcggcaac atcgtggacg 1740
   aggtggccta ccacgagaag taccccacca tctaccacct gagaaagaaa ctggtggaca 1800
   gcaccgacaa ggccgacctg cggctgatct atctggccct ggcccacatg atcaagttcc 1860
   ggggccactt cctgatcgag ggcgacctga accccgacaa cagcgacgtg gacaagctgt 1920
   tcatccagct ggtgcagacc tacaaccagc tgttcgagga aaaccccatc aacgccagcg 1980
   gcgtggacgc caaggccatc ctgtctgcca gactgagcaa gagcagacgg ctggaaaatc 2040
   tgatcgccca gctgcccggc gagaagaaga atggcctgtt cggaaacctg attgccctga 2100
   gcctgggcct gacccccaac ttcaagagca acttcgacct ggccgaggat gccaaactgc 2160
   agctgagcaa ggacacctac gacgacgacc tggacaacct gctggcccag atcggcgacc 2220
   agtacgccga cctgtttctg gccgccaaga acctgtccga cgccatcctg ctgagcgaca 2280
   tcctgagagt gaacaccgag atcaccaagg cccccctgag cgcctctatg atcaagagat 2340
   acgacgagca ccaccaggac ctgaccctgc tgaaagctct cgtgcggcag cagctgcctg 2400
   agaagtacaa agagattttc ttcgaccaga gcaagaacgg ctacgccggc tacattgacg 2460
   gcggagccag ccaggaagag ttctacaagt tcatcaagcc catcctggaa aagatggacg 2520
   gcaccgagga actgctcgtg aagctgaaca gagaggacct gctgcggaag cagcggacct 2580
   tcgacaacgg cagcatcccc caccagatcc acctgggaga gctgcacgcc attctgcggc 2640
   ggcaggaaga tttttaccca ttcctgaagg acaaccggga aaagatcgag aagatcctga 2700
   ccttccgcat cccctactac gtgggccctc tggccagggg aaacagcaga ttcgcctgga 2760
   tgaccagaaa gagcgaggaa accatcaccc cctggaactt cgaggaagtg gtggacaagg 2820
   gcgcttccgc ccagagcttc atcgagcgga tgaccaactt cgataagaac ctgcccaacg 2880
   agaaggtgct gcccaagcac agcctgctgt acgagtactt caccgtgtat aacgagctga 2940
   ccaaagtgaa atacgtgacc gagggaatga gaaagcccgc cttcctgagc ggcgagcaga 3000
   aaaaggccat cgtggacctg ctgttcaaga ccaaccggaa agtgaccgtg aagcagctga 3060
   aagaggacta cttcaagaaa atcgagtgct tcgactccgt ggaaatctcc ggcgtggaag 3120
   atcggttcaa cgcctccctg ggcacatacc acgatctgct gaaaattatc aaggacaagg 3180
   acttcctgga caatgaggaa aacgaggaca ttctggaaga tatcgtgctg accctgacac 3240
   tgtttgagga cagagagatg atcgaggaac ggctgaaaac ctatgcccac ctgttcgacg 3300
   acaaagtgat gaagcagctg aagcggcgga gatacaccgg ctggggcagg ctgagccgga 3360
   agctgatcaa cggcatccgg gacaagcagt ccggcaagac aatcctggat ttcctgaagt 3420
   ccgacggctt cgccaacaga aacttcatgc agctgatcca cgacgacagc ctgaccttta 3480
   aagaggacat ccagaaagcc caggtgtccg gccagggcga tagcctgcac gagcacattg 3540
   ccaatctggc cggcagcccc gccattaaga agggcatcct gcagacagtg aaggtggtgg 3600
   acgagctcgt gaaagtgatg ggccggcaca agcccgagaa catcgtgatc gaaatggcca 3660
   gagagaacca gaccacccag aagggacaga agaacagccg cgagagaatg aagcggatcg 3720
   aagagggcat caaagagctg ggcagccaga tcctgaaaga acaccccgtg gaaaacaccc 3780
   agctgcagaa cgagaagctg tacctgtact acctgcagaa tgggcgggat atgtacgtgg 3840
   accaggaact ggacatcaac cggctgtccg actacgatgt ggaccatatc gtgcctcaga 3900
   gctttctgaa ggacgactcc atcgacaaca aggtgctgac cagaagcgac aagaaccggg 3960
   gcaagagcga caacgtgccc tccgaagagg tcgtgaagaa gatgaagaac tactggcggc 4020
   agctgctgaa cgccaagctg attacccaga gaaagttcga caatctgacc aaggccgaga 4080
   gaggcggcct gagcgaactg gataaggccg gcttcatcaa gagacagctg gtggaaaccc 4140
   ggcagatcac aaagcacgtg gcacagatcc tggactcccg gatgaacact aagtacgacg 4200
   agaatgacaa gctgatccgg gaagtgaaag tgatcaccct gaagtccaag ctggtgtccg 4260
   atttccggaa ggatttccag ttttacaaag tgcgcgagat caacaactac caccacgccc 4320
   acgacgccta cctgaacgcc gtcgtgggaa ccgccctgat caaaaagtac cctaagctgg 4380
   aaagcgagtt cgtgtacggc gactacaagg tgtacgacgt gcggaagatg atcgccaaga 4440
   gcgagcagga aatcggcaag gctaccgcca agtacttctt ctacagcaac atcatgaact 4500
   ttttcaagac cgagattacc ctggccaacg gcgagatccg gaagcggcct ctgatcgaga 4560
   caaacggcga aaccggggag atcgtgtggg ataagggccg ggattttgcc accgtgcgga 4620
   aagtgctgag catgccccaa gtgaatatcg tgaaaaagac cgaggtgcag acaggcggct 4680
   tcagcaaaga gtctatcctg cccaagagga acagcgataa gctgatcgcc agaaagaagg 4740
   actgggaccc taagaagtac ggcggcttcg acagccccac cgtggcctat tctgtgctgg 4800
   tggtggccaa agtggaaaag ggcaagtcca agaaactgaa gagtgtgaaa gagctgctgg 4860
   ggatcaccat catggaaaga agcagcttcg agaagaatcc catcgacttt ctggaagcca 4920
   agggctacaa agaagtgaaa aaggacctga tcatcaagct gcctaagtac tccctgttcg 4980
   agctggaaaa cggccggaag agaatgctgg cctctgccgg cgaactgcag aagggaaacg 5040
   aactggccct gccctccaaa tatgtgaact tcctgtacct ggccagccac tatgagaagc 5100
   tgaagggctc ccccgaggat aatgagcaga aacagctgtt tgtggaacag cacaagcact 5160
   acctggacga gatcatcgag cagatcagcg agttctccaa gagagtgatc ctggccgacg 5220
   ctaatctgga caaagtgctg tccgcctaca acaagcaccg ggataagccc atcagagagc 5280
   aggccgagaa tatcatccac ctgtttaccc tgaccaatct gggagcccct gccgccttca 5340
   agtactttga caccaccatc gaccggaaga ggtacaccag caccaaagag gtgctggacg 5400
   ccaccctgat ccaccagagc atcaccggcc tgtacgagac acggatcgac ctgtctcagc 5460
   tgggaggcga caaaaggccg gcggccacga aaaaggccgg ccaggcaaaa aagaaaaagt 5520
   aagaattcct agagctcgct gatcagcctc gactgtgcct tctagttgcc agccatctgt 5580
   tgtttgcccc tcccccgtgc cttccttgac cctggaaggt gccactccca ctgtcctttc 5640
   ctaataaaat gaggaaattg catcgcattg tctgagtagg tgtcattcta ttctgggggg 5700
   tggggtgggg caggacagca agggggagga ttgggaagag aatagcaggc atgctgggga 5760
   gcggccgcag gaacccctag tgatggagtt ggccactccc tctctgcgcg ctcgctcgct 5820
   cactgaggcc gggcgaccaa aggtcgcccg acgcccgggc tttgcccggg cggcctcagt 5880
   gagcgagcga gcgcgcagct gcctgcaggg gcgcctgatg cggtattttc tccttacgca 5940
   tctgtgcggt atttcacacc gcatacgtca aagcaaccat agtacgcgcc ctgtagcggc 6000
   gcattaagcg cggcgggtgt ggtggttacg cgcagcgtga ccgctacact tgccagcgcc 6060
   ctagcgcccg ctcctttcgc tttcttccct tcctttctcg ccacgttcgc cggctttccc 6120
   cgtcaagctc taaatcgggg gctcccttta gggttccgat ttagtgcttt acggcacctc 6180
   gaccccaaaa aacttgattt gggtgatggt tcacgtagtg ggccatcgcc ctgatagacg 6240
   gtttttcgcc ctttgacgtt ggagtccacg ttctttaata gtggactctt gttccaaact 6300
   ggaacaacac tcaaccctat ctcgggctat tcttttgatt tataagggat tttgccgatt 6360
   tcggcctatt ggttaaaaaa tgagctgatt taacaaaaat ttaacgcgaa ttttaacaaa 6420
   atattaacgt ttacaatttt atggtgcact ctcagtacaa tctgctctga tgccgcatag 6480
   ttaagccagc cccgacaccc gccaacaccc gctgacgcgc cctgacgggc ttgtctgctc 6540
   ccggcatccg cttacagaca agctgtgacc gtctccggga gctgcatgtg tcagaggttt 6600
   tcaccgtcat caccgaaacg cgcgagacga aagggcctcg tgatacgcct atttttatag 6660
   gttaatgtca tgataataat ggtttcttag acgtcaggtg gcacttttcg gggaaatgtg 6720
   cgcggaaccc ctatttgttt atttttctaa atacattcaa atatgtatcc gctcatgaga 6780
   caataaccct gataaatgct tcaataatat tgaaaaagga agagtatgag tattcaacat 6840
   ttccgtgtcg cccttattcc cttttttgcg gcattttgcc ttcctgtttt tgctcaccca 6900
   gaaacgctgg tgaaagtaaa agatgctgaa gatcagttgg gtgcacgagt gggttacatc 6960
   gaactggatc tcaacagcgg taagatcctt gagagttttc gccccgaaga acgttttcca 7020
   atgatgagca cttttaaagt tctgctatgt ggcgcggtat tatcccgtat tgacgccggg 7080
   caagagcaac tcggtcgccg catacactat tctcagaatg acttggttga gtactcacca 7140
   gtcacagaaa agcatcttac ggatggcatg acagtaagag aattatgcag tgctgccata 7200
   accatgagtg ataacactgc ggccaactta cttctgacaa cgatcggagg accgaaggag 7260
   ctaaccgctt ttttgcacaa catgggggat catgtaactc gccttgatcg ttgggaaccg 7320
   gagctgaatg aagccatacc aaacgacgag cgtgacacca cgatgcctgt agcaatggca 7380
   acaacgttgc gcaaactatt aactggcgaa ctacttactc tagcttcccg gcaacaatta 7440
   atagactgga tggaggcgga taaagttgca ggaccacttc tgcgctcggc ccttccggct 7500
   ggctggttta ttgctgataa atctggagcc ggtgagcgtg gaagccgcgg tatcattgca 7560
   gcactggggc cagatggtaa gccctcccgt atcgtagtta tctacacgac ggggagtcag 7620
   gcaactatgg atgaacgaaa tagacagatc gctgagatag gtgcctcact gattaagcat 7680
   tggtaactgt cagaccaagt ttactcatat atactttaga ttgatttaaa acttcatttt 7740
   taatttaaaa ggatctaggt gaagatcctt tttgataatc tcatgaccaa aatcccttaa 7800
   cgtgagtttt cgttccactg agcgtcagac cccgtagaaa agatcaaagg atcttcttga 7860
   gatccttttt ttctgcgcgt aatctgctgc ttgcaaacaa aaaaaccacc gctaccagcg 7920
   gtggtttgtt tgccggatca agagctacca actctttttc cgaaggtaac tggcttcagc 7980
   agagcgcaga taccaaatac tgtccttcta gtgtagccgt agttaggcca ccacttcaag 8040
   aactctgtag caccgcctac atacctcgct ctgctaatcc tgttaccagt ggctgctgcc 8100
   agtggcgata agtcgtgtct taccgggttg gactcaagac gatagttacc ggataaggcg 8160
   cagcggtcgg gctgaacggg gggttcgtgc acacagccca gcttggagcg aacgacctac 8220
   accgaactga gatacctaca gcgtgagcta tgagaaagcg ccacgcttcc cgaagggaga 8280
   aaggcggaca ggtatccggt aagcggcagg gtcggaacag gagagcgcac gagggagctt 8340
   ccagggggaa acgcctggta tctttatagt cctgtcgggt ttcgccacct ctgacttgag 8400
   cgtcgatttt tgtgatgctc gtcagggggg cggagcctat ggaaaaacgc cagcaacgcg 8460
   gcctttttac ggttcctggc cttttgctgg ccttttgctc acatgt 8506
<210> 151
   <211> 8436
   <212> DNA
   <213> Artificial Sequence
<220>
   <223> synthetic DNA
<400> 151
   gagggcctat ttcccatgat tccttcatat ttgcatatac gatacaaggc tgttagagag 60
   ataattggaa ttaatttgac tgtaaacaca aagatattag tacaaaatac gtgacgtaga 120
   aagtaataat ttcttgggta gtttgcagtt ttaaaattat gttttaaaat ggactatcat 180
   atgcttaccg taacttgaaa gtatttcgat ttcttggctt tatatatctt gtggaaagga 240
   cgaaacaccg gatcccggga gctttaccgg ttttagagct agaaatagca agttaaaata 300
   aggctagtcc gttatcaact tgaaaaagtg gcaccgagtc ggtgcttttt tgttttagag 360
   ctagaaatag caagttaaaa taaggctagt ccgtttttag cgcgtgcgcc aattctgcag 420
   acaaatggct ctagaggtac ccgttacata acttacggta aatggcccgc ctggctgacc 480
   gcccaacgac ccccgcccat tgacgtcaat agtaacgcca atagggactt tccattgacg 540
   tcaatgggtg gagtatttac ggtaaactgc ccacttggca gtacatcaag tgtatcatat 600
   gccaagtacg ccccctattg acgtcaatga cggtaaatgg cccgcctggc attgtgccca 660
   gtacatgacc ttatgggact ttcctacttg gcagtacatc tacgtattag tcatcgctat 720
   taccatggtc gaggtgagcc ccacgttctg cttcactctc cccatctccc ccccctcccc 780
   acccccaatt ttgtatttat ttatttttta attattttgt gcagcgatgg gggcgggggg 840
   gggggggggg cgcgcgccag gcggggcggg gcggggcgag gggcggggcg gggcgaggcg 900
   gagaggtgcg gcggcagcca atcagagcgg cgcgctccga aagtttcctt ttatggcgag 960
   gcggcggcgg cggcggccct ataaaaagcg aagcgcgcgg cgggcgggag tcgctgcgac 1020
   gctgccttcg ccccgtgccc cgctccgccg ccgcctcgcg ccgcccgccc cggctctgac 1080
   tgaccgcgtt actcccacag gtgagcgggc gggacggccc ttctcctccg ggctgtaatt 1140
   agctgagcaa gaggtaaggg tttaagggat ggttggttgg tggggtatta atgtttaatt 1200
   acctggagca cctgcctgaa atcacttttt ttcaggttgg accggtgcca ccatgtaccc 1260
   atacgatgtt ccagattacg cttcgccgaa gaaaaagcgc aaggtcgaag cgtccgacaa 1320
   gaagtacagc atcggcctgg ccatcggcac caactctgtg ggctgggccg tgatcaccga 1380
   cgagtacaag gtgcccagca agaaattcaa ggtgctgggc aacaccgacc ggcacagcat 1440
   caagaagaac ctgatcggag ccctgctgtt cgacagcggc gaaacagccg aggccacccg 1500
   gctgaagaga accgccagaa gaagatacac cagacggaag aaccggatct gctatctgca 1560
   agagatcttc agcaacgaga tggccaaggt ggacgacagc ttcttccaca gactggaaga 1620
   gtccttcctg gtggaagagg ataagaagca cgagcggcac cccatcttcg gcaacatcgt 1680
   ggacgaggtg gcctaccacg agaagtaccc caccatctac cacctgagaa agaaactggt 1740
   ggacagcacc gacaaggccg acctgcggct gatctatctg gccctggccc acatgatcaa 1800
   gttccggggc cacttcctga tcgagggcga cctgaacccc gacaacagcg acgtggacaa 1860
   gctgttcatc cagctggtgc agacctacaa ccagctgttc gaggaaaacc ccatcaacgc 1920
   cagcggcgtg gacgccaagg ccatcctgtc tgccagactg agcaagagca gacggctgga 1980
   aaatctgatc gcccagctgc ccggcgagaa gaagaatggc ctgttcggca acctgattgc 2040
   cctgagcctg ggcctgaccc ccaacttcaa gagcaacttc gacctggccg aggatgccaa 2100
   actgcagctg agcaaggaca cctacgacga cgacctggac aacctgctgg cccagatcgg 2160
   cgaccagtac gccgacctgt ttctggccgc caagaacctg tccgacgcca tcctgctgag 2220
   cgacatcctg agagtgaaca ccgagatcac caaggccccc ctgagcgcct ctatgatcaa 2280
   gagatacgac gagcaccacc aggacctgac cctgctgaaa gctctcgtgc ggcagcagct 2340
   gcctgagaag tacaaagaga ttttcttcga ccagagcaag aacggctacg ccggctacat 2400
   tgacggcgga gccagccagg aagagttcta caagttcatc aagcccatcc tggaaaagat 2460
   ggacggcacc gaggaactgc tcgtgaagct gaacagagag gacctgctgc ggaagcagcg 2520
   gaccttcgac aacggcagca tcccccacca gatccacctg ggagagctgc acgccattct 2580
   gcggcggcag gaagattttt acccattcct gaaggacaac cgggaaaaga tcgagaagat 2640
   cctgaccttc cgcatcccct actacgtggg ccctctggcc aggggaaaca gcagattcgc 2700
   ctggatgacc agaaagagcg aggaaaccat caccccctgg aacttcgagg aagtggtgga 2760
   caagggcgct tccgcccaga gcttcatcga gcggatgacc aacttcgata agaacctgcc 2820
   caacgagaag gtgctgccca agcacagcct gctgtacgag tacttcaccg tgtataacga 2880
   gctgaccaaa gtgaaatacg tgaccgaggg aatgagaaag cccgccttcc tgagcggcga 2940
   gcagaaaaag gccatcgtgg acctgctgtt caagaccaac cggaaagtga ccgtgaagca 3000
   gctgaaagag gactacttca agaaaatcga gtgcttcgac tccgtggaaa tctccggcgt 3060
   ggaagatcgg ttcaacgcct ccctgggcac ataccacgat ctgctgaaaa ttatcaagga 3120
   caaggacttc ctggacaatg aggaaaacga ggacattctg gaagatatcg tgctgaccct 3180
   gacactgttt gaggacagag agatgatcga ggaacggctg aaaacctatg cccacctgtt 3240
   cgacgacaaa gtgatgaagc agctgaagcg gcggagatac accggctggg gcaggctgag 3300
   ccggaagctg atcaacggca tccgggacaa gcagtccggc aagacaatcc tggatttcct 3360
   gaagtccgac ggcttcgcca acagaaactt catgcagctg atccacgacg acagcctgac 3420
   ctttaaagag gacatccaga aagcccaggt gtccggccag ggcgatagcc tgcacgagca 3480
   cattgccaat ctggccggca gccccgccat taagaagggc atcctgcaga cagtgaaggt 3540
   ggtggacgag ctcgtgaaag tgatgggccg gcacaagccc gagaacatcg tgatcgaaat 3600
   ggccagagag aaccagacca cccagaaggg acagaagaac agccgcgaga gaatgaagcg 3660
   gatcgaagag ggcatcaaag agctgggcag ccagatcctg aaagaacacc ccgtggaaaa 3720
   cacccagctg cagaacgaga agctgtacct gtactacctg cagaatgggc gggatatgta 3780
   cgtggaccag gaactggaca tcaaccggct gtccgactac gatgtggacc atatcgtgcc 3840
   tcagagcttt ctgaaggacg actccatcga caacaaggtg ctgaccagaa gcgacaagaa 3900
   ccggggcaag agcgacaacg tgccctccga agaggtcgtg aagaagatga agaactactg 3960
   gcggcagctg ctgaacgcca agctgattac ccagagaaag ttcgacaatc tgaccaaggc 4020
   cgagagaggc ggcctgagcg aactggataa ggccggcttc atcaagagac agctggtgga 4080
   aacccggcag atcacaaagc acgtggcaca gatcctggac tcccggatga acactaagta 4140
   cgacgagaat gacaagctga tccgggaagt gaaagtgatc accctgaagt ccaagctggt 4200
   gtccgatttc cggaaggatt tccagtttta caaagtgcgc gagatcaaca actaccacca 4260
   cgcccacgac gcctacctga acgccgtcgt gggaaccgcc ctgatcaaaa agtaccctaa 4320
   gctggaaagc gagttcgtgt acggcgacta caaggtgtac gacgtgcgga agatgatcgc 4380
   caagagcgag caggaaatcg gcaaggctac cgccaagtac ttcttctaca gcaacatcat 4440
   gaactttttc aagaccgaga ttaccctggc caacggcgag atccggaagc ggcctctgat 4500
   cgagacaaac ggcgaaaccg gggagatcgt gtgggataag ggccgggatt ttgccaccgt 4560
   gcggaaagtg ctgagcatgc cccaagtgaa tatcgtgaaa aagaccgagg tgcagacagg 4620
   cggcttcagc aaagagtcta tcctgcccaa gaggaacagc gataagctga tcgccagaaa 4680
   gaaggactgg gaccctaaga agtacggcgg cttcgacagc cccaccgtgg cctattctgt 4740
   gctggtggtg gccaaagtgg aaaagggcaa gtccaagaaa ctgaagagtg tgaaagagct 4800
   gctggggatc accatcatgg aaagaagcag cttcgagaag aatcccatcg actttctgga 4860
   agccaagggc tacaaagaag tgaaaaagga cctgatcatc aagctgccta agtactccct 4920
   gttcgagctg gaaaacggcc ggaagagaat gctggcctct gccggcgaac tgcagaaggg 4980
   aaacgaactg gccctgccct ccaaatatgt gaacttcctg tacctggcca gccactatga 5040
   gaagctgaag ggctcccccg aggataatga gcagaaacag ctgtttgtgg aacagcacaa 5100
   gcactacctg gacgagatca tcgagcagat cagcgagttc tccaagagag tgatcctggc 5160
   cgacgctaat ctggacaaag tgctgtccgc ctacaacaag caccgggata agcccatcag 5220
   agagcaggcc gagaatatca tccacctgtt taccctgacc aatctgggag cccctgccgc 5280
   cttcaagtac tttgacacca ccatcgaccg gaagaggtac accagcacca aagaggtgct 5340
   ggacgccacc ctgatccacc agagcatcac cggcctgtac gagacacgga tcgacctgtc 5400
   tcagctggga ggcgacagcc ccaagaagaa gagaaaggtg gaggccagct aagaattcct 5460
   agagctcgct gatcagcctc gactgtgcct tctagttgcc agccatctgt tgtttgcccc 5520
   tcccccgtgc cttccttgac cctggaaggt gccactccca ctgtcctttc ctaataaaat 5580
   gaggaaattg catcgcattg tctgagtagg tgtcattcta ttctgggggg tggggtgggg 5640
   caggacagca agggggagga ttgggaagag aatagcaggc atgctgggga gcggccgcag 5700
   gaacccctag tgatggagtt ggccactccc tctctgcgcg ctcgctcgct cactgaggcc 5760
   gggcgaccaa aggtcgcccg acgcccgggc tttgcccggg cggcctcagt gagcgagcga 5820
   gcgcgcagct gcctgcaggg gcgcctgatg cggtattttc tccttacgca tctgtgcggt 5880
   atttcacacc gcatacgtca aagcaaccat agtacgcgcc ctgtagcggc gcattaagcg 5940
   cggcgggtgt ggtggttacg cgcagcgtga ccgctacact tgccagcgcc ctagcgcccg 6000
   ctcctttcgc tttcttccct tcctttctcg ccacgttcgc cggctttccc cgtcaagctc 6060
   taaatcgggg gctcccttta gggttccgat ttagtgcttt acggcacctc gaccccaaaa 6120
   aacttgattt gggtgatggt tcacgtagtg ggccatcgcc ctgatagacg gtttttcgcc 6180
   ctttgacgtt ggagtccacg ttctttaata gtggactctt gttccaaact ggaacaacac 6240
   tcaaccctat ctcgggctat tcttttgatt tataagggat tttgccgatt tcggcctatt 6300
   ggttaaaaaa tgagctgatt taacaaaaat ttaacgcgaa ttttaacaaa atattaacgt 6360
   ttacaatttt atggtgcact ctcagtacaa tctgctctga tgccgcatag ttaagccagc 6420
   cccgacaccc gccaacaccc gctgacgcgc cctgacgggc ttgtctgctc ccggcatccg 6480
   cttacagaca agctgtgacc gtctccggga gctgcatgtg tcagaggttt tcaccgtcat 6540
   caccgaaacg cgcgagacga aagggcctcg tgatacgcct atttttatag gttaatgtca 6600
   tgataataat ggtttcttag acgtcaggtg gcacttttcg gggaaatgtg cgcggaaccc 6660
   ctatttgttt atttttctaa atacattcaa atatgtatcc gctcatgaga caataaccct 6720
   gataaatgct tcaataatat tgaaaaagga agagtatgag tattcaacat ttccgtgtcg 6780
   cccttattcc cttttttgcg gcattttgcc ttcctgtttt tgctcaccca gaaacgctgg 6840
   tgaaagtaaa agatgctgaa gatcagttgg gtgcacgagt gggttacatc gaactggatc 6900
   tcaacagcgg taagatcctt gagagttttc gccccgaaga acgttttcca atgatgagca 6960
   cttttaaagt tctgctatgt ggcgcggtat tatcccgtat tgacgccggg caagagcaac 7020
   tcggtcgccg catacactat tctcagaatg acttggttga gtactcacca gtcacagaaa 7080
   agcatcttac ggatggcatg acagtaagag aattatgcag tgctgccata accatgagtg 7140
   ataacactgc ggccaactta cttctgacaa cgatcggagg accgaaggag ctaaccgctt 7200
   ttttgcacaa catgggggat catgtaactc gccttgatcg ttgggaaccg gagctgaatg 7260
   aagccatacc aaacgacgag cgtgacacca cgatgcctgt agcaatggca acaacgttgc 7320
   gcaaactatt aactggcgaa ctacttactc tagcttcccg gcaacaatta atagactgga 7380
   tggaggcgga taaagttgca ggaccacttc tgcgctcggc ccttccggct ggctggttta 7440
   ttgctgataa atctggagcc ggtgagcgtg gaagccgcgg tatcattgca gcactggggc 7500
   cagatggtaa gccctcccgt atcgtagtta tctacacgac ggggagtcag gcaactatgg 7560
   atgaacgaaa tagacagatc gctgagatag gtgcctcact gattaagcat tggtaactgt      7620
   cagaccaagt ttactcatat atactttaga ttgatttaaa acttcatttt taatttaaaa      7680
   ggatctaggt gaagatcctt tttgataatc tcatgaccaa aatcccttaa cgtgagtttt      7740
   cgttccactg agcgtcagac cccgtagaaa agatcaaagg atcttcttga gatccttttt      7800
   ttctgcgcgt aatctgctgc ttgcaaacaa aaaaaccacc gctaccagcg gtggtttgtt      7860
   tgccggatca agagctacca actctttttc cgaaggtaac tggcttcagc agagcgcaga      7920
   taccaaatac tgtccttcta gtgtagccgt agttaggcca ccacttcaag aactctgtag      7980
   caccgcctac atacctcgct ctgctaatcc tgttaccagt ggctgctgcc agtggcgata      8040
   agtcgtgtct taccgggttg gactcaagac gatagttacc ggataaggcg cagcggtcgg      8100
   gctgaacggg gggttcgtgc acacagccca gcttggagcg aacgacctac accgaactga      8160
   gatacctaca gcgtgagcta tgagaaagcg ccacgcttcc cgaagggaga aaggcggaca      8220
   ggtatccggt aagcggcagg gtcggaacag gagagcgcac gagggagctt ccagggggaa      8280
   acgcctggta tctttatagt cctgtcgggt ttcgccacct ctgacttgag cgtcgatttt      8340
   tgtgatgctc gtcagggggg cggagcctat ggaaaaacgc cagcaacgcg gcctttttac      8400
   ggttcctggc cttttgctgg ccttttgctc acatgt                                8436
<210> 152
   <211> 8435
   <212> DNA
   <213> Artificial Sequence
<220>
   <223> synthetic DNA
<400> 152
   gagggcctat ttcccatgat tccttcatat ttgcatatac gatacaaggc tgttagagag      60
   ataattggaa ttaatttgac tgtaaacaca aagatattag tacaaaatac gtgacgtaga     120
   aagtaataat ttcttgggta gtttgcagtt ttaaaattat gttttaaaat ggactatcat     180
   atgcttaccg taacttgaaa gtatttcgat ttcttggctt tatatatctt gtggaaagga     240
   cgaaacaccc tcgccgcggt aaagctccgt tttagagcta gaaatagcaa gttaaaataa     300
   ggctagtccg ttatcaactt gaaaaagtgg caccgagtcg gtgctttttt gttttagagc     360
   tagaaatagc aagttaaaat aaggctagtc cgtttttagc gcgtgcgcca attctgcaga     420
   caaatggctc tagaggtacc cgttacataa cttacggtaa atggcccgcc tggctgaccg     480
   cccaacgacc cccgcccatt gacgtcaata gtaacgccaa tagggacttt ccattgacgt     540
   caatgggtgg agtatttacg gtaaactgcc cacttggcag tacatcaagt gtatcatatg     600
   ccaagtacgc cccctattga cgtcaatgac ggtaaatggc ccgcctggca ttgtgcccag     660
   tacatgacct tatgggactt tcctacttgg cagtacatct acgtattagt catcgctatt     720
   accatggtcg aggtgagccc cacgttctgc ttcactctcc ccatctcccc cccctcccca 780
   cccccaattt tgtatttatt tattttttaa ttattttgtg cagcgatggg ggcggggggg 840
   gggggggggc gcgcgccagg cggggcgggg cggggcgagg ggcggggcgg ggcgaggcgg 900
   agaggtgcgg cggcagccaa tcagagcggc gcgctccgaa agtttccttt tatggcgagg 960
   cggcggcggc ggcggcccta taaaaagcga agcgcgcggc gggcgggagt cgctgcgacg 1020
   ctgccttcgc cccgtgcccc gctccgccgc cgcctcgcgc cgcccgcccc ggctctgact 1080
   gaccgcgtta ctcccacagg tgagcgggcg ggacggccct tctcctccgg gctgtaatta 1140
   gctgagcaag aggtaagggt ttaagggatg gttggttggt ggggtattaa tgtttaatta 1200
   cctggagcac ctgcctgaaa tcactttttt tcaggttgga ccggtgccac catgtaccca 1260
   tacgatgttc cagattacgc ttcgccgaag aaaaagcgca aggtcgaagc gtccgacaag 1320
   aagtacagca tcggcctggc catcggcacc aactctgtgg gctgggccgt gatcaccgac 1380
   gagtacaagg tgcccagcaa gaaattcaag gtgctgggca acaccgaccg gcacagcatc 1440
   aagaagaacc tgatcggagc cctgctgttc gacagcggcg aaacagccga ggccacccgg 1500
   ctgaagagaa ccgccagaag aagatacacc agacggaaga accggatctg ctatctgcaa 1560
   gagatcttca gcaacgagat ggccaaggtg gacgacagct tcttccacag actggaagag 1620
   tccttcctgg tggaagagga taagaagcac gagcggcacc ccatcttcgg caacatcgtg 1680
   gacgaggtgg cctaccacga gaagtacccc accatctacc acctgagaaa gaaactggtg 1740
   gacagcaccg acaaggccga cctgcggctg atctatctgg ccctggccca catgatcaag 1800
   ttccggggcc acttcctgat cgagggcgac ctgaaccccg acaacagcga cgtggacaag 1860
   ctgttcatcc agctggtgca gacctacaac cagctgttcg aggaaaaccc catcaacgcc 1920
   agcggcgtgg acgccaaggc catcctgtct gccagactga gcaagagcag acggctggaa 1980
   aatctgatcg cccagctgcc cggcgagaag aagaatggcc tgttcggcaa cctgattgcc 2040
   ctgagcctgg gcctgacccc caacttcaag agcaacttcg acctggccga ggatgccaaa 2100
   ctgcagctga gcaaggacac ctacgacgac gacctggaca acctgctggc ccagatcggc 2160
   gaccagtacg ccgacctgtt tctggccgcc aagaacctgt ccgacgccat cctgctgagc 2220
   gacatcctga gagtgaacac cgagatcacc aaggcccccc tgagcgcctc tatgatcaag 2280
   agatacgacg agcaccacca ggacctgacc ctgctgaaag ctctcgtgcg gcagcagctg 2340
   cctgagaagt acaaagagat tttcttcgac cagagcaaga acggctacgc cggctacatt 2400
   gacggcggag ccagccagga agagttctac aagttcatca agcccatcct ggaaaagatg 2460
   gacggcaccg aggaactgct cgtgaagctg aacagagagg acctgctgcg gaagcagcgg 2520
   accttcgaca acggcagcat cccccaccag atccacctgg gagagctgca cgccattctg 2580
   cggcggcagg aagattttta cccattcctg aaggacaacc gggaaaagat cgagaagatc 2640
   ctgaccttcc gcatccccta ctacgtgggc cctctggcca ggggaaacag cagattcgcc 2700
   tggatgacca gaaagagcga ggaaaccatc accccctgga acttcgagga agtggtggac 2760
   aagggcgctt ccgcccagag cttcatcgag cggatgacca acttcgataa gaacctgccc 2820
   aacgagaagg tgctgcccaa gcacagcctg ctgtacgagt acttcaccgt gtataacgag 2880
   ctgaccaaag tgaaatacgt gaccgaggga atgagaaagc ccgccttcct gagcggcgag 2940
   cagaaaaagg ccatcgtgga cctgctgttc aagaccaacc ggaaagtgac cgtgaagcag 3000
   ctgaaagagg actacttcaa gaaaatcgag tgcttcgact ccgtggaaat ctccggcgtg 3060
   gaagatcggt tcaacgcctc cctgggcaca taccacgatc tgctgaaaat tatcaaggac 3120
   aaggacttcc tggacaatga ggaaaacgag gacattctgg aagatatcgt gctgaccctg 3180
   acactgtttg aggacagaga gatgatcgag gaacggctga aaacctatgc ccacctgttc 3240
   gacgacaaag tgatgaagca gctgaagcgg cggagataca ccggctgggg caggctgagc 3300
   cggaagctga tcaacggcat ccgggacaag cagtccggca agacaatcct ggatttcctg 3360
   aagtccgacg gcttcgccaa cagaaacttc atgcagctga tccacgacga cagcctgacc 3420
   tttaaagagg acatccagaa agcccaggtg tccggccagg gcgatagcct gcacgagcac 3480
   attgccaatc tggccggcag ccccgccatt aagaagggca tcctgcagac agtgaaggtg 3540
   gtggacgagc tcgtgaaagt gatgggccgg cacaagcccg agaacatcgt gatcgaaatg 3600
   gccagagaga accagaccac ccagaaggga cagaagaaca gccgcgagag aatgaagcgg 3660
   atcgaagagg gcatcaaaga gctgggcagc cagatcctga aagaacaccc cgtggaaaac 3720
   acccagctgc agaacgagaa gctgtacctg tactacctgc agaatgggcg ggatatgtac 3780
   gtggaccagg aactggacat caaccggctg tccgactacg atgtggacca tatcgtgcct 3840
   cagagctttc tgaaggacga ctccatcgac aacaaggtgc tgaccagaag cgacaagaac 3900
   cggggcaaga gcgacaacgt gccctccgaa gaggtcgtga agaagatgaa gaactactgg 3960
   cggcagctgc tgaacgccaa gctgattacc cagagaaagt tcgacaatct gaccaaggcc 4020
   gagagaggcg gcctgagcga actggataag gccggcttca tcaagagaca gctggtggaa 4080
   acccggcaga tcacaaagca cgtggcacag atcctggact cccggatgaa cactaagtac 4140
   gacgagaatg acaagctgat ccgggaagtg aaagtgatca ccctgaagtc caagctggtg 4200
   tccgatttcc ggaaggattt ccagttttac aaagtgcgcg agatcaacaa ctaccaccac 4260
   gcccacgacg cctacctgaa cgccgtcgtg ggaaccgccc tgatcaaaaa gtaccctaag 4320
   ctggaaagcg agttcgtgta cggcgactac aaggtgtacg acgtgcggaa gatgatcgcc 4380
   aagagcgagc aggaaatcgg caaggctacc gccaagtact tcttctacag caacatcatg 4440
   aactttttca agaccgagat taccctggcc aacggcgaga tccggaagcg gcctctgatc 4500
   gagacaaacg gcgaaaccgg ggagatcgtg tgggataagg gccgggattt tgccaccgtg 4560
   cggaaagtgc tgagcatgcc ccaagtgaat atcgtgaaaa agaccgaggt gcagacaggc 4620
   ggcttcagca aagagtctat cctgcccaag aggaacagcg ataagctgat cgccagaaag 4680
   aaggactggg accctaagaa gtacggcggc ttcgacagcc ccaccgtggc ctattctgtg 4740
   ctggtggtgg ccaaagtgga aaagggcaag tccaagaaac tgaagagtgt gaaagagctg 4800
   ctggggatca ccatcatgga aagaagcagc ttcgagaaga atcccatcga ctttctggaa 4860
   gccaagggct acaaagaagt gaaaaaggac ctgatcatca agctgcctaa gtactccctg 4920
   ttcgagctgg aaaacggccg gaagagaatg ctggcctctg ccggcgaact gcagaaggga 4980
   aacgaactgg ccctgccctc caaatatgtg aacttcctgt acctggccag ccactatgag 5040
   aagctgaagg gctcccccga ggataatgag cagaaacagc tgtttgtgga acagcacaag 5100
   cactacctgg acgagatcat cgagcagatc agcgagttct ccaagagagt gatcctggcc 5160
   gacgctaatc tggacaaagt gctgtccgcc tacaacaagc accgggataa gcccatcaga 5220
   gagcaggccg agaatatcat ccacctgttt accctgacca atctgggagc ccctgccgcc 5280
   ttcaagtact ttgacaccac catcgaccgg aagaggtaca ccagcaccaa agaggtgctg 5340
   gacgccaccc tgatccacca gagcatcacc ggcctgtacg agacacggat cgacctgtct 5400
   cagctgggag gcgacagccc caagaagaag agaaaggtgg aggccagcta agaattccta 5460
   gagctcgctg atcagcctcg actgtgcctt ctagttgcca gccatctgtt gtttgcccct 5520
   cccccgtgcc ttccttgacc ctggaaggtg ccactcccac tgtcctttcc taataaaatg 5580
   aggaaattgc atcgcattgt ctgagtaggt gtcattctat tctggggggt ggggtggggc 5640
   aggacagcaa gggggaggat tgggaagaga atagcaggca tgctggggag cggccgcagg 5700
   aacccctagt gatggagttg gccactccct ctctgcgcgc tcgctcgctc actgaggccg 5760
   ggcgaccaaa ggtcgcccga cgcccgggct ttgcccgggc ggcctcagtg agcgagcgag 5820
   cgcgcagctg cctgcagggg cgcctgatgc ggtattttct ccttacgcat ctgtgcggta 5880
   tttcacaccg catacgtcaa agcaaccata gtacgcgccc tgtagcggcg cattaagcgc 5940
   ggcgggtgtg gtggttacgc gcagcgtgac cgctacactt gccagcgccc tagcgcccgc 6000
   tcctttcgct ttcttccctt cctttctcgc cacgttcgcc ggctttcccc gtcaagctct 6060
   aaatcggggg ctccctttag ggttccgatt tagtgcttta cggcacctcg accccaaaaa 6120
   acttgatttg ggtgatggtt cacgtagtgg gccatcgccc tgatagacgg tttttcgccc 6180
   tttgacgttg gagtccacgt tctttaatag tggactcttg ttccaaactg gaacaacact 6240
   caaccctatc tcgggctatt cttttgattt ataagggatt ttgccgattt cggcctattg 6300
   gttaaaaaat gagctgattt aacaaaaatt taacgcgaat tttaacaaaa tattaacgtt 6360
   tacaatttta tggtgcactc tcagtacaat ctgctctgat gccgcatagt taagccagcc 6420
   ccgacacccg ccaacacccg ctgacgcgcc ctgacgggct tgtctgctcc cggcatccgc 6480
   ttacagacaa gctgtgaccg tctccgggag ctgcatgtgt cagaggtttt caccgtcatc 6540
   accgaaacgc gcgagacgaa agggcctcgt gatacgccta tttttatagg ttaatgtcat 6600
   gataataatg gtttcttaga cgtcaggtgg cacttttcgg ggaaatgtgc gcggaacccc 6660
   tatttgttta tttttctaaa tacattcaaa tatgtatccg ctcatgagac aataaccctg 6720
   ataaatgctt caataatatt gaaaaaggaa gagtatgagt attcaacatt tccgtgtcgc 6780
   ccttattccc ttttttgcgg cattttgcct tcctgttttt gctcacccag aaacgctggt 6840
   gaaagtaaaa gatgctgaag atcagttggg tgcacgagtg ggttacatcg aactggatct 6900
   caacagcggt aagatccttg agagttttcg ccccgaagaa cgttttccaa tgatgagcac 6960
   ttttaaagtt ctgctatgtg gcgcggtatt atcccgtatt gacgccgggc aagagcaact 7020
   cggtcgccgc atacactatt ctcagaatga cttggttgag tactcaccag tcacagaaaa 7080
   gcatcttacg gatggcatga cagtaagaga attatgcagt gctgccataa ccatgagtga 7140
   taacactgcg gccaacttac ttctgacaac gatcggagga ccgaaggagc taaccgcttt 7200
   tttgcacaac atgggggatc atgtaactcg ccttgatcgt tgggaaccgg agctgaatga 7260
   agccatacca aacgacgagc gtgacaccac gatgcctgta gcaatggcaa caacgttgcg 7320
   caaactatta actggcgaac tacttactct agcttcccgg caacaattaa tagactggat 7380
   ggaggcggat aaagttgcag gaccacttct gcgctcggcc cttccggctg gctggtttat 7440
   tgctgataaa tctggagccg gtgagcgtgg aagccgcggt atcattgcag cactggggcc 7500
   agatggtaag ccctcccgta tcgtagttat ctacacgacg gggagtcagg caactatgga 7560
   tgaacgaaat agacagatcg ctgagatagg tgcctcactg attaagcatt ggtaactgtc 7620
   agaccaagtt tactcatata tactttagat tgatttaaaa cttcattttt aatttaaaag 7680
   gatctaggtg aagatccttt ttgataatct catgaccaaa atcccttaac gtgagttttc 7740
   gttccactga gcgtcagacc ccgtagaaaa gatcaaagga tcttcttgag atcctttttt 7800
   tctgcgcgta atctgctgct tgcaaacaaa aaaaccaccg ctaccagcgg tggtttgttt 7860
   gccggatcaa gagctaccaa ctctttttcc gaaggtaact ggcttcagca gagcgcagat 7920
   accaaatact gtccttctag tgtagccgta gttaggccac cacttcaaga actctgtagc 7980
   accgcctaca tacctcgctc tgctaatcct gttaccagtg gctgctgcca gtggcgataa 8040
   gtcgtgtctt accgggttgg actcaagacg atagttaccg gataaggcgc agcggtcggg 8100
   ctgaacgggg ggttcgtgca cacagcccag cttggagcga acgacctaca ccgaactgag 8160
   atacctacag cgtgagctat gagaaagcgc cacgcttccc gaagggagaa aggcggacag 8220
   gtatccggta agcggcaggg tcggaacagg agagcgcacg agggagcttc cagggggaaa 8280
   cgcctggtat ctttatagtc ctgtcgggtt tcgccacctc tgacttgagc gtcgattttt      8340
   gtgatgctcg tcaggggggc ggagcctatg gaaaaacgcc agcaacgcgg cctttttacg      8400
   gttcctggcc ttttgctggc cttttgctca catgt                                  8435

## Description

### CROSS REFERENCE TO PRIOR APPLICATIONS

This application claims priority under the Paris Convention to US Provisional Patent Application 62/130,258, filed March 9, 2015, and US Provisional Patent Application 62/130,270, filed March 9, 2015.

### FIELD OF THE DISCLOSURE

The present description relates generally to the fields of cell and molecular biology. More particularly, the description relates to molecular tools, methods and kits for controlling division of animal cells and genetically modified cells related to same.

### BACKGROUND OF THE DISCLOSURE

Human pluripotent stem (hPS) cells, may be used as tools for understanding normal cellular development, disease development and for use in cellular therapeutics for treating currently incurable disorders, such as, for example, genetic disorders, degenerative diseases and/or various injuries. The pluripotent nature of these cells renders them able to differentiate into any cell type after a period of self-renewal in the stem cell state (Rossant and Nagy, 1999). The gold standard of hPS cells are the human embryonic stem (hES) cells reported in 1998 (Thomson et al., 1998). In 2006 and 2007 a method for reprogramming differentiated somatic cells, such as skin fibroblasts, into ES cell-like "induced pluripotent stem" (iPS) cells was reported and expanded the types of pluripotent cells (Takahashi and Yamanaka, 2006; Takahashi et al., 2007). The methods of generation of iPS cells and their applications toward many directions including cell-based therapies for treating diseases and aberrant physiological conditions have been developed further in the years since.

One concern regarding pluripotent cell-based therapies is safety. For example, malignant growth originating from a cell graft is of concern. The process of reprogramming differentiated cells into iPS cells is also relevant to safety, as it has been reported that reprogramming methods can cause genome damage and aberrant epigenetic changes (Hussein et al., 2011; Laurent et al., 2011; Lister et al., 2011), which may pose a risk for malignant transformation of iPS cell-derived cells.

One challenge with cell-based therapies involving pluripotent cells expanded *in vitro* is the pluripotent nature of the cells themselves. For example, if pluripotent cells remain among differentiated therapeutic cells, the pluripotent cells may develop into teratomas (Yoshida and Yamanaka, 2010). Attempts to increase the safety of pluripotent cell-derived products and therapies have included efforts to eliminate pluripotent cells from cell cultures after *in vitro* differentiation. For example: cytotoxic antibodies have been used to eliminate cells having pluripotent-specific antigens (Choo et al., 2008; Tan et al., 2009); cells have been sorted based on pluripotency cell surface markers (Ben-David et al., 2013a; Fong et al., 2009; Tang et al., 2011); tumour progression genes have been genetically altered in cells (Blum et al., 2009; Menendez et al., 2012); transgenes for assisting with separation of differentiated cells have been introduced into cells (Chung et al., 2006; Eiges et al., 2001; Huber et al., 2007); suicide genes have been introduced into cells and used to eliminate residual pluripotent stem cells after differentiation (Rong et al., 2012; Schuldiner et al., 2003); and undesired pluripotent cells have been ablated using chemicals (Ben-David et al., 2013b; Dabir et al., 2013; Tohyama et al., 2013). It is possible that even if residual pluripotent cells are eliminated from differentiated cultures, the differentiated derivatives of pluripotent cells may have oncogenic properties (Ghosh et al., 2011). Related oncogenic events could occur in therapeutic cells i) during *in vitro* preparation of cells; or ii) following grafting of cells into a host.

Most current strategies for eliminating or preventing unwanted cell growth and/or differentiation are based on the herpes simplex virus - thymidine kinase (HSV-TK) / ganciclovir (GCV) negatively selectable system, which may be used to eliminate a graft entirely, if malignancy develops (Schuldiner et al., 2003) or to eliminate only the pluripotent cells 'contaminating' the intended differentiated derivatives (Ben-David and Benvenisty, 2014; Lim et al., 2013). The mechanism of GCV-induced cell killing and apoptosis is well understood. It creates a replication-dependent formation of DNA double-strand breaks (Halloran and Fenton, 1998), which leads to apoptosis (Tomicic et al., 2002). However, many HSV-TK/GCV-based systems are unreliably expressed, at least because they rely on random integration or transient expression of HSV-TK. Strategies involving negative selectable markers with different killing mechanisms, such as, for example, Caspase 9 (Di Stasi et al., 2011) have been tested, but reliable expression of the negative selectable marker has not been shown. Cell-based therapies may require millions or billions of cells, which may amplify any issues caused by unwanted cell growth and/or differentiation.

It is an object of the present disclosure to mitigate and/or obviate one or more of the above deficiencies.

### SUMMARY OF THE DISCLOSURE

According to a first aspect, the present invention provides a method of controlling proliferation of an animal cell, the method comprising:
a) genetically modifying one or more endogenous genes in the animal cell selected from the group consisting of Cdk1, Top2A, Cenpa, Birc5, and Eef2, the genetic modification comprising a DNA sequence encoding a negative selectable marker that is transcriptionally linked to a DNA sequence encoding each of the one or more genes, such that the negative selectable marker and the one or more genes are co-expressed; and
b) controlling proliferation of the genetically modified animal cell with an inducer of the negative selectable marker.

The negative selectable marker may comprise herpes simplex virus-thymidine kinase, cytosine deaminase, carboxyl esterase, or iCasp9, wherein preferably the negative selectable marker is herpes simplex virus-thymidine kinase. The gene may be Cdk1 and the negative selectable marker may be herpes simplex virus-thymidine kinase.

Controlling proliferation of the genetically modified animal cell may comprise:
i) permitting proliferation of the cell by maintaining the cell in the absence of an inducer of the negative selectable marker; or
ii) ablating or inhibiting proliferation of the cell by exposing the cell to the inducer of the negative selectable marker.

The genetic modification of the one or more genes may comprise performing targeted modification of each of the one or more genes with a DNA vector comprising DNA encoding the negative selectable marker. The genetic modification may be homozygous, heterozygous, hemizygous or compound heterozygous.

In the method of the invention:
i) the negative selectable marker may comprise herpes simplex virus-thymidine kinase and the inducer comprises ganciclovir;
ii) the negative selectable marker may comprise cytosine deaminase and the inducer comprises 5-fluorocytosine;
iii) the negative selectable marker may comprise carboxyl esterase and the inducer comprises irinotecan; or
iv) the negative selectable marker may comprise iCasp9 and the inducer comprises AP1903.

In the method of the invention:
a) the animal cell may be a mammalian cell selected from the group consisting of a human, mouse, rat, hamster, guinea pig, cat, dog, cow, horse, deer, elk, bison, oxen, camel, llama, rabbit, pig, goat, sheep, and non-human primate cell or is an avian cell; or
b) the animal cell may be or may be derived from a pluripotent stem cell a multipotent cell, or a monopotent progenitor cell, or is a terminally differentiated cell.

In a second aspect, the invention provides a method of controlling proliferation of an animal cell population according to the method of the invention.

In a third aspect, the invention provides an isolated, genetically modified animal cell comprising a genetic modification of one or more endogenous genes selected from the group consisting of Cdk1, Top2A, Cenpa, Birc5, and Eef2, wherein the genetic modification comprises a DNA sequence encoding a negative selectable marker that is transcriptionally linked to a DNA sequence encoding each of the one or more genes such that the negative selectable marker and the one or more genes are co-expressed, and wherein, in the presence of an inducer of the negative selectable marker, the cell is killed or its proliferation inhibited. The negative selectable marker may comprise herpes simplex virus-thymidine kinase, cytosine deaminase, carboxyl esterase, or iCasp9, wherein preferably the negative selectable marker is herpes simplex virus-thymidine kinase. The essential gene may be Cdk1 and the negative selectable marker may be herpes simplex virus-thymidine kinase. The genetic modification of the one or more genes may be generated with a DNA vector comprising DNA encoding the negative selectable marker. The genetic modification may be homozygous, heterozygous, hemizygous or compound heterozygous.

In an embodiment, the invention provides the isolated, genetically modified animal cell of the third aspect of the invention wherein:
i) the negative selectable marker comprises herpes simplex virus-thymidine kinase and the inducer comprises ganciclovir;
ii) the negative selectable marker comprises cytosine deaminase and the inducer comprises 5-fluorocytosine;
iii) the negative selectable marker comprises carboxyl esterase and the inducer comprises irinotecan; or
iv) the negative selectable marker comprises iCasp9 and the inducer comprises AP1903.

In an embodiment, the invention provides the isolated, genetically modified animal cell of the third aspect of the invention wherein:
a) the animal cell is a mammalian cell selected from the group consisting of a human, mouse, rat, hamster, guinea pig, cat, dog, cow, horse, deer, elk, bison, oxen, camel, llama, rabbit, pig, goat, sheep, and non-human primate cell or is an avian cell; or
b) the animal cell is or is derived from a pluripotent stem cell a multipotent cell, or a monopotent progenitor cell, or is a terminally differentiated cell.

According to a fourth aspect, the invnetion provides a DNA vector for modifying expression of an endogenous gene selected from the group consisting of Cdk1, Top2A, Cenpa, Birc5, and Eef2 in an animal cell, comprising a DNA sequence encoding a negative selectable marker that is transcriptionally linked to a DNA sequence encoding the gene,
wherein integration of the DNA sequence encoding the negative selectable marker that is transcriptionally linked to a DNA sequence encoding the gene into one or more host cells promotes death of proliferating host cells upon exposure of the host cells to an inducer of the negative selectable marker. The negative selectable marker may comprise herpes simplex virus-thymidine kinase, cytosine deaminase, carboxyl esterase, or iCasp9, wherein preferably the negative selectable marker is herpes simplex virus-thymidine kinase. The gene may ve Cdk1 and the negative selectable marker may be herpes simplex virus-thymidine kinase.

In an embodiment, the invention provides the DNA vector of the fourth aspect of the invention, wherein:
i) the negative selectable marker comprises herpes simplex virus-thymidine kinase and the inducer comprises ganciclovir;
ii) the negative selectable marker comprises cytosine deaminase and the inducer comprises 5-fluorocytosine;
iii) the negative selectable marker comprises carboxyl esterase and the inducer comprises irinotecan; or
iv) the negative selectable marker comprises iCasp9 and the inducer comprises AP1903.

The invention further provides an animal cell of the invention for use in medicine.

Disclosed herein is a method of controlling proliferation of an animal cell. The method comprises: providing an animal cell; genetically modifying in the animal cell a cell division locus (CDL), the CDL being one or more loci whose transcription product(s) is expressed by dividing cells; the genetic modification of the CDL comprising one or more of: a) an ablation link (ALINK) system, the ALINK system comprising a DNA sequence encoding a negative selectable marker that is transcriptionally linked to a DNA sequence encoding the CDL; and b) an inducible exogenous activator of regulation of a CDL (EARC) system, the EARC system comprising an inducible activator-based gene expression system that is operably linked to the CDL; controlling proliferation of the genetically modified animal cell comprising the ALINK system with an inducer of the negative selectable marker; and/or controlling proliferation of the genetically modified animal cell comprising the EARC system with an inducer of the inducible activator-based gene expression system.

The method of controlling proliferation of an animal cell provided herein may comprise, the controlling of the ALINK-modified animal cell which may comprise one or more of: permitting proliferation of the genetically modified animal cell comprising the ALINK system by maintaining the genetically modified animal cell comprising the ALINK system in the absence of an inducer of the negative selectable marker; and ablating or inhibiting proliferation of the genetically modified animal cell comprising the ALINK system by exposing the animal cell comprising the ALINK system to the inducer of the negative selectable marker.

The method of controlling proliferation of an animal cell provided herein may comprise the controlling of the EARC-modified animal cell which may comprise one or more of: permitting proliferation of the genetically modified animal cell comprising the EARC system by exposing the genetically modified animal cell comprising the EARC system to an inducer of the inducible activator-based gene expression system; and preventing or inhibiting proliferation of the genetically modified animal cell comprising the EARC system by maintaining the animal cell comprising the EARC system in the absence of the inducer of the inducible activator-based gene expression system.

In the method of controlling proliferation of an animal cell provided herein, the genetic modification of the CDL may comprise preforming targeted replacement of the CDL with one or more of: a) a DNA vector comprising the ALINK system; b) a DNA vector comprising the EARC system; and c) a DNA vector comprising the ALINK system and the EARC system.

In the method of controlling proliferation of an animal cell provided herein, the ALINK genetic modification of the CDL may be homozygous, heterozygous, hemizygous or compound heterozygous and/or wherein the EARC genetic modification ensures that functional CDL modification can only be generated through EARC-modified alleles.

In the method of controlling proliferation of an animal cell provided herein, the CDL may be one or more loci recited in Table 2. The CDL may encode a gene product whose function is involved with one or more of: cell cycle, DNA replication, RNA transcription, protein translation, and metabolism. The CDL may be one or more of Cdk1/CDK1,Top2A/TOP2A, Cenpa/CENPA, Birc5/BIRC5, and Eef2/EEF2, preferably the CDL is Cdk1 or CDK1.

In the method of controlling proliferation of an animal cell provided herein, the ALINK system may comprise a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In the method of controlling proliferation of an animal cell provided herein, the EARC system may be a dox-bridge system, a cumate switch inducible system, an ecdysone inducible system, a radio wave inducible system, or a ligand-reversible dimerization system, preferably the EARC system is a dox-bridge system.

In the method of controlling proliferation of an animal cell provided herein, the animal cell may be a mammalian cell or an avian cell.The mammalian cell may be a human, mouse, rat, hamster, guinea pig, cat, dog, cow, horse, deer, elk, bison, oxen, camel, llama, rabbit, pig, goat, sheep, or non-human primate cell, preferably the mammalian cell is a human cell.

In the method of controlling proliferation of an animal cell provided herein, the animal cell may be a pluripotent stem cell a multipotent cell, a monopotent progenitor cell, or a terminally differentiated cell.

In the method of controlling proliferation of an animal cell provided herein, the animal cell may be derived from a pluripotent stem cell, a multipotent cell, a monopotent progenitor cell, or a terminally differentiated cell.

As disclosed herein, an animal cell may be genetically modified to comprise at least one mechanism for controlling cell proliferation is provided. The genetically modified animal cell may comprise: a genetic modification of one or more cell division locus (CDL), the CDL being one or more loci whose transcription product(s) is expressed by dividing cells. The genetic modification may be one or more of: a) an ablation link (ALINK) system, the ALINK system comprising a DNA sequence encoding a negative selectable marker that is transcriptionally linked to a DNA sequence encoding the CDL; and b) an exogenous activator of regulation of a CEDL (EARC) system, the EARC system comprising an inducible activator-based gene expression system that is operably linked to the CDL.

In the animal cell genetically modified to comprise at least one mechanism for controlling cell proliferation provided herein, the genetic modification of the CDL may comprise preforming targeted replacement of the CDL with one or more of: a) a DNA vector comprising the ALINK system; b) a DNA vector comprising the EARC system; and c) a DNA vector comprising the ALINK system and the EARC system.

In the animal cell genetically modified to comprise at least one mechanism for controlling cell proliferation provided herein, the ALINK genetic modification of the CDL may be homozygous, heterozygous, hemizygous or compound heterozygous and/or wherein the EARC genetic modification ensures that functional CDL modification can only be generated through EARC-modified alleles.

In the animal cell genetically modified to comprise at least one mechanism for controlling cell proliferation provided herein, the CDL may be one or more loci recited in Table 2. The CDL may encode a gene product whose function is involved with one or more of: cell cycle, DNA replication, RNA transcription, protein translation, and metabolism. The CDL may be one or more of Cdk1/CDK1, Top2A/TOP2A, Cenpa/CENPA, Birc5/BIRC5, and Eef2/EEF2, preferably the CDL is Cdk1 or CDK1.

In the animal cell genetically modified to comprise at least one mechanism for controlling cell proliferation provided herein, the ALINK system may comprise a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In the animal cell genetically modified to comprise at least one mechanism for controlling cell proliferation provided herein, the EARC system may be a dox-bridge system, a cumate switch inducible system, an ecdysone inducible system, a radio wave inducible system, or a ligand-reversible dimerization system, preferably the EARC system is a dox-bridge system.

In the animal cell genetically modified to comprise at least one mechanism for controlling cell proliferation provided herein, the animal cell may be a mammalian cell or an avian cell.The mammalian cell may be a human, mouse, rat, hamster, guinea pig, cat, dog, cow, horse, deer, elk, bison, oxen, camel, llama, rabbit, pig, goat, sheep, or non-human primate cell, preferably the mammalian cell is a human cell.

In the animal cell genetically modified to comprise at least one mechanism for controlling cell proliferation provided herein, the animal cell may be a pluripotent stem cell a multipotent cell, a monopotent progenitor cell, or a terminally differentiated cell.

In the animal cell genetically modified to comprise at least one mechanism for controlling cell proliferation provided herein, the animal cell may be derived from a pluripotent stem cell, a multipotent cell, a monopotent progenitor cell, or a terminally differentiated cell.

The disclosure further provides a DNA vector for modifying expression of a cell division locus (CDL), the CDL may be one or more loci whose transcription product(s) is expressed by dividing cells is provided. The DNA vector may comprise: an ablation link (ALINK) system, the ALINK system comprising a DNA sequence encoding a negative selectable marker that is transcriptionally linked to the CDL, wherein if the DNA vector is inserted into one or more host cells, proliferating host cells comprising the DNA vector will be killed if the proliferating host cells comprising the DNA vector are exposed to an inducer of the negative selectable marker.

In a DNA vector for modifying expression of a cell division essential locus (CDL) disclosed herein, the CDL may be one or more loci whose transcription product(s) is expressed by dividing cells. The DNA vector may comprise: an exogenous activator of regulation of a CDL (EARC) system, the EARC system may comprise an inducible activator-based gene expression system that is operably linked to the CDL, wherein if the DNA vector is inserted into one or more host cells, proliferating host cells comprising the DNA vector will be killed if the proliferating host cells comprising the DNA vector are not exposed to an inducer of the inducible activator-based gene expression system.

In a DNA vector for modifying expression of a cell division essential locus (CDL) disclosed herein, the CDL may be one or more loci whose transcription product(s) is expressed by dividing cells. The DNA vector may comprise: an ablation link (ALINK) system, the ALINK system may be a DNA sequence encoding a negative selectable marker that is transcriptionally linked to the CDL; and an exogenous activator of regulation of CDL (EARC) system, the EARC system comprising an inducible activator-based gene expression system that is operably linked to the CDL, wherein if the DNA vector is inserted into one or more host cells, proliferating host cells comprising the DNA vector will be killed if the proliferating host cells comprising the DNA vector are exposed to an inducer of the negative selectable marker and if the proliferating host cells comprising the DNA vector are not exposed to an inducer of the inducible activator-based gene expression system.

In the DNA vectors provided herein, the CDL may be one or more loci recited in Table 2. The CDL may encode a gene product whose function is involved with one or more of: cell cycle, DNA replication, RNA transcription, protein translation, and metabolism. The CDL may be one or more of Cdk1/CDK1, Top2A/TOP2A, Cenpa/CENPA, Birc5/BIRC5, and Eef2/EEF2, preferably the CDL is Cdk1 or CDK1.

In the DNA vectors provided herein, the ALINK system may comprise a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In the DNA vectors provided herein, the EARC system may be a dox-bridge system, a cumate switch inducible system, an ecdysone inducible system, a radio wave inducible system, or a ligand-reversible dimerization system, preferably the EARC system is a dox-bridge system.

Also disclosed herein is a kit for controlling proliferation of an animal cell by genetically modifying one or more cell division essential locus/loci (CDL), the CDL being one or more loci whose transcription product(s) is expressed by dividing cells is provided. The kit may comprise: a DNA vector comprising an ablation link (ALINK) system, the ALINK system comprising a DNA sequence encoding a negative selectable marker that is transcriptionally linked to a DNA sequence encoding the CDL; and/or a DNA vector comprising an exogenous activator of regulation of a CDL (EARC) system, the EARC system comprising an inducible activator-based gene expression system that is operably linked to the CDL; and/or a DNA vector comprising an ALINK system and an EARC system, the ALINK and EARC systems each being operably linked to the CDL; and instructions for targeted replacement of the CDL in an animal cell using one or more of the DNA vectors.

In the kit provided herein, the CDL may be one or more loci recited in Table 2. The CDL may encode a gene product whose function is involved with one or more of: cell cycle, DNA replication, RNA transcription, protein translation, and metabolism. The CDL may be one or more of Cdk1/CDK1,Top2A/TOP2A, Cenpa/CENPA, Birc5/BIRC5, and Eef2/EEF2, preferably the CDL is Cdk1 or CDK1.

In the kit provided herein, the ALINK system may comprise a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In the kit provided herein, the EARC system may be a dox-bridge system, a cumate switch inducible system, an ecdysone inducible system, a radio wave inducible system, or a ligand-reversible dimerization system, preferably the EARC system is a dox-bridge system.

### DESCRIPTION OF THE DRAWINGS

The patent or application file contains at least one drawing in color. Copies of this patent or patent application publication with color drawings will be provided by the Office upon request and payment of the necessary fee.

These and other features of the disclosure will become more apparent in the following detailed description in which reference is made to the appended drawings wherein:

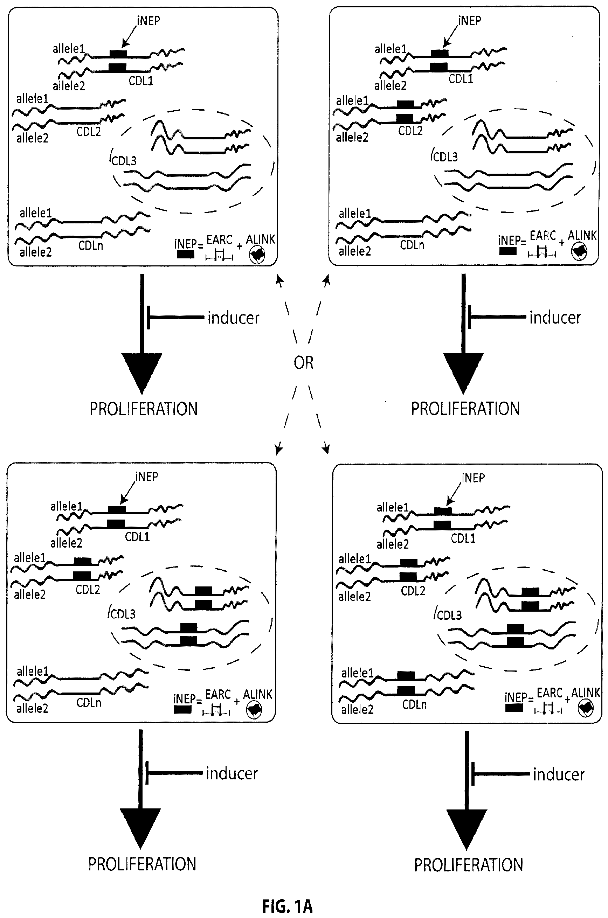

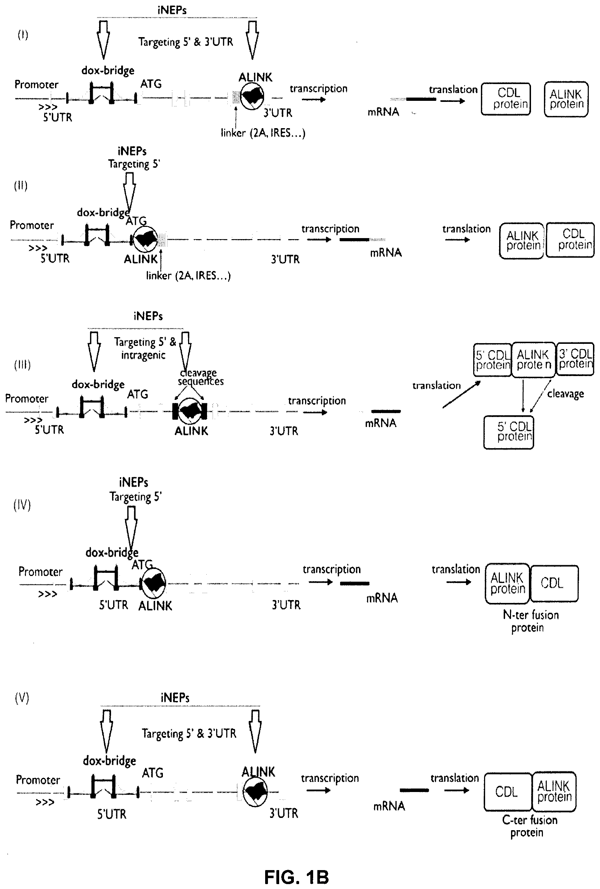

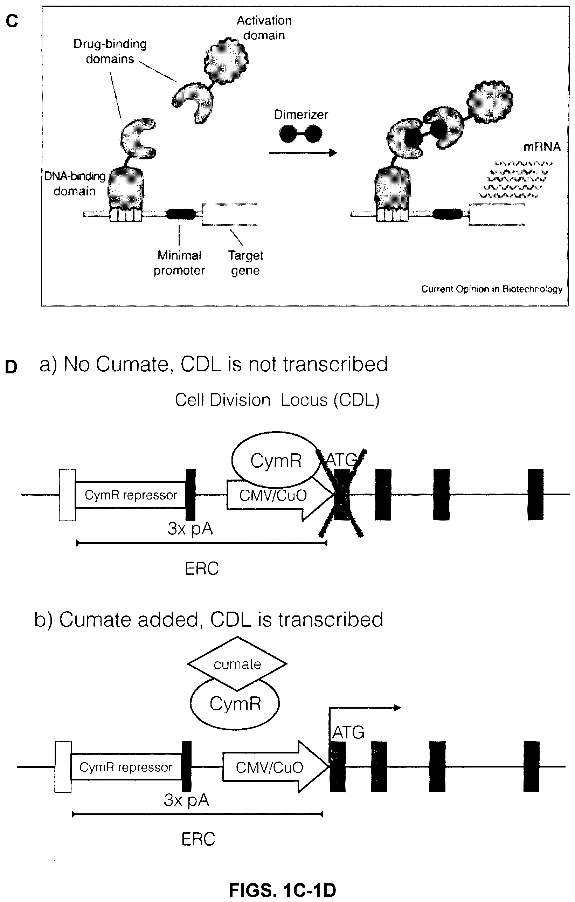

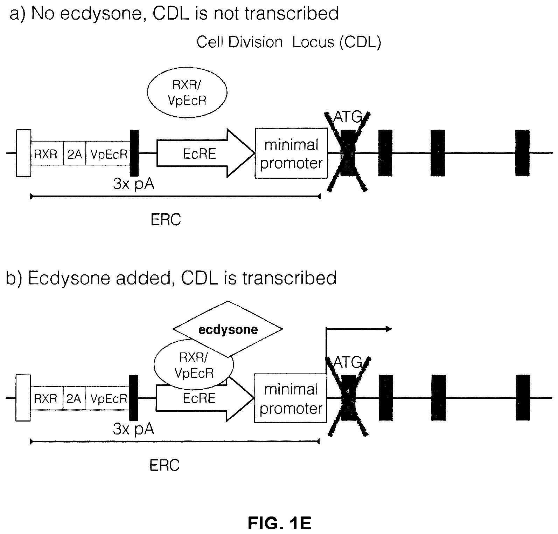

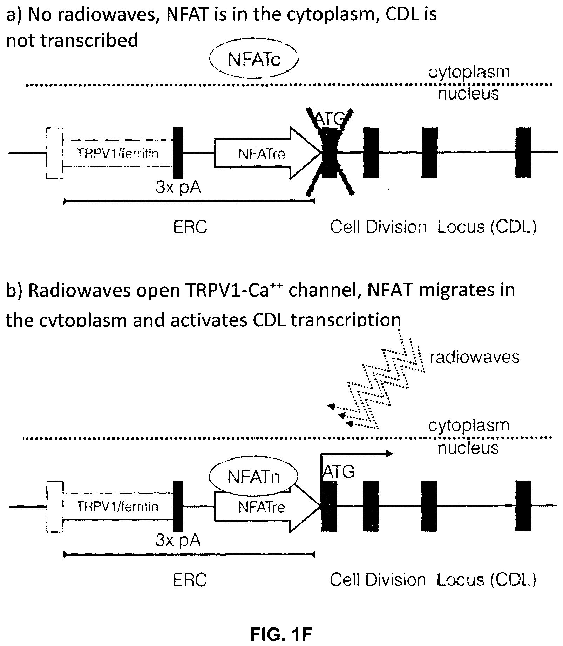

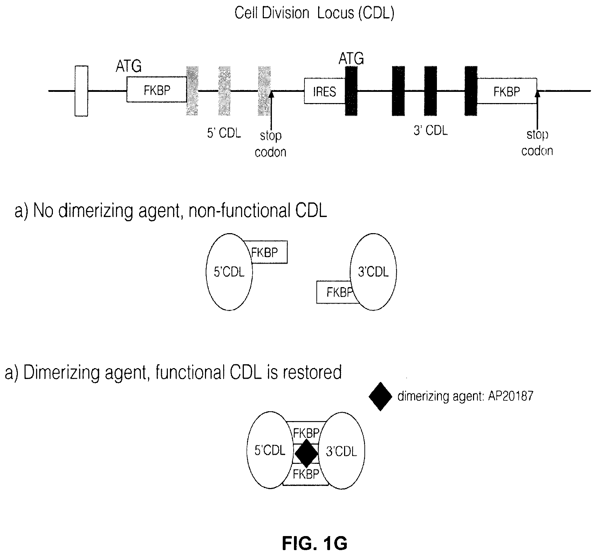

Figures 1A-1G depict schematics illustrating the concept of induced negative effectors of proliferation (iNEPs) and examples of iNEP systems contemplated for use in the methods and tools provided herein. FIG. 1A depicts a schematic representing different examples of iNEP-modified CDLs, including a homozygous modification in CDL1, homozygous insertions in CDL1 and CDL2, CDL comprising two separate loci that together are essential for cell division (CDL3). FIG. 1B depicts schematics representing examples of iNEP comprising an ablation link (ALINK) and an exogenous activator of regulation of a CDL (EARC) in different configurations. FIG. 1C depicts a schematic illustrating transcription activator-like effector (TALE) technology combined with dimerizer-regulated expression induction. FIG. 1D depicts a schematic illustrating a reverse-cumate-Trans-Activator (rcTA) system. FIG. 1E depicts a schematic illustrating a retinoid X receptor (RXR) and an N-terminal truncation of ecdysone receptor (EcR) fused to the activation domain of Vp16 (VpEcR). FIG. 1F depicts a schematic illustrating a transient receptor potential vanilloid-1 (TRPV1), together with ferritin, which is one example of an iNEP system, as set forth herein. FIG. 1G depicts a schematic illustrating how an IRES and a dimerization agent may be used as an iNEP.

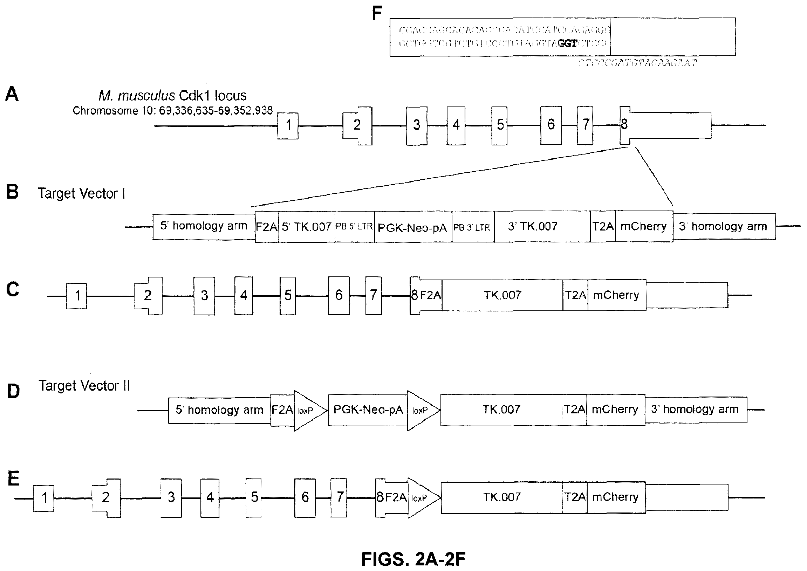

Figures 2A-2F depict schematics illustrating targeting HSV-TK into the 3'UTR of the Cdk1 locus to generate an ALINK, which enables elimination of dividing modified CDK1-expressing cells. FIG. 2A shows a schematic of the mouse *Cdk1* locus. FIG. 2B shows a schematic of mouse target vector I. FIG. 2C shows a schematic of a Cdk1^{TC} allele. FIG. 2D shows a schematic of mouse target vector II. FIG. 2E shows a schematic of a Cdk1^{TClox} allele. FIG. 2F depicts the position of the CRISPR guide RNA; the sequence in the yellow box is the 8th exon of Cdk1.

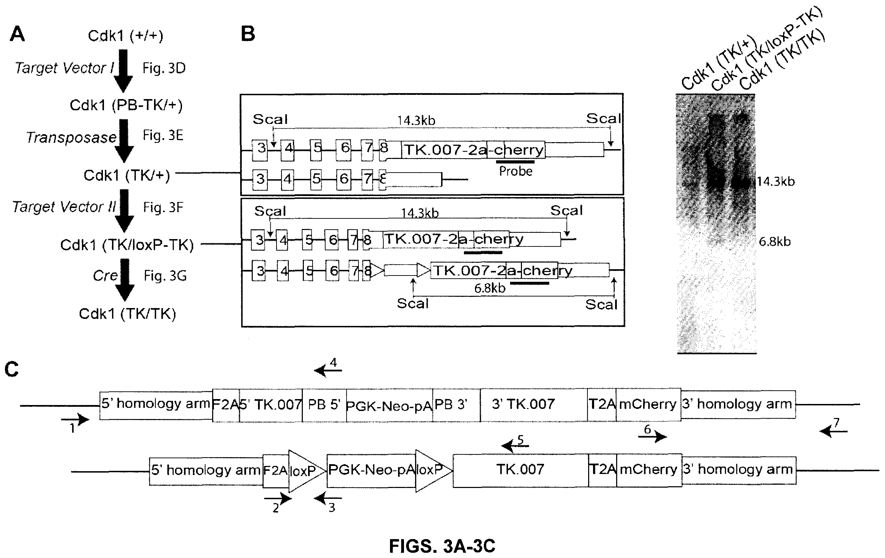

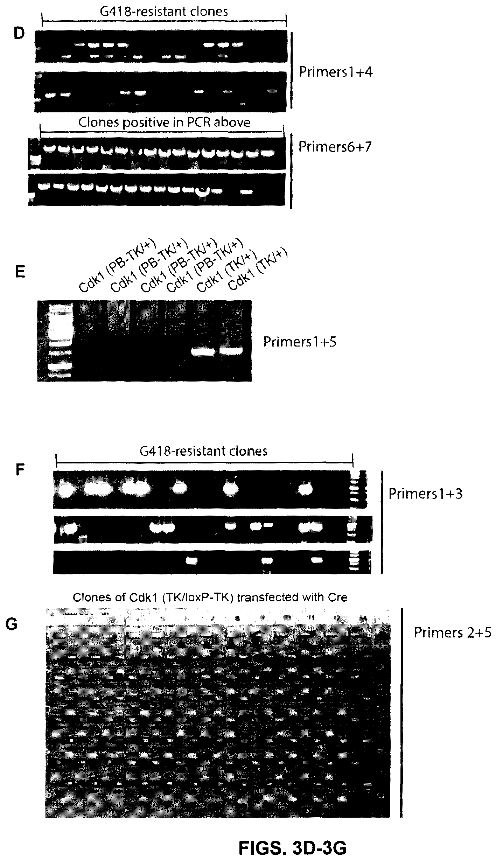

Figures 3A-3G depict generation of ALINK example, HSV-TK-mCherry into the 3'UTR of the CDK1 locus to generate ALINK in mouse ES cell lines. Fig. 3A shows the overall steps of generating ALINK in mouse C2 ES cells. Fig. 3B shows southern blotting result of correct genotyping of Cdk1(TK/+), Cdk1(TK, IoxP-TK), and Cdk1(TK/TK). Fig. 3C shows the locations of the primers used in ALINK genotyping in mouse cells. Fig. 3D includes PCR results illustrating targeting of Targeting Vector I into the 3'UTR of the CDK1 locus. Fig. 3E shows PCR results illustrating the excision event of selection marker in a mouse ES cell line already correctly targeted with Targeting Vector I to activate the expression of HSV-TK-mCherry. Fig. 3F shows PCR results illustrating targeting of Targeting Vector II into Cdk1(TK/+) cells. Fig. 3G shows PCR results illustrating the excision event of selection marker in Cdk1(TK, IoxP-TK) to activate the 2^{nd} allele expression of HSV-TK-mCherry, thus generating Cdk1(TK/TK).

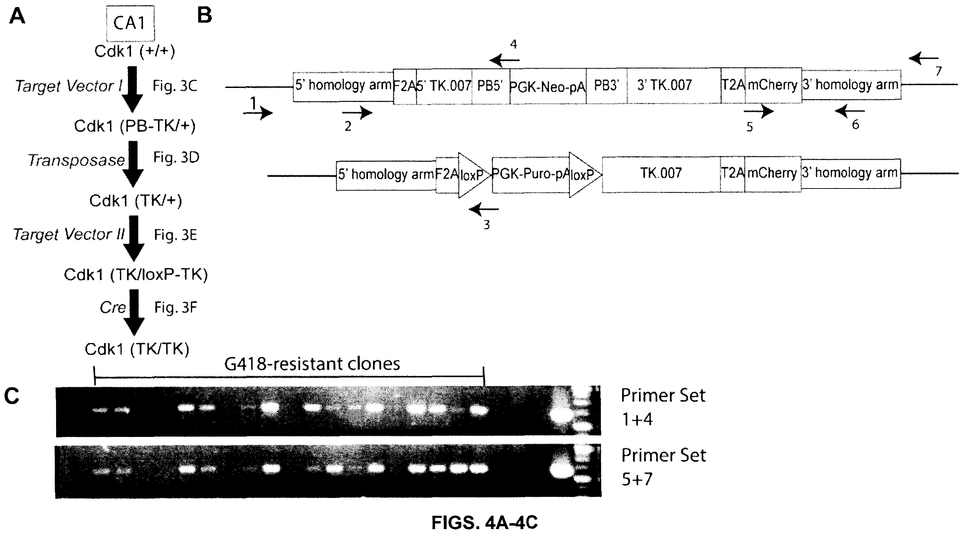

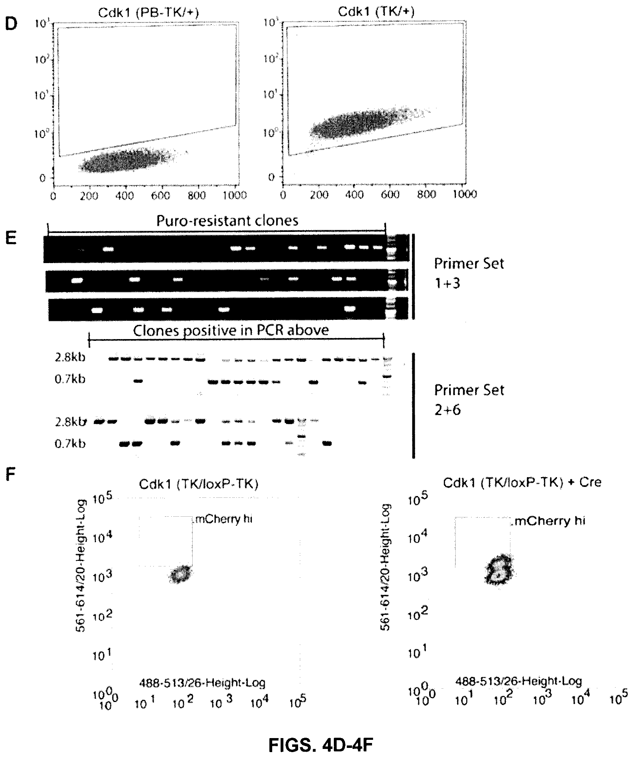

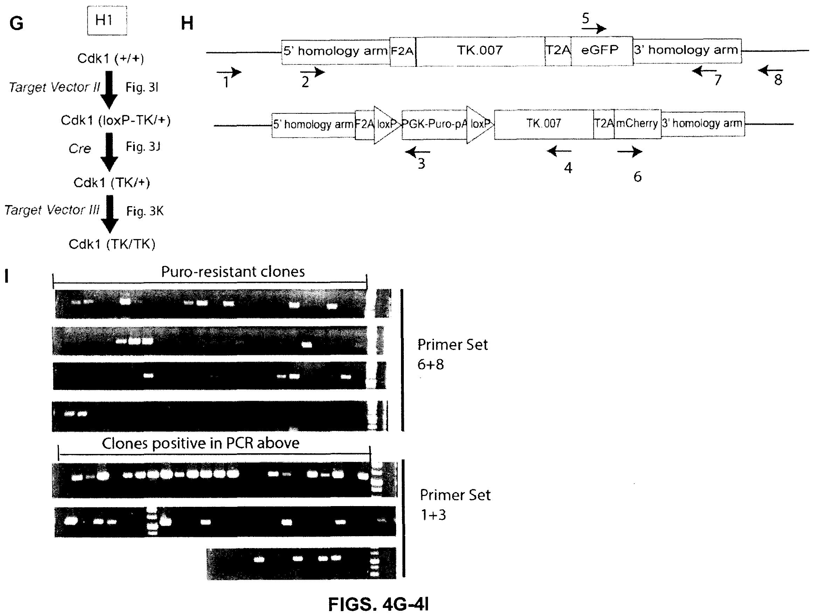

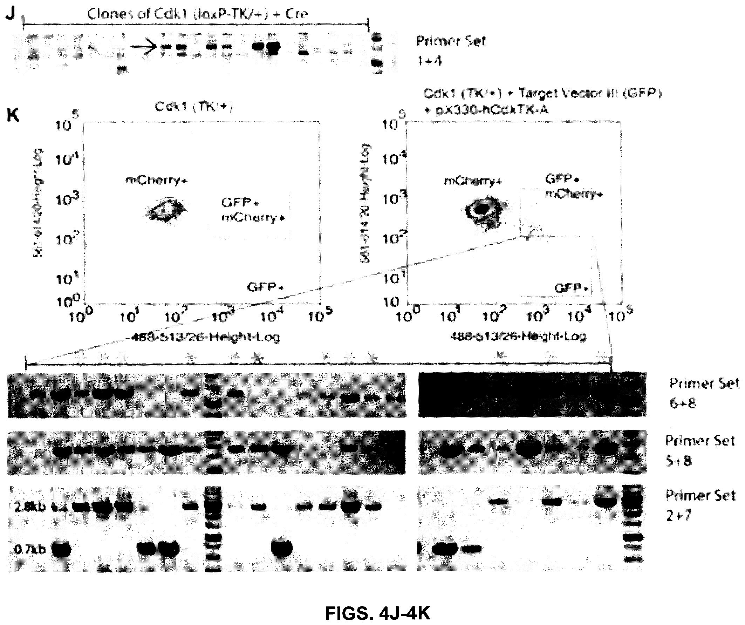

Figures 4A-4K depict generation of an ALINK modification, HSV-TK-mCherry into the 3'UTR of the CDK1 locus, in human ES cell lines. Fig. 4A shows the overall steps of generating ALINK in human CA1 ES cells. Fig. 4B shows the locations of the primers used in ALINK genotyping in human CA1 cells. Fig. 4C shows PCR results illustrating targeting of Targeting Vector I into the 3'UTR of the CDK1 locus. Fig. 4D shows flow cytometry illustrating the excision event of selection marker in human Cdk1(PB-TK/+) ES cell line to activate the expression of HSV-TK-mCherry; the Y-axis shows the mCherry expression level, while the X-axis is an autofluorescence channel. Fig. 4E shows PCR results illustrating targeting of Targeting Vector II (puro-version) into Cdk1 (TK/+) cells; the upper panel is PCR using primers flanking the 5'homology arm; the lower panel is PCR using primers inside 5' and 3' homology arm, so absence of 0.7kb band and presence of 2.8kb band means that the clone is homozygous in ALINK, and presence of 0.7kb band means that the clone is heterozygous in ALINK or the population is not clonal. Fig. 4F shows flow cytometry analysis illustrating the excision event of selection marker in Cdk1(TK, IoxP-TK) to activate the 2nd allele expression of HSV-TK-mCherry; the Y-axis shows the mCherry expression level, while the X-axis is an autofluorescence channel. Fig. 4G shows the overall steps of generating ALINK in human H1 ES cells. Fig. 4H shows the locations of the primers used in ALINK genotyping in human H1 cells. Fig. 4I shows PCR results illustrating targeting of Targeting Vector II into the 3'UTR of the CDK1 locus. Fig. 4J shows PCR results illustrating the excision event of selection marker in human H1 Cdk1(IoxP-TK/+) to activate the expression of HSV-TK-mCherry; the Y-axis shows the mCherry expression level, while the X-axis is an autofluorescence channel. Fig. 4K shows fluorescence-activated cell sorting (FACS) of targeting of Targeting Vector III (GFP-version) into Cdk1(TK/+) cells. After FACS sorting, clones picked from sparse plating were genotyped with mCherry-allele-specific primers, eGFP-allele-specific primers and primers in 5' and 3' homology arms; clones labeled with orange star sign are homozygous ALINK with one allele of mCherry and one allele of eGFP; the one clone labeled with green star sign is homozygous ALINK with two alleles of eGFP.

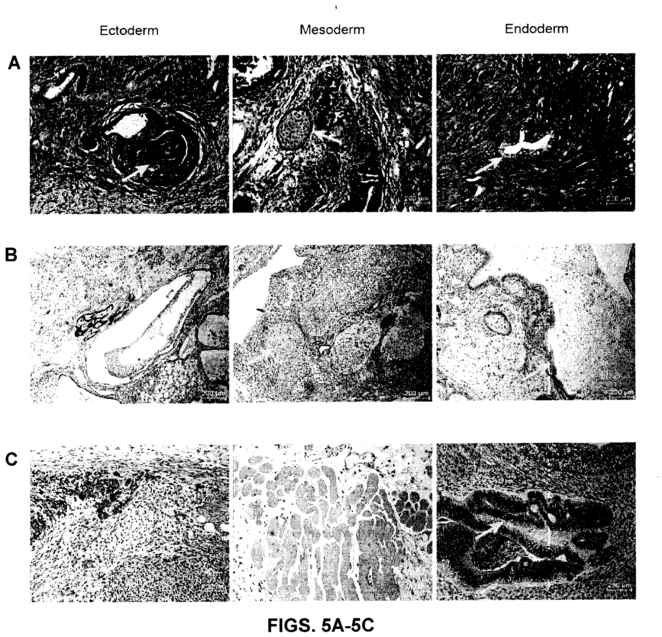

Figures 5A-C depict teratoma histology (endoderm, mesoderm and ectoderm portions of the teratoma are shown from left to right, respectively). FIG. 5A depicts photomicrographs of a teratoma derived from a mouse ES cdk1^{+/+,alink/alink} cell. FIG 5B depicts photomicrographs of a teratoma derived from a mouse ES Cdk1^{earc/earc,alink/alink} cell. FIG. 5C depicts photomicrographs of a teratoma derived from a human ES Cdk1^{+/+, alink/alink} cell.

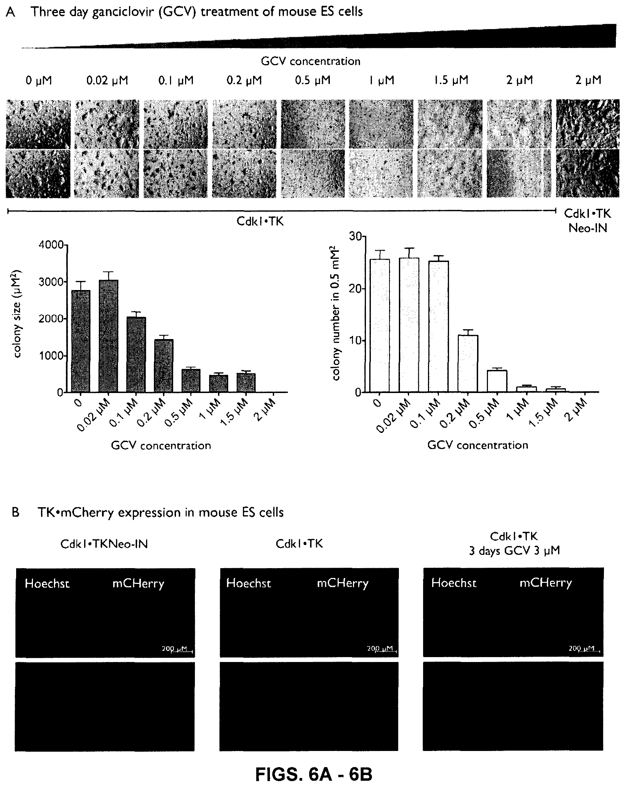

Figures 6A-6B depict *in vitro* functional analysis of mouse ES cells with an HSV-TK - mCherry knock-in into the 3'UTR of the CDK1 locus. FIG. 6A illustrates killing efficiency provided by the TK.007 gene after cells were exposed to different concentrations of GCV for 3 days. Colony size and number are directly proportional to GCV concentration. The second lowest concentration of 0.01µM did not affect the colony number but slowed down cell growth as evidenced by the reduced colony size (n=5). FIG. 6B illustrates expression of mCherry before (Cdk1•HSV-TK•NeoIN) and after (Cdk1•HSV-TK) PB-mediated removal of the neo-cassette.

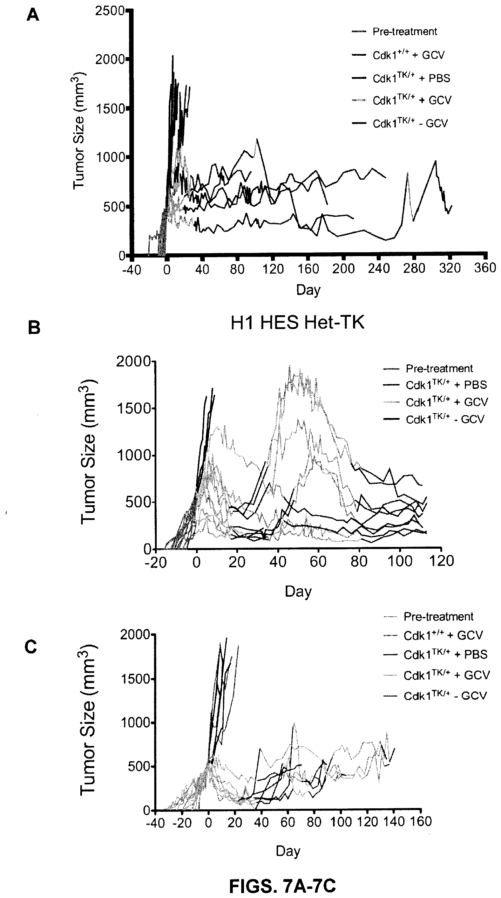

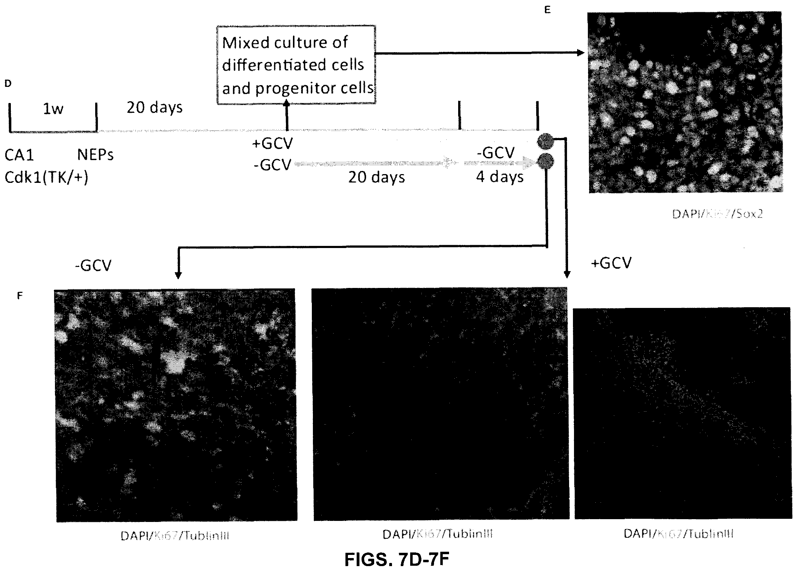

Figures 7A-F depict results of cellular experiments using ALINK-modified cells. FIG. 7A graphically depicts results of GCV treatment of subcutaneous teratomas comprising ALINK-modified mouse C2 cells. FIG. 7B graphically depicts results of GCV treatment of subcutaneous teratomas comprising ALINK-modified H1 ES cells. FIG. 7C graphically depicts results of GCV treatment of mammary gland tumors comprising ALINK-modified cells. FIG. 7D schematically depicts experimental design of neural assay. FIG. 7E is a microscopic image of Neural Epithelial Progenitor (NEP) cells derived from Cdk1^{+/+,+/alink} human CA1 ES cells. FIG. 7F depicts microscopic images illustrating GCV-induced killing of dividing ALINK-modified NEPs and non-killing of non-dividing neurons.

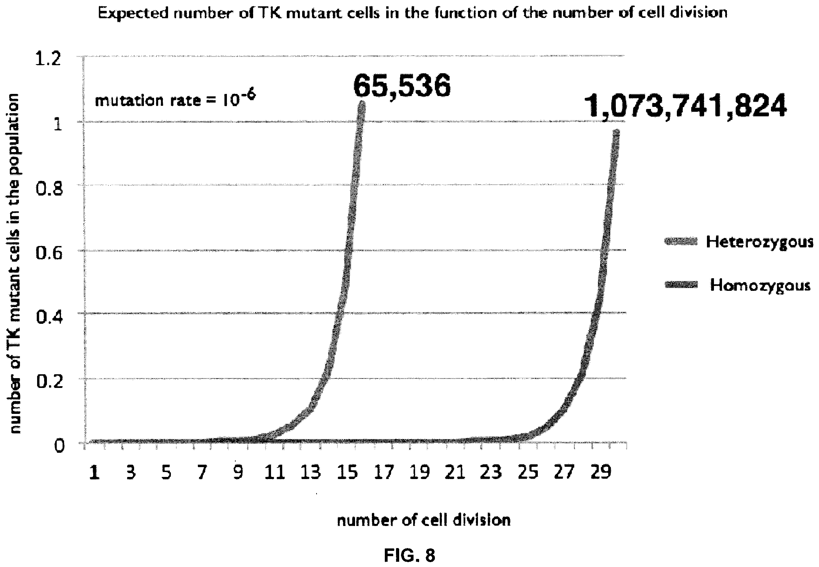

Figure 8 depicts a graph showing the expected number of cells comprising spontaneous mutations in the HSV-TK gene as a population is expanded from heterozygous (blue line) and homozygous (red line) ALINK cells.

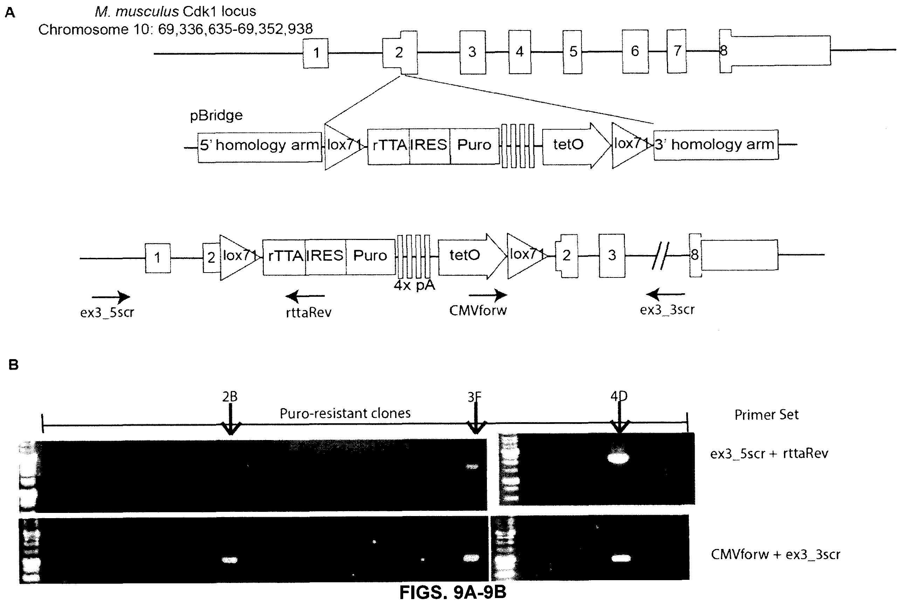

Figures 9A-9B depict targeting of a dox-bridge into the 5'UTR of the mouse Cdk1 locus to generate EARC and behavior of the bridge after insertion into Cdk1. FIG. 9A is a schematic illustrating the structure of the mouseCdk1 locus, the target vector, and the position of the primers used for genotyping for homologous recombination events. FIG. 9B depicts PCR results showing the genotyping of the puromycin resistant colonies to identify those that integrated the dox-bridge to the Cdk1 5'UTR.

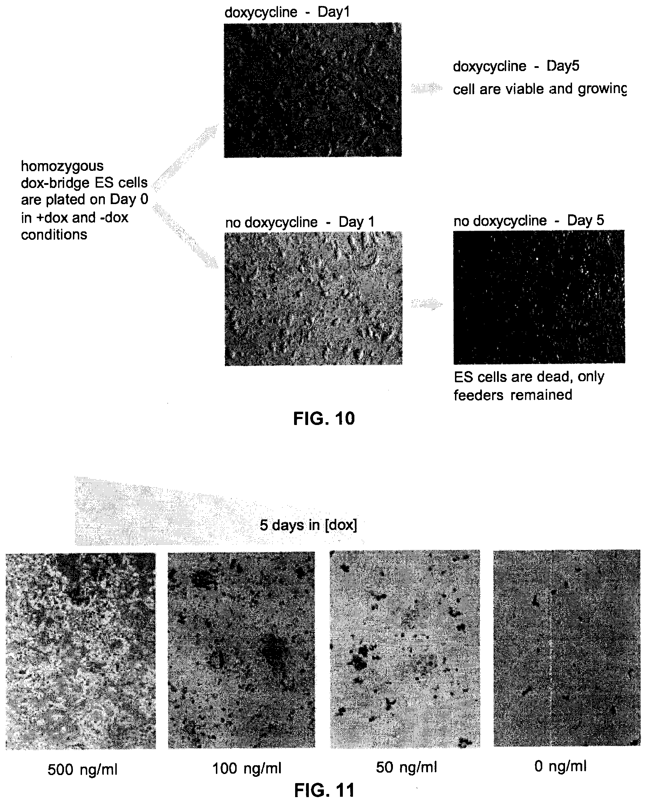

Figure 10 depicts a flow chart illustrating that ES cells having a homozygous dox-bridge knock-in survive and divide only in the presence of doxycycline (or drug with doxycycline overlapping function).
Figure 11 depicts representative photomicrographs illustrating that homozygous dox-bridge knock-in ES cells show doxycycline concentration dependent survival and growth.

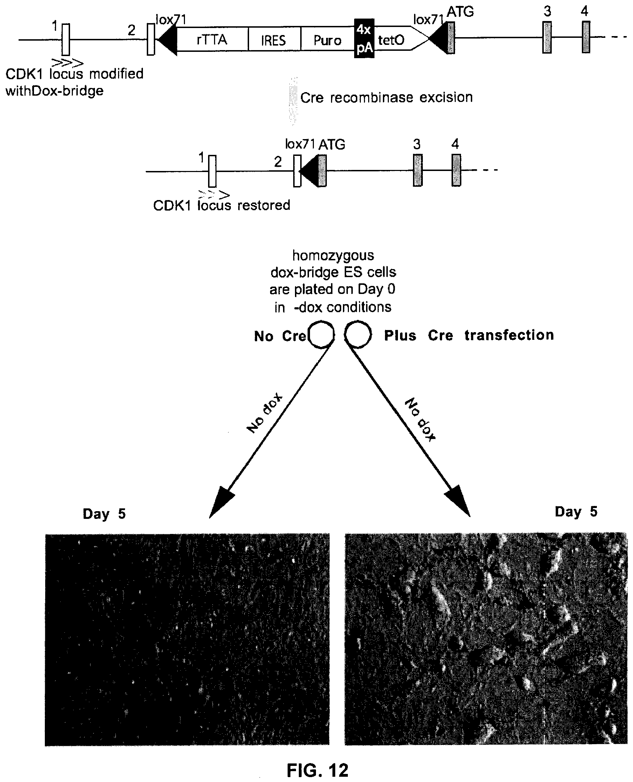

Figure 12 depicts dox-bridge removal with Cre recombinase-mediated excision, which rescues the doxycycline dependent survival of the ES cells.

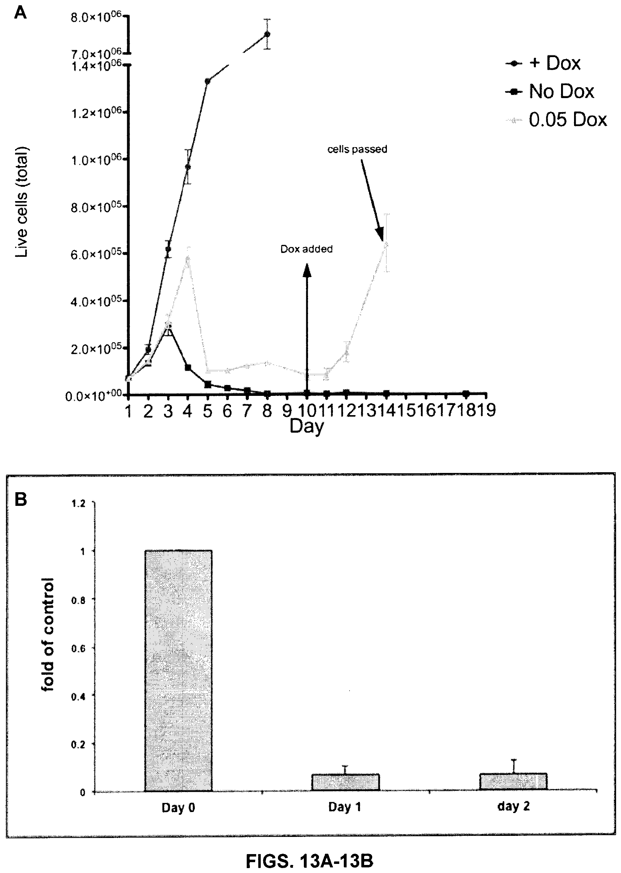

Figures 13A-13B depict the effect of doxycycline withdrawal on the growth of dox-bridged ES cells. FIG. 13A depicts a graph showing that in the presence of doxycycline the cells grew exponentially (red line with circle), indicating their normal growth. Upon doxycycline withdrawal on Day 1, the cells grew only for two days and then they started disappearing from the plates until no cell left on Day 9 on (dark blue line with square). The 20x lower doxycycline concentration (50ng/ml) after an initial 3 days of growth kept a constant number of cells on the plate for at least five days (Fig. 13, light blue line with triangle). On Day 10 the normal concentration of doxycycline was added back to the plates and the cells started growing again as normal ES cells. FIG. 13B depicts a bar graph showing the level of Cdk1 mRNA (as measured by quantitative-PCR) after 0, 1 and 2 days of Dox removal. Expression levels are normalized to beta-actin.

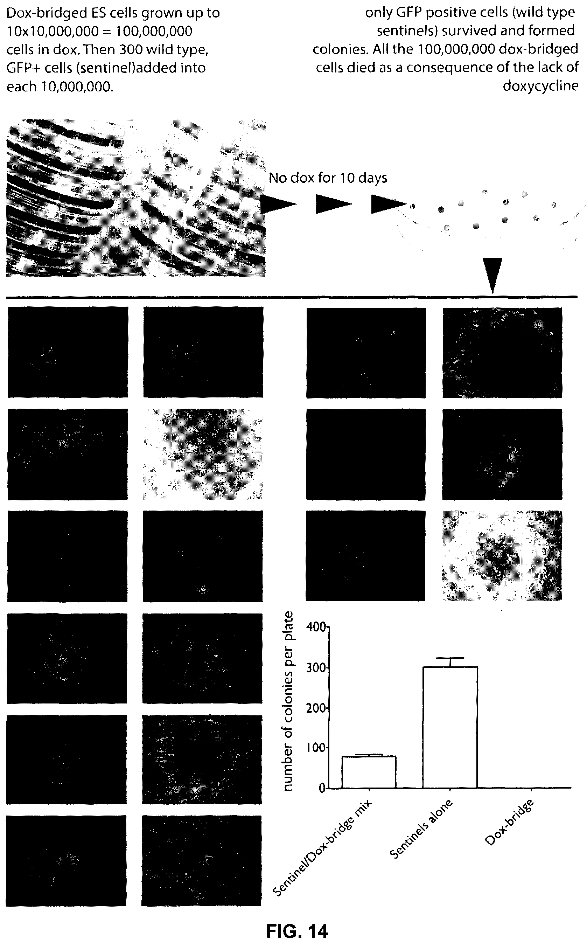

Figure 14 depicts the process of growing dox-bridged ES cells and illustrates that no escaper cells were found among 100,000,000 dox-bridged ES cells when doxycycline was withdrawn from the media, but the sentinel (wild type, GFP positive) cells survived with high efficiency.

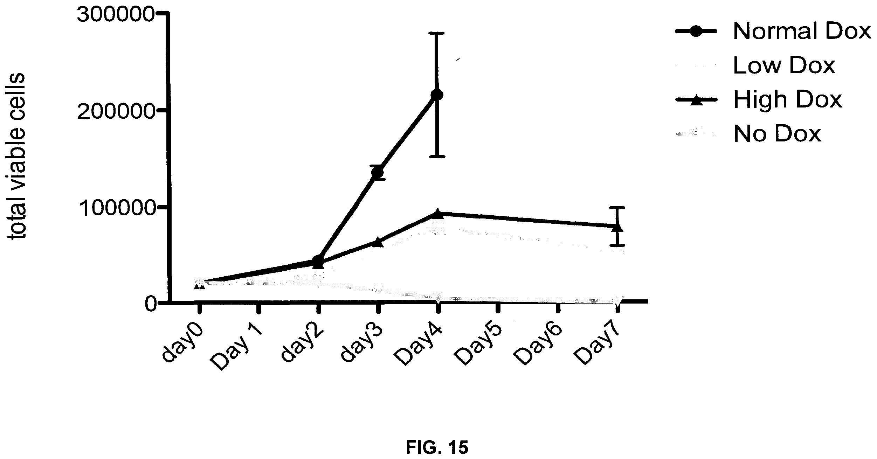

Figure 15 depicts a graph showing the effect of high doxycycline concentration (10 µg/ml) on dox-bridged ES cells: in the presence of high doxycycline, the cells slow down their growth rate similarly to when in low-doxycycline (high dox was 10 µg/ml, normal dox was 1 µg/ml, low dox was 0.05 µg/ml), indicating that there is a window for Dox concentration defining optimal level of CDK1 expression for cell proliferation.

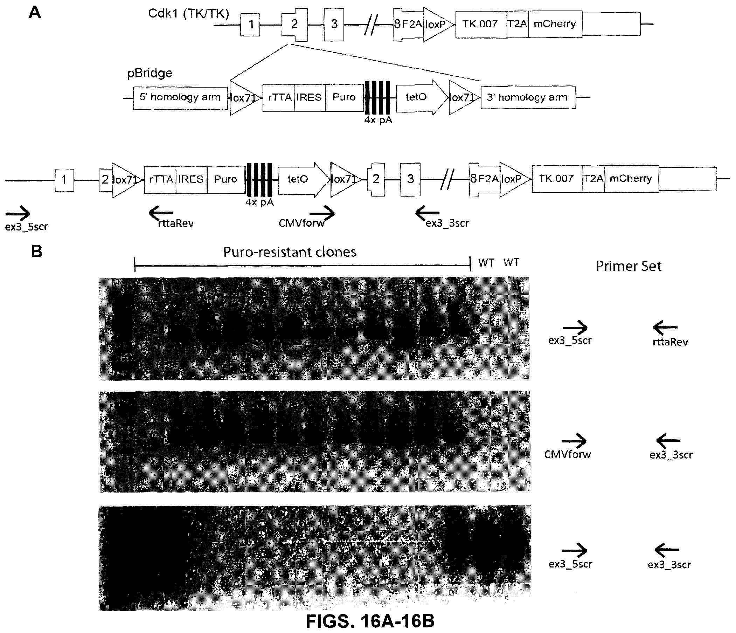

Figures 16A-16B depict targeting of dox-bridge into the 5'UTR of the Cdk1 locus of mouse cells comprising ALINK modifications (i.e., Cdk1(TK/TK) cells; the cell product described in FIGS. 3A-3G). FIG. 16A is a schematic illustrating the structure of the Cdk1 locus in Cdk1(TK/TK) cells, the bridge target vector, and the location of genotyping primers. FIG. 16B depicts PCR results showing the genotyping of the puromycin resistant colonies to identify those that integrated the dox-bridge to the Cdk1 5'UTR in mouse Cdk1(TK/TK) cells, thus generating mouse cell product Cdk1^{earc/earc,alink/alink}.

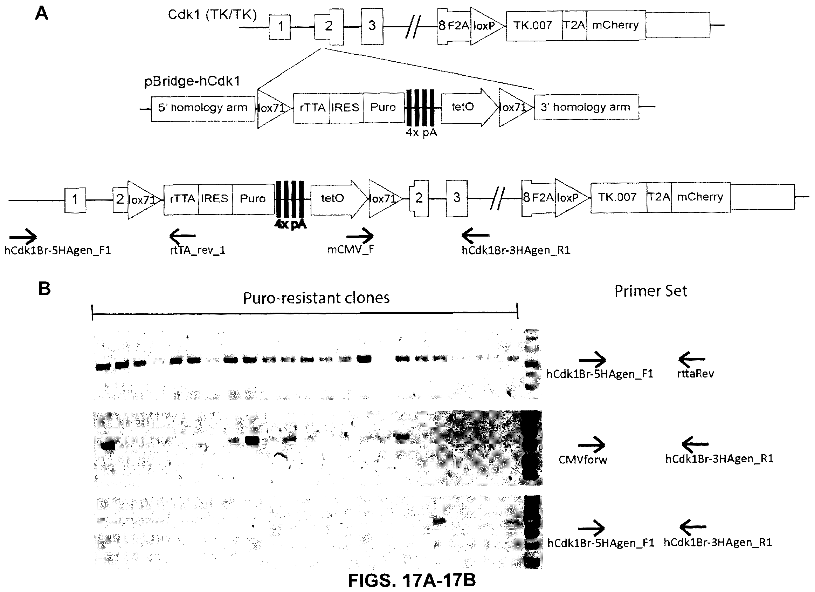

Figures 17A-17B depict targeting of dox-bridge into the 5'UTR of the Cdk1 locus of human cells comprising ALINK modifications (i.e., Cdk1(TK/TK) cells; the cell product described in FIGS. 4A-4F). FIG. 17A is a schematic illustrating the structure of the Cdk1 locus in Cdk1(TK/TK) cells, the bridge target vector, and the location of genotyping primers. Fig. 17B depicts PCR results showing the genotyping of the puromycin resistant colonies to identify those that integrated the dox-bridge to the Cdk1 5'UTR in human Cdk1 (TK/TK) cells, thus generating human cell product Cdk1^{earc/earc,alink/alink}.

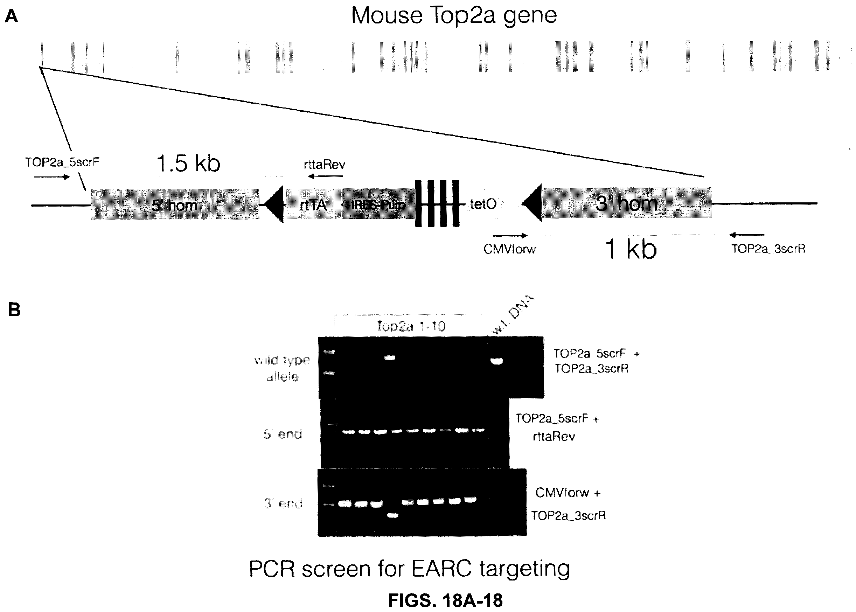

Figures 18A-18B depict targeting of a dox-bridge into the 5'UTR of the Top2 locus to generate EARC insertion into Top2a. FIG. 18A is a schematic illustrating the structure of the Top2a locus and the target vector. TOP2a_5scrF, rttaRev, CMVforw and TOP2a_3scrR indicate the position of the primers used for genotyping for homologous recombination events. FIG. 18B depicts PCR results showing the genotyping of the puro resistant colonies to identify those that integrated the dox-bridge to the Top2a 5'UTR. Nine of these cell lines was found to be homozygous targeted comprising a dox-bridge inserted by homologous recombination into the 5'UTR of both alleles of Top2a.

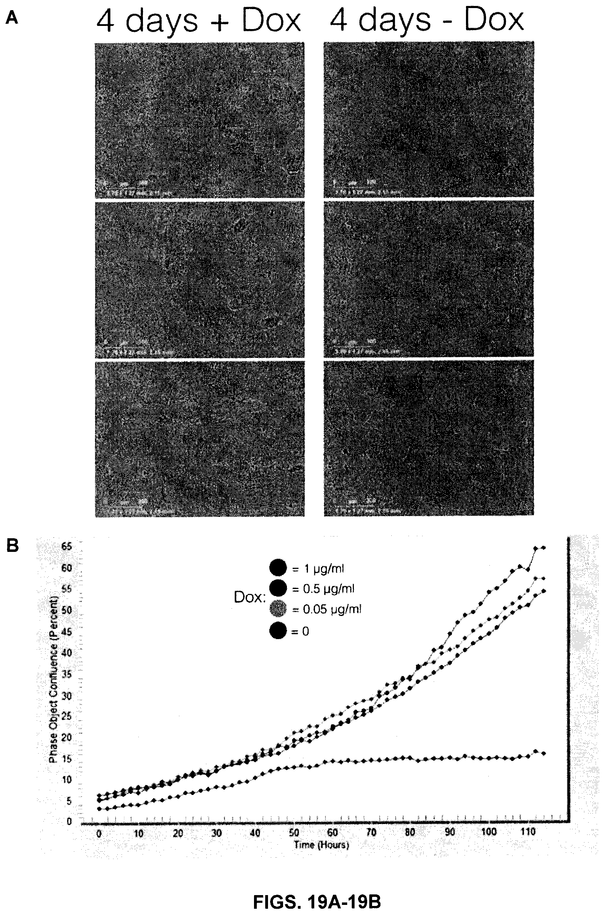

Figures 19A-19B depict the effect of doxycycline withdrawal on the growth of Top2a-EARC ES cells. FIG. 19A shows that withdrawal of doxycycline results in complete elimination of mitotically active ES cells within 4 days. FIG. 19B depicts how different concentrations of doxycycline affected proliferation of the dox-bridge ES cells by measuring cell growth for 4 days. ES cells in the presence of doxycycline grew exponentially, indicating their normal growth. In contrast, two days after doxycycline removal, cells growth was completely arrested.

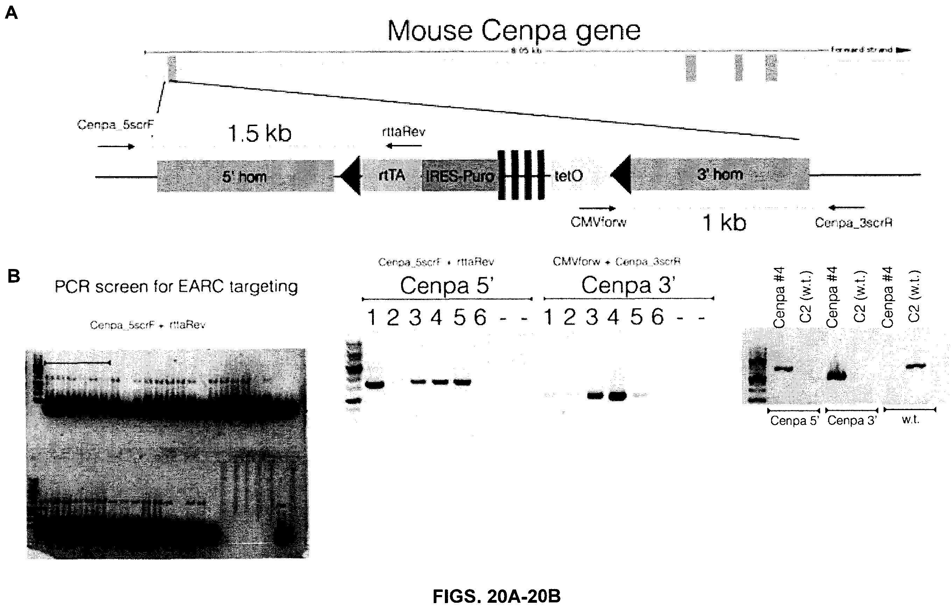

Figures 20A-20B depict targeting of a dox-bridge into the 5'UTR of the Cenpa locus to generate EARC insertion into Cenpa. FIG. 20A is a schematic illustrating the structure of the Cenpa locus and the target vector. Cenpa_5scrF, rttaRev, CMVforw and Cenpa_3scrR indicate the position of the primers used for genotyping for homologous recombination events. FIG. 20B depicts PCR results showing the genotyping of the puro resistant colonies to identify those that integrated the dox-bridge to the Cenpa 5'UTR. Six of these cells were found to have a correct insertion at the 5' and 3', and at least one clone (Cenpa#4), was found to have homozygous targeting comprising a dox-bridge inserted by homologous recombination into the 5'UTR of both alleles of Cenpa.

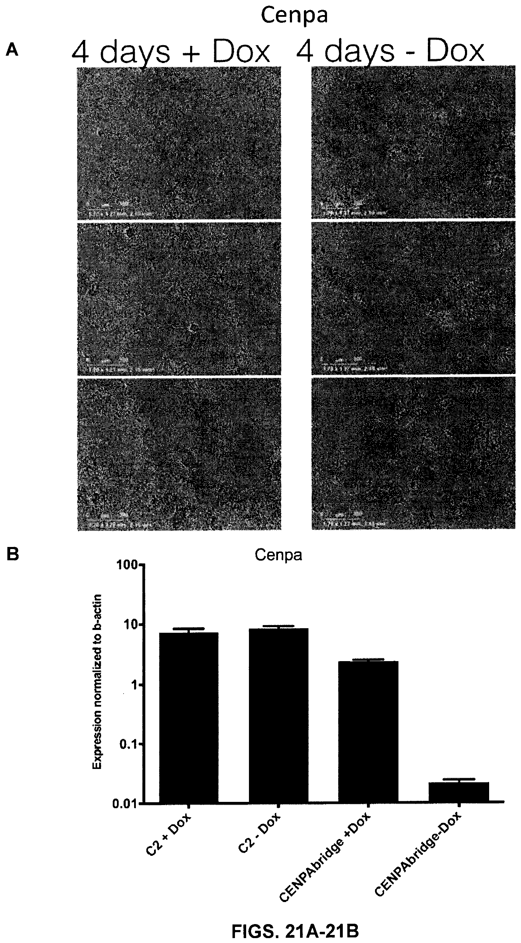

Figures 21A-21B depict the effect of doxycycline withdrawal on the growth of Cenpa-EARC ES cells. FIG. 21A depicts that withdrawal of doxycycline results in complete elimination of mitotically active ES cells within 4 days. FIG. 21B is the Cenpa gene expression level (determined by q-PCR) in Cenpa-EARC cells with Dox and after 2 days of Dox removal, and compared it to the expression level in wild type mouse ES cells (C2). As expected Cenpa expression level is greatly reduced in Cenpa-EARC cells without Dox for 2 days.

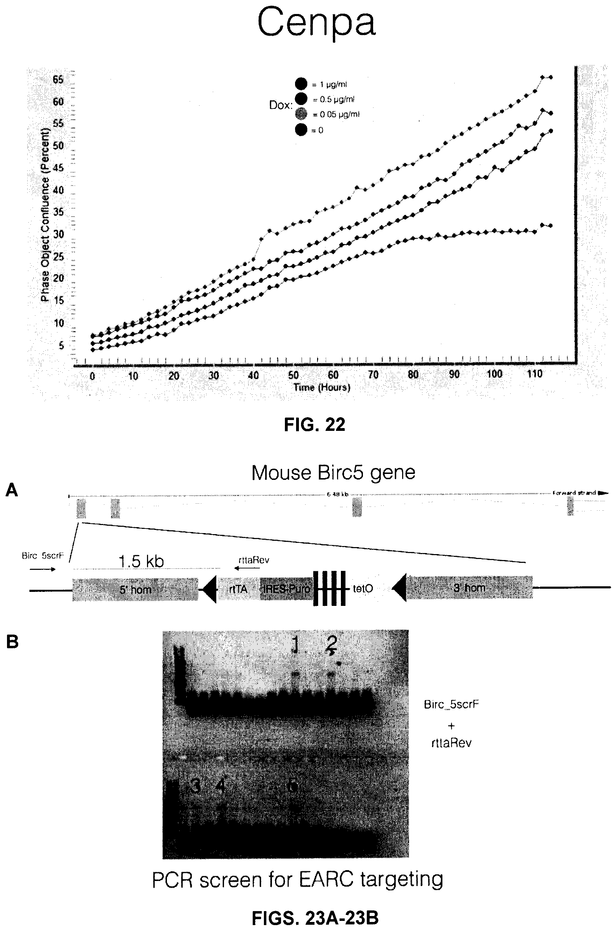

Figure 22 depicts how different concentrations of doxycycline affected proliferation of the Cenpa-EARC ES cells by measuring cell growth for 4 days. ES cells in the presence of doxycycline grew exponentially, indicating their normal growth. In contrast, 80 hours after doxycycline removal, cells growth was completely arrested.
Figures 23A-23B depict targeting of a dox-bridge into the 5'UTR of the Birc5 locus to generate EARC insertion into Birc5. FIG. 23A is a schematic illustrating the structure of the Birc5 locus and the target vector. Birc_5scrF and rttaRev indicate the position of the primers used for genotyping for homologous recombination events. FIG. 23B depicts PCR results showing the genotyping of the puro resistant colonies to identify those that integrated the dox-bridge to the Birc5 5'UTR. Five clones were found to be correctly targeted comprising a dox-bridge inserted by recombination into the 5'UTR of both alleles of Birc5. One of these clones was Birc#3, was found to stop growing or die in the absence of Dox.

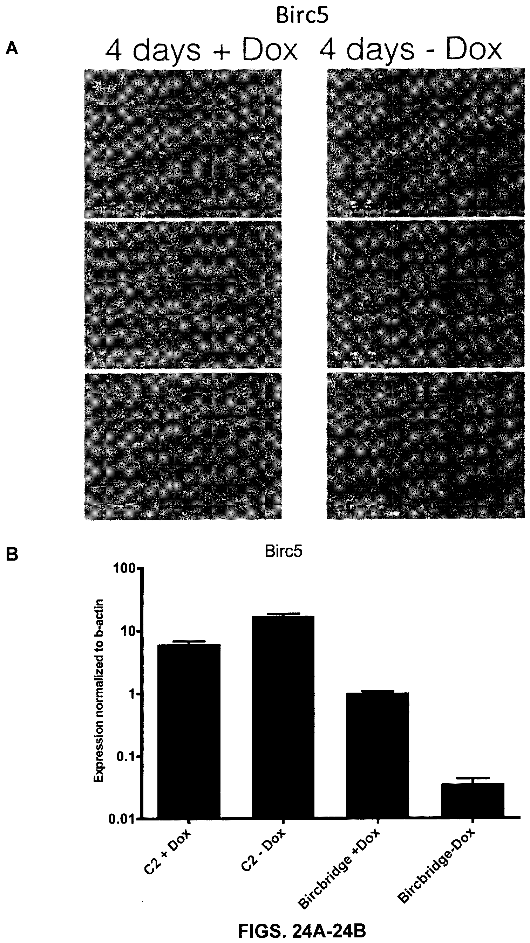

Figures 24A-24B depict the effect of doxycycline withdrawal on the growth of Birc5-EARC ES cells. FIG. 24A depicts that withdrawal of doxycycline results in complete elimination of mitotically active ES cells within 4 days. FIG. 24B is the Birc5 gene expression level (determined by q-PCR) in Birc5-EARC cells with Dox and after 2 days of Dox removal, and compared it to the expression level in wild type mouse ES cells (C2). As expected Birc5 expression level is greatly reduced in Birc5-EARC cells without Dox for 2 days.

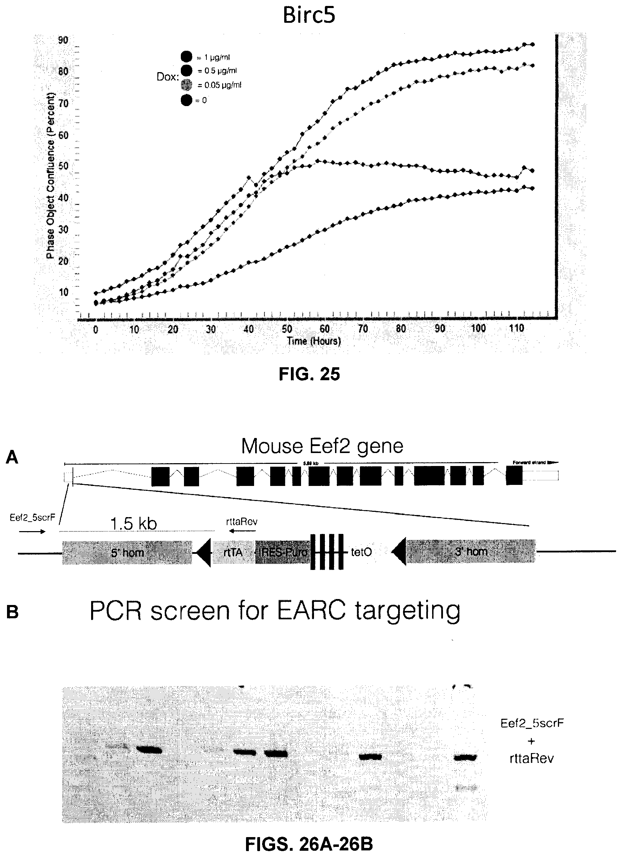

Figure 25 depicts how different concentrations of doxycycline affected proliferation of the Birc5-EARC ES cells by measuring cell growth for 4 days. ES cells in the presence of doxycycline grew exponentially, indicating their normal growth. In contrast, 50 hours after doxycycline removal, cells growth was completely arrested. Interestingly, it appears that lower Dox concentrations (0.5 and 0.05 µg/ml) promote better cell growth than a higher concentration (1 µg/ml) .
Figures 26A-26B depict targeting of a dox-bridge into the 5'UTR of the Eef2 locus to generate EARC insertion into Eef2. FIG. 26A is a schematic illustrating the structure of the Eef2 locus and the target vector. Eef2_5scrF and rttaRev indicate the position of the primers used for genotyping for homologous recombination events. FIG. 26B depicts PCR results showing the genotyping of the puro resistant colonies to identify those that integrated the dox-bridge to the Eef2 5'UTR. Nine of these cell lines was found to be correctly targeted with at least one clone growing only in Dox-media.

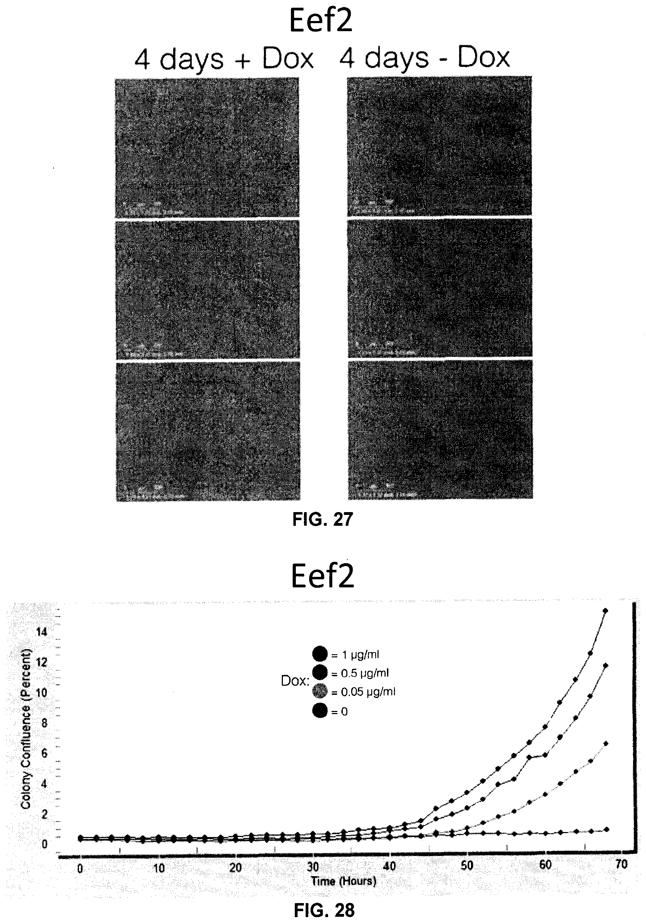

Figures 27 depict the effect of doxycycline withdrawal on the growth of Eef2-EARC ES cells. Withdrawal of doxycycline results in complete elimination of mitotically active ES cells within 4 days.
Figure 28 depicts how different concentrations of doxycycline affected proliferation of the Eef2-EARC ES cells by measuring cell growth for 4 days. ES cells in the presence of doxycycline grew exponentially, indicating their normal growth. In contrast, without doxycycline cells completely fail to grow.

### DETAILED DESCRIPTION OF THE DISCLOSURE

Unless defined otherwise, all technical and scientific terms used herein have the same meaning as commonly understood by one of ordinary skill in the art to which this disclosure belongs.

### Definitions

The terms "cell division locus", "cell division loci", and "CDL" as used herein, refer to a genomic locus (or loci) whose transcription product(s) is expressed by dividing cells. When a CDL comprises a single locus, absence of CDL expression in a cell (or its derivatives) means that tumour initiation and/or formation is prohibited either because the cell(s) will be ablated in the absence of CDL expression or because proliferation of the cell(s) will be blocked or compromised in the absence of CDL expression. When a CDL comprises multiple loci, absence of expression by all or subsets of the loci in a cell (or its derivatives) means that tumour initiation and/or formation is prohibited either because the cell(s) will be ablated in the absence of CDL expression or because proliferation of the cell(s) will be blocked or compromised in the absence of CDL expression. A CDL may or may not be expressed in non-dividing and/or non-proliferating cells. A CDL may be endogenous to a host cell or it may be a transgene. If a CDL is a transgene, it may be from the same or different species as a host cell or it may be of synthetic origin. A CDL may be a single locus that is transcribed during cell division. For example, in an embodiment, a single locus CDL is CDK1. A CDL may comprise two or more loci that are transcribed during cell division. For example, in an embodiment, a multi-locus CDL comprises two MYC genes (c-Myc and N-myc) (Scognamiglio et al., 2016). A multi-locus CDL may comprise AURORA B and C kinases, which may have overlapping functions (Fernandez-Miranda et al., 2011). Cell division and cell proliferation are terms that may be used interchangeably herein.

The terms "normal rate of cell division", "normal cell division rate", "normal rate of cell proliferation", and "normal cell proliferation rate" as used herein, refer to a rate of cell division and/or proliferation that is typical of a non-cancerous healthy cell. A normal rate of cell division and/or proliferation may be specific to cell type. For example, it is widely accepted that the number of cells in the epidermis, intestine, lung, blood, bone marrow, thymus, testis, uterus and mammary gland is maintained by a high rate of cell division and a high rate of cell death. In contrast, the number of cells in the pancreas, kidney, cornea, prostate, bone, heart and brain is maintained by a low rate of cell division and a low rate of cell death (Pellettieri and Sánchez Alvarado, 2007).

The terms "inducible negative effector of proliferation" and "iNEP" as used herein, refer to a genetic modification that facilitates use of CDL expression to control cell division and/or proliferation by: i) inducibly stopping or blocking CDL expression, thereby prohibiting cell division and proliferation; ii) inducibly ablating at least a portion of CDL-expressing cells (i.e., killing at least a portion of proliferating cells); or iii) inducibly slowing the rate of cell division relative to a cell's normal cell division rate, such that the rate of cell division would not be fast enough to contribute to tumor formation.

The terms "ablation link" and "ALINK" as used herein, refer to an example of an iNEP, which comprises a transcriptional link between a CDL and a sequence encoding a negative selectable marker. The ALINK modification allows a user to inducibly kill proliferating host cells comprising the ALINK or inhibit the host cell's proliferation by killing at least a portion of proliferating cells by exposing the ALINK-modified cells to an inducer of the negative selectable marker. For example, a cell modified to comprise an ALINK at a CDL may be treated with an inducer (e.g., a prodrug) of the negative selectable marker in order to ablate proliferating cells or to inhibit cell proliferation by killing at least a portion of proliferating cells (Figure 1B).

The terms "exogenous activator of regulation of CDL" and "EARC" as used herein, refer to an example of an iNEP, which comprises a mechanism or system that facilitates exogenous alteration of non-coding or coding DNA transcription or corresponding translation via an activator. An EARC modification allows a user to inducibly stop or inhibit division of cells comprising the EARC by removing from the EARC-modified cells an inducer that permits transcription and/or translation of the EARC-modified CDL. For example, an inducible activator-based gene expression system may be operably linked to a CDL and used to exogenously control expression of a CDL or CDL translation, such that the presence of a drug inducible activator and corresponding inducer drug are required for CDL transcription and/or translation. In the absence of the inducer drug, cell division and/or proliferation would be stopped or inhibited (e.g., slowed to a normal cell division rate). For example, the CDL Cdk1/CDK1 may be modified to comprise a dox-bridge (Figure 1B), such that expression of Cdk1/CDK1 and cell division and proliferation are only possible in the presence of an inducer (e.g., doxycycline).

The term "proliferation antagonist system" as used herein, refers to a natural or engineered compound(s) whose presence inhibits (completely or partially) proliferation of a cell.

### General Description of Tools and Methods

As described herein, the inventors have provided molecular tools, methods and kits for using one or more cell division loci (CDL) in an animal cell to generate genetically modified cells in which cell division and/or proliferation can be controlled by a user through one or more iNEPs (FIG. 1A). For example, division of cells generated using one or more tools and/or methods provided herein could be stopped, blocked or inhibited by a user such that a cell's division rate would not be fast enough to contribute to tumor formation. For example, proliferation of cells generated using one or more tools and/or methods provided herein could be stopped, blocked or inhibited by a user, by killing or stopping at least a portion of proliferating cells, such that a cell's proliferation rate or volume may be maintained at a rate or size, respectively, desired by the user.

Tools and methods for controlling cell division and/or proliferation are desirable, for example, in instances wherein faster cell division rates (relative to normal cell division rates) are undesirable. For example, cells that divide at faster than normal rates may form tumors *in situ,* which may be harmful to a host. The genetically modified animal cells provided herein may comprise one or more mechanisms for allowing normal cell division and/or proliferation and for stopping, ablating, blocking and/or slowing cell division and/or proliferation, such that undesirable cell division and/or proliferation may be controlled by a user (FIG. 1B). Referring to FIG. 1B, in example (I) EARC is inserted at the 5' UTR of the CDL and ALINK is inserted at the 3' UTR, the product of transcription is a bi-cistronic mRNA that get processed in two proteins. In example (II) both EARC and ALINK are inserted at the 5' UTR of the CDL, the product of transcription is a bi-cistronic mRNA that get processed in two proteins. In example (III) EARC is inserted at the 5' UTR of the CDL and ALINK is inserted within the CDL coding sequence, the product of transcription is a mRNA that get processed in a precursor protein that will generate two separate protein upon cleavage of specifically designed cleavage sequences. In example (IV) both EARC and ALINK are inserted at the 5' UTR of the CDL, the product of transcription is a mRNA that get processed into a fusion protein that maintains both CDL and ALINK functions. In example (V) EARC is inserted at the 5' UTR of the CDL and ALINK is inserted at the 3' UTR, the product of transcription is a mRNA that get processed into a fusion protein that maintains both CDL and ALINK functions.

For example, the genetically modified animal cells provided herein may be used in a cell therapeutic treatment applied to a subject. If one or more of the genetically modified animal cells provided to the subject were to begin dividing at an undesirable rate (e.g., faster than normal), then a user could stop or slow division of cells dividing at the undesirable rate or block, slow or stop cells proliferating at the undesirable rate by i) applying to the cells dividing at the undesirable rate an inducer corresponding to the genetic modification in the cells; or ii) restricting access of the cells dividing at the undesirable rate to an inducer corresponding to the genetic modification in the cells, i) or ii) being determined based on the type of iNEP(s) provided in the genetically modified animal cells.

The genetically modified animal cells provided herein may be referred to as "fail-safe cells". A fail-safe cell contains one or more homozygous, heterozygous, hemizygous or compound heterozygous ALINKs in one or more CDLs. A fail-safe cell may further comprise one or more EARCs in one or more CDL. A fail-safe cell may comprise a CDL comprising both ALINK and EARC modifications.

As used herein, the term "fail-safe", refers to the probability (designated as pFS) defining a cell number. For example, the number of cells that can be grown from a single fail-safe cell (clone volume) where the probability of obtaining a clone containing cells, which have lost all ALINKs is less than an arbitrary value (pFS). For example, a pFS = 0.01 refers to a scenario wherein if clones were grown from a single cell comprising an ALINK-modified CDL 100 times, only one clone expected to have cells, which lost ALINK function (the expression of the negative selectable marker) while still capable of cell division. The fail-safe volume will depend on the number of ALINKs and the number of ALINK-targeted CDLs. The fail-safe property is further described in Table 1.

**Table 1. Fail-safe cell volumes and their relationship to a human body were calculated using mathematical modelling. The model did not take into a count the events when CDL expression was co-lost with the loss of negative selectable marker activity, compromising cell proliferation. Therefore the values are underestimates and were calculated assuming 10-6 forward mutation rate for the negative selectable marker. The estimated number of cells in a human body as 3.72x10¹³ was taken from (Bianconi et al., 2013).**

| CDL# | ALINK# | Genotype in CDLs | Fail-safe volume (#cells) | Relative (x) to a human body=3.72x10¹³ cells | Estimated weight of clones |
|---|---|---|---|---|---|
| 1 | 1 | het | 512 | 0.0000000000137 | 1µg |
| 1 | 2 | hom | 16777216 | 0.000000451 | 31 mg |
| 2 | 3 | het, hom | 1.374E+11 | 0.004 | 0.26 kg |
| 2 | 4 | hom, hom | 1.13E+15 | 30 | 2100 kg |

It is contemplated herein that fail-safe cells may be of use in cell-based therapies wherein it may be desirable to eliminate cells exhibiting undesirable growth rates, irrespective of whether such cells are generated before or after grafting the cells into a host.

### Cell Division Loci (CDLs)

The systems, methods and compositions provided herein are based on the identification of one or more CDLs, such as, for example, the CDLs set forth in Table 2. It is contemplated herein that various CDLs could be targeted using the methods provided herein.

A CDL may be a locus identified as an "essential gene" as set forth in Wang et al., 2015. Essential genes in Wang et al., 2015, were identified by computing a score (i.e., a CRISPR score) for each gene that reflects the fitness cost imposed by inactivation of the gene.A CDL may have a CRISPR score of less than about -1.0 (Table 2, column 5).

A CDL may be a locus/loci that encodes a gene product that is relevant to cell division and/or replication (Table 2, column 6). For example, a CDL may be a locus/loci that encodes a gene product that is relevant to one or more of: i) cell cycle; ii) DNA replication; iii) RNA transcription and/or protein translation; and iv) metabolism (Table 2, column 7).

A CDL may be one or more cyclin-dependent kinases that are involved with regulating progression of the cell cycle (e.g., control of G1/S G2/M and metaphase-to-anaphase transition), such as CDK1, CDK2, CDK3, CDK4, CDK5, CDK6, CDK7, CDK8, CDK9 and/or CDK11 (Morgan, 2007). A CDL may be one or more cyclins that are involved with controlling progression of the cell cycle by activating one or more CDK, such as, for example, cyclinB, cyclinE, cyclinA, cyclinC, cyclinD, cyclinH, cyclinC, cyclinT, cyclinL and/or cyclinF (FUNG and POON, 2005). A CDL may be one or more loci involved in the anaphase-promoting complex that controls the progression of metaphase to anaphase transition in the M phase of the cell cycle (Peters, 2002). A CDL may be one or more loci involved with kinetochore components that control the progression of metaphase to anaphase transition in the M phase of the cell cycle (Fukagawa, 2007). A CDL may be one or more loci involved with microtuble components that control microtubule dynamics required for the cell cycle (Cassimeris, 1999).

A CDL may be a locus/loci involved with housekeeping. As used herein, the term "housekeeping gene" or "housekeeping locus" refers to one or more genes that are required for the maintenance of basic cellular function. Housekeeping genes are expressed in all cells of an organism under normal and patho-physiological conditions.

A CDL may be a locus/loci that encodes a gene product that is relevant to cell division and/or proliferation and has a CRISPR score of less than about -1.0. For example, a CDL may be a locus/loci that encodes a gene product that is relevant to one or more of: i) cell cycle; ii) DNA replication; iii) RNA transcription and/or protein translation; and iv) metabolism, and has a CRISPR score of less than about -1.0.The CDL may also be a housekeeping gene.

To identify potential CDLs, the inventors examined early mouse embryonic lethal phenotypes of gene knockouts (KOs; Table 2, column 8). For example, the inventors found that mouse embryos homozygous null for *Cdk1* (cyclin-dependent kinase 1, also referred to as cell division cycle protein 2 homolog (CDC2)) null mutation die at the 2-cell stage (E1.5) (Santamaría et al., 2007). Cdk1 (referred to as CDK1 in humans) is a highly conserved serine/threonine kinase whose function is critical in regulating the cell cycle. Protein complexes of Cdk1 phosphorylate a large number of target substrates, which leads to cell cycle progression. In the absence of Cdk1 expression, a cell cannot transition through the G2 to M phase of the cell cycle.

Cdk1/CDK1 is one example of a single locus CDL. Genetic modifications of Cdk1/CDK1, in which transcription of the locus is ablated by insertion of an ALINK modification and/or exogenously controlled by insertion of an EARC modification, are examined herein as set forth in Examples 1, 2 and 3. Top2A/TOP2A is one example of a CDL. Cenpa/CEPNA is one example of a CDL. Birc5/BIRC5 is one example of a CDL. Eef2/EEF2 is one example of a CDL. Genetic modifications of Top2a, Cenpa, Birc5, and Eef2 in which transcription of the locus can be exogenously controlled by insertion of an EARC modification are examined herein as set forth in Examples 4-7, respectively.

It is contemplated herein that alternative and/or additional loci are CDLs that could be targeted using the method provided herein.

For example, RNAi screening of human cell lines identified a plurality of genes essential for cell proliferation (Harborth et al., 2001; Kittler et al., 2004). The inventors predicted that a subset of these loci were CDLs after confirming the loci's early embryonic lethal phenotype of mouse deficient of the orthologues and/or analyzing the Loci's GO term and/or genecards (Table 2, column 8).

### Targeting a CDL with an Ablation Link (ALINK) Genetic Modification

The disclosure provides molecular tools, methods and kits for modifying a CDL by linking the expression of a CDL with that of a DNA sequence encoding a negative selectable marker, thereby allowing drug-induced ablation of mitotically active cells consequently expressing the CDL and the negative selectable marker. Ablation of proliferating cells may be desirable, for example, when cell proliferation is uncontrolled and/or accelerated relative to a cell's normal division rate (e.g., uncontrolled cell division exhibited by cancerous cells). Ablation of proliferating cells may be achieved via a genetic modification to the cell, referred to herein as an "ablation link" (ALINK), which links the expression of a DNA sequence encoding a negative selectable marker to that of a CDL, thereby allowing elimination or sufficient inhibition of ALINK-modified proliferating cells consequently expressing the CDL locus (sufficient inhibition being inhibition of cell expansion rate to a rate that is too low to contribute to tumour formation). In the presence of a pro-drug or other inducer of the negatively selectable system, cells expressing the negative selectable marker will stop proliferating or die, depending on the mechanism of action of the selectable marker. Cells may be modified to comprise homozygous, heterozygous, hemizygous or compound heterozygous ALINKS. To improve fidelity of ablation, a negative selectable marker may be introduced into all alleles functional of a CDL. A negative selectable marker may be introduced into all functional alleles of a CDL.

An ALINK may be inserted in any position of CDL, which allows co-expression of the CDL and the negative selectable marker.

As discussed further below in Example 1, DNA encoding a negatively selectable marker (e.g., HSV-TK), may be inserted into a CDL (e.g., CDK1) in a host cell, such that expression of the negative selectable marker causes host cells expressing the negative selectable marker and, necessarily, the CDL, to be killed in the presence of an inducer (e.g., prodrug) of the negative selectable marker (e.g., ganciclovir (GCV)). In this example, host cells modified with the ALINK will produce thymidine kinase (TK) and the TK protein will convert GCV into GCV monophosphate, which is then converted into GCV triphosphate by cellular kinases. GCV triphosphate incorporates into the replicating DNA during S phase, which leads to the termination of DNA elongation and cell apoptosis (Halloran and Fenton, 1998).

A modified HSV-TK gene (Preuβ et al., 2010) is disclosed herein as one example of DNA encoding a negative selectable marker that may be used in an ALINK genetic modification to selectively ablate cells comprising undesirable cell division rate.

It is contemplated herein that alternative and/or additional negative selectable systems could be used in the tools and/or methods provided herein. Various negative selectable marker systems are known in the art (e.g., dCK.DM (Neschadim et al., 2012)).

For example, various negative selectable system having clinical relevance have been under active development in the field of "gene-direct enzyme/prodrug therapy" (GEPT), which aims to improve therapeutic efficacy of conventional cancer therapy with no or minimal side-effects (Hedley et al., 2007; Nawa et al., 2008). Frequently, GEPT involves the use of viral vectors to deliver a gene into cancer cells or into the vicinity of cancer cells in an area of the cancer cells that is not found in mammalian cells and that produces enzymes, which can convert a relatively non-toxic prodrug into a toxic agent.

HSV-TK/GCV, cytosine deaminase/5-fluorocytosine (CD/5-FC), and carboxyl esterase/irinotecan (CE/CPT-11) are examples of negative selectable marker systems being evaluated in GEPT pre- and clinical trials (Danks et al., 2007; Shah, 2012).

To overcome the potential immunogenicity issue of Herpes Simplex Virus type 1 thymidine kinase/ganciclovir (TK/GCV) system, a "humanized" suicide system has been developed by engineering the human deoxycytidine kinase enzyme to become thymidine-active and to work as a negative selectable (suicide) system with non-toxic prodrugs: bromovinyl-deoxyuridine (BVdU), L-deoxythymidine (LdT) or L-deoxyuridine (LdU) (Neschadim et al., 2012).

The CD/5-FC negative selectable marker system is a widely used "suicide gene" system. Cytosine deaminase (CD) is a non-mammalian enzyme that may be obtained from bacteria or yeast (e.g., from *Escherichia coli* or *Saccharomyces cerevisiae,* respectively) (Ramnaraine et al., 2003). CD catalyzes conversion of cytosine into uracil and is an important member of the pyrimidine salvage pathway in prokaryotes and fungi, but it does not exist in mammalian cells. 5-fluorocytosine (5-FC) is an antifungal prodrug that causes a low level of cytotoxicity in humans (Denny, 2003). CD catalyzes conversion of 5-FC into the genotoxic agent 5-FU, which has a high level of toxicity in humans (Ireton et al., 2002).

The CE/CPT-11 system is based on the carboxyl esterase enzyme, which is a serine esterase found in a different tissues of mammalian species (Humerickhouse et al., 2000). The anti-cancer agent CPT-11 is a prodrug that is activated by CE to generate an active referred to as 7-ethyl-10-hydroxycamptothecin (SN-38), which is a strong mammalian topoisomerase I inhibitor (Wierdl et al., 2001). SN-38 induces accumulation of double-strand DNA breaks in dividing cells (Kojima et al., 1998).

Another example of a negative selectable marker system is the iCasp9/AP1903 suicide system, which is based on a modified human caspase 9 fused to a human FK506 binding protein (FKBP) to allow chemical dimerization using a small molecule AP1903, which has tested safely in humans. Administration of the dimerizing drug induces apoptosis of cells expressing the engineered caspase 9 components. This system has several advantages, such as, for example, including low potential immunogenicity, since it consists of human gene products, the dimerizer drug only effects the cells expressing the engineered caspase 9 components (Straathof et al., 2005). The iCasp/AP1903 suicide system is being tested in clinical settings (Di Stasi et al., 2011).

It is contemplated herein that the negative selectable marker system of the ALINK system could be replaced with a proliferation antagonist system. The term "proliferation antagonist" as used herein, refers to a natural or engineered compound(s) whose presence inhibits (completely or partially) division of a cell. For example, Omomyc^{ER} is the fusion protein of MYC dominant negative Omomyc with mutant murine estrogen receptor (ER) domain. When induced with tamoxifen (TAM), the fusion protein Omomyc^{ER} localizes to the nucleus, where the dominant negative Omomyc dimerizes with C-Myc, L-Myc and N-Myc, sequestering them in complexes that are unable to bind the Myc DNA binding consensus sequences (Soucek et al., 2002). As a consequence of the lack of Myc activity, cells are unable to divide (Oricchio et al., 2014). Another example of a proliferation antagonist is A-Fos, a dominant negative to activation protein-1 (AP1) (a heterodimer of the oncogenes Fos and Jun) that inhibits DNA binding in an equimolar competition (Olive et al., 1997). A-Fos can also be fused to ER domain, rendering its nuclear localization to be induced by TAM. Omomyc^{ER} / tamoxifen or A-Fos^{ER} / tamoxifen could be a replacement for TK/GCV to be an ALINK.

### Targeting a CDL with an EARC Genetic Modification

The disclosure provides molecular tools, methods and kits for exogenously controlling a CDL by operably linking the CDL with an EARC, such as an inducible activator-based gene expression system. Under these conditions, the CDL will only be expressed (and the cell can only divide) in the presence of the inducer of the inducible activator-based gene expression system. Under these conditions, EARC-modified cells stop dividing, significantly slow down, or die in the absence of the inducer, depending on the mechanism of action of the inducible activator-based gene expression system and CDL function. Cells may be modified to comprise homozygous or compound heterozygous EARCs or may be altered such that only EARC-modified alleles could produce functional CDLs.An EARC modification may be introduced into all alleles of a CDL, for example, to provide a mechanism for cell division control.

An EARC may be inserted in any position of CDL that permits co-expression of the CDL and the activator component of the inducible system in the presence of the inducer.

An "activator" based gene expression system may be preferable to a "repressor" based gene expression system. For example, if a repressor is used to suppress a CDL a loss of function mutation of the repressor could release CDL expression, thereby allowing cell proliferation. In a case of an activation-based suppression of cell division, the loss of activator function (mutation) would shut down CDL expression, thereby disallowing cell proliferation.

As discussed further below in Examples 2-6, a dox-bridge may be inserted into a CDL (e.g., CDK1) in a host cell, such that in the presence of an inducer (e.g., doxycycline or "DOX") the dox-bridge permits CDL expression, thereby allowing cell division and proliferation. Host cells modified with a dox-bridge EARC may comprise a reverse tetracycline Trans-Activator (rtTA) gene (Urlinger et al., 2000) under the transcriptional control of a promoter, which is active in dividing cells (e.g., in the CDL). This targeted insertion makes the CDL promoter no longer available for CDL transcription. To regain CDL transcription, a tetracycline responder element promoter (for example TRE (Agha-Mohammadi et al., 2004)) is inserted in front of the CDL transcript, which will express the CDL gene only in a situation when rtTA is expressed and doxycycline is present. When the only source of CDL expression is dox-bridged alleles, there is no CDL gene expression in the absence of doxycycline. The lack of CDL expression causes the EARC-modified cells to be compromised in their proliferation, either by death, stopping cell division, or by rendering the cell mitotic rate so slow that the EARC-modified cell could not contribute to tumor formation.

The term "dox-bridge" as used herein, refers to a mechanism for separating activity of a promoter from a target transcribed region by expressing rtTA (Gossen et al., 1995) by the endogenous or exogenous promoter and rendering the transcription of target region under the control of TRE. As used herein, "rtTA" refers to the reverse tetracycline transactivator elements of the tetracycline inducible system (Gossen et al., 1995) and "TRE" refers to a promoter consisting of *TetO* operator sequences upstream of a minimal promoter. Upon binding of rtTA to the TRE promoter in the presence of doxycycline, transcription of loci downstream of the TRE promoter increases. The rtTA sequence may be inserted in the same transcriptional unit as the CDL or in a different location of the genome, so long as the transcriptional expression's permissive or non-permissive status of the target region is controlled by doxycycline. A dox-bridge is an example of an EARC.

Introduction of an EARC system into the 5' regulatory region of a CDL is also contemplated herein.

It is contemplated herein that alternative and/or additional inducible activator-based gene expression systems could be used in the tools and or methods provided herein to produce EARC modifications. Various inducible activator-based gene expression systems are known in the art.

For example, destabilizing protein domains (Banaszynski et al., 2006) fused with an acting protein product of a coding CDL could be used in conjunction with a small molecule synthetic ligand to stabilize a CDL fusion protein when cell division and/or proliferation is desirable. In the absence of a stabilizer, destabilized-CDL-protein will be degraded by the cell, which in turn would stop proliferation. When the stabilizer compound is added, it would bind to the destabilized-CDL-protein, which would not be degraded, thereby allowing the cell to proliferate.

For example, transcription activator-like effector (TALE) technology (Maeder et al., 2013) could be combined with dimerizer-regulated expression induction (Pollock and Clackson, 2002). The TALE technology could be used to generate a DNA binding domain designed to be specific to a sequence, placed together with a minimal promoter replacing the promoter of a CDL. The TALE DNA binding domain also extended with a drug dimerizing domain. The latter can bind to another engineered protein having corresponding dimerizing domain and a transcriptional activation domain. (FIG. 1C)

For example, referring to FIG. 1D, a reverse-cumate-Trans-Activator (rcTA) may be inserted in the 5' untranslated region of the CDL, such that it will be expressed by the endogenous CDL promoter. A 6-times repeat of a Cumate Operator (6xCuO) may be inserted just before the translational start (ATG) of CDL. In the absence of cumate in the system, rcTA cannot bind to the 6xCuO, so the CDL will not be transcribed because the 6xCuO is not active. When cumate is added, it will form a complex with rcTA, enabling binding to 6xCuO and enabling CDL transcription (Mullick et al., 2006).

For example, referring to FIG. 1E, a retinoid X receptor (RXR) and an N-terminal truncation of ecdysone receptor (EcR) fused to the activation domain of Vp16 (VpEcR) may be inserted in the 5' untranslated region of a CDL such that they are co-expressed by an endogenous CDL promoter. Ecdysone responsive element (EcRE), with a downstream minimal promoter, may also be inserted in the CDL, just upstream of the starting codon. Co-expressed RXR and VpEcR can heterodimerize with each other. In the absence of ecdysone or a synthetic drug analog muristerone A, dimerized RXR/VpEcR cannot bind to EcRE, so the CDL is not transcribed. In the presence of ecdysone or muristerone A, dimerized RXR/VpEcR can bind to EcRE, such that the CDL is transcribed (No et al., 1996).

For example, referring to FIG. 1F, a transient receptor potential vanilloid-1 (TRPV1), together with ferritin, may be inserted in the 5' untranslated region of a CDL and co-expressed by an endogenous CDL promoter. A promoter inducible by NFAT (NFATre) may also be inserted in the CDL, just upstream of the starting codon. In a normal environment, the NFAT promoter is not active. However, upon exposure to low-frequency radio waves, TRPV1 and ferritin create a wave of Ca⁺⁺ entering the cell, which in turn converts cytoplasmatic-NFAT (NFATc) to nuclear-NFAT (NFATn), that ultimately will activate the NFATre and transcribe the CDL (Stanley et al., 2015).

For example, referring to FIG. 1G, a CDL may be functionally divided in to parts/domains: 5'-CDL and 3'CDL, and a FKBP peptide sequence may be inserted into each domain. An IRES (internal ribosomal entry site) sequence may be placed between the two domains, which will be transcribed simultaneously by a CDL promoter but will generate two separate proteins. Without the presence of an inducer, the two separate CDL domains will be functionally inactive. Upon introduction of a dimerization agent, such as rapamycin or AP20187, the FKBP peptides will dimerize, bringing together the 5' and 3' CDL parts and reconstituting an active protein (Rollins et al., 2000).

### Methods of Controlling Division of an Animal Cell

A method of controlling division of an animal cell is provided herein.

The method comprises providing an animal cell. For example, the animal cell may be an avian or mammalian cell. For example, the mammalian cell may be an isolated human or non-human cell that is pluripotent (e.g., embryonic stem cell or iPS cell), multipotent, monopotent progenitor, or terminally differentiated. The mammalian cell may be derived from a pluripotent, multipotent, monopotent progenitor, or terminally differentiated cell. The mammalian cell may be a somatic stem cell, a multipotent or monopotent progenitor cell, a multipotent somatic cell or a cell derived from a somatic stem cell, a multipotent progenitor cell or a somatic cell. Preferably, the animal cell is amenable to genetic modification. Preferably, the animal cell is deemed by a user to have therapeutic value, meaning that the cell may be used to treat a disease, disorder, defect or injury in a subject in need of treatment for same. The non-human mammalian cell may be a mouse, rat, hamster, guinea pig, cat, dog, cow, horse, deer, elk, bison, oxen, camel, llama, rabbit, pig, goat, sheep, or non-human primate cell. The animal cell may be a human cell.

The method further comprises genetically modifying in the animal cell a CDL. The step of genetically modifying the CDL comprises introducing into the host animal cell an iNEP, such as one or more ALINK systems or one or more of an ALINK system and an EARC system. Techniques for introducing into animal cells various genetic modifications, such as negative selectable marker systems and inducible activator-based gene expression systems, are known in the art, including techniques for targeted (i.e., non-random), compound heterozygous and homozygous introduction of same. In cases involving use of EARC modifications, the modification should ensure that functional CDL expression can only be generated through EARC-modified alleles. For example, targeted replacement of a CDL or a CDL with a DNA vector comprising one or more of an ALINKalone or together with one or more EARC systems may be carried out to genetically modify the host animal cell.

The method further comprises permitting division of the genetically modified animal cell(s) comprising the iNEP system.

For example, permitting division of ALINK-modified cells by maintaining the genetically modified animal cells comprising the ALINK system in the absence of an inducer of the corresponding ALINK negative selectable marker. Cell division and proliferation may be carried out *in vitro* and/or *in vivo.* For example, genetically modified cells may be allowed to proliferate and expand *in vitro* until a population of cells that is large enough for therapeutic use has been generated. For example, one or more of the genetically modified animal cell(s) cells that have been proliferated and expanded may be introduced into a host (e.g., by grafting) and allowed to proliferate further *in vivo.* Ablating and/or inhibiting division of the genetically modified animal cell(s) comprising an ALINK system, may be done, *in vitro* and/or *in vivo,* by exposing the genetically modified animal cell(s) comprising the ALINK system to the inducer of the corresponding negative selectable marker. Such exposure will ablate proliferating cells and/or inhibit the genetically modified animal cell's rate of proliferation by killing at least a portion of proliferating cells. Ablation of genetically modified cells and/or inhibition of cell proliferation of the genetically modified animal cells may be desirable if, for example, the cells begin dividing at a rate that is faster than normal *in vitro* or *in vivo,* which could lead to tumor formation and/or undesirable cell growth.

For example, permitting division of EARC-modified cells by maintaining the genetically modified animal cell comprising the EARC system in the presence of an inducer of the inducible activator-based gene expression system. Cell division and proliferation may be carried out *in vitro* and/or *in vivo.* For example, genetically modified cells may be allowed to proliferate and expand *in vitro* until a population of cells that is large enough for therapeutic use has been generated. For example, one or more of the genetically modified animal cell(s) cells that have been proliferated and expanded may be introduced into a host (e.g., by grafting) and allowed to proliferate further *in vivo.* Ablating and/or inhibiting division of the genetically modified animal cell(s) comprising the EARC system, may be done, *in vitro* and/or *in vivo,* by preventing or inhibiting exposure the genetically modified animal cell(s) comprising the EARC system to the inducer of the inducible activator-based gene expression system. The absence of the inducer will ablate proliferating cells and/or inhibit the genetically modified animal cell's expansion by proliferation such that it is too slow to contribute to tumor formation. Ablation and/or inhibition of cell division of the genetically modified animal cells may be desirable if, for example, the cells begin dividing at a rate that is faster than normal *in vitro* or *in vivo,* which could lead to tumor formation and/or undesirable cell growth.

For example, in the method provided herein, set forth in various Examples below, the inducers are doxycycline and ganciclovir.

Doxycycline may be delivered to cells *in vitro* by adding to cell growth media a concentrated solution of the inducer, such as, for example, about 1 mg/ml of Dox dissolved in H₂O to a final concentration in growth media of about 1 µg/ml. *In vivo,* doxycycline may be administered to a subject orally, for example through drinking water (e.g., at a dosage of about 5-10 mg/kg) or eating food (e.g., at a dosage of about 100 mg/kg), by injection (e.g., I.V. or I.P. at a dosage of about 50 mg/kg) or by way of tablets (e.g., at a dosage of about 1-4 mg/kg).

Ganciclovir may be delivered to cells *in vitro* by adding to cell growth media a concentrated solution of the inducer, such as, for example, about 10 mg/ml of GCV dissolved in H₂O to a final concentration in growth media of about 0.25-25 µg/ml. *In vivo,* GCV may be administered to a subject orally, for example through drinking water (e.g., at a dosage of about 4-20 mg/kg) or eating food (e.g., at a dosage of about 4-20 mg/kg), by injection (e.g., at a dosage of about I.V. or I.P. 50 mg/kg) or by way of tablets (e.g., at a dosage of about 4-20 mg/kg).

To assess whether the inducers are working *in vitro,* cell growth and cell death may be measured (e.g., by cell counting and viability assay), for example every 24 hours after treatment begins. To assess whether the inducers are working *in vivo,* the size of teratomas generated from genetically modified pluripotent cells may be measured, for example, every 1-2 days after treatment begins.

In the method provided herein, an animal cell may be genetically modified to comprise both ALINK and EARC systems. The ALINK and EARC systems may target the same or different CDLs. Such cells may be desirable for certain applications, for example, because they provide a user with at least two mechanisms for ablating and/or inhibiting cell division and/or ablating and/or inhibiting proliferation by killing at least a portion of proliferating cells.

It is contemplated herein that the method provided herein may be used to control division and/or proliferation of an avian cell, such as, for example, a chicken cell.

### Cells Engineered to Comprise at Least One Mechanism for Controlling Cell Division

An animal cell genetically modified to comprise at least one mechanism for controlling cell division and/or proliferation, and populations of same, are provided herein. For example, the mammalian cell may be an isolated human or non-human cell that is pluripotent (e.g., embryonic stem cell or iPS cell), multipotent, monopotent progenitor, or terminally differentiated. The mammalian cell may be derived from a pluripotent, multipotent, monopotent progenitor, or terminally differentiated cell. The mammalian cell may be a somatic stem cell, a multipotent, monopotent progenitor, progenitor cell or a somatic cell or a cell derived from a somatic stem cell, a multipotent or monopotent progenitor cell or a somatic cell. Preferably, the animal cell is amenable to genetic modification. Preferably, the animal cell is deemed by a user to have therapeutic value, meaning that the cell may be used to treat a disease, disorder, defect or injury in a subject in need of treatment for same. The non-human mammalian cell may be a mouse, rat, hamster, guinea pig, cat, dog, cow, horse, deer, elk, bison, oxen, camel, llama, rabbit, pig, goat, sheep, or non-human primate cell.

The genetically modified cells provided herein comprise one or more genetic modification of one or more CDL. The genetic modification of a CDL being an ALINK system and, in the case of CDLs, one or more of an ALINK system and an EARC system, such as, for example, one or more of the ALINK and/or EARC systems described herein. For example, a genetically modified animal cell provided herein may comprise: an ALINK system in one or more CDLs; an EARC system in one or more CDLs; or ALINK and EARC systems in one or more CDLS, wherein the ALINK and EARC systems correspond to the same or different CDLs. The genetically modified cells may comprise homozygous, heterozygous, hemizygous or compound heterozygous ALINK genetic modifications. In the case of EARC modifications, the modification should ensure that functional CDL expression can only be generated through EARC-modified alleles.

It is contemplated that the genetically modified cells provided herein may be useful in cellular therapies directed to treat a disease, disorder or injury and/or in cellular therapeutics that comprise controlled cellular delivery of compounds and/or compositions (e.g., natural or engineered biologics). As indicated above, patient safety is a concern in cellular therapeutics, particularly with respect to the possibility of malignant growth arising from therapeutic cell grafts. For cell-based therapies where intensive proliferation of the therapeutic cell graft is not required, it is contemplated that the genetically modified cells comprising one or more iNEP modifications, as described herein, would be suitable for addressing therapeutic and safety needs. For cell-based therapies where intensive proliferation of the therapeutic cell graft is required, it is contemplated that the genetically modified cells comprising two or more iNEP modifications, as described herein, would be suitable for addressing therapeutic and safety needs.

It is contemplated herein that avian cells, such as chicken cells, may be provided, wherein the avian cells comprise the above genetic modifications.

### Molecular Tools for Targeting CDLs

Various DNA vectors for modifying expression of a CDL are provided herein.

The DNA vector may comprise an ALINK system, the ALINK system comprising a DNA sequence encoding a negative selectable marker. The expression of the negative selectable marker may be linked to that of a CDL.

The DNA vector may comprise an EARC system, the EARC system may comprise an inducible activator-based gene expression system that is operably linked to a CDL, wherein expression of the CDL is inducible by an inducer of the inducible activator-based gene expression system.

The DNA vector may comprise an ALINK system, as described herein, and an EARC system, as described herein. When such a cassette is inserted into a host cell, CDL transcription product expression may be prevented and/or inhibited by an inducer of the negative selectable marker of the ALINK system and expression of the CDL may be inducible by an inducer of the inducible activator-based gene expression system of the EARC system.

The CDL in the DNA vector may be a CDL listed in Table 2.

The ALINK system in the DNA vector may be a herpes simplex virus-thymidine kinase/ ganciclovir system, a cytosine deaminase/5-fluorocytosine system, a carboxyl esterase/irinotecan system or an iCasp9/AP1903 system.

The EARC system in the DNA vector may be a dox-bridge system, a cumate switch inducible system, an ecdysone inducible system, a radio wave inducible system, or a ligand-reversible dimerization system.

### Kits

The present disclosure contemplates kits for carrying out the methods disclosed herein. Such kits typically comprise two or more components required for using CDLs and/or CDLs to control cell proliferation. Components of the kit include, but are not limited to, one or more of compounds, reagents, containers, equipment and instructions for using the kit. Accordingly, the methods described herein may be performed by utilizing pre-packaged kits provided herein. The kit may comprise one or more DNA vectors and instructions. The instructions may comprise one or more protocols for introducing the one or more DNA vectors into host cells. The kit may comprise one or more controls.

The kit may comprise one or more DNA vector for modifying expression of a CDL, as described herein. By way of example, the kit may contain a DNA vector comprising an ALINK system; and/or a DNA vector comprising an EARC system; and/or a DNA vector comprising an ALINK system and an EARC system; and instructions for targeted replacement of a CDL and/or CDL in an animal cell using one or more of the DNA vectors. The kit may further comprise one or more inducers (e.g., drug inducer) that correspond with the ALINK and/or EARC systems provided in the DNA vector(s) of the kit.

The following non-limiting examples illustrative of the disclosure are provided.

### Example 1: Generation of ALINK-Modified Cells (Mouse and Human)

In Example 1, construction of ALINK (HSV-TK) vectors targeting Cdk1/CDK1 and use of same to control cell proliferation in mouse and human reference ES cells, by way of killing at least a portion of proliferating cells, is described. In this example, Cdk1/CDK1 is the CDL and HSV-TK is the negative selectable marker.

Cdk1/CDK1 is expressed in all mitotically active (i.e., dividing) cells. In cells modified to comprise a homozygous ALINK between the CDK1 locus and HSV-TK, all mitotically active cells express CDK1 and HSV-TK. Thus, the ALINK-modified mitotically active cells can be eliminated by treatment with GCV (the pro-drug of HSV-TK). If all the functional CDK1 expressing allele is ALINK modified and the cells were to silence HSV-TK expression then likely CDK1 expression would also be silenced and the cells would no longer be able to divide. Quiescent (i.e., non-dividing) cells do not express Cdk1/CDK1. Thus, ALINK-modified quiescent cells would not express the Cdk1/CDK1-HSV-TK link.

In Example 1, the transcriptional link between Cdk1/CDK1 and HSV-TK was achieved by homologous recombination-based knock-ins.

### METHODS

### Generation of Target Vectors

*Mouse Target Vector* I: The mouse *Cdk1* genomic locus is shown in FIG. 2A. Referring to FIG. 2B, two DNA fragments: 5TK (SEQ ID NO: 1) and 3TK (SEQ ID NO: 2) (Sall-F2A-5'TK.007-PB 5'LTR-Notl-Sacll and Sall-Sacll-3'TK.007-PB 3'LTR-3'TK.007-T2A-Xhol-mCherry-Nhel) were obtained by gene synthesis in a pUC57 vector (GenScript). Fragment 5TK was digested with Sall + Sacll and cloned into 3TK with the same digestion to generate pUC57-5TK-3TK. A PGK-Neomycin cassette was obtained by cutting the plasmid pBluescript-M214 (SEQ ID NO: 3) with Notl + HindiII and it was ligated into the Notl + Sacll site of pUC57-5TK-3TK to generate the ALINK cassette to be inserted at the 3' end of Cdk1 (i.e., the CDL).

*Homology arms for the insertion ALINK at the 3' of the CDL:* Cdk1 DNA coding sequences were cloned by recombineering: DH10B *E. coli cell* strain containing bacterial artificial chromosomes (BACs) with the genomic sequences of Cdk1 (SEQ ID NO: 4), which were purchased from The Center for Applied Genomics (TCAG). The recombineering process was mediated by the plasmid pSC101-BAD-γβα Red/ET (pRET) (GeneBridges, Heidelberg Germany). pRET was first electroporated into BAC-containing DH10B *E. coli* at 1.8kV, 25µF, 400Ohms (BioRad GenePulserI/II system, BioRad, ON, CA) and then selected for choloramphenicol and tetracycline resistance. Short homology arms (50bp) (SEQ ID NOs: 5 and 6 respectively) spanning the ALINK insertion point (5' and 3' of the Cdk1 stop codon) were added by PCR to the cassette, F2A-5'TK-PB-PGKneo-PB-3'TK-T2A-cherry. This PCR product was then electroporated into Bac+pRET DH10B *E. coli* under the conditions described above and then selected for kanamycin resistance. The final targeting cassette, consisting of 755bp and 842 base pair (bp) homology arms (SEQ ID NOs: 7 and 8, respectively), was retrieved by PCR with primers (SEQ ID NOs: 9 and 10, respectively) and cloned into a pGemT-Easy vector to generate mouse Target Vector I. The critical junction regions of the vector were sequenced at TCAG and confirmed.

*Mouse Target Vector II:* referring to FIG. 2D, F2A-IoxP-PGK-neo-pA-IoxP-AscI (SEQ ID NO: 11) was PCR amplified from pLoxPNeo1 vector and TA cloned into a pDrive vector (Qiagen). AscI-TK-T2A-mCherry-EcoRI (SEQ ID NO: 12) was PCR amplified from excised TC allele I, and TA cloned into the pDrive vector. The latter fragment was then cloned into the former vector by BamHI + AscI restriction sites. This F2A-IoxP-PGK-neo-pA-IoxP-TK-T2A-mCherry cassette was inserted between mouse Cdk1 homology arms by GeneArt® Seamless Cloning and Assembly Kit (Life Technologies). To generate the puromycin (puro) version vector, PGK-puro-pA fragment (SEQ ID NO: 13) was cut from pNewDockZ with BamHI + Notl and T4 blunted. The neo version vector was cut with Ascl+Clal, T4 blunted and ligated with PGK-puro-pA.

*Human Target Vector I:* Similar to mouse Target Vector I, 847 bp upstream of human CDK1 stop codon (SEQ ID NO: 14) + F2A-5'TK-PB-PGKneo-PB-3'TK-T2A-cherry (SEQ ID NO: 15) + 831 bp downstream of human CDK1 stop codon (SEQ ID NO: 16) was generated by recombineering technology. A different version of the vector containing a puromycin resistant cassette for selection, was generated to facilitate one-shot generation of homozygous targeting: Agel-PGK-puro-pA-Fsel (SEQ ID NO: 17) was amplified from pNewDockZ vector, digested and cloned into neo version vector cut by Agel+Fsel.

*Human Target Vector II:* BamHI-F2A-IoxP-PGK-neo-pA-IoxP-TK-T2A-mCherry (SEQ ID NO: 18) and BamHI-F2A-IoxP-PGK-puro-pA-IoxP-TK-T2A-mCherry (SEQ ID NO: 19) were amplified from the corresponding mouse Target Vector II, and digested with BamHI+SgrAI. The mCherry (3' 30bp)-hCDK13'HA-pGemEasy-hCDK15'HA-BamHI (SEQ ID NO: 20) was PCR-amplified and also digested with BamHI+SgrAI. The neo and puromycin version of human Target Vector II were generated by ligation of the homology arm backbone and the neo or puromycin version ALINK cassette.

*Human Target Vector III: Target vectors with no selection cassette were made for targeting with fluorescent marker (mCherry or eGFP) by FACS and avoiding the step of excision of selection cassette.* BamHI-F2A-TK-T2A-mCherry-SgrAI (SEQ ID NO: 58) was PCR amplified from excised TC allele I, digested with BamHI+SgrAI, and ligated with digested mCherry (3' 30bp)-hCDK13'HA-pGemEasy-hCDK15'HA-BamHI (SEQ ID NO: 20). The CRISPR PAM site in the target vector was mutagenized with primers PAM_fwd (SEQ ID NO: 59) and PAM_rev (SEQ ID NO: 60) using site-directed PCR-based mutagenesis protocol. The GFP version vector was generated by fusion of PCR-amplified Xhol-GFP (SEQ ID NO: 61) and pGemT-hCdk1-TK-PAMmut (SEQ ID NO: 62) with NEBuiler HiFi DNA Assembly Cloning Kit (New England Biolabs Inc.).

### Generation of CRISPR/Cas9 plasmids

CRISPR/Cas9-assisted gene targeting was used to achieve high targeting efficiency (Cong et al., 2013). Guide sequences for CRISPR/Cas9 were analyzed using the online CRISPR design tool (http://crispr.mit.edu) (Hsu et al., 2013).

CRISPR/Cas9 plasmids pX335-mCdkTK-A (SEQ ID NO: 21) and pX335-mCdkTK-B (SEQ ID NO: 22) were designed to target mouse Cdk1 at SEQ ID NO: 23.

CRISPR/Cas9 plasmids pX330-hCdkTK-A (SEQ ID NO: 24) and pX459-hCdkTK-A (SEQ ID NO: 25) were designed to target the human Cdk1 at SEQ ID NO: 26.

CRISPRs were generated according to the suggested protocol with backbone plasmids purchased from Addgene. (Ran et al., 2013).

### Generation of ALINK-Modified Mouse ES Cells

*Mouse ES Cell Culture:* Mouse ES cells are cultured in Dulbecco's modified Eagle's medium (DMEM) (high glucose, 4500 mg/liter) (Invitrogen), supplemented with 15% Fetal Bovine Serum (Invitrogen), 1mM Sodium pyruvate (Invitrogen), 0.1 mM MEM Non-essential Amino-acids (Invitrogen), 2mM GlutaMAX (Invitrogen), 0.1 mM 2-mEARCaptoethanol (Sigma), 50U/ml each Penicillin/ Streptomycin (Invitrogen) and 1000U/ml Leukemia-inhibiting factor (LIF) (Chemicon). Mouse ES cells are passed with 0.25% trypsin and 0.1% EDTA.

*Targeting:* 5x10⁵ mouse C57BL/6 C2 ES cells (Gertsenstein et al., 2010) were transfected with 2ug DNA (Target Vector:0.5 µg, CRISPR vector: 1.5 µg ) by JetPrime transfection (Polyplus). 48h after transfection cells were selected for G418 or/and puromycin-resistant. Resistant clones were picked independently and transferred to 96-well plates. 96-well plates were replicated for freezing and genotyping (SEQ ID NOs: 27, 28, 29 and 30). PCR-positive clones were expanded, frozen to multiple vials, and genotyped by southern blotting.

*Excision of the selection cassette:* correctly targeted ES clones were transfected with Episomal-hyPBase (for Target Vector I) (SEQ ID NO: 34) or pCAGGs-NLS-Cre-Ires-Puromycin(for Target Vector II) (SEQ ID NO: 35). 2-3 days following transfection, cells were trypsinized and plated clonally (1000-2000 cells per 10cm plate). mCherry-positive clones were picked and transferred to 96-well plates independently and genotyped by PCR (SEQ ID NOs: 31 and 36) and Southern blots to confirm the excision event. The junctions of the removal region were PCR-amplified, sequenced and confirmed to be intact and seamless without frame shift.

*Homozygous targeting:* ES clones that had already been correctly targeted with a neo version target vector and excised of selection cassette were transfected again with a puromycin-resistant version of the target vector. Selection of puromycin was added after 48 hours of transfection, then colonies were picked and analyzed, as described above (SEQ ID NOs: 31 and 32). Independent puro-resistant clones were grown on gelatin, then DNA was extracted for PCR to confirm the absence of a wild-type allele band (SEQ ID NOs: 31, 33).

### Generation of ALINK-Modified Human ES Cells

*Human ES Cell Culture:* Human CA1 or H1 (Adewumi et al., 2007) ES cells were cultured with mTeSR1 media (STEMCELL Technologies) plus penicillin-streptomycin (Gibco by Life Technologies) on Geltrex (Life Technologies) feeder-free condition. Cells were passed by TrypIE Express (Life Technologies) or Accutase (STEMCELL Technologies) and plated on mTeSR media plus ROCK inhibitor (STEMCELL Technologies) for the first 24h, then changed to mTeSR media. Half of cells from a fully confluent 6-well plate were frozen in 1ml 90% FBS (Life Technologies) + 10%DMSO (Sigma).

*Targeting:* 6x10⁶ CA1 hES cells were transfected by Neon protocol 14 with 24ug DNA (Target Vector: pX330-hCdkTK-A = 18ug:6ug). After transfection, cells were plated on four 10-cm plates. G418 and/or puromycin selection was started 48h after transfection. Independent colonies were picked to 96-well plates. Each plate was duplicated for further growth and genotyping (SEQ ID NOs: 37, 38, 39 and 40). PCR-positive clones were expanded, frozen to multiple vials and genotyped with southern blotting.

*Excision of the selection cassette:* ALINK-targeted ES clones were transfected with hyPBase or pCAGGs-NLS-Cre-IRES-Puromycin and plated in a 6-well plate. When cells reached confluence in 6-well plates, cells were suspended in Hanks Balanced Salt Solution (HBSS) (Ca2+/ Mg2+ Free) (25 mM HEPES pH7.0, 1% Fetal Calf Serum), and mCherry-positive cells were sorted to a 96-well plate using an ASTRIOS EQ cell sorter (Beckman Coulter).

*Homozygous Targeting:* Homozygous targeting can be achieved by the same way as in the mouse system or by transfecting mCherry and eGFP human target vector III plus pX330-hCdkTK-A or pX459-hCdkTK-A followed by FACS sorting for mCherry-and-eGFP double-positive cells.

### Teratoma Assay

Matrigel Matrix High Concentration (Corning) was diluted 1:3 with cold DMEM media on ice. 5-10x10⁶ cells were suspended into 100ul of 66% DMEM + 33% Matrigel media and injected subcutaneously into either or both dorsal flanks of B6N mice (for mouse C2 ES cells) and NOD-SCID mice (for human ES cells). Teratomas formed 2-4 weeks after injection. Teratoma size was measured by caliper, and teratoma volume was calculated using the formula V= (LxWxH)π/6. GCV/PBS treatment was performed by daily injection with 50mg/kg into the peritoneal cavity with different treatment durations. At the end of treatment, mice were sacrificed and tumors were dissected and fixed in 4% paraformaldehyde for histology analysis.

### Mammary gland tumor assay

Chimeras of Cdk1^{+/+,+/loxp-alink} mouse C2 ES and CD-1 backgrounds were generated through diploid aggregation, and then were bred with B6N WT mice to generate Cdk1^{+/+,+/loxp-alink} mice through germline transmission. Qdk1^{+/+,+/loxp-alink} mice were bred with Ella-Cre mice to generate Cdk1^{+/+,+/alink} mice. Cdk1^{+/+,+/alink} mice were then bred with MMTV-PyMT mice (Guy et al., 1992) to get double-positive pups with mammary gland tumors and ALINK modification. Mammary gland tumors with fail-safe modification were isolated, cut into 1mm³ pieces, and transplanted into the 4^{th} mammary gland of wild-type B6N females. GCV/PBS treatment was injected every other day at the dosage of 50mg/kg into the peritoneal cavity with different treatment durations. Mammary gland tumor size was measured by calipers and calculated with the formula V=Length*Width*Height* π/6.

### Neuronal progenitor vs. neuron killing assay

Cdk1^{+/+,+/alink} human CA1 ES cells were differentiated to neural epithelial progenitor cells (NEPs). NEPs were subsequently cultured under conditions for differentiation into neurons, thereby generating a mixed culture of non-dividing neurons and dividing NEPs, which were characterized by immunostaining of DAPI, Ki67 and Sox2. GCV (10uM) was provided to the mixed culture every other day for 20 days. Then, GCV was withdrawn from culture for 4 days before cells were fixed by 4% PFA. Fixed cells were immunostained for proliferation marker Ki67 to check whether all the leftover cells have exited cell cycle, and mature neutron marker beta-Tublinlll.

### RESULTS

The mouse *Cdk1* genomic locus is shown in FIG. 2a. Two vectors targeting murine *Cdk1* were generated (FIGs. 2B and D), each configured to modify the 3'UTR of the *Cdk1* gene (FIG. 2A) by replacing the STOP codon of the last exon with an F2A (Szymczak et al., 2004) sequence followed by an enhanced *HSV-TK* (TK.007 (Preuß et al., 2010)) gene connected to an mCherry reporter with a T2A (Szymczak et al., 2004) sequence.

Referring to FIG. 2B and mouse target vector I, the PGK-neo-pA selectable marker (necessary for targeting) was inserted into the TK.007 open-reading-frame with a *piggyBac* transposon, interrupting TK expression. The *piggyBac* transposon insertion was designed such that transposon removal restored the normal ORF of TK.007, resulting in expression of functional thymidine kinase (FIG. 2C).

Referring to FIG. 2D and mouse target vector II, the neo cassette was IoxP-flanked and inserted between the F2A and TK.007.

Target vectors I and II had short (-800 bp) homology arms, which were sufficient for CRISPRs assisted homologous recombination targeting and made the PCR genotyping for identifying targeting events easy and reliable. The CRISPRs facilitated high targeting frequency at 40% PCR-positive of drug-resistant clones (FIG. 3D).

Both the *piggyBac*-inserted and the IoxP-flanked neo cassettes were removed by transient expression of the piggyBac transposase and Cre recombinase, respectively, resulting in cell lines comprising alleles shown in FIGs. 2C and 2E, respectively. Referring to FIG. 2E, the remaining IoxP site was in frame with TK and added 13 amino acids to the N-terminus of TK. The TK functionality test (GCV killing) proved that this N-terminus insertion did not interfere with TK function.

Referring to FIG. 4, assisted with CRISPR-Cas9 technology, homozygous ALINK can also be generated efficiently in two different human ES cell lines, CA1 and H1 (Adewumi et al., 2007).

Referring to FIG. 5A and 5C, the data indicate that: i) the TK.007 insertion into the 3'UTR of Cdk1 does not interfere with Cdk1 expression; ii) the ALINK-modified homozygous mouse C2 ES cells properly self-renew under ES cell conditions and differentiate in vivo and form complex teratomas; iii) the ALINK-modified homozygous human CA1 ES cells properly self-renew under ES cell conditions and differentiate in vivo and form complex teratomas.

Referring to FIG. 6, the data indicate that: i) TK.007 is properly expressed; GCV treatment of undifferentiated ES cells ablates both homozygously- and heterozygously-modified cells (FIG. 6A); and ii) the T2A-linked mCherry is constitutively expressed in ES cells (FIG. 6B).

Referring to FIG. 7A, the data indicate that in hosts comprising ALINK-modified cell grafts, GCV treatment of subcutaneous teratomas comprising the ALINK-modified ES cells stops teratoma growth by ablating dividing cells. GCV treatment did not affect quiescent cells of the teratoma. A brief (3 week) GCV treatment period of the recipient was sufficient to render the teratomas dormant. Referring to FIG. 7B, in NOD scid gamma mouse hosts comprising ALINK-modified human cell grafts, two rounds of GCV treatment (1st round 15 days + 2^{nd} round 40 days) rendered the teratomas to dormancy.

Referring to FIG. 7C, in B6N hosts comprising ALINK-modified MMTV-PyMT-transformed mammary epithelial tumorigenic cell grafts, GCV treatment was able to render the mammary gland tumors to dormancy.

Referring to FIGS. 7D-F, in a mixed culture of non-dividing neurons and dividing NEPs, all cells having been derived from Cdk1^{+/+,+/alink} human CA1 ES cells, GCV killed the dividing NEPs but did not kill the non-dividing neurons.

It is contemplated that one or more dividing cells could escape GCV-mediated ablation if an inactivating mutation were to occur in the HSV-TK component of the CDL-HSV-TK transcriptional link. To address the probability of cell escape, the inventors considered the general mutation rate per cell division (i.e., 10⁻⁶) and determined that the expected number of cell divisions required to create 1 mutant cell would be 16 in cells comprising a heterozygous Cdk1 - HSV-TK transcriptional link, and 30 cell divisions in cells comprising a homozygous Cdk1 - HSV-TK transcriptional link. This means that if a single heterozygous ALINK-modified cell is expanded to 2¹⁶ (i.e., 65,000 cells) and a single homozygous ALINK-modified cell is expanded to 2³⁰ (i.e., 1 billion cells), then an average of one mutant cell comprising lost HSV-TK activity per heterozygous and homozygous cell population would be generated (FIG. 8). Accordingly, the inventors have determined that homozygous ALINK-modified cells would be very safe for use in cell-based therapies. Another way of calculating the level of safety of cell therapy was presented above.

### Example 2: Generation of EARC-Modified Mouse ES Cells in the Cdk1 Locus

In Example 2, construction of EARC (dox-bridge) vectors targeting Cdk1 and use of same to control cell division in mouse ES cells is described. In this example, Cdk1/CDK1 is the CDL, which is targeted with an inducible gene expression system, wherein a dox-bridge is inserted and doxycycline induces expression of the CDL.

As described above, Cdk1/CDK1 is expressed in all mitotically active (i.e., dividing) cells. In cells modified to comprise an EARC (dox-bridge) insertion at the Cdk1 locus, cell division is only possible in the presence of the inducer (doxycycline), which permits expression of Cdk1. Thus, cell division of EARC-modified mitotically active cells can be eliminated in the absence of doxycycline.

In Example 2, dox-bridge insertion into the 5'UTR of the *Cdk1* gene was achieved by homologous recombination knock-in technology.

### METHODS

### Construction of EARC Targeting Vector Comprising a Dox-bridge

A fragment containing an rTTA coding sequence (SEQ ID NO: 41) followed by a 3x SV40 pA signal was amplified by PCR from a pPB-CAGG-rtta plasmid, using primers containing a Iox71 site added at the 5' of the rTTA (rtta3xpaFrw1 (SEQ ID NO: 63), rtta3xpaRev1 (SEQ ID NO: 64)). This fragment was subcloned into a pGemT plasmid, to generate pGem-bridge-step1. Subsequently, a Sacll fragment containing a TetO promoter (SEQ ID NO: 42) (derived from pPB-TetO-IRES-mCherry) was cloned into the Sacll site of the pGem-bridge-step1, generating a pGem-bridge-step2. The final element of the bridge was cloned by inserting a BamHI IRES-Puromycin fragment (SEQ ID NO: 43) into the BamHI site of the pGem-bridge-step2, generating a pGem-bridge-step3. The 5' homology arm was cloned by PCR-amplifying a 900 bp fragment (SEQ ID NO: 44) from C57/B6 genomic DNA (primers cdk5FrwPst (SEQ ID NO: 45) and cdk5RevSpe (SEQ ID NO: 46) and cloning it into Sbfl and Spel of the pGem-bridge-step3. Similarly, the 3' homology arm (900 bp) (SEQ ID NO: 47) was amplified by PCR using primers dkex3_5'FSpe (SEQ ID NO: 48), cdkex3_3Iox (SEQ ID NO: 49) and cloned into Sphl and Ncol to generate a final targeting vector, referred to as pBridge (SEQ ID NO: 148).

### Construction of CRISPR/Cas9 Plasmids

A double-nickase strategy was chosen to minimize the possibility of off-target mutations. Guide RNA sequences (SEQ ID NOs: 50, 51, 52 and 53) were cloned into pX335 (obtained from Addgene, according to the suggested protocol) (Ran et al., 2013).

### Generation of EARC-modified Mouse ES cells

*Mouse ES cell culture:* All genetic manipulations were performed on a C57BL/6N mouse ES cell line previously characterized (C2) (Gertsenstein et al., 2010). Mouse ES cells were grown in media based on high-glucose DMEM (Invitrogen), supplemented with 15% ES cell-grade FBS (Gibco), 0.1 mM 2-mEARCaptophenol, 2 mM L-glutamine, 1 mM sodium pyruvate, 0.1 mM non-essential amino acids, and 2,000 units/ml leukemia inhibitory factor (LIF). Cells were maintained at 37°C in 5% CO₂ on mitomycin C-treated mouse embryonic fibroblasts (MEFs).

*Targeting:* Plasmids containing the CRISPR/Cas9 components (pX335-cdk-ex3A (SEQ ID NO: 151) and px335-cdk-ex3B (SEQ ID NO: 152)) and the targeting plasmid (pBridge; SEQ ID NO: 148) were co-transfected in mouse ES cells using FuGENE HD (Clontech), according to the manufacturer's instructions, using a FuGENE:DNA ratio of 8:2, (2 µg total DNA: 250 ng for each pX330 and 1500 ng for pBridge). Typical transfection was performed on 3x10⁵ cells, plated on 35 mm plates. Upon transfection, doxycycline was added to the media to a final concentration of 1 µg/ml. 2 days following transfection, cells were plated on a 100 mm plate and selection was applied with 1 µg/ml of puromycin. Puromycin-resistant colonies were picked 8-10 days after start of selection and maintained in 96 well plates until PCR-screening.

*Genotyping:* DNA was extracted from ES cells directly in 96 well plates according to (Nagy et al., 2003). Clones positive for correct insertion by homologous recombination of pBridge in the 5' of the Cdk1 gene were screened by PCR using primers spanning the 5' and 3' homology arms (primers rttaRev (SEQ ID NO: 54), ex3_5scr (SEQ ID NO: 55) for the 5' arm, primers CMVforw (SEQ ID NO: 56), ex3_3scr (SEQ ID NO: 57) for the 3' arm).

*Targeted cell growth:* F3-bridge targeted cells were trypsinized and plated on gelatinized 24 well plates at a density of 5x10⁴ cells per well. Starting one day after plating, cell counting was performed by trypsinizing 3 wells for each condition and counting live cells using a Countess automated cell counter (Life Technologies). Doxycycline was removed or reduced to 0.05 ng/ml 2 days after plating and live cells were counted every day up to 18 days in the different conditions.

*Cre-excision:* F3-bridge cells (grown in Dox+ media) were trypsinized and transfected with 2 µg of a plasmid expressing Cre (pCAGG-NLS-Cre). Transfection was performed using JetPrime (Polyplus) according to the manufacturer's protocol. After transfection, doxycycline was removed and colonies were trypsinized and expanded as a pool.

*Quantitative PCR:* Total RNA was extracted from cells treated for 2 days with 1 µg/ml and 0 µg/ml of Dox using the GeneElute total RNA miniprep kit (Sigma) according to the manufacturer's protocol. cDNA was generated by reverse transcription of 1 µg of RNA using the QuantiTect reverse transcription kit (Qiagen), according to the manufacturer's protocol. Real-time qPCR were set up in a BioRad CFX thermocycler, using SensiFast-SYBR qPCR mix (Bioline). The primers used were: qpcrcdk1_F (SEQ ID NO: 65), qpcrcdk1_R (SEQ ID NO:66) and actBf (SEQ ID NO: 67), actBr (SEQ ID NO: 68). Results were analyzed with the ΔΔCT method and normalized for beta-actin.

### RESULTS

Referring to FIG. 9, the dox-bridge target vector, depicted in FIG. 9A, was used to generate three targeted C2 mouse ES cell lines (FIG. 9B). One of these cell lines was found to be a homozygous targeted line (3F in FIG. 9B) comprising a dox-bridge inserted by homologous recombination into the 5'UTR of both alleles of Cdk1.

As expected, this ES cell line grows only in the presence of doxycycline. In the presence of doxycycline, the *Cdk1* promoter activity produced rtTA binds to TRE and initiates transcription of the *Cdk1.* Similarly to the 3' modification, the dox-bridge may be inserted into the 5'UTR into both alleles of *Cdk1,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 5 days (FIG. 10). Lowering the doxycycline concentration by 20x (50 ng/ml) compared to the concentration used for derivation and maintenance of the doc-bridged cell line, allowed some cells/colonies to survive the 5 days period (FIG. 11).

Referring to FIG. 12, the dox-bridge was removable with a Cre recombinase mediated excision of the segment between the two Iox71 sites, which restore the original endogenous expression regulation of the allele and rescues the cell lethality from the lack of doxycycline. These data indicate that the dox-bridge was working in the cells as predicted.

Referring to FIG. 13, the inventors determined how doxycycline withdrawal affected elimination of the dox-bridge ES cells by measuring cell growth in the presence and absent of doxycycline. ES cells in the presence of doxycycline grew exponentially, indicating their normal growth. In contrast, upon withdrawal of doxycycline (Day 1) cells grew for only two days and then cells death began until no live cells were present on Day 9. A 20x lower doxycycline concentration (50ng/ml) provided after an initial 3 days of cell growth was sufficient to maintain a constant number of cells on the plates for at least five days (FIG. 13, light blue line). When the normal concentration of doxycycline was added back to the plate on day 10, cells started growing again as normal ES cells.

It is contemplated that dividing cells could escape EARC (dox-bridge)-modification of Cdk1 when grown in media lacking doxycycline. To address the probability of cell escape, EARC (dox-bridge)-modified mouse ES cells were grown up to 100,000,000 cells/plate on ten plates in medium containing doxycycline. 300 GFP-positive wild-type ES cells (sentinels) were then mixed into each 10 plate of modified ES cells and doxycycline was withdrawn from the culture medium. Only GFP positive colonies were recovered (FIG. 14) indicating that there were no escapee dox-bridged ES cells among the 100,000,000 cells in the culture. Accordingly, the inventors have determined that EARC (dox-bridge)-modified ES cells would add an additional level of safety to ALINK modification for certain cell therapy applications, because loss of the dox-bridge is unlikely to occur by mutation and cell division is not possible in the absence of the inducer (doxycycline) due to the block of CDL expression.

Referring to FIG. 15, the effect of high doxycycline concentration (10 µg/ml) on the growth of dox-bridged ES cells was examined. In the presence of high concentration doxycycline, the growth rate of dox-bridged ES cells slowed to a rate similar to that of cells grown in low concentration doxycycline. These data suggest that there is a range of doxycycline concentrations that may permit optimal Cdk1 expression for wild-type cell-like proliferation.

### Example 3: Generation of EARC-ALINK Modified Cells in the CDK1 locus (Mouse and Human)

In Example 3, construction of EARC (dox-bridge) vectors targeting CDK1 and use of same to control cell division in both mouse and human ALINK-modified ES cells is described. In this example, Cdk1/CDK1 is the CDL, the dox-bridge is the EARC, and HSV-TK is the ALINK. CDL Cdk1 is modified with both EARC and ALINK systems in the homozygous form, wherein doxycycline is required to induce expression of the CDL, and wherein doxycycline and GCV together provide a way of killing the modified proliferating cells.

In Example 3, dox-bridge insertion into the 5'UTR of the *CDK1* gene was achieved by homologous recombination knock-in technology.

### METHODS

Construction of mouse EARC targeting vector, CRISPR/Cas9 plasmids for mouse targeting are the same as in Example 2. Targeting and genotyping methods are also the same as described in Example 2 except that instead of C2 WT cells, Cdk1 (TK/TK) cells generated in Example 1 (FIG. 3A-3G) were used for transfection.

### Construction of EARC Targeting Vector Comprising a Dox-bridge for human CDK1

The 5' homology arm (SEQ ID NO: 69) was cloned by PCR-amplifying a 981 bp fragment from CA1 genomic DNA (primers hcdk5'F (SEQ ID NO: 70) and hcdk5'R (SEQ ID NO: 71) and cloning it into *SbfI of the* pGem-bridge-step3. Similarly, the 3' homology arm (943 bp; SEQ ID NO: 72) was amplified by PCR using primers hcdk3'F (SEQ ID NO: 73) and hcdk3'R (SEQ ID NO: 74) and cloned into *SphI* and *NcoI* to generate a final targeting vector, referred to as pBridge-hCdk1 (SEQ ID NO: 75).

### Construction of CRISPR/Cas9 Plasmids for human targeting

Guide RNA (hCdk1A_up (SEQ ID NO: 76), hCdk1A_low (SEQ ID NO: 77), hCdk1B_up (SEQ ID NO: 78), hCdk1B_low (SEQ ID NO: 79)) were cloned in to pX335 (SEQ ID NO: 149) and pX330 (SEQ ID NO: 150) to generate pX335-1A (SEQ ID NO: 80), pX335-1B (SEQ ID NO: 81) and pX330-1B (SEQ ID NO: 82).

### Generation of EARC-modified Human ES cells

*Targeting:* 2x10⁶ CA1 Cdk1(TK/TK) (i.e., the cell product described in FIGS. 4A-4F) hES cells were transfected by Neon protocol 14 with 8ug DNA (Target Vector: pX330-hCdkTK-A = 6ug:2ug). After transfection, cells were plated on four 10-cm plates. Upon transfection, doxycycline was added to the media to a final concentration of 1 µg/ml. 2 days following transfection, selection was applied with 0.75 µg/ml of puromycin. Puromycin-resistant colonies were picked to 96-well plates, duplicated for further growth and genotyping with primers (hCdk1Br-5HAgen_F1 (SEQ ID NO: 83), rtTA_rev_1 (SEQ ID NO: 84), mCMV_F (SEQ ID NO:85), hCdk1Br-3HAgen_R1 (SEQ ID NO: 86)).

### RESULTS

Referring to FIG. 16A, the mouse dox-bridge target vector, pBridge was used to target mouse cell products generated in Example 1, Cdk1(TK/TK), generating mouse Cdk1^{earc/earc,alink/alink} cells. Nine Cdk1^{earc/earc,alink/alink} clones were generated by one-shot transfection (FIG. 16B).

Referring to FIG. 5B, the data indicate that the EARC-and-ALINK-modified homozygous mouse C2 ES Cdk1 ^{earc/earc,alink/alink} cells properly self-renewed under ES cell conditions, differentiated in vivo, and formed complex teratomas.

Referring to FIG. 17A, the human dox-bridge target vector, pBridge-hCdk1 was used to target human CA1 cell products generated in Example 1, Cdk1(TK/TK), generating human Cdk1 ^{earc/earc,alink/alink} cells. At least Cdk1 ^{earc/earc,alink/alink} CA1 clones were generated by one-shot transfection (FIG. 17B).

### Example 4: Generation of EARC-Modified Mouse ES Cells in the Top2a locus

In Example 4, construction of EARC (dox-bridge) vectors targeting Top2a and use of same to control cell division in mouse ES cells is described. In this example, Top2a/TOP2A is the CDL, which is targeted with an inducible gene expression system, wherein a dox-bridge is inserted and doxycycline induces expression of the CDL.

As described above, Top2a/TOP2A is expressed in all mitotically active (i.e., dividing) cells. In cells modified to comprise an EARC (dox-bridge) insertion at the Top2a locus, cell division is only possible in the presence of the inducer (doxycycline), which permits expression of Top2a. Thus, cell division of EARC-modified mitotically active cells can be eliminated in the absence of doxycycline.

In Example 4, dox-bridge insertion into the 5'UTR of the *Top2a* gene was achieved by homologous recombination knock-in technology.

### METHODS

### Construction of EARC Targeting Vector Comprising a Dox-bridge for Top2a

The 5' homology arm (SEQ ID NO: 87) was cloned by PCR-amplifying a 870 bp fragment from C57/B6 genomic DNA (primers Top5F (SEQ ID NO: 88) and Top5R (SEQ ID NO: 89) and cloning it into Sbfl and Spel of the pGem-bridge-step3. Similarly, the 3' homology arm (818 bp; SEQ ID NO: 90) was amplified by PCR using primers Top3F (SEQ ID NO: 91), Top3R (SEQ ID NO: 92) and cloned into Sphl and Ncol to generate a final targeting vector, referred to as pBridge-Top2a (SEQ ID NO: 93).

### Construction of CRISPR/Cas9 Plasmids

A double-nickase strategy was chosen to minimize the possibility of off-target mutations. Guide RNA sequences were cloned into pX335 (Addgene) using oligos:TOP2A1BF (SEQ ID NO: 94), TOP2A1BR (SEQ ID NO: 95), TOP2A1AF (SEQ ID NO: 96), TOP2A1AR (SEQ ID NO: 97), according to the suggested protocol (Ran et al., 2013), generating the CRISPR vectors pX335-Top2aA (SEQ ID NO: 98) and px335-Top2aB (SEQ ID NO: 99).

### Generation of EARC-modified Mouse ES cells

*Mouse ES cell culture:* All genetic manipulations were performed on a C57/B6 mouse ES cell line previously characterized (C2) (Gertsenstein et al., 2010). Mouse ES cells were grown in media based on high-glucose DMEM (Invitrogen), supplemented with 15% ES cell-grade FBS (Gibco), 0.1 mM 2-mEARCaptophenol, 2 mM L-glutamine, 1 mM sodium pyruvate, 0.1 mM non-essential amino acids, and 2,000 units/ml leukemia inhibitory factor (LIF). Cells were maintained at 37°C in 5% CO₂ on mitomycin C-treated mouse embryonic fibroblasts (MEFs).

*Targeting:* Plasmids containing the CRISPR/Cas9 components (pX335-Top2aA (SEQ ID NO: 98) and px335-Top2aB (SEQ ID NO: 99)) and the targeting plasmid (pBridge-Top2a (SEQ ID NO: 93)) were co-transfected in mouse ES cells using FuGENE HD (Clontech), according to the manufacturer's instructions, using a FuGENE:DNA ratio of 8:2, (2 µg total DNA: 250 ng for each pX335 and 1500 ng for pBridge-Top2a). Typical transfection was performed on 3x10⁵ cells, plated on 35 mm plates. Upon transfection, doxycycline was added to the media to a final concentration of 1 µg/ml. 2 days following transfection, cells were plated on a 100 mm plate and selection was applied with 1 µg/ml of puromycin. Puromycin-resistant colonies were picked 8-10 days after start of selection and maintained in 96 well plates until PCR-screening.

*Genotyping:* DNA was extracted from ES cells directly in 96 well plates according to (Nagy et al., 2003). Clones positive for correct insertion by homologous recombination of pBridge-Top2a in the 5' of the Top2a gene were screened by PCR using primers spanning the 5' and 3' homology arms (primers rttaRev (SEQ ID NO: 54), top2a_5scrF (SEQ ID NO: 55) for the 5' arm, primers CMVforw (SEQ ID NO: 56), top2a_3scrR (SEQ ID NO: 57) for the 3' arm).

*Targeted cell growth:* Top2a homozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluency.

### RESULTS

Referring to FIG. 18, the dox-bridge target vector, depicted in FIG. 18A, was used to generate several targeted C2 mouse ES cell lines (FIG. 18B). Nine of these cell lines were found to be homozygous targeted (FIG. 18B) comprising a dox-bridge inserted by homologous recombination into the 5'UTR of both alleles of Top2a.

As expected, this ES cell lines grows only in the presence of doxycycline. In the presence of doxycycline, the rtTA produced by *Top2a* promoter, binds to TRE and initiates transcription of the *Top2a* coding sequence. The dox-bridge may be inserted into the 5'UTR into both alleles of *Top2a* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 19A).

Referring to FIG. 19B, the inventors determined how different concentrations of doxycycline affected proliferation of the dox-bridge ES cells by measuring cell growth for 4 days. ES cells in the presence of doxycycline grew exponentially, indicating their normal growth. In contrast, two days after doxycycline removal, cells growth of EARC-modified cells was completely arrested.

### Example 5: Generation of EARC-Modified Mouse ES Cells in the Cenpa locus

In Example 5, construction of EARC (dox-bridge) vectors targeting Cenpa and use of same to control cell division in mouse ES cells is described. In this example, Cenpa/CENPA is the CDL, which is targeted with an inducible gene expression system, wherein a dox-bridge is inserted and doxycycline induces expression of the CDL.

As described above, Cenpa/CENPA is expressed in all mitotically active (i.e., dividing) cells. In cells modified to comprise an EARC (dox-bridge) insertion at the Cenpa locus, cell division is only possible in the presence of the inducer (doxycycline), which permits expression of Cenpa. Thus, cell division of EARC-modified mitotically active cells can be eliminated in the absence of doxycycline.

In Example 5, dox-bridge insertion into the 5'UTR of the *Cenpa* gene was achieved by homologous recombination knock-in technology.

### METHODS

### Construction of EARC Targeting Vector Comprising a Dox-bridge

The 5' homology arm (SEQ ID NO: 100) was cloned by PCR-amplifying a 874 bp fragment from C57/B6 genomic DNA (primers Cenpa5F (SEQ ID NO: 101) and Cenpa5R (SEQ ID NO: 102) and cloning it into Sbfl and Spel of the pGem-bridge-step3. Similarly, the 3' homology arm (825 bp; SEQ ID NO: 103) was amplified by PCR using primers Cenpa3F (SEQ ID NO: 104), Cenpa3R (SEQ ID NO: 105) and cloned into Sphl and Ncol to generate a final targeting vector, referred to as pBridge-Cenpa (SEQ ID NO: 106).

### Construction of CRISPR/Cas9 Plasmids

A double-nickase strategy was chosen to minimize the possibility of off-target mutations. Guide RNA sequences were cloned into pX335 (Addgene) using oligos CenpaAF (SEQ ID NO: 107), CenpaAR (SEQ ID NO: 108), CenpaBF (SEQ ID NO: 109), CenpaBR (SEQ ID NO: 110), according to the suggested protocol (Ran et al., 2013), generating the CRISPR vectors pX335-CenpaA (SEQ ID NO: 111) and px335-CenpaB (SEQ ID NO: 112).

### Generation of EARC-modified Mouse ES cells

### Mouse ES cell culture: As in Example 4.

*Targeting:* Plasmids containing the CRISPR/Cas9 components (pX335-CenpaA; SEQ ID NO: 111, and px335-CenpaB; SEQ ID NO: 112) and the targeting plasmid (pBridge-Cenpa; SEQ ID NO: 106) were co-transfected in mouse ES cells using FuGENE HD (Clontech), as in Example 4.

*Genotyping:* DNA was extracted as in Example 4. Clones positive for correct insertion by homologous recombination of pBridge-Cenpa in the 5' of the Cenpa gene were screened by PCR using primers spanning the 5' and 3' homology arms (primers rttaRev (SEQ ID NO: 54), Cenpa_5scr (SEQ ID NO: 113) for the 5' arm, primers CMVforw (SEQ ID NO: 114), Cenpa_3scr (SEQ ID NO: 115) for the 3' arm).

*Targeted cell growth:* Cenpa homozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluency.

*Quantitative PCR:* Total RNA was extracted from cells treated for 2 days with 1 µg/ml and 0 µg/ml of Dox using the GeneElute total RNA miniprep kit (Sigma) according to the manufacturer's protocol. cDNA was generated by reverse transcription of 1 µg of RNA using the QuantiTect reverse transcription kit (Qiagen), according to the manufacturer's protocol. Real-time qPCR were set up in a BioRad CFX thermocycler, using SensiFast-SYBR qPCR mix (Bioline). The primers used were: qpcrcenpa_F (SEQ ID NO: 116), qpcrcenpa_R (SEQ ID NO: 117) and actBf (SEQ ID NO: 67), actBr (SEQ ID NO: 68). Results were analyzed with the ΔΔCT method and normalized for beta-actin.

### RESULTS

Referring to FIG. 20, the dox-bridge target vector, depicted in FIG. 20A, was used to generate several targeted C2 mouse ES cell lines (FIG. 20B). Six of these cells were found to have a correct insertion at the 5' and 3', and at least one clone (Cenpa#4), was found to have homozygous targeting (FIG. 20B) comprising a dox-bridge inserted by homologous recombination into the 5'UTR of both alleles of Cenpa.

As expected, this ES cell lines grows only in the presence of doxycycline. In the presence of doxycycline, the rtTA produced by *Cenpa* promoter, binds to TRE and initiates transcription of the *Cenpa* coding sequence. The dox-bridge may be inserted into the 5'UTR into both alleles of *Cenpa,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 21A).

Referring to FIG. 21B, the inventors determined by qPCR the Cenpa gene expression level in Cenpa-EARC cells with Dox and after 2 days of Dox removal, and compared it to the expression level in wild type mouse ES cells (C2). As expected Cenpa expression level is greatly reduced in Cenpa-EARC cells without Dox for 2 days.

Referring to FIG. 22, the inventors determined how different concentrations of doxycycline affected proliferation of the dox-bridge ES cells by measuring cell growth for 4 days. ES cells in the presence of doxycycline grew exponentially, indicating their normal growth. In contrast, 80 hours after doxycycline removal, cells growth was completely arrested.

### Example 6: Generation of EARC-Modified Mouse ES Cells in the Birc5 locus

In Example 6, construction of EARC (dox-bridge) vectors targeting Birc5 and use of same to control cell division in mouse ES cells is described. In this example, Birc5/BIRC5 is the CDL, which is targeted with an inducible gene expression system, wherein a dox-bridge is inserted and doxycycline induces expression of the CDL.

As described above, Birc5/BIRC5 is expressed in all mitotically active (i.e., dividing) cells. In cells modified to comprise an EARC (dox-bridge) insertion at the Birc5 locus, cell division is only possible in the presence of the inducer (doxycycline), which permits expression of Birc5. Thus, cell division of EARC-modified mitotically active cells can be eliminated in the absence of doxycycline.

In Example 6, dox-bridge insertion into the 5'UTR of the *Birc5* gene was achieved by homologous recombination knock-in technology.

### METHODS

### Construction of EARC Targeting Vector Comprising a Dox-bridge

The 3' homology arm (SEQ ID NO: 118) was cloned by PCR-amplifying a 775 bp fragment from C57/B6 genomic DNA (primers Birc3F (SEQ ID NO: 119), Birc3R (SEQ ID NO: 120)), and cloning it into Sbfl and Ncol of the pGem-bridge-step3. Similarly, the 5' homology arm (617 bp; SEQ ID NO: 121) was amplified by PCR using primers Birc5F (SEQ ID NO: 122) and Birc5R Pstl (SEQ ID NO: 123) and Spel and cloned into to generate a final targeting vector, referred to as pBridge-Birc5 (SEQ ID NO: 124).

### Construction of CRISPR/Cas9 Plasmids

A double-nickase strategy was chosen to minimize the possibility of off-target mutations. Guide RNA sequences were cloned into pX335 (Addgene) using oligos Birc5AF (SEQ ID NO: 125), Birc5AR (SEQ ID NO: 126), Birc5BF (SEQ ID NO: 127), Birc5BR (SEQ ID NO: 128), according to the suggested protocol (Ran et al., 2013), generating the CRISPR vectors pX335-Birc5A (SEQ ID NO: 129) and px335-Birc5B (SEQ ID NO: 130).

### Generation of EARC-modified Mouse ES cells

*Mouse ES cell culture:* As in Example 4.

*Targeting:* Plasmids containing the CRISPR/Cas9 components (pX335-Birc5A and px335-Birc5B) and the targeting plasmid (pBridge-Birc5) were co-transfected in mouse ES cells using FuGENE HD (Clontech), as in Example 4.

*Genotyping:* DNA was extracted as in Example 4. Clones positive for correct insertion by homologous recombination of pBridge-Birc5 in the 5' of the Birc5 gene were screened by PCR using primers spanning the 5' homology arm (primers rttaRev (SEQ ID NO: 54), Birc_5scrF (SEQ ID NO: 131)).

*Targeted cell growth:* Birc5 homozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluence.

*Quantitative PCR:* Total RNA was extracted from cells treated for 2 days with 1 µg/ml and 0 µg/ml of Dox using the GeneElute total RNA miniprep kit (Sigma) according to the manufacturer's protocol. cDNA was generated by reverse transcription of 1 µg of RNA using the QuantiTect reverse transcription kit (Qiagen), according to the manufacturer's protocol. Real-time qPCR were set up in a BioRad CFX thermocycler, using SensiFast-SYBR qPCR mix (Bioline). The primers used were: qpcrbirc_F (SEQ ID NO: 132), qpcrbirc_R (SEQ ID NO: 133) and actBf (SEQ ID NO: 67), actBr (SEQ ID NO: 68). Results were analyzed with the ΔΔCT method and normalized for beta-actin.

### RESULTS

Referring to FIG. 23, the dox-bridge target vector, depicted in FIG. 23A, was used to generate targeted C2 mouse ES cell lines (FIG. 23B). Five clones were found to be correctly targeted (FIG. 23B) comprising a dox-bridge inserted by recombination into the 5'UTR of both alleles of Birc5. One of these clones was Birc#3, was found to stop growing or die in the absence of Dox.

As expected, this ES cell lines grows only in the presence of doxycycline. In the presence of doxycycline, the rtTA produced by *Birc5* promoter, binds to TRE and initiates transcription of the *Birc5* coding sequence. The dox-bridge may be inserted into the 5'UTR into both alleles of *Birc5,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 24A).

Referring to FIG. 24B, the inventors determined by qPCR the Birc5 gene expression level in Birc5-EARC cells with Dox and after 2 days of Dox removal, and compared it to the expression level in wild type mouse ES cells (C2). As expected Birc5 expression level is greatly reduced in Birc5-EARC cells without Dox for 2 days.

Referring to FIG. 25, the inventors determined how different concentrations of doxycycline affected proliferation of the dox-bridge ES cells by measuring cell growth for 4 days. ES cells in the presence of doxycycline grew exponentially, indicating their normal growth. In contrast, 50 hours after doxycycline removal, cells growth was completely arrested. Interestingly, it appears that lower Dox concentrations (0.5 and 0.05 µg/ml) promote better cell growth than a higher concentration (1 µg/ml).

### Example 7: Generation of EARC-Modified Mouse ES Cells in the Eef2 locus

In Example 7, construction of EARC (dox-bridge) vectors targeting Eef2 and use of same to control cell division in mouse ES cells is described. In this example, Eef2/EEF2 is the CDL, which is targeted with an inducible gene expression system, wherein a dox-bridge is inserted and doxycycline induces expression of the CDL.

As described above, Eef2/EEF2 is expressed in all mitotically active (i.e., dividing) cells. In cells modified to comprise an EARC (dox-bridge) insertion at the Eef2 locus, cell division is only possible in the presence of the inducer (doxycycline), which permits expression of Eef2. Thus, cell division of EARC-modified mitotically active cells can be eliminated in the absence of doxycycline.

In Example 7, dox-bridge insertion into the 5'UTR of the *Eef2* gene was achieved by homologous recombination knock-in technology.

### METHODS

### Construction of EARC Targeting Vector Comprising a Dox-bridge

The 5' homology arm was cloned by PCR-amplifying a 817 bp fragment(SEQ ID NO: 134) from C57/B6 genomic DNA (primers Eef2_5F (SEQ ID NO: 135) and Eef2_5R (SEQ ID NO: 136) and cloning it into Sbfl and Spel of the pGem-bridge-step3. Similarly, the 3' homology arm (826 bp; SEQ ID NO: 137) was amplified by PCR using primers Eef2_3F (SEQ ID NO: 138), Eef2_3R (SEQ ID NO: 139) and cloned into Sphl to generate a final targeting vector, referred to as pBridge-Eef2 (SEQ ID NO: 140).

### Construction of CRISPR/Cas9 Plasmids

A double-nickase strategy was chosen to minimize the possibility of off-target mutations. Guide RNA sequences were cloned into pX335 (Addgene) using oligos Eef2aFWD (SEQ ID NO: 141), Eef2aREV (SEQ ID NO: 142), Eef2bFWD (SEQ ID NO: 143), Eef2bREV (SEQ ID NO: 144), according to the suggested protocol (Ran et al., 2013), generating the CRISPR vectors pX335-Eef2A (SEQ ID NO: 145) and px335-Eef2B (SEQ ID NO: 146).

### Generation of EARC-modified Mouse ES cells

### Mouse ES cell culture: As in Example 4.

*Targeting:* Plasmids containing the CRISPR/Cas9 components (pX335-Eef2A and px335-Eef2B) and the targeting plasmid (pBridge-Eef2) were co-transfected in mouse ES cells using FuGENE HD (Clontech), as in Example 4.

*Genotyping:* DNA was extracted as in Example 4. Clones positive for correct insertion by homologous recombination of pBridge-Eef2 in the 5' of the Eef2 gene were screened by PCR using primers spanning the 5' homology arm (primers rttaRev (SEQ ID NO: 54), Eef2_5scrF (SEQ ID NO: 147)).

*Targeted cell growth:* Eef2 homozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluence.

### RESULTS

Referring to FIG. 26, the dox-bridge target vector, depicted in FIG. 26A, was used to generate several targeted C2 mouse ES cell lines (FIG. 26B). Nine of these cell lines was found to be correctly targeted (FIG. 26B) with at least one clone growing only in Dox-media.

As expected, this ES cell lines grows only in the presence of doxycycline. In the presence of doxycycline, the rtTA produced by *Eef2* promoter, binds to TRE and initiates transcription of the *Eef2* coding sequence. The dox-bridge may be inserted into the 5'UTR into both alleles of *Eef2,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 27).

Referring to FIG. 28, the inventors determined how different concentrations of doxycycline affected proliferation of the dox-bridge ES cells by measuring cell growth for 4 days. ES cells in the presence of doxycycline grew exponentially, indicating their normal growth. In contrast, without doxycycline cells completely failed to grow.

Although the disclosure has been described with reference to certain specific embodiments, various modifications thereof will be apparent to those skilled in the art. Any examples provided herein are included solely for the purpose of illustrating the disclosure and are not intended to limit the disclosure in any way. Any drawings provided herein are solely for the purpose of illustrating the disclosure and are not intended to be drawn to scale or to limit the disclosure in any way. The scope of the claims appended hereto should not be limited by the preferred embodiments set forth in the above description, but should be given the broadest interpretation consistent with the present specification as a whole.

Table 2: Predicted CDLs (ID refers to EntrezGene identification number; CS score refers to the CRISPR score average provided in Wang et al., 2015; function refers to the known or predicted function of the locus, predictions being based on GO terms, as set forth in the Gene Ontology Consortium website http://geneontology.org/; functional category refers to 4 categories of cell functions based on the GO term-predicted function; CDL (basis) refers to information that the inventors used to predict that a gene is a CDL, predictions being based on CS score, available gene knockout (KO) data, gene function, and experimental data provided herein) .

| Name (mouse) | ID (mouse) | Name (human) | ID (human) | CS score | Function (GO term) | Functional category | CDL (basis) | Citation |
|---|---|---|---|---|---|---|---|---|
| Actr8 | 56249 | ACTR8 | 93973 | -1.88 | chromatin remodeling | Cell cycle | CS score, function | |
| Alg11 | 207958 | ALG11 | 440138 | -1.27 | dolichol-linked oligosaccharide biosynthetic process | Cell cycle | CS score, function | |
| Anapc11 | 66156 | ANAPC1 1 | 51529 | -2.68 | protein ubiquitination involved in ubiquitin-dependent protein catabolic process | Cell cycle | CS score, function | |
| Anapc2 | 99152 | ANAPC2 | 29882 | -2.88 | mitotic cell cycle | Cell cycle | CS score, mouse K.O., function | Wirth KG, et al. Genes Dev. 2004 Jan 1;18(1):88-98 |
| Anapc4 | 52206 | ANAPC4 | 29945 | -1.79 | regulation of mitotic metaphase/anaphas e transition | Cell cycle | CS score, function | |
| Anapc5 | 59008 | ANAPC5 | 51433 | -1.66 | mitotic cell cycle | Cell cycle | CS score, function | |
| Aurka | 20878 | AURKA | 6790 | -2.26 | meiotic spindle organization | Cell cycle | CS score, mouse K.O., function | Sasai K, et al. Oncogene. 2008 Jul 3;27(29):4122-7 |
| Banf1 | 23825 | BANF1 | 8815 | -2.14 | mitotic cell cycle | Cell cycle | CS score, function | |
| Birc5 | 11799 | BIRC5 | 332 | -2.24 | regulation of signal transduction | Cell cycle | CS score, mouse K.O., function | Uren AG, et al. Curr Biol. 2000 Nov 2;10(21):1319-28 |
| Bub3 | 12237 | BUB3 | 9184 | -3.15 | mitotic sister chromatid segregation | Cell cycle | CS score, mouse K.O., function | Kalitsis P, et al. Genes Dev. 2000 Sep 15;14(18):2277-82 |
| Casc5 | 76464 | CASC5 | 57082 | -1.16 | mitotic cell cycle | Cell cycle | CS score, mouse K.O., function | Overbeek PA, et al. MGI Direct Data Submission. 2011 |
| Ccna2 | 12428 | CCNA2 | 890 | -1.59 | regulation of cyclindependent protein serine/threonine kinase activity | Cell cycle | CS score, mouse K.O., function | Kalaszczynska I, et al. Cell. 2009 Jul 23;138(2):352-65 |
| Ccnh | 66671 | CCNH | 902 | -2.01 | regulation of cyclindependent protein serine/threonine kinase activity | Cell cycle | CS score, function | |
| Cdc123 | 98828 | CDC123 | 8872 | -2.45 | cell cycle | Cell cycle | CS score, function | |
| Cdc16 | 69957 | CDC16 | 8881 | -3.58 | cell division | Cell cycle | CS score, function | |
| Cdc20 | 107995 | CDC20 | 991 | -2.97 | mitotic cell cycle | Cell cycle | CS score, mouse K.O., function | Li M, et al. Mol Cell Biol. 2007 May;27(9):3481-8 |
| Cdc23 | 52563 | CDC23 | 8697 | -2.28 | mitotic cell cycle | Cell cycle | CS score, function | |
| Cdk1 | 12534 | CDK1 | 983 | -2.44 | cell cycle | Cell cycle | CS score, mouse K.O., function | Diril MK, et al. Proc Natl Acad Sci U S A. 2012 Mar 6;109(10):3826-31 |
| Cenpa | 12615 | CENPA | 1058 | -1.87 | cell cycle | Cell cycle | CS score, mouse K.O., function | Howman EV, et al. Proc Natl Acad Sci U S A. 2000 Feb 1;97(3): 1148-53 |
| Cenpm | 66570 | CENPM | 79019 | -2.53 | mitotic cell cycle | Cell cycle | CS score, function | |
| Chek1 | 12649 | CHEK1 | 1111 | -1.67 | protein phosphorylation | Cell cycle | CS score, mouse K.O., function | Takai H, et al. Genes Dev. 2000 Jun 15;14(12):1439-47 |
| Chmp2a | 68953 | CHMP2A | 27243 | -2.40 | vacuolar transport | Cell cycle | CS score, function | |
| Ckap5 | 75786 | CKAP5 | 9793 | -2.94 | G2/M transition of mitotic cell cycle | Cell cycle | CS score, mouse K.O., function | Barbarese E, et al. PLoS One. 2013;8(8):e69989 |
| Cltc | 67300 | CLTC | 1213 | -1.75 | intracellular protein transport | Cell cycle | CS score, function | |
| Cops5 | 26754 | COPS5 | 10987 | -1.75 | protein deneddylation | Cell cycle | CS score, mouse K.O., function | Tian L, et al. Oncogene. 2010 Nov 18;29(46):6125-37 |
| Dctn2 | 69654 | DCTN2 | 10540 | -1.48 | G2/M transition of mitotic cell cycle | Cell cycle | CS score, function | |
| Dctn3 | 53598 | DCTN3 | 11258 | -1.77 | G2/M transition of mitotic cell cycle | Cell cycle | CS score, function | |
| Dhfr | 13361 | DHFR | 1719 | -2.84 | G1/S transition of mitotic cell cycle | Cell cycle | CS score, function | |
| Dtl | 76843 | DTL | 51514 | -2.69 | protein polyubiquitination | Cell cycle | CS score, mouse K.O., function | Liu CL, et al. J Biol Chem. 2007 Jan 12;282(2):1109-18 |
| Dync1 h1 | 13424 | DYNC1H 1 | 1778 | -3.44 | G2/M transition of mitotic cell cycle | Cell cycle | CS score, mouse K.O., function | Harada A, et al. J Cell Biol. 1998 Apr 6;141(1):51-9 |
| Ecd | 70601 | ECD | 11319 | -3.18 | regulation of glycolytic process | Cell cycle | CS score, function | |
| Ect2 | 13605 | ECT2 | 1894 | -1.80 | cell morphogenesis | Cell cycle | CS score, mouse K.O., function | Hansen J, et al. Proc Natl Acad Sci U S A. 2003 Aug 19;100(17):9918-22 |
| Ep300 | 328572 | EP300 | 2033 | -2.04 | G2/M transition of mitotic cell cycle | Cell cycle | CS score, mouse K.O., function | Yao TP, et al. Cell. 1998 May 1;93(3):361-72 |
| Ercc3 | 13872 | ERCC3 | 2071 | -2.10 | nucleotide-excision repair | Cell cycle | CS score, mouse K.O., function | Andressoo JO, et al. Mol Cell Biol. 2009 Mar;29(5):1276-90 |
| Espl1 | 105988 | ESPL1 | 9700 | -3.24 | proteolysis | Cell cycle | CS score, mouse K.O., function | Wirth KG, et al. J Cell Biol. 2006 Mar 13;172(6):847-60 |
| Fntb | 110606 | FNTB | 2342 | -2.42 | phototransduction, visible light | Cell cycle | CS score, mouse K.O., function | Mijimolle N, et al. Cancer Cell. 2005 Apr;7(4):313-24 |
| Gadd45gi p1 | 102060 | GADD45 GIP1 | 90480 | -1.81 | organelle organization | Cell cycle | CS score, mouse K.O., function | Kwon MC, et al. EMBO J. 2008 Feb 20;27(4):642-53 |
| Gins1 | 69270 | GINS1 | 9837 | -1.84 | mitotic cell cycle | Cell cycle | CS score, mouse K.O., function | Ueno M, et al. Mol Cell Biol. 2005 Dec;25(23):10528-32 |
| Gnb2l1 | 14694 | GNB2L1 | 10399 | -2.84 | osteoblast differentiation | Cell cycle | CS score, function | |
| Gspt1 | 14852 | GSPT1 | 2935 | -1.77 | G1/S transition of mitotic cell cycle | Cell cycle | CS score, function | |
| Haus1 | 225745 | HAUS1 | 115106 | -1.92 | spindle assembly | Cell cycle | CS score, function | |
| Haus3 | 231123 | HAUS3 | 79441 | -1.38 | mitotic nuclear division | Cell cycle | CS score, function | |
| Haus5 | 71909 | HAUS5 | 23354 | -2.55 | spindle assembly | Cell cycle | CS score, function | |
| Haus8 | 76478 | HAUS8 | 93323 | -1.73 | mitotic nuclear division | Cell cycle | CS score, function | |
| Hdac3 | 15183 | HDAC3 | 8841 | -2.12 | histone deacetylation | Cell cycle | CS score, mouse K.O., function | Bhaskara S, et al. Mol Cell. 2008 Apr 11;30(1):61-72 |
| Kif11 | 16551 | KIF11 | 3832 | -3.23 | microtubule-based movement | Cell cycle | CS score, mouse K.O., function | Castillo A, et al. Biochem Biophys Res Commun. 2007 Jun 8;357(3):694-9 |
| Kif23 | 71819 | KIF23 | 9493 | -1.59 | microtubule-based movement | Cell cycle | CS score, function | |
| Kpnb1 | 16211 | KPNB1 | 3837 | -3.19 | nucleocytoplasmic transport | Cell cycle | CS score, mouse K.O., function | Miura K, et al. Biochem Biophys Res Commun. 2006 Mar 3;341(1):132-8 |
| Mastl | 67121 | MASTL | 84930 | -2.36 | protein phosphorylation | Cell cycle | CS score, mouse K.O., function | Alvarez-Fernandez M, et al. Proc Natl Acad Sci U S A. 2013 Oct 22;110(43):17374-9 |
| Mau2 | 74549 | MAU2 | 23383 | -2.71 | mitotic cell cycle | Cell cycle | CS score, mouse K.O., function | Smith TG, et al. Genesis. 2014 Jul;52(7):687-94 |
| Mcm3 | 17215 | MCM3 | 4172 | -2.52 | G1/S transition of mitotic cell cycle | Cell cycle | CS score, function | |
| Mcm4 | 17217 | MCM4 | 4173 | -1.87 | G1/S transition of mitotic cell cycle | Cell cycle | CS score, mouse K.O., function | Shima N, et al. Nat Genet. 2007 Jan;39(1):93-8 |
| Mcm7 | 17220 | MCM7 | 4176 | -2.39 | G1/S transition of mitotic cell cycle | Cell cycle | CS score, function | |
| Mnat1 | 17420 | MNAT1 | 4331 | -1.22 | regulation of cyclindependent protein serine/threonine kinase activity | Cell cycle | CS score, mouse K.O., function | Rossi DJ, et al. EMBO J. 2001 Jun 1 ;20(11):2844-56 |
| Mybbp1a | 18432 | MYBBP1 A | 10514 | -2.17 | osteoblast differentiation | Cell cycle | CS score, mouse K.O., function | Mori S, et al. PLoS One. 2012;7(10):e39723 |
| Ncapd2 | 68298 | NCAPD2 | 9918 | -2.03 | mitotic chromosome condensation | Cell cycle | CS score, function | |
| Ncaph | 215387 | NCAPH | 23397 | -2.33 | mitotic chromosome condensation | Cell cycle | CS score, mouse K.O., function | Nishide K, et al. PLoS Genet. 2014 Dec;10(12):e10048 47 |
| Ndc80 | 67052 | NDC80 | 10403 | -2.98 | attachment of mitotic spindle microtubules to kinetochore | Cell cycle | CS score, function | |
| Nle1 | 217011 | NLE1 | 54475 | -1.88 | somitogenesis | Cell cycle | CS score, mouse K.O., function | Hentges KE, et al. Gene Expr Patterns. 2006 Aug;6(6):653-65 |
| Nsl1 | 381318 | NSL1 | 25936 | -1.90 | mitotic cell cycle | Cell cycle | CS score, function | |
| Nude | 18221 | NUDC | 10726 | -1.93 | mitotic cell cycle | Cell cycle | CS score, function | |
| Nuf2 | 66977 | NUF2 | 83540 | -1.78 | mitotic nuclear division | Cell cycle | CS score, function | |
| Nup133 | 234865 | NUP133 | 55746 | -2.26 | mitotic cell cycle | Cell cycle | CS score, mouse K.O., function | Garcia-Garcia MJ, et al. Proc Natl Acad Sci U S A. 2005 Apr 26;102(17):5913-9 |
| Nup160 | 59015 | NUP160 | 23279 | -2.64 | mitotic cell cycle | Cell cycle | CS score, function | |
| Nup188 | 227699 | NUP188 | 23511 | -1.16 | mitotic cell cycle | Cell cycle | CS score, function | |
| Nup214 | 227720 | NUP214 | 8021 | -2.70 | mitotic cell cycle | Cell cycle | CS score, mouse K.O., function | van Deursen J, et al. EMBO J. 1996 Oct 15;15(20):5574-83 |
| n/a | n/a | NUP62 | 23636 | -2.35 | mitotic cell cycle | Cell cycle | CS score, function | |
| Nup85 | 445007 | NUP85 | 79902 | -2.47 | mitotic cell cycle | Cell cycle | CS score, function | |
| Orc3 | 50793 | ORC3 | 23595 | -1.67 | G1/S transition of mitotic cell cycle | Cell cycle | CS score, function | |
| Pafah1b1 | 18472 | PAFAH1 B1 | 5048 | -2.34 | G2/M transition of mitotic cell cycle | Cell cycle | CS score, mouse K.O., function | Cahana A, et al. Proc Natl Acad Sci U S A. 2001 May 22;98(11):6429-34 |
| Pcid2 | 234069 | PCID2 | 55795 | -1.98 | negative regulation of apoptotic process | Cell cycle | CS score, function | |
| Pfas | 237823 | PFAS | 5198 | -2.58 | purine nucleotide biosynthetic process | Cell cycle | CS score, function | |
| Phb2 | 12034 | PHB2 | 11331 | -2.98 | protein import into nucleus, translocation | Cell cycle | CS score, mouse K.O., function | Park SE, et al. Mol Cell Biol. 2005 Mar;25(5):1989-99 |
| Pkmyt1 | 268930 | PKMYT1 | 9088 | -1.93 | regulation of cyclindependent protein serine/threonine kinase activity | Cell cycle | CS score, function | |
| Plk1 | 18817 | PLK1 | 5347 | -2.83 | protein phosphorylation | Cell cycle | CS score, mouse K.O., function | Lu LY, et al. Mol Cell Biol. 2008 Nov;28(22):6870-6 |
| Pmf1 | 67037 | PMF1 | 11243 | -2.15 | mitotic cell cycle | Cell cycle | CS score, function | |
| Pole2 | 18974 | POLE2 | 5427 | -3.08 | G1/S transition of mitotic cell cycle | Cell cycle | CS score, function | |
| Ppat | 231327 | PPAT | 5471 | -2.15 | G1/S transition of mitotic cell cycle | Cell cycle | CS score, function | |
| Psma6 | 26443 | PSMA6 | 5687 | -3.51 | G1/S transition of mitotic cell cycle | Cell cycle | CS score, function | |
| Psma7 | 26444 | PSMA7 | 5688 | -2.91 | G1/S transition of mitotic cell cycle | Cell cycle | CS score, function | |
| Psmb1 | 19170 | PSMB1 | 5689 | -1.63 | G1/S transition of mitotic cell cycle | Cell cycle | CS score, function | |
| Psmb4 | 19172 | PSMB4 | 5692 | -2.91 | G1/S transition of mitotic cell cycle | Cell cycle | CS score, function | |
| Psmd12 | 66997 | PSMD12 | 5718 | -1.69 | G1/S transition of mitotic cell cycle | Cell cycle | CS score, function | |
| Psmd13 | 23997 | PSMD13 | 5719 | -1.57 | G1/S transition of mitotic cell cycle | Cell cycle | CS score, function | |
| Psmd14 | 59029 | PSMD14 | 10213 | -3.01 | G1/S transition of mitotic cell cycle | Cell cycle | CS score, function | |
| Psmd7 | 17463 | PSMD7 | 5713 | -2.18 | G1/S transition of mitotic cell cycle | Cell cycle | CS score, mouse K.O., function | Soriano P, et al. Genes Dev. 1987 Jun;1 (4):366-75 |
| Racgap1 | 26934 | RACGAP 1 | 29127 | -1.94 | mitotic spindle assembly | Cell cycle | CS score, mouse K.O., function | Van de Putte T, et al. Mech Dev. 2001 Apr;102(1-2):33-44 |
| Rad21 | 19357 | RAD21 | 5885 | -2.12 | mitotic cell cycle | Cell cycle | CS score, function | |
| Rae1 | 66679 | RAE1 | 8480 | -2.15 | mitotic cell cycle | Cell cycle | CS score, mouse K.O., function | Babu JR, et al. J Cell Biol. 2003 Feb 3;160(3):341-53 |
| Rcc1 | 100088 | RCC1 | 1104 | -2.91 | G1/S transition of mitotic cell cycle | Cell cycle | CS score, function | |
| Rfc3 | 69263 | RFC3 | 5983 | -2.74 | mitotic cell cycle | Cell cycle | CS score, function | |
| Rps27a | 78294 | RPS27A | 6233 | -2.74 | G1/S transition of mitotic cell cycle | Cell cycle | CS score, function | |
| Rrm2 | 20135 | RRM2 | 6241 | -3.09 | G1/S transition of mitotic cell cycle | Cell cycle | CS score, function | |
| Sae1 | 56459 | SAE1 | 10055 | -2.08 | cellular protein modification process | Cell cycle | CS score, function | |
| Sec13 | 110379 | SEC13 | 6396 | -2.96 | mitotic cell cycle | Cell cycle | CS score, function | |
| Smarcb1 | 20587 | SMARCB 1 | 6598 | -1.98 | chromatin remodeling | Cell cycle | CS score, mouse K.O., function | Guidi CJ, et al. Mol Cell Biol. 2001 May 15;21(10):3598-603 |
| Smc2 | 14211 | SMC2 | 10592 | -2.13 | mitotic chromosome condensation | Cell cycle | CS score, mouse K.O., function | Nishide K, et al. PLoS Genet. 2014 Dec;10(12):e10048 47 |
| Smc4 | 70099 | SMC4 | 10051 | -1.47 | chromosome organization | Cell cycle | CS score, function | |
| Son | 20658 | SON | 6651 | -1.99 | microtubule cytoskeleton organization | Cell cycle | CS score, function | |
| Spc24 | 67629 | SPC24 | 147841 | -2.83 | mitotic cell cycle | Cell cycle | CS score, function | |
| Spc25 | 66442 | SPC25 | 57405 | -1.63 | mitotic cell cycle | Cell cycle | CS score, function | |
| Terf2 | 21750 | TERF2 | 7014 | -2.17 | telomere maintenance | Cell cycle | CS score, mouse K.O., function | Celli GB, et al. Nat Cell Biol. 2005 Jul;7(7):712-8 |
| Tpx2 | 72119 | TPX2 | 22974 | -2.08 | apoptotic process | Cell cycle | CS score, mouse K.O., function | Aguirre-Portoles C, et al. Cancer Res. 2012 Mar 15;72(6):1518-28 |
| Tubg1 | 103733 | TUBG1 | 7283 | -2.08 | microtubule nucleation | Cell cycle | CS score, mouse K.O., function | Yuba-Kubo A, et al. Dev Biol. 2005 Jun 15;282(2):361-73 |
| Tubgcp2 | 74237 | TUBGCP 2 | 10844 | -2.78 | microtubule cytoskeleton organization | Cell cycle | CS score, function | |
| Tubgcp5 | 233276 | TUBGCP 5 | 114791 | -1.76 | microtubule cytoskeleton organization | Cell cycle | CS score, function | |
| Tubgcp6 | 328580 | TUBGCP 6 | 85378 | -1.52 | microtubule cytoskeleton organization | Cell cycle | CS score, function | |
| Txnl4a | 27366 | TXNL4A | 10907 | -3.89 | mitotic nuclear division | Cell cycle | CS score, function | |
| Usp39 | 28035 | USP39 | 10713 | -2.85 | spliceosomal complex assembly | Cell cycle | CS score, function | |
| Wdr43 | 72515 | WDR43 | 23160 | -3.02 | reproduction | Cell cycle | CS score, function | |
| Zfp830 | 66983 | ZNF830 | 91603 | -1.52 | blastocyst growth | Cell cycle | CS score, mouse K.O., function | Houlard M, et al. Cell Cycle. 2011 Jan 1;10(1):108-17 |
| Aatf | 56321 | AATF | 26574 | -1.46 | cellular response to DNA damage stimulus | DNA replication , DNA repair | CS score, mouse K.O., function | Thomas T, et al. Dev Biol. 2000 Nov 15;227(2):324-42 |
| Alyref | 21681 | ALYREF | 10189 | -1.92 | regulation of DNA recombination | DNA replication , DNA repair | CS score, function | |
| Brf2 | 66653 | BRF2 | 55290 | -2.30 | DNA-templated transcription, initiation | DNA replication , DNA repair | CS score, function | |
| Cdc45 | 12544 | CDC45 | 8318 | -3.69 | DNA replication checkpoint | DNA replication , DNA repair | CS score, mouse K.O., function | Yoshida K, et al. Mol Cell Biol. 2001 Jul;21 (14):4598-603 |
| Cdc6 | 23834 | CDC6 | 990 | -1.87 | DNA replication initiation | DNA replication , DNA repair | CS score, function | |
| Cdt1 | 67177 | CDT1 | 81620 | -2.74 | DNA replication checkpoint | DNA replication, DNA repair | CS score, function | |
| Cinp | 67236 | CINP | 51550 | -1.64 | DNA replication | DNA replication, DNA repair | CS score, function | |
| Cirh1a | 21771 | CIRH1A | 84916 | -2.62 | transcription, DNA-templated | DNA replication, DNA repair | CS score, function | |
| Ddb1 | 13194 | DDB1 | 1642 | -2.14 | nucleotide-excision repair, DNA damage removal | DNA replication, DNA repair | CS score, mouse K.O., function | Cang Y, et al. Cell. 2006 Dec 1;127(5):929-40 |
| Ercc2 | 13871 | ERCC2 | 2068 | -2.80 | DNA duplex unwinding | DNA replication, DNA repair | CS score, mouse K.O., function | de Boer J, et al. Cancer Res. 1998 Jan 1;58(1):89-94 |
| Gabpb1 | 14391 | GABPB1 | 2553 | -1.74 | transcription, DNA-templated | DNA replication, DNA repair | CS score, mouse K.O., function | Xue HH, et al. Mol Cell Biol. 2008 Jul;28(13):4300-9 |
| Gtf2b | 229906 | GTF2B | 2959 | -2.76 | regulation of transcription, DNA-templated | DNA replication, DNA repair | CS score, function | |
| Gtf2h4 | 14885 | GTF2H4 | 2968 | -1.93 | nucleotide-excision repair, DNA damage removal | DNA replication, DNA repair | CS score, function | |
| Gtf3a | 66596 | GTF3A | 2971 | -2.25 | regulation of transcription, DNA-templated | DNA replication, DNA repair | CS score, function | |
| Gtf3c1 | 233863 | GTF3C1 | 2975 | -2.45 | transcription, DNA-templated | DNA replication, DNA repair | CS score, function | |
| Gtf3c2 | 71752 | GTF3C2 | 2976 | -2.09 | transcription, DNA-templated | DNA replication, DNA repair | CS score, function | |
| Hinfp | 102423 | HINFP | 25988 | -2.35 | DNA damage checkpoint | DNA replication, DNA repair | CS score, mouse K.O., function | Xie R, et al. Proc Natl Acad Sci U S A. 2009 Jul 9 |
| n/a | n/a | HIST2H2 AA3 | 8337 | -1.71 | DNA repair | DNA replication, DNA repair | CS score, function | |
| Ints3 | 229543 | INTS3 | 65123 | -3.14 | DNA repair | DNA replication, DNA repair | CS score, function | |
| Kin | 16588 | KIN | 22944 | -1.99 | DNA replication | DNA replication, DNA repair | CS score, function | |
| Mcm2 | 17216 | MCM2 | 4171 | -2.86 | DNA replication initiation | DNA replication, DNA repair | CS score, function | |
| Mcm6 | 17219 | MCM6 | 4175 | -1.55 | DNA replication | DNA replication, DNA repair | CS score, function | |
| Mcrs1 | 51812 | MCRS1 | 10445 | -1.23 | DNA repair | DNA replication, DNA repair | CS score, function | |
| Med11 | 66172 | MED11 | 400569 | -2.39 | transcription, DNA-templated | DNA replication, DNA repair | CS score, function | |
| Mtpap | 67440 | MTPAP | 55149 | -1.86 | transcription, DNA-templated | DNA replication, DNA repair | CS score, function | |
| Myc | 17869 | MYC | 4609 | -2.49 | regulation of transcription, DNA-templated | DNA replication, DNA repair | CS score, mouse K.O., function | Trumpp A, et al. Nature. 2001 Dec 13;414(6865):768-73 |
| Ndnl2 | 66647 | NDNL2 | 56160 | -2.03 | DNA repair | DNA replication, DNA repair | CS score, function | |
| Nol11 | 68979 | NOL11 | 25926 | -1.59 | transcription, DNA-templated | DNA replication, DNA repair | CS score, function | |
| Nol8 | 70930 | NOL8 | 55035 | -1.35 | DNA replication | DNA replication, DNA repair | CS score, function | |
| Pcna | 18538 | PCNA | 5111 | -3.60 | DNA replication | DNA replication, DNA repair | CS score, mouse K.O., function | Roa S, et al. Proc Natl Acad Sci U S A. 2008 Oct 21;105(42):16248-53 |
| Pola1 | 18968 | POLA1 | 5422 | -2.28 | DNA-dependent DNA replication | DNA replication, DNA repair | CS score, function | |
| Pold2 | 18972 | POLD2 | 5425 | -2.51 | DNA replication | DNA replication, DNA repair | CS score, function | |
| Pole | 18973 | POLE | 5426 | -2.90 | DNA replication | DNA replication, DNA repair | CS score, function | |
| Polr1a | 20019 | POLR1A | 25885 | -2.62 | transcription, DNA-templated | DNA replication, DNA repair | CS score, function | |
| n/a | n/a | POLR2J2 | 246721 | -3.08 | transcription, DNA-templated | DNA replication, DNA repair | CS score, function | |
| Polr3a | 218832 | POLR3A | 11128 | -2.43 | transcription, DNA-templated | DNA replication, DNA repair | CS score, function | |
| Polr3c | 74414 | POLR3C | 10623 | -2.02 | transcription, DNA-templated | DNA replication, DNA repair | CS score, function | |
| Polr3h | 78929 | POLR3H | 171568 | -2.66 | transcription, DNA-templated | DNA replication, DNA repair | CS score, function | |
| Prmt1 | 15469 | PRMT1 | 3276 | -2.40 | regulation of transcription, DNA-templated | DNA replication, DNA repair | CS score, mouse K.O., function | Pawlak MR, et al. Mol Cell Biol. 2000 Jul;20(13):4859-69 |
| Prmt5 | 27374 | PRMT5 | 10419 | -2.69 | regulation of transcription, DNA-templated | DNA replication, DNA repair | CS score, mouse K.O., function | Tee WW, et al. Genes Dev. 2010 Dec 15;24(24):2772-7 |
| Puf60 | 67959 | PUF60 | 22827 | -2.69 | transcription, DNA-templated | DNA replication, DNA repair | CS score, function | |
| Rad51 | 19361 | RAD51 | 5888 | -2.29 | DNA repair | DNA replication, DNA repair | CS score, mouse K.O., function | Tsuzuki T, et al. Proc Natl Acad Sci U S A. 1996 Jun 25;93(13):6236-40 |
| Rad51c | 114714 | RAD51C | 5889 | -1.62 | DNA repair | DNA replication, DNA repair | CS score, mouse K.O., function | Smeenk G, et al. Mutat Res. 2010 Jul 7;689(1-2):50-58 |
| Rbx1 | 56438 | RBX1 | 9978 | -2.19 | DNA repair | DNA replication, DNA repair | CS score, mouse K.O., function | Tan M, et al. Proc Natl Acad Sci U S A. 2009 Apr 14;106(15):6203-8 |
| Rfc2 | 19718 | RFC2 | 5982 | -2.88 | DNA-dependent DNA replication | DNA replication, DNA repair | CS score, function | |
| Rfc4 | 106344 | RFC4 | 5984 | -1.92 | DNA-dependent DNA replication | DNA replication, DNA repair | CS score, function | |
| Rfc5 | 72151 | RFC5 | 5985 | -2.78 | DNA-dependent DNA replication | DNA replication, DNA repair | CS score, function | |
| Rpa1 | 68275 | RPA1 | 6117 | -2.61 | DNA replication | DNA replication, DNA repair | CS score, mouse K.O., function | Wang Y, et al. Nat Genet. 2005 Jul;37(7):750-5 |
| Rps3 | 27050 | RPS3 | 6188 | -2.75 | DNA repair | DNA replication, DNA repair | CS score, function | |
| Rrm1 | 20133 | RRM1 | 6240 | -4.16 | DNA replication | DNA replication, DNA repair | CS score, function | |
| Ruvbl1 | 56505 | RUVBL1 | 8607 | -3.26 | DNA duplex unwinding | DNA replication, DNA repair | CS score, function | |
| Ruvbl2 | 20174 | RUVBL2 | 10856 | -3.91 | DNA repair | DNA replication, DNA repair | CS score, function | |
| Sap30bp | 57230 | SAP30BP | 29115 | -2.18 | regulation of transcription, DNA-templated | DNA replication, DNA repair | CS score, function | |
| Smc1a | 24061 | SMC1A | 8243 | -2.76 | DNA repair | DNA replication, DNA repair | CS score, function | |
| Smc3 | 13006 | SMC3 | 9126 | -3.22 | DNA repair | DNA replication, DNA repair | CS score, mouse K.O., function | White JK, et al. Cell. 2013 Jul 18;154(2):452-64 |
| Snapc4 | 227644 | SNAPC4 | 6621 | -2.78 | regulation of transcription, DNA-templated | DNA replication, DNA repair | CS score, function | |
| Snapc5 | 330959 | SNAPC5 | 10302 | -2.24 | regulation of transcription, DNA-templated | DNA replication, DNA repair | CS score, function | |
| Snip1 | 76793 | SNIP1 | 79753 | -1.78 | regulation of transcription, DNA-templated | DNA replication, DNA repair | CS score, function | |
| Srrt | 83701 | SRRT | 51593 | -2.18 | transcription, DNA-templated | DNA replication, DNA repair | CS score, mouse K.O., function | Wilson MD, et al. Mol Cell Biol. 2008 Mar;28(5):1503-14 |
| Ssrp1 | 20833 | SSRP1 | 6749 | -1.45 | DNA replication | DNA replication, DNA repair | CS score, mouse K.O., function | Cao S, et al.5 mouse embryos. Mol Cell Biol. 2003 Aug ;23(15):5301-7 |
| Taf10 | 24075 | TAF10 | 6881 | -1.38 | DNA-templated transcription, initiation | DNA replication, DNA repair | CS score, mouse K.O., function | Mohan WS Jr, et al. Mol Cell Biol. 2003 Jun;23(12):4307-18 |
| Taf1c | 21341 | TAF1C | 9013 | -1.80 | chromatin silencing at rDNA | DNA replication, DNA repair | CS score, function | |
| Taf6 | 21343 | TAF6 | 6878 | -1.84 | DNA-templated transcription, initiation | DNA replication, DNA repair | CS score, function | |
| Taf6l | 67706 | TAF6L | 10629 | -1.53 | DNA-templated transcription, initiation | DNA replication, DNA repair | CS score, function | |
| Ticrr | 77011 | TICRR | 90381 | -2.03 | DNA replication | DNA replication, DNA repair | CS score, function | |
| Top1 | 21969 | TOP1 | 7150 | -2.02 | DNA topological change | DNA replication, DNA repair | CS score, mouse K.O., function | Morham SG, et al. Mol Cell Biol. 1996 Dec;16(12):6804-9 |
| Top2a | 21973 | TOP2A | 7153 | -1.50 | DNA replication | DNA replication, DNA repair | CS score, function | |
| Trrap | 100683 | TRRAP | 8295 | -2.36 | DNA repair | DNA replication, DNA repair | CS score, mouse K.O., function | Herceg Z, et al. Nat Genet. 2001 Oct;29(2):206-11 |
| Zbtb11 | 271377 | ZBTB11 | 27107 | -2.34 | transcription, DNA-templated | DNA replication, DNA repair | CS score, function | |
| Actl6a | 56456 | ACTL6A | 86 | -2.33 | neural retina development | DNA replication, DNA repair | CS score, mouse K.O., function | Krasteva V, et al. Blood. 2012 Dec 6;120(24):4720-32 |
| Atr | 245000 | ATR | 545 | -2.01 | double-strand break repair via homologous recombination | DNA replication, DNA repair | CS score, mouse K.O., function | de Klein A, et al. Curr Biol. 2000 Apr 20;10(8):479-82 |
| Chd4 | 107932 | CHD4 | 1108 | -1.71 | chromatin organization | DNA replication, DNA repair | CS score, function | |
| Ciao1 | 26371 | ClAO1 | 9391 | -1.94 | chromosome segregation | DNA replication, DNA repair | CS score, function | |
| Ddx21 | 56200 | DDX21 | 9188 | -2.84 | osteoblast differentiation | DNA replication, DNA repair | CS score, function | |
| Dnaja3 | 83945 | DNAJA3 | 9093 | -2.19 | mitochondrion organization | DNA replication, DNA repair | CS score, mouse K.O., function | Lo JF, et al. Mol Cell Biol. 2004 Mar;24(6):2226-36 |
| Dnmt1 | 13433 | DNMT1 | 1786 | -1.97 | methylation | DNA replication, DNA repair | CS score, mouse K.O., function | Lei H, et al. Development. 1996 Oct;122(10):3195-205 |
| Gins2 | 272551 | GINS2 | 51659 | -3.32 | double-strand break repair via break-induced replication | DNA replication, DNA repair | CS score, function | |
| Gtf2h3 | 209357 | GTF2H3 | 2967 | -1.84 | nucleotide-excision repair | DNA replication, DNA repair | CS score, function | |
| n/a | n/a | HIST2H2 BF | 440689 | -1.70 | chromatin organization | DNA replication, DNA repair | CS score, function | |
| Mms22l | 212377 | MMS22L | 253714 | -1.38 | double-strand break repair via homologous recombination | DNA replication, DNA repair | CS score, function | |
| Mtor | 56717 | MTOR | 2475 | -1.98 | double-strand break repair via homologous recombination | DNA replication, DNA repair | CS score, mouse K.O., function | Murakami M, et al. Mol Cell Biol. 2004 Aug;24(15):6710-8 |
| Narfl | 67563 | NARFL | 64428 | -2.13 | response to hypoxia | DNA replication, DNA repair | CS score, mouse K.O., function | Song D, et al. J Biol Chem. 2011 Mar 2 |
| Ndufa13 | 67184 | NDUFA1 3 | 51079 | -1.31 | positive regulation of peptidase activity | DNA replication, DNA repair | CS score, mouse K.O., function | Huang G, et al. Mol Cell Biol. 2004 Oct;24(19):8447-56 |
| Nol12 | 97961 | NOL12 | 79159 | -1.61 | poly(A) RNA binding | DNA replication, DNA repair | CS score, function | |
| Nup107 | 103468 | NUP107 | 57122 | -1.30 | transport | DNA replication, DNA repair | CS score, function | |
| Oraov1 | 72284 | ORAOV1 | 220064 | -2.26 | biological process | DNA replication, DNA repair | CS score, function | |
| Pam16 | 66449 | PAM16 | 51025 | -2.13 | protein import into mitochondrial matrix | DNA replication, DNA repair | CS score, function | |
| Pola2 | 18969 | POLA2 | 23649 | -2.84 | protein import into nucleus, translocation | DNA replication, DNA repair | CS score, function | |
| Ppie | 56031 | PPIE | 10450 | -1.63 | protein peptidyl-prolyl isomerization | DNA replication, DNA repair | CS score, function | |
| Prpf19 | 28000 | PRPF19 | 27339 | -3.96 | generation of catalytic spliceosome for first transesterification step | DNA replication, DNA repair | CS score, mouse K.O., function | Fortschegger K, et al. Mol Cell Biol. 2007 Apr;27(8):3123-30 |
| Psmc5 | 19184 | PSMC5 | 5705 | -2.57 | ER-associated ubiquitin-dependent protein catabolic process | DNA replication, DNA repair | CS score, function | |
| Rbbp5 | 213464 | RBBP5 | 5929 | -1.70 | chromatin organization | DNA replication, DNA repair | CS score, function | |
| Rbbp6 | 19647 | RBBP6 | 5930 | -1.78 | in utero embryonic development | DNA replication, DNA repair | CS score, mouse K.O., function | Li L, et al. Proc Natl Acad Sci U S A. 2007 May 8;104(19):7951-6 |
| Rptor | 74370 | RPTOR | 57521 | -2.43 | TOR signaling | DNA replication, DNA repair | CS score, mouse K.O., function | Guertin DA, et al. Dev Cell. 2006 Dec;11(6):859-71 |
| Rrn3 | 106298 | RRN3 | 54700 | -1.85 | in utero embryonic development | DNA replication, DNA repair | CS score, mouse K.O., function | Yuan X, et al. Mol Cell. 2005 Jul 1:19(1):77-87 |
| Smg1 | 233789 | SMG1 | 23049 | -1.94 | double-strand break repair via homologous recombination | DNA replication, DNA repair | CS score, mouse K.O., function | Roberts TL, et al. Proc Natl Acad Sci U S A. 2013 Jan 22;110(4):E285-94 |
| Supt6 | 20926 | SUPT6H | 6830 | -1.78 | chromatin remodeling | DNA replication, DNA repair | CS score, mouse K.O., function | Dietrich JE, et al. EMBO Rep. 2015 Aug;16(8):1005-21 |
| Tada2b | 231151 | TADA2B | 93624 | -1.23 | chromatin organization | DNA replication, DNA repair | CS score, function | |
| Tfip11 | 54723 | TFIP11 | 24144 | -2.19 | spliceosomal complex disassembly | DNA replication, DNA repair | CS score, function | |
| Tonsl | 66914 | TONSL | 4796 | -3.03 | double-strand break repair via homologous recombination | DNA replication, DNA repair | CS score, function | |
| Tpt1 | 22070 | TPT1 | 7178 | -2.05 | calcium ion transport | DNA replication, DNA repair | CS score, mouse K.O., function | Susini L, et al. Cell Death Differ. 2008 Auc;15(8):1211-20 |
| Uba1 | 22201 | UBA1 | 7317 | -2.90 | protein ubiquitination | DNA replication, DNA repair | CS score, function | |
| Vps25 | 28084 | VPS25 | 84313 | -2.31 | protein targeting to vacuole involved in ubiquitin-dependent protein catabolic process via the multivesicular body sorting pathway | DNA replication, DNA repair | CS score, function | |
| Wbscr22 | 66138 | WBSCR2 2 | 114049 | -2.70 | methylation | DNA replication, DNA repair | CS score, function | |
| Wdr5 | 140858 | WDR5 | 11091 | -1.99 | skeletal system development | DNA replication, DNA repair | CS score, function | |
| Xab2 | 67439 | XAB2 | 56949 | -2.86 | generation of catalytic spliceosome for first transesterification step | DNA replication, DNA repair | CS score, mouse K.O., function | Yonemasu R, et al. DNA Repair (Amst). 2005 Apr 4;4(4):479-91 |
| Zmat2 | 66492 | ZMAT2 | 153527 | -2.17 | histidine-tRNA ligase activity | DNA replication, DNA repair | CS score, function | |
| Zfp335 | 329559 | ZNF335 | 63925 | -1.58 | in utero embryonic development | DNA replication, DNA repair | CS score, mouse K.O., function | Yang YJ, et al. Cell. 2012 Nov 21;151(5):1097-112 |
| Acly | 104112 | ACLY | 47 | -1.54 | acetyl-CoA metabolic process | Metabolism | CS score, mouse K.O., function | Beigneux AP, et al. J Biol Chem. 2004 Mar 5;279(10):9557-64 |
| Adsl | 11564 | ADSL | 158 | -2.39 | metabolic process | Metabolism | CS score, function | |
| Ahcy | 269378 | AHCY | 191 | -2.07 | sulfur amino acid metabolic process | Metabolism | CS score, function | |
| Arl2 | 56327 | ARL2 | 402 | -2.29 | energy reserve metabolic process | Metabolism | CS score, function | |
| Chka | 12660 | CHKA | 1119 | -1.64 | lipid metabolic process | Metabolism | CS score, mouse K.O., function | Wu G, et al. J Biol Chem. 2008 Jan 18;283(3):1456-62 |
| Coasy | 71743 | COASY | 80347 | -1.82 | vitamin metabolic process | Metabolism | CS score, function | |
| Cox4i1 | 12857 | COX4l1 | 1327 | -2.00 | generation of precursor metabolites and energy | Metabolism | CS score, function | |
| n/a | n/a | COX7C | 1350 | -1.59 | generation of precursor metabolites and energy | Metabolism | CS score, function | |
| n/a | n/a | CTPS1 | 1503 | -2.52 | nucleobase-containing compound metabolic process | Metabolism | CS score, function | |
| Ddx10 | 77591 | DDX10 | 1662 | -2.02 | metabolic process | Metabolism | CS score, function | |
| Ddx20 | 53975 | DDX20 | 11218 | -2.49 | metabolic process | Metabolism | CS score, mouse K.O., function | Mouillet JF, et al. Endocrinology. 2008 May;149(5):2168-75 |
| Dhdds | 67422 | DHDDS | 79947 | -2.86 | metabolic process | Metabolism | CS score, function | |
| Dhx30 | 72831 | DHX30 | 22907 | -1.93 | metabolic process | Metabolism | CS score, function | |
| Dhx8 | 217207 | DHX8 | 1659 | -2.61 | metabolic process | Metabolism | CS score, function | |
| Dhx9 | 13211 | DHX9 | 1660 | -1.73 | metabolic process | Metabolism | CS score, mouse K.O., function | Lee CG, et al. Proc Natl Acad Sci U S A. 1998 Nov 10;95(23):13709-13 |
| Dlst | 78920 | DLST | 1743 | -1.93 | metabolic process | Metabolism | CS score, function | |
| Dpagt1 | 13478 | DPAGT1 | 1798 | -2.80 | UDP-N-acetylglucosamine metabolic process | Metabolism | CS score, mouse K.O., function | Marek KW, et al. Glycobiology. 1999 Nov;9(11):1263-71 |
| Gfpt1 | 14583 | GFPT1 | 2673 | -1.81 | fructose 6-phosphate metabolic process | Metabolism | CS score, function | |
| Gmps | 229363 | GMPS | 8833 | -1.80 | purine nucleobase metabolic process | Metabolism | CS score, function | |
| Gpn1 | 74254 | GPN1 | 11321 | -1.79 | metabolic process | Metabolism | CS score, function | |
| Gpn3 | 68080 | GPN3 | 51184 | -3.12 | metabolic process | Metabolism | CS score, function | |
| Guk1 | 14923 | GUK1 | 2987 | -2.67 | purine nucleotide metabolic process | Metabolism | CS score, function | |
| Hsd17b10 | 15108 | HSD17B1 0 | 3028 | -1.84 | lipid metabolic process | Metabolism | CS score, function | |
| Lrr1 | 69706 | LRR1 | 122769 | -3.44 | metabolic process | Metabolism | CS score, function | |
| Mtg2 | 52856 | MTG2 | 26164 | -2.04 | metabolic process | Metabolism | CS score, function | |
| Myh9 | 17886 | MYH9 | 4627 | -1.70 | metabolic process | Metabolism | CS score, mouse K.O., function | Matsushita T, et al. Biochem Biophys Res Commun. 2004 Dec 24;325(4):1163-71 |
| Nampt | 59027 | NAMPT | 10135 | -2.40 | vitamin metabolic process | Metabolism | CS score, mouse K.O., function | Revollo JR, et al. Cell Metab. 2007 Nov;6(5):363-75 |
| Ncbp1 | 433702 | NCBP1 | 4686 | -1.62 | RNA metabolic process | Metabolism | CS score, function | |
| Nfs1 | 18041 | NFS1 | 9054 | -2.40 | metabolic process | Metabolism | CS score, function | |
| Ppcdc | 66812 | PPCDC | 60490 | -1.98 | metabolic process | Metabolism | CS score, function | |
| Qrsl1 | 76563 | QRSL1 | 55278 | -1.67 | metabolic process | Metabolism | CS score, function | |
| Rpp14 | 67053 | RPP14 | 11102 | -1.72 | fatty acid metabolic process | Metabolism | CS score, function | |
| Smarca4 | 20586 | SMARCA 4 | 6597 | -1.89 | metabolic process | Metabolism | CS score, mouse K.O., function | Bultman S, et al. Mol Cell. 2000 Dec;6(6):1287-95 |
| Snrnp200 | 320632 | SNRNP2 00 | 23020 | -2.50 | metabolic process | Metabolism | CS score, function | |
| Srbd1 | 78586 | SRBD1 | 55133 | -2.35 | nucleobase-containing compound metabolic process | Metabolism | CS score, function | |
| Srcap | 100043 597 | SRCAP | 10847 | -1.43 | metabolic process | Metabolism | CS score, function | |
| Ube2i | 22196 | UBE2l | 7329 | -2.55 | metabolic process | Metabolism | CS score, mouse K.O., function | Nacerddine K, et al. Dev Cell. 2005 Dec;9(6):769-79 |
| Ube2m | 22192 | UBE2M | 9040 | -2.39 | metabolic process | Metabolism | CS score, function | |
| Vcp | 269523 | VCP | 7415 | -2.85 | metabolic process | Metabolism | CS score, mouse K.O., function | Muller JM, et al. Biochem Biophys Res Commun. 2007 Mar 9;354(2):459-465 |
| Aamp | 227290 | AAMP | 14 | -2.37 | angiogenesis | Metabolism | CS score, function | |
| Acin1 | 56215 | ACIN1 | 22985 | -1.53 | positive regulation of defense response to virus by host | Metabolism | CS score, function | |
| Aco2 | 11429 | ACO2 | 50 | -2.08 | tricarboxylic acid cycle | Metabolism | CS score, function | |
| Adss | 11566 | ADSS | 159 | -2.46 | purine nucleotide biosynthetic process | Metabolism | CS score, function | |
| Alg2 | 56737 | ALG2 | 85365 | -2.29 | biosynthetic process | Metabolism | CS score, function | |
| Ap2s1 | 232910 | AP2S1 | 1175 | -2.00 | intracellular protein transport | Metabolism | CS score, function | |
| Arcn1 | 213827 | ARCN1 | 372 | -1.91 | intracellular protein transport | Metabolism | CS score, function | |
| Armc7 | 276905 | ARMC7 | 79637 | -2.02 | molecular_function | Metabolism | CS score, function | |
| Atp2a2 | 11938 | ATP2A2 | 488 | -3.01 | calcium ion transmembrane transport | Metabolism | CS score, mouse K.O., function | Andersson KB, et al. Cell Calcium. 2009 Sep;46(3):219-25 |
| Atp5a1 | 11946 | ATP5A1 | 498 | -1.99 | negative regulation of endothelial cell proliferation | Metabolism | CS score, function | |
| Atp5d | 66043 | ATP5D | 513 | -2.21 | oxidative phosphorylation | Metabolism | CS score, function | |
| Atp5o | 28080 | ATP5O | 539 | -1.17 | ATP biosynthetic process | Metabolism | CS score, function | |
| Atp6v0b | 114143 | ATP6V0B | 533 | -3.01 | cellular iron ion homeostasis | Metabolism | CS score, function | |
| Atp6v0c | 11984 | ATP6V0C | 527 | -3.84 | cellular iron ion homeostasis | Metabolism | CS score, mouse K.O., function | Sun-Wada GH, et al. Dev Biol. 2000 Dec 15;228(2):315-25 |
| Atp6v1a | 11964 | ATP6V1A | 523 | -3.58 | proton transport | Metabolism | CS score, function | |
| Atp6v1 b2 | 11966 | ATP6V1B 2 | 526 | -2.94 | cellular iron ion homeostasis | Metabolism | CS score, function | |
| Atp6v1 d | 73834 | ATP6V1D | 51382 | -2.58 | transmembrane transport | Metabolism | CS score, function | |
| Aurkaip1 | 66077 | AURKAIP 1 | 54998 | -1.56 | organelle organization | Metabolism | CS score, function | |
| n/a | n/a | C1orf109 | 54955 | -2.43 | molecular_function | Metabolism | CS score, function | |
| n/a | n/a | C21orf59 | 56683 | -2.77 | cell projection morphogenesis | Metabolism | CS score, function | |
| Ccdc84 | 382073 | CCDC84 | 338657 | -1.86 | molecular_function | Metabolism | CS score, function | |
| Cct2 | 12461 | CCT2 | 10576 | -3.23 | protein folding | Metabolism | CS score, function | |
| Cct3 | 12462 | CCT3 | 7203 | -3.31 | protein folding | Metabolism | CS score, function | |
| Cct4 | 12464 | CCT4 | 10575 | -2.62 | protein folding | Metabolism | CS score, function | |
| Cct5 | 12465 | CCT5 | 22948 | -2.84 | protein folding | Metabolism | CS score, function | |
| Cct7 | 12468 | CCT7 | 10574 | -2.47 | protein folding | Metabolism | CS score, function | |
| Cct8 | 12469 | CCT8 | 10694 | -2.03 | protein folding | Metabolism | CS score, function | |
| Cdipt | 52858 | CDIPT | 10423 | -2.53 | phospholipid biosynthetic process | Metabolism | CS score, function | |
| Cenpi | 102920 | CENPI | 2491 | -1.81 | centromere complex assembly | Metabolism | CS score, function | |
| Chordc1 | 66917 | CHORDC 1 | 26973 | -1.52 | regulation of centrosome duplication | Metabolism | CS score, mouse K.O., function | Ferretti R, et al. Dev Cell. 2010 Mar 16;18(3):486-95 |
| Coa5 | 76178 | COA5 | 493753 | -2.33 | mitochondrion | Metabolism | CS score, function | |
| Cog4 | 102339 | COG4 | 25839 | -1.39 | Golgi vesicle transport | Metabolism | CS score, function | |
| Copa | 12847 | COPA | 1314 | -1.63 | intracellular protein transport | Metabolism | CS score, function | |
| Copb1 | 70349 | COPB1 | 1315 | -2.30 | intracellular protein transport | Metabolism | CS score, function | |
| Copb2 | 50797 | COPB2 | 9276 | -2.65 | intracellular protein transport | Metabolism | CS score, function | |
| Cope | 59042 | COPE | 11316 | -2.93 | ER to Golgi vesicle-mediated transport | Metabolism | CS score, function | |
| Copz1 | 56447 | COPZ1 | 22818 | -1.87 | transport | Metabolism | CS score, function | |
| Coq4 | 227683 | COQ4 | 51117 | -1.29 | ubiquinone biosynthetic process | Metabolism | CS score, function | |
| Cox15 | 226139 | COX15 | 1355 | -2.14 | mitochondrial electron transport, cytochrome c to oxygen | Metabolism | CS score, mouse K.O., function | Viscomi C, et al. Cell Metab. 2011 Jul 6;14(1):80-90 |
| Cox17 | 12856 | COX17 | 10063 | -1.97 | copper ion transport | Metabolism | CS score, mouse K.O., function | Takahashi Y, et al. Mol Cell Biol. 2002 Nov;22(21 ):7614-21 |
| Cse1l | 110750 | CSE1L | 1434 | -2.31 | protein export from nucleus | Metabolism | CS score, mouse K.O., function | Bera TK, et al. Mol Cell Biol. 2001 Oct;21(20):7020-4 |
| Csnk2b | 13001 | CSNK2B | 1460 | -1.94 | regulation of protein kinase activity | Metabolism | CS score, mouse K.O., function | Buchou T, et al. Mol Cell Biol. 2003 Feb;23(3):908-15 |
| Cycs | 13063 | CYCS | 54205 | -2.36 | response to reactive oxygen species | Metabolism | CS score, mouse K.O., function | Li K, et al. Cell. 2000 May 12;101(4):389-99 |
| Dad1 | 13135 | DAD1 | 1603 | -2.21 | protein glycosylation | Metabolism | CS score, mouse K.O., function | Brewster JL, et al. Genesis. 2000 Apr;26(4):271-8 |
| Dap3 | 65111 | DAP3 | 7818 | -1.70 | apoptotic process | Metabolism | CS score, mouse K.O., function | Kim HR, et al. FASEB J. 2007 Jan;21 (1):188-96 |
| Dctn5 | 59288 | DCTN5 | 84516 | -2.39 | antigen processing and presentation of exogenous peptide antigen via MHC class II | Metabolism | CS score, function | |
| Ddost | 13200 | DDOST | 1650 | -2.38 | protein N-linked glycosylation via asparagine | Metabolism | CS score, function | |
| Dgcr8 | 94223 | DGCR8 | 54487 | -2.10 | gene expression | Metabolism | CS score, mouse K.O., function | Ouchi Y, et al. J Neurosci. 2013 May 29;33(22):9408-19 |
| Dhodh | 56749 | DHODH | 1723 | -2.57 | de novo' pyrimidine nucleobase biosynthetic process | Metabolism | CS score, function | |
| Dnlz | 52838 | DNLZ | 728489 | -1.92 | protein folding | Metabolism | CS score, function | |
| Dnm1l | 74006 | DNM1L | 10059 | -3.25 | mitochondrial fission | Metabolism | CS score, mouse K.O., function | Wakabayashi J, et al. J Cell Biol. 2009 Sep 21;186(6):805-16 |
| Dnm2 | 13430 | DNM2 | 1785 | -3.98 | endocytosis | Metabolism | CS score, mouse K.O., function | Ferguson SM, et al. Dev Cell. 2009 Dec;17(6):811-22 |
| Dohh | 102115 | DOHH | 83475 | -1.76 | peptidyl-lysine modification to peptidyl-hypusine | Metabolism | CS score, function | |
| Dolk | 227697 | DOLK | 22845 | -2.38 | dolichol-linked oligosaccharide biosynthetic process | Metabolism | CS score, function | |
| Donson | 60364 | DONSON | 29980 | -2.30 | multicellular organismal development | Metabolism | CS score, function | |
| Dph3 | 105638 | DPH3 | 285381 | -1.62 | peptidyl-diphthamide biosynthetic process from peptidyl-histidine | Metabolism | CS score, mouse K.O., function | Liu S, et al. Mol Cell Biol. 2006 May;26(10):3835-41 |
| Dtymk | 21915 | DTYMK | 1841 | -3.54 | phosphorylation | Metabolism | CS score, function | |
| Eif2b2 | 217715 | EIF2B2 | 8892 | -2.24 | ovarian follicle development | Metabolism | CS score, function | |
| Eif2s2 | 67204 | EIF2S2 | 8894 | -2.33 | in utero embryonic development | Metabolism | CS score, mouse K.O., function | Heaney JD, et al. Hum Mol Genet. 2009 Apr 15;18(8):1395-404 |
| Emc1 | 230866 | EMC1 | 23065 | -1.34 | protein folding in endoplasmic reticulum | Metabolism | CS score, function | |
| Emc7 | 73024 | EMC7 | 56851 | -2.27 | biological process | Metabolism | CS score, function | |
| Eno1 | 13806 | ENO1 | 2023 | -2.03 | glycolytic process | Metabolism | CS score, mouse K.O., function | Couldrey C, et al. Dev Dyn. 1998 Jun;212(2):284-92 |
| Fam50a | 108160 | FAM50A | 9130 | -3.16 | spermatogenesis | Metabolism | CS score, function | |
| Fam96b | 68523 | FAM96B | 51647 | -1.90 | iron-sulfur cluster assembly | Metabolism | CS score, function | |
| Fdps | 110196 | FDPS | 2224 | -2.41 | isoprenoid biosynthetic process | Metabolism | CS score, function | |
| Gapdh | 14433 | GAPDH | 2597 | -2.40 | oxidation-reduction process | Metabolism | CS score, function | |
| Gart | 14450 | GART | 2618 | -1.87 | purine nucleobase biosynthetic process | Metabolism | CS score, function | |
| Gemin4 | 276919 | GEMIN4 | 50628 | -1.56 | spliceosomal snRNP assembly | Metabolism | CS score, function | |
| Gemin5 | 216766 | GEMIN5 | 25929 | -2.51 | spliceosomal snRNP assembly | Metabolism | CS score, function | |
| Ggps1 | 14593 | GGPS1 | 9453 | -1.62 | cholesterol biosynthetic process | Metabolism | CS score, function | |
| Gmppb | 331026 | GMPPB | 29925 | -3.22 | biosynthetic process | Metabolism | CS score, function | |
| Gnb1l | 13972 | GNB1L | 54584 | -1.93 | G-potein coupled receptor signaling pathway | Metabolism | CS score, function | |
| n/a | n/a | GOLGA6 L1 | 283767 | -3.15 | | Metabolism | CS score, function | |
| Gosr2 | 56494 | GOSR2 | 9570 | -1.13 | protein targeting to vacuole | Metabolism | CS score, function | |
| Gpkow | 209416 | GPKOW | 27238 | -1.36 | biological process | Metabolism | CS score, function | |
| Gpn2 | 100210 | GPN2 | 54707 | -3.71 | biological process | Metabolism | CS score, function | |
| Gps1 | 209318 | GPS1 | 2873 | -2.11 | inactivation of MAPK activity | Metabolism | CS score, function | |
| Grpel1 | 17713 | GRPEL1 | 80273 | -2.61 | protein folding | Metabolism | CS score, function | |
| Grwd1 | 101612 | GRWD1 | 83743 | -1.90 | poly(A) RNA binding | Metabolism | CS score, function | |
| Hmgcr | 15357 | HMGCR | 3156 | -2.94 | cholesterol biosynthetic process | Metabolism | CS score, mouse K.O., function | Ohashi K, et al. J Biol Chem. 2003 Oct 31 ;278(44):42936-41 |
| Hmgcs1 | 208715 | HMGCS1 | 3157 | -2.41 | liver development | Metabolism | CS score, function | |
| Hspa5 | 14828 | HSPA5 | 3309 | -3.86 | platelet degranulation | Metabolism | CS score, mouse K.O., function | Luo S, et al. Mol Cell Biol. 2006 Aug;26(15):5688-97 |
| Hspa9 | 15526 | HSPA9 | 3313 | -3.55 | protein folding | Metabolism | CS score, function | |
| Hspd1 | 15510 | HSPD1 | 3329 | -1.95 | response to hypoxia | Metabolism | CS score, mouse K.O., function | Christensen JH, et al. Cell Stress Chaperones. 2010 Nov;15(6):851-63 |
| Hspe1 | 15528 | HSPE1 | 3336 | -3.75 | osteoblast differentiation | Metabolism | CS score, function | |
| Hyou1 | 12282 | HYOU1 | 10525 | -2.06 | response to ischemia | Metabolism | CS score, function | |
| lpo13 | 230673 | IPO13 | 9670 | -2.84 | intracellular protein transport | Metabolism | CS score, function | |
| Iscu | 66383 | ISCU | 23479 | -2.40 | cellular iron ion homeostasis | Metabolism | CS score, function | |
| Itpk1 | 217837 | ITPK1 | 3705 | -1.55 | phosphorylation | Metabolism | CS score, function | |
| Kansl2 | 69612 | KANSL2 | 54934 | -1.19 | chromatin organization | Metabolism | CS score, function | |
| Kansl3 | 226976 | KANSL3 | 55683 | -1.53 | chromatin organization | Metabolism | CS score, function | |
| Kri1 | 215194 | KRI1 | 65095 | -2.49 | poly(A) RNA binding | Metabolism | CS score, function | |
| Lamtor2 | 83409 | LAMTOR 2 | 28956 | -1.62 | activation of MAPKK activity | Metabolism | CS score, mouse K.O., function | Teis D, et al. J Cell Biol. 2006 Dec 18;175(6):861-8 |
| Leng8 | 232798 | LENG8 | 114823 | -1.75 | biological_process | Metabolism | CS score, function | |
| Ltv1 | 353258 | LTV1 | 84946 | -1.81 | nucleoplasm | Metabolism | CS score, function | |
| Mak16 | 67920 | MAK16 | 84549 | -2.30 | poly(A) RNA binding | Metabolism | CS score, function | |
| Mat2a | 232087 | MAT2A | 4144 | -2.34 | S-adenosylmethionine biosynthetic process | Metabolism | CS score, function | |
| Mcm3ap | 54387 | MCM3AP | 8888 | -1.58 | immune system process | Metabolism | CS score, mouse K.O., function | Yoshida M, et al. Genes Cells. 2007 Oct;12(10):1205-13 |
| Mdn1 | 100019 | MDN1 | 23195 | -1.68 | protein complex assembly | Metabolism | CS score, function | |
| n/a | n/a | MFAP1 | 4236 | -1.94 | biological process | Metabolism | CS score, function | |
| Mmgt1 | 236792 | MMGT1 | 93380 | -1.55 | magnesium ion transport | Metabolism | CS score, function | |
| Mrpl16 | 94063 | MRPL16 | 54948 | -1.80 | organelle organization | Metabolism | CS score, function | |
| Mrpl17 | 27397 | MRPL17 | 63875 | -1.80 | mitochondrial genome maintenance | Metabolism | CS score, function | |
| Mrpl33 | 66845 | MRPL33 | 9553 | -1.62 | organelle organization | Metabolism | CS score, function | |
| Mrpl38 | 60441 | MRPL38 | 64978 | -1.95 | organelle organization | Metabolism | CS score, function | |
| Mrpl39 | 27393 | MRPL39 | 54148 | -1.71 | organelle organization | Metabolism | CS score, function | |
| Mrpl45 | 67036 | MRPL45 | 84311 | -1.75 | organelle organization | Metabolism | CS score, function | |
| Mrpl46 | 67308 | MRPL46 | 26589 | -1.83 | organelle organization | Metabolism | CS score, function | |
| Mrpl53 | 68499 | MRPL53 | 116540 | -1.84 | organelle organization | Metabolism | CS score, function | |
| Mrps22 | 64655 | MRPS22 | 56945 | -1.32 | organelle organization | Metabolism | CS score, function | |
| Mrps25 | 64658 | MRPS25 | 64432 | -1.63 | organelle organization | Metabolism | CS score, function | |
| Mrps35 | 232536 | MRPS35 | 60488 | -1.60 | organelle organization | Metabolism | CS score, function | |
| Mrps5 | 77721 | MRPS5 | 64969 | -1.65 | organelle organization | Metabolism | CS score, function | |
| Mvd | 192156 | MVD | 4597 | -3.24 | isoprenoid biosynthetic process | Metabolism | CS score, function | |
| Mvk | 17855 | MVK | 4598 | -1.73 | isoprenoid biosynthetic process | Metabolism | CS score, function | |
| Naa25 | 231713 | NAA25 | 80018 | -2.40 | peptide alpha-N-acetyltransferase activity | Metabolism | CS score, function | |
| Napa | 108124 | NAPA | 8775 | -2.31 | intracellular protein transport | Metabolism | CS score, function | |
| Nat10 | 98956 | NAT10 | 55226 | -2.16 | biological process | Metabolism | CS score, function | |
| Mdor1 | 78797 | NDOR1 | 27158 | -2.10 | cell death | Metabolism | CS score, function | |
| Ndufab1 | 70316 | NDUFAB 1 | 4706 | -1.83 | fatty acid biosynthetic process | Metabolism | CS score, function | |
| Nol10 | 217431 | NOL10 | 79954 | -1.79 | poly(A) RNA binding | Metabolism | CS score, function | |
| Nop9 | 67842 | NOP9 | 161424 | -1.44 | biological_process | Metabolism | CS score, function | |
| Nrde2 | 217827 | NRDE2 | 55051 | -2.69 | biological process | Metabolism | CS score, function | |
| Nsf | 18195 | NSF | 4905 | -2.76 | intra-Golgi vesicle-mediated transport | Metabolism | CS score, function | |
| Nubp1 | 26425 | NUBP1 | 4682 | -2.05 | cellular iron ion homeostasis | Metabolism | CS score, function | |
| Nudcd3 | 209586 | NUDCD3 | 23386 | -1.71 | molecular_function | Metabolism | CS score, function | |
| Nup155 | 170762 | NUP155 | 9631 | -1.59 | nucleocytoplasmic transport | Metabolism | CS score, mouse K.O., function | Zhang X, et al. Cell. 2008 Dec 12;135(6):1017-27 |
| Nup93 | 71805 | NUP93 | 9688 | -2.11 | protein import into nucleus | Metabolism | CS score, function | |
| Nus1 | 52014 | NUS1 | 116150 | -1.94 | angiogenesis | Metabolism | CS score, mouse K.O., function | Park EJ, et al. Cell Metab. 2014 Sep 2;20(3):448-57 |
| Nvl | 67459 | NVL | 4931 | -2.61 | positive regulation of telomerase activity | Metabolism | CS score, function | |
| Ogdh | 18293 | OGDH | 4967 | -2.98 | tricarboxylic acid cycle | Metabolism | CS score, function | |
| Osbp | 76303 | OSBP | 5007 | -2.06 | lipid transport | Metabolism | CS score, function | |
| Pak1ip1 | 68083 | PAK1IP1 | 55003 | -2.28 | cell proliferation | Metabolism | CS score, function | |
| Pfdn2 | 18637 | PFDN2 | 5202 | -1.32 | protein folding | Metabolism | CS score, function | |
| Pgam1 | 18648 | PGAM1 | 5223 | -2.37 | glycolytic process | Metabolism | CS score, function | |
| Pkm | 18746 | PKM | 5315 | -1.68 | glycolytic process | Metabolism | CS score, mouse K.O., function | Lewis SE, et al. 1983:267-78. Plenum Publ. Corp. |
| Pmpcb | 73078 | PMPCB | 9512 | -1.77 | proteolysis | Metabolism | CS score, function | |
| Ppil2 | 66053 | PPIL2 | 23759 | -3.01 | protein polyubiquitination | Metabolism | CS score, function | |
| Ppp4c | 56420 | PPP4C | 5531 | -2.89 | protein dephosphorylation | Metabolism | CS score, mouse K.O., function | Toyo-oka K, et al. J Cell Biol. 2008 Mar 24;180(6):1133-47 |
| Prelid1 | 66494 | PRELID1 | 27166 | -2.27 | apoptotic process | Metabolism | CS score, function | |
| Prpf31 | 68988 | PRPF31 | 26121 | -3.20 | spliceosomal tri-snRNP complex assembly | Metabolism | CS score, mouse K.O., function | Bujakowska K, et al. Invest Ophthalmol Vis Sci. 2009 Dec;50(12):5927-33 |
| Prpf6 | 68879 | PRPF6 | 24148 | -2.96 | spliceosomal tri-snRNP complex assembly | Metabolism | CS score, function | |
| Psma1 | 26440 | PSMA1 | 5682 | -2.39 | proteasomal ubiqu itin-independent protein catabolic process | Metabolism | CS score, function | |
| Psma2 | 19166 | PSMA2 | 5683 | -2.23 | proteasomal ubiqu itin-independent protein catabolic process | Metabolism | CS score, function | |
| Psma3 | 19167 | PSMA3 | 5684 | -2.30 | proteasomal ubiqu itin-independent protein catabolic process | Metabolism | CS score, function | |
| Psmb2 | 26445 | PSMB2 | 5690 | -2.12 | proteasomal ubiqu itin-independent protein catabolic process | Metabolism | CS score, function | |
| Psmb3 | 26446 | PSMB3 | 5691 | -2.78 | proteolysis involved in cellular protein catabolic process | Metabolism | CS score, function | |
| Psmb5 | 19173 | PSMB5 | 5693 | -1.67 | proteasomal ubiqu itin-independent protein catabolic process | Metabolism | CS score, function | |
| Psmb6 | 19175 | PSMB6 | 5694 | -2.42 | proteasomal ubiqu itin-independent protein catabolic process | Metabolism | CS score, function | |
| Psmb7 | 19177 | PSMB7 | 5695 | -2.69 | proteasomal ubiqu itin-independent protein catabolic process | Metabolism | CS score, function | |
| Psmc2 | 19181 | PSMC2 | 5701 | -2.35 | protein catabolic process | Metabolism | CS score, function | |
| Psmc3 | 19182 | PSMC3 | 5702 | -2.76 | ER-associated ubiquitin-dependent protein catabolic process | Metabolism | CS score, mouse K.O., function | Sakao Y, et al. Genomics. 2000 Jul 1;67(1):1-7 |
| Psmc4 | 23996 | PSMC4 | 5704 | -2.36 | blastocyst development | Metabolism | CS score, mouse K.O., function | Sakao Y, et al. Genomics. 2000 Jul 1;67(1):1-7 |
| Psmd1 | 70247 | PSMD1 | 5707 | -1.88 | regulation of protein catabolic process | Metabolism | CS score, function | |
| Psmd2 | 21762 | PSMD2 | 5708 | -2.16 | regulation of protein catabolic process | Metabolism | CS score, function | |
| Psmd3 | 22123 | PSMD3 | 5709 | -2.10 | regulation of protein catabolic process | Metabolism | CS score, function | |
| Psmd4 | 19185 | PSMD4 | 5710 | -1.77 | ubiquitin-dependent protein catabolic process | Metabolism | CS score, mouse K.O., function | Soriano P, et al. Genes Dev. 1987 Jun;1 (4):366-75 |
| Psmd6 | 66413 | PSMD6 | 9861 | -2.27 | proteasome-mediated ubiquitin-dependent protein catabolic process | Metabolism | CS score, function | |
| Psmg3 | 66506 | PSMG3 | 84262 | -2.57 | molecular_function | Metabolism | CS score, function | |
| Ptpmt1 | 66461 | PTPMT1 | 114971 | -2.89 | protein dephosphorylation | Metabolism | CS score, mouse K.O., function | Shen J, et al. Mol Cell Biol. 2011 Dec;31(24):4902-16 |
| Ptpn23 | 104831 | PTPN23 | 25930 | -1.59 | negative regulation of epithelial cell migration | Metabolism | CS score, mouse K.O., function | Gingras MC, et al. Int J Dev Biol. 2009;53(7):1069-74 |
| Rabggta | 56187 | RABGGT A | 5875 | -3.18 | protein prenylation | Metabolism | CS score, function | |
| Rabggtb | 19352 | RABGGT B | 5876 | -2.44 | protein geranylgeranylation | Metabolism | CS score, function | |
| Rbm19 | 74111 | RBM19 | 9904 | -2.03 | multicellular organismal development | Metabolism | CS score, mouse K.O., function | Zhang J, et al. BMC Dev Biol. 2008;8:115 |
| Rfk | 54391 | RFK | 55312 | -1.56 | riboflavin biosynthetic process | Metabolism | CS score, mouse K.O., function | Yazdanpanah B, et al. Nature. 2009 Aug 27;460(7259):1159-63 |
| Rheb | 19744 | RHEB | 6009 | -1.38 | signal transduction | Metabolism | CS score, mouse K.O., function | Zou J, et al. Dev Cell. 2011 Jan 18;20(1):97-108 |
| Riok1 | 71340 | RIOK1 | 83732 | -1.27 | protein phosphorylation | Metabolism | CS score, function | |
| Rpn1 | 103963 | RPN1 | 6184 | -2.13 | protein glycosylation | Metabolism | CS score, function | |
| Rtfdc1 | 66404 | RTFDC1 | 51507 | -2.09 | biological process | Metabolism | CS score, function | |
| Sacm1I | 83493 | SACM1L L | 22908 | -1.80 | protein dephosphorylation | Metabolism | CS score, function | |
| Samm50 | 68653 | SAMM50 | 25813 | -1.62 | protein targeting to mitochondrion | Metabolism | CS score, function | |
| Sco2 | 100126 824 | SCO2 | 9997 | -1.60 | eye development | Metabolism | CS score, mouse K.O., function | Yang H, et al. Hum Mol Genet. 2010 Jan 1;19(1):170-80 |
| Sdha | 66945 | SDHA | 6389 | -2.20 | tricarboxylic acid cycle | Metabolism | CS score, function | |
| Sdhb | 67680 | SDHB | 6390 | -2.33 | tricarboxylic acid cycle | Metabolism | CS score, function | |
| Sec61a1 | 53421 | SEC61A1 | 29927 | -2.42 | protein transport | Metabolism | CS score, function | |
| Slc20a1 | 20515 | SLC20A1 | 6574 | -2.38 | sodium ion transport | Metabolism | CS score, mouse K.O., function | Festing MH, et al. Genesis. 2009 Dec;47(12):858-63 |
| Slc7a6os | 66432 | SLC7A6O S | 84138 | -2.30 | hematopoietic progenitor cell differentiation | Metabolism | CS score, function | |
| Smn1 | 20595 | SMN1 | 6606 | -1.58 | spliceosomal complex assembly | Metabolism | CS score, mouse K.O., function | Hsieh-Li HM, et al. Nat Genet. 2000 Jan;24(1):66-70 |
| Smu1 | 74255 | SMU1 | 55234 | -3.65 | molecular_function | Metabolism | CS score, function | |
| Snrpd1 | 20641 | SNRPD1 | 6632 | -2.79 | spliceosomal complex assembly | Metabolism | CS score, function | |
| Snrpd3 | 67332 | SNRPD3 | 6634 | -3.62 | spliceosomal complex assembly | Metabolism | CS score, function | |
| Snrpe | 20643 | SNRPE | 6635 | -2.74 | spliceosomal complex assembly | Metabolism | CS score, function | |
| Spata5 | 57815 | SPATA5 | 166378 | -1.50 | multicellular organismal development | Metabolism | CS score, function | |
| Spata5l1 | 214616 | SPATA5L 1 | 79029 | -2.70 | molecular_function | Metabolism | CS score, function | |
| Tango6 | 272538 | TANGO6 | 79613 | -2.29 | integral component of membrane | Metabolism | CS score, function | |
| n/a | n/a | TBC1D3B | 414059 | -1.67 | positive regulation of GTPase activity | Metabolism | CS score, function | |
| n/a | n/a | TBC1D3 C | 414060 | -2.01 | positive regulation of GTPase activity | Metabolism | CS score, function | |
| Tbcb | 66411 | TBCB | 1155 | -1.97 | nervous system development | Metabolism | CS score, function | |
| Tbcc | 72726 | TBCC | 6903 | -3.02 | cell morphogenesis | Metabolism | CS score, function | |
| Tbcd | 108903 | TBCD | 6904 | -1.82 | microtubule cytoskeleton organization | Metabolism | CS score, function | |
| Tcp1 | 21454 | TCP1 | 6950 | -2.34 | protein folding | Metabolism | CS score, function | |
| Telo2 | 71718 | TELO2 | 9894 | -2.34 | regulation of TOR signaling | Metabolism | CS score, mouse K.O., function | Takai H, et al. Cell. 2007 Dec 28;131(7):1248-59 |
| Tex10 | 269536 | TEX10 | 54881 | -1.26 | integral component of membrane | Metabolism | CS score, function | |
| Tfrc | 22042 | TFRC | 7037 | -3.40 | cellular iron ion homeostasis | Metabolism | CS score, mouse K.O., function | Levy JE, et al. Nat Genet. 1999 Apr;21(4):396-9 |
| Timm10 | 30059 | TIMM10 | 26519 | -1.99 | protein targeting to mitochondrion | Metabolism | CS score, function | |
| Timm13 | 30055 | TIMM13 | 26517 | -1.62 | protein targeting to mitochondrion | Metabolism | CS score, function | |
| Timm23 | 53600 | TIMM23 | 100287 932 | -2.00 | protein targeting to mitochondrion | Metabolism | CS score, mouse K.O., function | Ahting U, et al. Biochim Biophys Acta. 2009 May;1787(5):371-6 |
| Timm44 | 21856 | TIMM44 | 10469 | -1.73 | protein import into mitochondrial matrix | Metabolism | CS score, function | |
| Tmx2 | 66958 | TMX2 | 51075 | -2.29 | biological process | Metabolism | CS score, function | |
| Tnpo3 | 320938 | TNPO3 | 23534 | -1.82 | splicing factor protein import into nucleus | Metabolism | CS score, function | |
| Trmt112 | 67674 | TRMT112 | 51504 | -3.70 | peptidyl-glutamine methylation | Metabolism | CS score, function | |
| Trnau1ap | 71787 | TRNAU1 AP | 54952 | -1.40 | selenocysteine incorporation | Metabolism | CS score, function | |
| Ttc1 | 66827 | TTC1 | 7265 | -1.74 | protein folding | Metabolism | CS score, function | |
| Ttc27 | 74196 | TTC27 | 55622 | -2.54 | biological process | Metabolism | CS score, function | |
| Tti1 | 75425 | TTI1 | 9675 | -2.91 | regulation of TOR signaling | Metabolism | CS score, function | |
| Tti2 | 234138 | TTI2 | 80185 | -1.94 | molecular_function | Metabolism | CS score, function | |
| n/a | n/a | TUBB | 203068 | -3.40 | microtubule-based process | Metabolism | CS score, function | |
| Txn2 | 56551 | TXN2 | 25828 | -1.41 | sulfate assimilation | Metabolism | CS score, mouse K.O., function | Nonn L, et al. Mol Cell Biol. 2003 Feb;23(3):916-22 |
| Uqcrc1 | 22273 | UQCRC1 | 7384 | -1.29 | oxidative phosphorylation | Metabolism | CS score, function | |
| Uqcrh | 66576 | UQCRH | 7388 | -1.28 | oxidative phosphorylation | Metabolism | CS score, function | |
| Urb2 | 382038 | URB2 | 9816 | -2.25 | molecular_function | Metabolism | CS score, function | |
| Vmp1 | 75909 | VMP1 | 81671 | -1.75 | exocytosis | Metabolism | CS score, function | |
| n/a | n/a | VPS28 | 51160 | -3.06 | protein targeting to vacuole involved in ubiquitin-dependent protein catabolic process via the multivesicular body sorting pathway | Metabolism | CS score, function | |
| Vps29 | 56433 | VPS29 | 51699 | -2.05 | intracellular protein transport | Metabolism | CS score, function | |
| Vps52 | 224705 | VPS52 | 6293 | -1.85 | ectodermal cell differentiation | Metabolism | CS score, mouse K.O., function | Sugimoto M, et al. Cell Rep. 2012 Nov 29;2(5):1363-74 |
| Wars2 | 70560 | WARS2 | 10352 | -1.16 | vasculogenesis | Metabolism | CS score, function | |
| Wdr7 | 104082 | WDR7 | 23335 | -1.47 | hematopoietic progenitor cell differentiation | Metabolism | CS score, function | |
| Wdr70 | 545085 | WDR70 | 55100 | -1.69 | enzyme binding | Metabolism | CS score, function | |
| Wdr74 | 107071 | WDR74 | 54663 | -2.84 | blastocyst formation | Metabolism | CS score, function | |
| Wdr77 | 70465 | WDR77 | 79084 | -2.19 | spliceosomal snRNP assembly | Metabolism | CS score, mouse K.O., function | Zhou L, et al. J Mol Endocrinol. 2006 Oct;37(2):283-300 |
| Yae1d1 | 67008 | YAE1D1 | 57002 | -1.71 | molecular_function | Metabolism | CS score, function | |
| Yrdc | 230734 | YRDC | 79693 | -2.33 | negative regulation of transport | Metabolism | CS score, function | |
| Znhit2 | 29805 | ZNHIT2 | 741 | -2.70 | metal ion binding | Metabolism | CS score, function | |
| Aars | 234734 | AARS | 16 | -2.48 | alanyl-tRNA aminoacylation | RNA transcription , protein translation | CS score, function | |
| Bms1 | 213895 | BMS1 | 9790 | -1.36 | ribosome assembly | RNA transcription , protein translation | CS score, function | |
| Bud31 | 231889 | BUD31 | 8896 | -2.46 | mRNA splicing, via spliceosome | RNA transcription , protein translation | CS score, function | |
| Bysl | 53414 | BYSL | 705 | -2.24 | maturation of SSU-rRNA from tricistronic rRNA transcript (SSU-rRNA, 5.8S rRNA, LSU-rRNA) | RNA transcription , protein translation | CS score, mouse K.O., function | Aoki R, et al. FEBS Lett. 2006 Nov 13;580(26):6062-8 |
| Cars | 27267 | CARS | 833 | -2.45 | tRNA aminoacylation for protein translation | RNA transcription , protein translation | CS score, function | |
| Cdc5l | 71702 | CDC5L | 988 | -2.09 | mRNA splicing, via spliceosome | RNA transcription , protein translation | CS score, function | |
| Cdc73 | 214498 | CDC73 | 79577 | -2.58 | negative regulation of transcription from RNA polymerase II promoter | RNA transcription , protein translation | CS score, mouse K.O., function | Wang P, et al. Mol Cell Biol. 2008 May;28(9):2930-40 |
| Cebpz | 12607 | CEBPZ | 10153 | -2.11 | transcription from RNA polymerase II promoter | RNA transcription , protein translation | CS score, function | |
| Clasrp | 53609 | CLASRP | 11129 | -1.30 | mRNA processing | RNA transcription , protein translation | CS score, function | |
| Clp1 | 98985 | CLP1 | 10978 | -3.47 | mRNA splicing, via spliceosome | RNA transcription , protein translation | CS score, mouse K.O., function | Hanada T, et al. Nature. 2013 Mar 28;495(7442):474-80 |
| Cox5b | 12859 | COX5B | 1329 | -1.50 | transcription initiation from RNA polymerase II promoter | RNA transcription , protein translation | CS score, function | |
| Cpsf1 | 94230 | CPSF1 | 29894 | -2.58 | mRNA splicing, via spliceosome | RNA transcription , protein translation | CS score, function | |
| Cpsf2 | 51786 | CPSF2 | 53981 | -2.55 | mRNA polyadenylation | RNA transcription , protein translation | CS score, function | |
| Cpsf3l | 71957 | CPSF3L | 54973 | -2.09 | snRNA processing | RNA transcription , protein translation | CS score, function | |
| Dars | 226414 | DARS | 1615 | -2.90 | translation | RNA transcription , protein translation | CS score, function | |
| Dbr1 | 83703 | DBR1 | 51163 | -3.75 | RNA splicing, via transesterification reactions | RNA transcription , protein translation | CS score, function | |
| Ddx18 | 66942 | DDX18 | 8886 | -2.33 | RNA secondary structure unwinding | RNA transcription , protein translation | CS score, function | |
| Ddx23 | 74351 | DDX23 | 9416 | -3.01 | RNA secondary structure unwinding | RNA transcription , protein translation | CS score, function | |
| Ddx24 | 27225 | DDX24 | 57062 | -1.40 | RNA secondary structure unwinding | RNA transcription , protein translation | CS score, function | |
| Ddx41 | 72935 | DDX41 | 51428 | -1.74 | RNA secondary structure unwinding | RNA transcription , protein translation | CS score, function | |
| Ddx46 | 212880 | DDX46 | 9879 | -2.79 | mRNA splicing, via spliceosome | RNA transcription , protein translation | CS score, function | |
| Ddx47 | 67755 | DDX47 | 51202 | -2.20 | RNA secondary structure unwinding | RNA transcription , protein translation | CS score, function | |
| Ddx49 | 234374 | DDX49 | 54555 | -3.20 | RNA secondary structure unwinding | RNA transcription , protein translation | CS score, function | |
| Ddx54 | 71990 | DDX54 | 79039 | -2.94 | RNA secondary structure unwinding | RNA transcription , protein translation | CS score, function | |
| Ddx56 | 52513 | DDX56 | 54606 | -2.85 | rRNA processing | RNA transcription , protein translation | CS score, function | |
| Dgcr14 | 27886 | DGCR14 | 8220 | -1.76 | mRNA splicing, via spliceosome | RNA transcription , protein translation | CS score, function | |
| Dhx15 | 13204 | DHX15 | 1665 | -2.58 | mRNA processing | RNA transcription , protein translation | CS score, function | |
| Dhx16 | 69192 | DHX16 | 8449 | -1.35 | mRNA processing | RNA transcription , protein translation | CS score, function | |
| Dhx38 | 64340 | DHX38 | 9785 | -1.76 | mRNA splicing, via spliceosome | RNA transcription , protein translation | CS score, function | |
| Diexf | 215193 | DIEXF | 27042 | -2.03 | maturation of SSU-rRNA from tricistronic rRNA transcript (SSU-rRNA, 5.8S rRNA, LSU-rRNA) | RNA transcription , protein translation | CS score, function | |
| Dimt1 | 66254 | DIMT1 | 27292 | -1.87 | rRNA methylation | RNA transcription , protein translation | CS score, function | |
| Dis3 | 72662 | DIS3 | 22894 | -1.77 | mRNA catabolic process | RNA transcription , protein translation | CS score, function | |
| Dkc1 | 245474 | DKC1 | 1736 | -2.37 | box H/ACA snoRNA 3'-end processing | RNA transcription , protein translation | CS score, mouse K.O., function | He J, et al. Oncogene. 2002 Oct 31;21(50):7740-4 |
| Dnajc17 | 69408 | DNAJC17 | 55192 | -2.25 | negative regulation of transcription from RNA polymerase II promoter | RNA transcription , protein translation | CS score, mouse K.O., function | Amendola E, et al. Endocrinology. 2010 Apr;151(4):1948-58 |
| Ears2 | 67417 | EARS2 | 124454 | -1.91 | tRNA aminoacylation for protein translation | RNA transcription , protein translation | CS score, function | |
| Ebna1bp2 | 69072 | EBNA1B P2 | 10969 | -1.52 | ribosome biogenesis | RNA transcription , protein translation | CS score, function | |
| Eef1a1 | 13627 | EEF1A1 | 1915 | -3.11 | translational elongation | RNA transcription , protein translation | CS score, function | |
| Eef1g | 67160 | EEF1G | 1937 | -1.42 | translation | RNA transcription , protein translation | CS score, function | |
| Eef2 | 13629 | EEF2 | 1938 | -3.53 | translation | RNA transcription , protein translation | CS score, function | |
| Eftud2 | 20624 | EFTUD2 | 9343 | -3.79 | mRNA splicing, via spliceosome | RNA transcription, protein translation | CS score, function | |
| Eif1ad | 69860 | EIF1AD | 84285 | -2.26 | translational initiation | RNA transcription , protein translation | CS score, function | |
| Eif2b1 | 209354 | EIF2B1 | 1967 | -2.23 | regulation of translational initiation | RNA transcription , protein translation | CS score, function | |
| Eif2b3 | 108067 | EIF2B3 | 8891 | -3.00 | translational initiation | RNA transcription , protein translation | CS score, function | |
| Eif2s1 | 13665 | EIF2S1 | 1965 | -3.93 | translation | RNA transcription , protein translation | CS score, function | |
| Eif3c | 56347 | EIF3C | 8663 | -2.59 | formation of translation preinitiation complex | RNA transcription , protein translation | CS score, function | |
| n/a | n/a | EIF3CL | 728689 | -2.71 | formation of translation preinitiation complex | RNA transcription , protein translation | CS score, function | |
| Eif3d | 55944 | EIF3D | 8664 | -3.23 | formation of translation preinitiation complex | RNA transcription , protein translation | CS score, function | |
| Eif3f | 66085 | EIF3F | 8665 | -1.44 | formation of translation preinitiation complex | RNA transcription , protein translation | CS score, function | |
| Eif3g | 53356 | EIF3G | 8666 | -3.10 | translational initiation | RNA transcription , protein translation | CS score, function | |
| Eif3i | 54709 | EIF3I | 8668 | -2.24 | formation of translation preinitiation complex | RNA transcription , protein translation | CS score, function | |
| Eif3l | 223691 | EIF3L | 51386 | -1.28 | translational initiation | RNA transcription , protein translation | CS score, function | |
| Eif4a1 | 13681 | EIF4A1 | 1973 | -1.97 | translational initiation | RNA transcription , protein translation | CS score, function | |
| Eif4a3 | 192170 | EIF4A3 | 9775 | -4.32 | RNA splicing | RNA transcription , protein translation | CS score, function | |
| Eif4g1 | 208643 | EIF4G1 | 1981 | -1.79 | nuclear-transcribed mRNA catabolic process, nonsense-mediated decay | RNA transcription , protein translation | CS score, function | |
| Eif5b | 226982 | EIF5B | 9669 | -2.93 | translational initiation | RNA transcription , protein translation | CS score, function | |
| Eif6 | 16418 | EIF6 | 3692 | -2.75 | mature ribosome assembly | RNA transcription , protein translation | CS score, mouse K.O., function | Gandin V, et al. Nature. 2008 Oct 2;455(7213):684-8 |
| Elac2 | 68626 | ELAC2 | 60528 | -2.06 | tRNA 3'-trailer cleavage, endonucleolytic | RNA transcription , protein translation | CS score, function | |
| Ell | 13716 | ELL | 8178 | -2.23 | transcription elongation from RNA polymerase II promoter | RNA transcription , protein translation | CS score, mouse K.O., function | Mitani K, et al. Biochem Biophys Res Commun. 2000 Dec 20;279(2):563-7 |
| Etf1 | 225363 | ETF1 | 2107 | -2.44 | translational termination | RNA transcription , protein translation | CS score, function | |
| Exosc2 | 227715 | EXOSC2 | 23404 | -1.66 | exonucleolytic trimming to generate mature 3'-end of 5.8S rRNA from tricistronic rRNA transcript (SSU-rRNA, 5.8S rRNA, LSU-rRNA) | RNA transcription , protein translation | CS score, function | |
| Exosc4 | 109075 | EXOSC4 | 54512 | -3.21 | nuclear-transcribed mRNA catabolic process, deadenylation-dependent decay | RNA transcription , protein translation | CS score, function | |
| Exosc5 | 27998 | EXOSC5 | 56915 | -2.09 | rRNA catabolic process | RNA transcription , protein translation | CS score, function | |
| n/a | n/a | EXOSC6 | 118460 | -3.20 | nuclear-transcribed mRNA catabolic process, deadenylation-dependent decay | RNA transcription , protein translation | CS score, function | |
| Exosc7 | 66446 | EXOSC7 | 23016 | -2.17 | nuclear-transcribed mRNA catabolic process, deadenylation-dependent decay | RNA transcription , protein translation | CS score, function | |
| Exosc8 | 69639 | EXOSC8 | 11340 | -2.08 | nuclear-transcribed mRNA catabolic process, deadenylation-dependent decay | RNA transcription , protein translation | CS score, function | |
| Fars2 | 69955 | FARS2 | 10667 | -1.90 | tRNA aminoacylation for protein translation | RNA transcription , protein translation | CS score, function | |
| Farsa | 66590 | FARSA | 2193 | -3.30 | phenylalanyl-tRNA aminoacylation | RNA transcription , protein translation | CS score, function | |
| Farsb | 23874 | FARSB | 10056 | -2.49 | phenylalanyl-tRNA aminoacylation | RNA transcription , protein translation | CS score, function | |
| Fau | 14109 | FAU | 2197 | -2.64 | translation | RNA transcription , protein translation | CS score, function | |
| Fip1l1 | 66899 | FIP1L1 | 81608 | -1.93 | mRNA processing | RNA transcription , protein translation | CS score, function | |
| Ftsj3 | 56095 | FTSJ3 | 117246 | -1.50 | rRNA methylation | RNA transcription , protein translation | CS score, function | |
| Gle1 | 74412 | GLE1 | 2733 | -1.89 | mRNA export from nucleus | RNA transcription , protein translation | CS score, function | |
| Gnl3l | 237107 | GNL3L | 54552 | -1.35 | ribosome biogenesis | RNA transcription , protein translation | CS score, function | |
| Gtf2e1 | 74197 | GTF2E1 | 2960 | -1.22 | transcriptional open complex formation at RNA polymerase II promoter | RNA transcription , protein translation | CS score, function | |
| Gtpbp4 | 69237 | GTPBP4 | 23560 | -2.25 | ribosome biogenesis | RNA transcription , protein translation | CS score, function | |
| Hars | 15115 | HARS | 3035 | -3.49 | histidyl-tRNA aminoacylation | RNA transcription , protein translation | CS score, function | |
| Hars2 | 70791 | HARS2 | 23438 | -1.92 | histidyl-tRNA aminoacylation | RNA transcription , protein translation | CS score, function | |
| Heatr1 | 217995 | HEATR1 | 55127 | -2.58 | maturation of SSU-rRNA from tricistronic rRNA transcript (SSU-rRNA, 5.8S rRNA, LSU-rRNA) | RNA transcription , protein translation | CS score, function | |
| Hnrnpc | 15381 | HNRNPC | 3183 | -1.95 | mRNA splicing, via spliceosome | RNA transcription , protein translation | CS score, mouse K.O., function | Williamson DJ, et al. Mol Cell Biol. 2000 Jun;20(11):4094-105 |
| Hnrnpk | 15387 | HNRNPK | 3190 | -2.39 | mRNA splicing, via spliceosome | RNA transcription , protein translation | CS score, function | |
| Hnrnpl | 15388 | HNRNPL | 3191 | -1.88 | mRNA processing | RNA transcription , protein translation | CS score, mouse K.O., function | Gaudreau MC, et al. J Immunol. 2012 Jun 1;188(11):5377-88 |
| Hnrnpu | 51810 | HNRNPU | 3192 | -2.44 | mRNA splicing, via spliceosome | RNA transcription , protein translation | CS score, mouse K.O., function | Roshon MJ, et al. Transgenic Res. 2005 Apr;14(2):179-92 |
| lars | 105148 | IARS | 3376 | -3.87 | isoleucyl-tRNA aminoacylation | RNA transcription , protein translation | CS score, function | |
| lars2 | 381314 | IARS2 | 55699 | -2.83 | tRNA aminoacylation for protein translation | RNA transcription , protein translation | CS score, function | |
| Imp3 | 102462 | IMP3 | 55272 | -3.46 | rRNA processing | RNA transcription , protein translation | CS score, function | |
| Imp4 | 27993 | IMP4 | 92856 | -2.01 | rRNA processing | RNA transcription , protein translation | CS score, function | |
| Ints1 | 68510 | INTS1 | 26173 | -1.93 | snRNA processing | RNA transcription , protein translation | CS score, mouse K.O., function | Nakayama M, et al. FASEB J. 2006 Aug;20(10):1718-20 |
| Ints4 | 101861 | INTS4 | 92105 | -1.75 | snRNA processing | RNA transcription , protein translation | CS score, function | |
| Ints5 | 109077 | INTS5 | 80789 | -2.10 | snRNA processing | RNA transcription , protein translation | CS score, function | |
| Ints8 | 72656 | INTS8 | 55656 | -1.35 | snRNA processing | RNA transcription , protein translation | CS score, function | |
| Ints9 | 210925 | INTS9 | 55756 | -2.26 | snRNA processing | RNA transcription , protein translation | CS score, function | |
| Isg20l2 | 229504 | ISG20L2 | 81875 | -2.27 | ribosome biogenesis | RNA transcription , protein translation | CS score, function | |
| Kars | 85305 | KARS | 3735 | -2.76 | tRNA aminoacylation for protein translation | RNA transcription , protein translation | CS score, function | |
| n/a | n/a | KIAA0391 | 9692 | -1.56 | tRNA processing | RNA transcription , protein translation | CS score, function | |
| Lars | 107045 | LARS | 51520 | -1.83 | tRNA aminoacylation for protein translation | RNA transcription , protein translation | CS score, function | |
| Lars2 | 102436 | LARS2 | 23395 | -1.60 | tRNA aminoacylation for protein translation | RNA transcription , protein translation | CS score, function | |
| Las1l | 76130 | LAS1L | 81887 | -2.12 | rRNA processing | RNA transcription , protein translation | CS score, function | |
| Lrpprc | 72416 | LRPPRC | 10128 | -1.39 | negative regulation of mitochondrial RNA catabolic process | RNA transcription , protein translation | CS score, mouse K.O., function | Ruzzenente B, et al. EMBO J. 2012 Jan 18;31(2):443-56 |
| Lsm2 | 27756 | LSM2 | 57819 | -2.96 | nuclear-transcribed mRNA catabolic process, deadenylation-dependent decay | RNA transcription , protein translation | CS score, function | |
| Lsm3 | 67678 | LSM3 | 27258 | -1.66 | nuclear-transcribed mRNA catabolic process, deadenylation-dependent decay | RNA transcription , protein translation | CS score, function | |
| Lsm7 | 66094 | LSM7 | 51690 | -1.96 | nuclear-transcribed mRNA catabolic process, deadenylation-dependent decay | RNA transcription , protein translation | CS score, function | |
| Magoh | 17149 | MAGOH | 4116 | -1.78 | nuclear-transcribed mRNA catabolic process, nonsense-mediated decay | RNA transcription , protein translation | CS score, mouse K.O., function | Silver DL, et al. Nat Neurosci. 2010 May;13(5):551-8 |
| Mars | 216443 | MARS | 4141 | -3.24 | methionyl-tRNA aminoacylation | RNA transcription , protein translation | CS score, function | |
| Mars2 | 212679 | MARS2 | 92935 | -2.31 | tRNA aminoacylation for protein translation | RNA transcription , protein translation | CS score, function | |
| Med17 | 234959 | MED17 | 9440 | -1.78 | regulation of transcription from RNA polymerase II promoter | RNA transcription , protein translation | CS score, function | |
| Med20 | 56771 | MED20 | 9477 | -2.00 | regulation of transcription from RNA polymerase II promoter | RNA transcription , protein translation | CS score, function | |
| Med22 | 20933 | MED22 | 6837 | -1.86 | regulation of transcription from RNA polymerase II promoter | RNA transcription , protein translation | CS score, function | |
| Med27 | 68975 | MED27 | 9442 | -1.48 | regulation of transcription from RNA polymerase II promoter | RNA transcription , protein translation | CS score, function | |
| Med30 | 69790 | MED30 | 90390 | -2.21 | regulation of transcription from RNA polymerase II promoter | RNA transcription , protein translation | CS score, function | |
| Med8 | 80509 | MED8 | 112950 | -1.64 | regulation of transcription from RNA polymerase II promoter | RNA transcription , protein translation | CS score, function | |
| Mepce | 231803 | MEPCE | 56257 | -2.08 | negative regulation of transcription from RNA polymerase II promoter | RNA transcription , protein translation | CS score, function | |
| Mettl16 | 67493 | METTL16 | 79066 | -2.10 | rRNA base methylation | RNA transcription , protein translation | CS score, function | |
| Mphosph 10 | 67973 | MPHOSP H10 | 10199 | -1.85 | RNA splicing, via transesterification reactions | RNA transcription , protein translation | CS score, function | |
| Mrpl10 | 107732 | MRPL10 | 124995 | -1.38 | translation | RNA transcription , protein translation | CS score, function | |
| Mrpl12 | 56282 | MRPL12 | 6182 | -1.56 | translation | RNA transcription , protein translation | CS score, function | |
| Mrpl21 | 353242 | MRPL21 | 219927 | -1.91 | translation | RNA transcription , protein translation | CS score, function | |
| Mrpl28 | 68611 | MRPL28 | 10573 | -1.50 | translation | RNA transcription , protein translation | CS score, function | |
| Mrpl3 | 94062 | MRPL3 | 11222 | -1.58 | translation | RNA transcription , protein translation | CS score, function | |
| Mrpl34 | 94065 | MRPL34 | 64981 | -1.66 | translation | RNA transcription , protein translation | CS score, function | |
| Mrpl4 | 66163 | MRPL4 | 51073 | -2.41 | translation | RNA transcription , protein translation | CS score, function | |
| Mrpl41 | 107733 | MRPL41 | 64975 | -2.15 | translation | RNA transcription , protein translation | CS score, function | |
| Mrpl51 | 66493 | MRPL51 | 51258 | -1.40 | translation | RNA transcription , protein translation | CS score, function | |
| Mrps14 | 64659 | MRPS14 | 63931 | -1.82 | translation | RNA transcription , protein translation | CS score, function | |
| Mrps15 | 66407 | MRPS15 | 64960 | -1.28 | translation | RNA transcription , protein translation | CS score, function | |
| Mrps16 | 66242 | MRPS16 | 51021 | -2.29 | translation | RNA transcription , protein translation | CS score, function | |
| Mrps18a | 68565 | MRPS18 A | 55168 | -1.55 | translation | RNA transcription , protein translation | CS score, function | |
| Mrps2 | 118451 | MRPS2 | 51116 | -1.59 | translation | RNA transcription , protein translation | CS score, function | |
| Mrps21 | 66292 | MRPS21 | 54460 | -1.51 | translation | RNA transcription , protein translation | CS score, function | |
| Mrps24 | 64660 | MRPS24 | 64951 | -1.71 | translation | RNA transcription , protein translation | CS score, function | |
| Mrps6 | 121022 | MRPS6 | 64968 | -1.65 | translation | RNA transcription , protein translation | CS score, function | |
| Nars | 70223 | NARS | 4677 | -3.31 | tRNA aminoacylation for protein translation | RNA transcription , protein translation | CS score, function | |
| Nars2 | 244141 | NARS2 | 79731 | -1.32 | tRNA aminoacylation for protein translation | RNA transcription , protein translation | CS score, function | |
| Ncbp2 | 68092 | NCBP2 | 22916 | -3.00 | mRNA cis splicing, via spliceosome | RNA transcription , protein translation | CS score, function | |
| Nedd8 | 18002 | NEDD8 | 4738 | -2.45 | regulation of transcription from RNA polymerase II promoter | RNA transcription , protein translation | CS score, function | |
| Ngdn | 68966 | NGDN | 25983 | -2.35 | maturation of SSU-rRNA from tricistronic rRNA transcript (SSU-rRNA, 5.8S rRNA, LSU-rRNA) | RNA transcription , protein translation | CS score, function | |
| Nhp2 | 52530 | NHP2 | 55651 | -1.74 | rRNA pseudouridine synthesis | RNA transcription , protein translation | CS score, function | |
| Nip7 | 66164 | NIP7 | 51388 | -2.03 | ribosome assembly | RNA transcription , protein translation | CS score, function | |
| Noc2l | 57741 | NOC2L | 26155 | -2.34 | negative regulation of transcription from RNA polymerase II promoter | RNA transcription , protein translation | CS score, function | |
| Noc4l | 100608 | NOC4L | 79050 | -2.11 | ribosome biogenesis | RNA transcription , protein translation | CS score, function | |
| Nol6 | 230082 | NOL6 | 65083 | -2.28 | rRNA processing | RNA transcription, protein translation | CS score, function | |
| Nol9 | 74035 | NOL9 | 79707 | -2.20 | cleavage in ITS2 between 5.8S rRNA and LSU-rRNA of tricistronic rRNA transcript (SSU-rRNA, 5.8S rRNA, LSU-rRNA) | RNA transcription , protein translation | CS score, function | |
| Nop16 | 28126 | NOP16 | 51491 | -2.10 | ribosomal large subunit biogenesis | RNA transcription , protein translation | CS score, function | |
| Nop2 | 110109 | NOP2 | 4839 | -2.14 | rRNA processing | RNA transcription , protein translation | CS score, function | |
| Nop58 | 55989 | NOP58 | 51602 | -2.54 | rRNA modification | RNA transcription , protein translation | CS score, function | |
| Nsa2 | 59050 | NSA2 | 10412 | -1.78 | rRNA processing | RNA transcription , protein translation | CS score, function | |
| Nudt21 | 68219 | NUDT21 | 11051 | -2.36 | mRNA polyadenylation | RNA transcription , protein translation | CS score, function | |
| Osgep | 66246 | OSGEP | 55644 | -1.98 | tRNA processing | RNA transcription , protein translation | CS score, function | |
| Pabpn1 | 54196 | PABPN1 | 8106 | -1.92 | mRNA splicing, via spliceosome | RNA transcription , protein translation | CS score, function | |
| Pdcd11 | 18572 | PDCD11 | 22984 | -1.47 | rRNA processing | RNA transcription , protein translation | CS score, function | |
| Pes1 | 64934 | PES1 | 23481 | -2.92 | maturation of LSU-rRNA from tricistronic rRNA transcript (SSU-rRNA, 5.8S rRNA, LSU-rRNA) | RNA transcription , protein translation | CS score, mouse K.O., function | Lerch-Gaggl A, et al. J Biol Chem. 2002 Nov 22;277(47):45347-55 |
| Phb | 18673 | PHB | 5245 | -2.26 | regulation of transcription from RNA polymerase II promoter | RNA transcription , protein translation | CS score, mouse K.O., function | He B, et al. Endocrinology. 2011 Mar;152(3):1047-56 |
| Phf5a | 68479 | PHF5A | 84844 | -3.52 | mRNA splicing, via spliceosome | RNA transcription , protein translation | CS score, function | |
| Pnn | 18949 | PNN | 5411 | -1.34 | mRNA splicing, via spliceosome | RNA transcription , protein translation | CS score, mouse K.O., function | Joo JH, et al. Dev Dyn. 2007 Aug;236(8):2147-58 |
| Polr1b | 20017 | POLR1B | 84172 | -3.23 | transcription from RNA polymerase I promoter | RNA transcription , protein translation | CS score, mouse K.O., function | Chen H, et al. Biochem Biophys Res Commun. 2008 Jan 25;365(4):636-42 |
| Polr1c | 20016 | POLR1C | 9533 | -2.79 | transcription from RNA polymerase I promoter | RNA transcription , protein translation | CS score, function | |
| Polr2a | 20020 | POLR2A | 5430 | -3.15 | transcription from RNA polymerase II promoter | RNA transcription , protein translation | CS score, function | |
| Polr2b | 231329 | POLR2B | 5431 | -3.09 | transcription from RNA polymerase II promoter | RNA transcription , protein translation | CS score, function | |
| Polr2c | 20021 | POLR2C | 5432 | -3.15 | mRNA splicing, via spliceosome | RNA transcription , protein translation | CS score, function | |
| Polr2d | 69241 | POLR2D | 5433 | -2.23 | nuclear-transcribed mRNA catabolic process, deadenylation-dependent decay | RNA transcription , protein translation | CS score, function | |
| Polr2f | 69833 | POLR2F | 5435 | -2.31 | transcription from RNA polymerase I promoter | RNA transcription , protein translation | CS score, function | |
| Polr2 | 67710 | POLR2G | 5436 | -2.78 | nuclear-transcribed mRNA catabolic process, exonucleolytic | RNA transcription , protein translation | CS score, function | |
| Polr2h | 245841 | POLR2H | 5437 | -1.83 | transcription from RNA polymerase I promoter | RNA transcription , protein translation | CS score, function | |
| Polr2i | 69920 | POLR2I | 5438 | -2.92 | maintenance of transcriptional fidelity during DNA-templated transcription elongation from RNA polymerase II promoter | RNA transcription , protein translation | CS score, function | |
| Polr2j | 20022 | POLR2J | 5439 | -3.31 | mRNA splicing, via spliceosome | RNA transcription , protein translation | CS score, function | |
| Polr2l | 66491 | POLR2L | 5441 | -3.55 | mRNA splicing, via spliceosome | RNA transcription , protein translation | CS score, function | |
| Polr3e | 26939 | POLR3E | 55718 | -2.33 | transcription from RNA polymerase III promoter | RNA transcription , protein translation | CS score, function | |
| Pop1 | 67724 | POP1 | 10940 | -1.79 | tRNA 5'-leader removal | RNA transcription , protein translation | CS score, function | |
| Pop4 | 66161 | POP4 | 10775 | -1.87 | RNA phosphodiester bond hydrolysis | RNA transcription , protein translation | CS score, function | |
| Ppa1 | 67895 | PPA1 | 5464 | -1.63 | tRNA aminoacylation for protein translation | RNA transcription , protein translation | CS score, function | |
| Ppan | 235036 | PPAN | 56342 | -1.62 | ribosomal large subunit assembly | RNA transcription , protein translation | CS score, function | |
| Ppp2ca | 19052 | PPP2CA | 5515 | -3.01 | nuclear-transcribed mRNA catabolic process, nonsense-mediated decay | RNA transcription , protein translation | CS score, mouse K.O., function | Gu P, et al. Genesis. 2012 May;50(5):429-36 |
| Prim1 | 19075 | PRIM1 | 5557 | -2.07 | DNA replication, synthesis of RNA primer | RNA transcription , protein translation | CS score, function | |
| Prpf38b | 66921 | PRPF38B | 55119 | -2.68 | mRNA processing | RNA transcription , protein translation | CS score, function | |
| Prpf4 | 70052 | PRPF4 | 9128 | -2.24 | RNA splicing | RNA transcription , protein translation | CS score, function | |
| Prpf8 | 192159 | PRPF8 | 10594 | -3.43 | mRNA splicing, via spliceosome | RNA transcription , protein translation | CS score, function | |
| Ptcd1 | 71799 | PTCD1 | 26024 | -1.77 | tRNA 3'-end processing | RNA transcription , protein translation | CS score, function | |
| Pwp2 | 110816 | PWP2 | 5822 | -2.52 | ribosomal small subunit assembly | RNA transcription , protein translation | CS score, function | |
| Qars | 97541 | QARS | 5859 | -3.35 | tRNA aminoacylation for protein translation | RNA transcription , protein translation | CS score, function | |
| Ran | 19384 | RAN | 5901 | -3.09 | ribosomal large subunit export from nucleus | RNA transcription , protein translation | CS score, function | |
| Rars | 104458 | RARS | 5917 | -2.30 | tRNA aminoacylation for protein translation | RNA transcription , protein translation | CS score, function | |
| Rars2 | 109093 | RARS2 | 57038 | -1.93 | arginyl-tRNA aminoacylation | RNA transcription , protein translation | CS score, function | |
| Rbm25 | 67039 | RBM25 | 58517 | -2.15 | regulation of alternative mRNA splicing, via spliceosome | RNA transcription , protein translation | CS score, function | |
| Rbm8a | 60365 | RBM8A | 9939 | -2.97 | nuclear-transcribed mRNA catabolic process, nonsense-mediated decay | RNA transcription , protein translation | CS score, function | |
| Rbmx | 19655 | RBMX | 27316 | -1.95 | regulation of alternative mRNA splicing, via spliceosome | RNA transcription , protein translation | CS score, function | |
| Rcl1 | 59028 | RCL1 | 10171 | -2.08 | endonucleolytic cleavage of tricistronic rRNA transcript (SSU-rRNA, 5.8S rRNA, LSU-rRNA) | RNA transcription , protein translation | CS score, function | |
| Rngtt | 24018 | RNGTT | 8732 | -2.90 | transcription from RNA polymerase II promoter | RNA transcription , protein translation | CS score, function | |
| Rnmt | 67897 | RNMT | 8731 | -1.45 | 7-methylguanosine mRNA capping | RNA transcription , protein translation | CS score, function | |
| Rnpc3 | 67225 | RNPC3 | 55599 | -1.95 | mRNA splicing, via spliceosome | RNA transcription , protein translation | CS score, function | |
| Rpap1 | 68925 | RPAP1 | 26015 | -2.58 | transcription from RNA polymerase II promoter | RNA transcription , protein translation | CS score, function | |
| Rpl10 | 110954 | RPL10 | 6134 | -3.76 | translation | RNA transcription , protein translation | CS score, function | |
| Rpl10a | 19896 | RPL10A | 4736 | -2.15 | nuclear-transcribed mRNA catabolic process, nonsense-mediated decay | RNA transcription , protein translation | CS score, function | |
| Rpl11 | 67025 | RPL11 | 6135 | -2.99 | translation | RNA transcription , protein translation | CS score, function | |
| Rpl12 | 269261 | RPL12 | 6136 | -2.64 | ribosomal large subunit assembly | RNA transcription , protein translation | CS score, function | |
| Rpl13 | 270106 | RPL13 | 6137 | -3.28 | translation | RNA transcription , protein translation | CS score, function | |
| Rpl14 | 67115 | RPL14 | 9045 | -2.92 | nuclear-transcribed mRNA catabolic process, nonsense-mediated decay | RNA transcription , protein translation | CS score, function | |
| Rpl15 | 66480 | RPL15 | 6138 | -3.50 | translation | RNA transcription , protein translation | CS score, function | |
| Rpl18 | 19899 | RPL18 | 6141 | -3.72 | translation | RNA transcription , protein translation | CS score, function | |
| Rpl18a | 76808 | RPL18A | 6142 | -3.37 | translation | RNA transcription , protein translation | CS score, function | |
| Rpl23 | 65019 | RPL23 | 9349 | -3.02 | translation | RNA transcription , protein translation | CS score, function | |
| n/a | n/a | RPL23A | 6147 | -4.25 | translation | RNA transcription , protein translation | CS score, function | |
| Rpl24 | 68193 | RPL24 | 6152 | -2.55 | ribosomal large subunit assembly | RNA transcription , protein translation | CS score, mouse K.O., function | Oliver ER, et al. Development. 2004 Aug;131(16):3907-20 |
| Rpl26 | 19941 | RPL26 | 6154 | -2.88 | translation | RNA transcription , protein translation | CS score, function | |
| Rpl27 | 19942 | RPL27 | 6155 | -2.25 | translation | RNA transcription , protein translation | CS score, function | |
| Rpl27a | 26451 | RPL27A | 6157 | -2.87 | translation | RNA transcription , protein translation | CS score, mouse K.O., function | Terzian T, et al. J Pathol. 2011 Aug;224(4):540-52 |
| Rpl3 | 27367 | RPL3 | 6122 | -3.27 | ribosomal large subunit assembly | RNA transcription , protein translation | CS score, function | |
| Rpl30 | 19946 | RPL30 | 6156 | -2.53 | nuclear-transcribed mRNA catabolic process, nonsense-mediated decay | RNA transcription , protein translation | CS score, function | |
| Rpl31 | 114641 | RPL31 | 6160 | -1.92 | translation | RNA transcription , protein translation | CS score, function | |
| Rpl32 | 19951 | RPL32 | 6161 | -3.70 | nuclear-transcribed mRNA catabolic process, nonsense-mediated decay | RNA transcription , protein translation | CS score, function | |
| n/a | n/a | RPL34 | 6164 | -2.37 | nuclear-transcribed mRNA catabolic process, nonsense-mediated decay | RNA transcription , protein translation | CS score, function | |
| Rpl35 | 66489 | RPL35 | 11224 | -2.25 | nuclear-transcribed mRNA catabolic process, nonsense-mediated decay | RNA transcription , protein translation | CS score, function | |
| Rpl35a | 57808 | RPL35A | 6165 | -3.20 | translation | RNA transcription , protein translation | CS score, function | |
| Rpl36 | 54217 | RPL36 | 25873 | -3.44 | nuclear-transcribed mRNA catabolic process, nonsense-mediated decay | RNA transcription , protein translation | CS score, function | |
| Rpl37 | 67281 | RPL37 | 6167 | -3.02 | translation | RNA transcription , protein translation | CS score, function | |
| Rpl37a | 19981 | RPL37A | 6168 | -2.62 | nuclear-transcribed mRNA catabolic process, nonsense-mediated decay | RNA transcription , protein translation | CS score, function | |
| Rpl38 | 67671 | RPL38 | 6169 | -2.57 | translation | RNA transcription , protein translation | CS score, mouse K.O., function | MORGAN WC, et al. J Hered. 1950 Aug;41(8)208-15 |
| Rpl4 | 67891 | RPL4 | 6124 | -2.67 | nuclear-transcribed mRNA catabolic process, nonsense-mediated decay | RNA transcription , protein translation | CS score, function | |
| Rpl5 | 100503 670 | RPL5 | 6125 | -3.20 | translation | RNA transcription , protein translation | CS score, function | |
| Rpl6 | 19988 | RPL6 | 6128 | -3.07 | translation | RNA transcription , protein translation | CS score, function | |
| Rpl7 | 19989 | RPL7 | 6129 | -2.15 | nuclear-transcribed mRNA catabolic process, nonsense-mediated decay | RNA transcription , protein translation | CS score, function | |
| Rpl7a | 27176 | RPL7A | 6130 | -3.45 | ribosome biogenesis | RNA transcription , protein translation | CS score, function | |
| Rpl7l1 | 66229 | RPL7L1 | 285855 | -1.86 | maturation of LSU-rRNA from tricistronic rRNA transcript (SSU-rRNA, 5.8S rRNA, LSU-rRNA) | RNA transcription , protein translation | CS score, function | |
| Rpl8 | 26961 | RPL8 | 6132 | -4.00 | translation | RNA transcription , protein translation | CS score, function | |
| Rpl9 | 20005 | RPL9 | 6133 | -3.57 | translation | RNA transcription , protein translation | CS score, function | |
| Rplp0 | 11837 | RPLP0 | 6175 | -2.61 | nuclear-transcribed mRNA catabolic process, nonsense-mediated decay | RNA transcription , protein translation | CS score, function | |
| Rpp21 | 67676 | RPP21 | 79897 | -2.96 | tRNA processing | RNA transcription , protein translation | CS score, function | |
| Rpp30 | 54364 | RPP30 | 10556 | -1.79 | tRNA processing | RNA transcription , protein translation | CS score, function | |
| Rps10 | 67097 | RPS10 | 6204 | -2.88 | ribosomal small subunit assembly | RNA transcription , protein translation | CS score, function | |
| Rps11 | 27207 | RPS11 | 6205 | -2.93 | translation | RNA transcription , protein translation | CS score, function | |
| Rps12 | 20042 | RPS12 | 6206 | -3.33 | nuclear-transcribed mRNA catabolic process, nonsense-mediated decay | RNA transcription , protein translation | CS score, function | |
| Rps13 | 68052 | RPS13 | 6207 | -3.13 | translation | RNA transcription , protein translation | CS score, function | |
| n/a | n/a | RPS14 | 6208 | -3.18 | translation | RNA transcription , protein translation | CS score, function | |
| Rps15 | 20054 | RPS15 | 6209 | -3.20 | ribosomal small subunit assembly | RNA transcription , protein translation | CS score, function | |
| Rps15a | 267019 | RPS15A | 6210 | -3.18 | translation | RNA transcription , protein translation | CS score, function | |
| Rps16 | 20055 | RPS16 | 6217 | -2.35 | translation | RNA transcription , protein translation | CS score, function | |
| Rps17 | 20068 | RPS17 | 6218 | -2.69 | ribosomal small subunit assembly | RNA transcription , protein translation | CS score, function | |
| Rps19 | 20085 | RPS19 | 6223 | -3.49 | translation | RNA transcription , protein translation | CS score, mouse K.O., function | Matsson H, et al. Mol Cell Biol. 2004 May;24(9):4032-7 |
| Rps2 | 16898 | RPS2 | 6187 | -2.50 | translation | RNA transcription , protein translation | CS score, function | |
| Rps21 | 66481 | RPS21 | 6227 | -1.84 | nuclear-transcribed mRNA catabolic process, nonsense-mediated decay | RNA transcription , protein translation | CS score, function | |
| Rps23 | 66475 | RPS23 | 6228 | -2.86 | translation | RNA transcription , protein translation | CS score, function | |
| Rps25 | 75617 | RPS25 | 6230 | -2.38 | nuclear-transcribed mRNA catabolic process, nonsense-mediated decay | RNA transcription , protein translation | CS score, function | |
| n/a | n/a | RPS3A | 6189 | -3.72 | translation | RNA transcription , protein translation | CS score, function | |
| Rps4x | 20102 | RPS4X | 6191 | -3.04 | translation | RNA transcription , protein translation | CS score, function | |
| Rps5 | 20103 | RPS5 | 6193 | -2.61 | translation | RNA transcription , protein translation | CS score, function | |
| Rps6 | 20104 | RPS6 | 6194 | -3.31 | translation | RNA transcription , protein translation | CS score, function | |
| Rps7 | 20115 | RPS7 | 6201 | -2.97 | nuclear-transcribed mRNA catabolic process, nonsense-mediated decay | RNA transcription , protein translation | CS score, function | |
| Rps8 | 20116 | RPS8 | 6202 | -3.44 | nuclear-transcribed mRNA catabolic process, nonsense-mediated decay | RNA transcription , protein translation | CS score, function | |
| Rps9 | 76846 | RPS9 | 6203 | -3.16 | translation | RNA transcription , protein translation | CS score, function | |
| Rpsa | 16785 | RPSA | 3921 | -3.06 | ribosomal small subunit assembly | RNA transcription , protein translation | CS score, mouse K.O., function | Han J, et al. MGI Direct Data Submission. 2008 |
| Rsl24d1 | 225215 | RSL24D1 | 51187 | -2.76 | translation | RNA transcription , protein translation | CS score, function | |
| Sars | 20226 | SARS | 6301 | -2.67 | tRNA aminoacylation for protein translation | RNA transcription , protein translation | CS score, function | |
| Sars2 | 71984 | SARS2 | 54938 | -2.25 | seryl-tRNA aminoacylation | RNA transcription , protein translation | CS score, function | |
| Sart1 | 20227 | SART1 | 9092 | -2.13 | maturation of 5S rRNA | RNA transcription , protein translation | CS score, function | |
| Sart3 | 53890 | SART3 | 9733 | -1.88 | RNA processing | RNA transcription , protein translation | CS score, function | |
| Sdad1 | 231452 | SDAD1 | 55153 | -1.96 | ribosomal large subunit export from nucleus | RNA transcription , protein translation | CS score, function | |
| Sf1 | 22668 | SF1 | 7536 | -3.04 | mRNA splicing, via spliceosome | RNA transcription , protein translation | CS score, mouse K.O., function | Shitashige M, et al. Cancer Sci. 2007 Dec;98(12):1862-7 |
| Sf3a1 | 67465 | SF3A1 | 10291 | -3.18 | mRNA 3'-splice site recognition | RNA transcription , protein translation | CS score, function | |
| Sf3a2 | 20222 | SF3A2 | 8175 | -2.66 | mRNA 3'-splice site recognition | RNA transcription , protein translation | CS score, function | |
| Sf3a3 | 75062 | SF3A3 | 10946 | -2.26 | RNA splicing, via transesterification reactions | RNA transcription, protein translation | CS score, function | |
| Sf3b2 | 319322 | SF3B2 | 10992 | -2.51 | mRNA splicing, via spliceosome | RNA transcription, protein translation | CS score, function | |
| Sf3b3 | 101943 | SF3B3 | 23450 | -4.13 | RNA splicing, via transesterification reactions | RNA transcription, protein translation | CS score, function | |
| Sf3b4 | 107701 | SF3B4 | 10262 | -2.60 | RNA splicing, via transesterification reactions | RNA transcription, protein translation | CS score, function | |
| Sfpq | 71514 | SFPQ | 6421 | -2.27 | negative regulation of transcription from RNA polymerase II promoter | RNA transcription, protein translation | CS score, function | |
| Sin3a | 20466 | SIN3A | 25942 | -1.74 | negative regulation of transcription from RNA polymerase II promoter | RNA transcription, protein translation | CS score, mouse K.O., function | Dannenberg JH, et al. Genes Dev. 2005 Jul 1;19(13):1581-95 |
| Smg5 | 229512 | SMG5 | 23381 | -2.35 | nuclear-transcribed mRNA catabolic process, nonsensemediated decay | RNA transcription, protein translation | CS score, function | |
| Smg6 | 103677 | SMG6 | 23293 | -1.18 | nuclear-transcribed mRNA catabolic process, nonsensemediated decay | RNA transcription , protein translation | CS score, function | |
| Snrnp25 | 78372 | SNRNP2 5 | 79622 | -2.43 | mRNA processing | RNA transcription, protein translation | CS score, function | |
| Snrnp27 | 66618 | SNRNP2 7 | 11017 | -1.36 | mRNA processing | RNA transcription, protein translation | CS score, function | |
| Snrpd2 | 107686 | SNRPD2 | 6633 | -2.47 | RNA splicing | RNA transcription, protein translation | CS score, function | |
| Snrpf | 69878 | SNRPF | 6636 | -3.58 | mRNA splicing, via spliceosome | RNA transcription, protein translation | CS score, function | |
| Srrm1 | 51796 | SRRM1 | 10250 | -1.81 | mRNA processing | RNA transcription, protein translation | CS score, function | |
| Srsf1 | 110809 | SRSF1 | 6426 | -2.75 | mRNA 5'-splice site recognition | RNA transcription, protein translation | CS score, mouse K.O., function | Xu X, et al. Cell. 2005 Jan 14;120(1):59-72 |
| Srsf2 | 20382 | SRSF2 | 6427 | -3.66 | regulation of alternative mRNA splicing, via spliceosome | RNA transcription , protein translation | CS score, mouse K.O., function | Ding JH, et al. EMBO J. 2004 Feb 25;23(4):885-96 |
| Srsf3 | 20383 | SRSF3 | 6428 | -2.28 | mRNA splicing, via spliceosome | RNA transcription, protein translation | CS score, mouse K.O., function | Jumaa H, et al. Curr Biol. 1999 Aug 26;9(16):899-902 |
| Srsf7 | 225027 | SRSF7 | 6432 | -2.06 | mRNA splicing, via spliceosome | RNA transcription, protein translation | CS score, function | |
| Ssu72 | 68991 | SSU72 | 29101 | -2.57 | mRNA polyadenylation | RNA transcription, protein translation | CS score, function | |
| Sugp1 | 70616 | SUGP1 | 57794 | -1.36 | RNA processing | RNA transcription, protein translation | CS score, function | |
| Tars | 110960 | TARS | 6897 | -2.53 | tRNA aminoacylation for protein translation | RNA transcription, protein translation | CS score, function | |
| Tars2 | 71807 | TARS2 | 80222 | -1.91 | threonyl-tRNA aminoacylation | RNA transcription, protein translation | CS score, function | |
| Tbl3 | 213773 | TBL3 | 10607 | -2.41 | maturation of SSU-rRNA from tricistronic rRNA transcript (SSU-rRNA, 5.8S rRNA, LSU-rRNA) | RNA transcription, protein translation | CS score, function | |
| Thoc2 | 331401 | THOC2 | 57187 | -2.52 | mRNA processing | RNA transcription, protein translation | CS score, function | |
| Thoc5 | 107829 | THOC5 | 8563 | -1.57 | mRNA processing | RNA transcription, protein translation | CS score, mouse K.O., function | Mancini A, et al. BMC Biol. 2010;8:1 |
| Thoc7 | 66231 | THOC7 | 80145 | -2.23 | mRNA processing | RNA transcription, protein translation | CS score, function | |
| Timeless | 21853 | TIMELES S | 8914 | -2.27 | negative regulation of transcription from RNA polymerase II promoter | RNA transcription, protein translation | CS score, mouse K.O., function | Gotter AL, et al. Nat Neurosci. 2000 Aug;3(8):755-6 |
| Tsen2 | 381802 | TSEN2 | 80746 | -1.41 | tRNA-type intron splice site recognition and cleavage | RNA transcription, protein translation | CS score, function | |
| Tsr1 | 104662 | TSR1 | 55720 | -1.76 | ribosome biogenesis | RNA transcription, protein translation | CS score, function | |
| Tsr2 | 69499 | TSR2 | 90121 | -2.82 | maturation of SSU-rRNA from tricistronic rRNA transcript (SSU-rRNA, 5.8S rRNA, LSU-rRNA) | RNA transcription, protein translation | CS score, function | |
| Tufm | 233870 | TUFM | 7284 | -1.92 | translational elongation | RNA transcription, protein translation | CS score, function | |
| Tut1 | 70044 | TUT1 | 64852 | -2.65 | mRNA polyadenylation | RNA transcription, protein translation | CS score, function | |
| Twistnb | 28071 | TWISTNB | 221830 | -2.17 | transcription from RNA polymerase I promoter | RNA transcription, protein translation | CS score, function | |
| U2af1 | 108121 | U2AF1 | 7307 | -2.41 | mRNA splicing, via spliceosome | RNA transcription, protein translation | CS score, function | |
| U2af2 | 22185 | U2AF2 | 11338 | -2.80 | mRNA processing | RNA transcription , protein translation | CS score, function | |
| Uba52 | 22186 | UBA52 | 7311 | -2.54 | translation | RNA transcription, protein translation | CS score, function | |
| Ubl5 | 66177 | UBL5 | 59286 | -2.56 | mRNA splicing, via spliceosome | RNA transcription, protein translation | CS score, function | |
| Upf1 | 19704 | UPF1 | 5976 | -2.63 | nuclear-transcribed mRNA catabolic process, nonsensemediated decay | RNA transcription, protein translation | CS score, mouse K.O., function | Medghalchi SM, et al. Hum Mol Genet. 2001 Jan 15;10(2):99-105 |
| Upf2 | 326622 | UPF2 | 26019 | -2.16 | nuclear-transcribed mRNA catabolic process, nonsensemediated decay | RNA transcription, protein translation | CS score, mouse K.O., function | Weischenfeldt J, et al. Genes Dev. 2008 May 15;22(10):1381-96 |
| Utp15 | 105372 | UTP15 | 84135 | -1.65 | maturation of SSU-rRNA from tricistronic rRNA transcript (SSU-rRNA, 5.8S rRNA, LSU-rRNA) | RNA transcription, protein translation | CS score, function | |
| Utp20 | 70683 | UTP20 | 27340 | -2.28 | endonucleolytic cleavage in ITS1 to separate SSU-rRNA from 5.8S rRNA and LSU-rRNA from tricistronic rRNA transcript (SSU-rRNA, 5.8S rRNA, LSU-rRNA) | RNA transcription, protein translation | CS score, function | |
| Utp23 | 78581 | UTP23 | 84294 | -2.54 | rRNA processing | RNA transcription, protein translation | CS score, function | |
| Utp3 | 65961 | UTP3 | 57050 | -1.58 | maturation of SSU-rRNA from tricistronic rRNA transcript (SSU-rRNA, 5.8S rRNA, LSU-rRNA) | RNA transcription, protein translation | CS score, function | |
| Utp6 | 216987 | UTP6 | 55813 | -1.99 | maturation of SSU-rRNA from tricistronic rRNA transcript (SSU-rRNA, 5.8S rRNA, LSU-rRNA) | RNA transcription, protein translation | CS score, function | |
| Vars | 22321 | VARS | 7407 | -3.35 | tRNA aminoacylation for protein translation | RNA transcription, protein translation | CS score, function | |
| Wars | 22375 | WARS | 7453 | -2.22 | tryptophanyl-tRNA aminoacylation | RNA transcription, protein translation | CS score, function | |
| Wdr12 | 57750 | WDR12 | 55759 | -2.16 | maturation of LSU-rRNA from tricistronic rRNA transcript (SSU-rRNA, 5.8S rRNA, LSU-rRNA) | RNA transcription, protein translation | CS score, function | |
| Wdr3 | 269470 | WDR3 | 10885 | -2.65 | maturation of SSU-rRNA from tricistronic rRNA transcript (SSU-rRNA, 5.8S rRNA, LSU-rRNA) | RNA transcription, protein translation | CS score, function | |
| Wdr33 | 74320 | WDR33 | 55339 | -2.63 | mRNA polyadenylation | RNA transcription, protein translation | CS score, function | |
| Wdr36 | 225348 | WDR36 | 134430 | -2.04 | rRNA processing | RNA transcription, protein translation | CS score, mouse K.O., function | Gallenberger M, et al. Hum Mol Genet. 2011 Feb 1;20(3):422-35 |
| Wdr46 | 57315 | WDR46 | 9277 | -2.41 | maturation of SSU-rRNA from tricistronic rRNA transcript (SSU-rRNA, 5.8S rRNA, LSU-rRNA) | RNA transcription, protein translation | CS score, function | |
| Wdr61 | 66317 | WDR61 | 80349 | -2.63 | nuclear-transcribed mRNA catabolic process, exonucleolytic, 3'-5' | RNA transcription, protein translation | CS score, function | |
| Wdr75 | 73674 | WDR75 | 84128 | -2.12 | regulation of transcription from RNA polymerase II promoter | RNA transcription, protein translation | CS score, function | |
| Xpo1 | 103573 | XPO1 | 7514 | -3.50 | ribosomal large subunit export from nucleus | RNA transcription, protein translation | CS score, function | |
| Yars | 107271 | YARS | 8565 | -2.78 | tRNA aminoacylation for protein translation | RNA transcription, protein translation | CS score, function | |
| Yars2 | 70120 | YARS2 | 51067 | -2.40 | translation | RNA transcription, protein translation | CS score, function | |
| Ythdc1 | 231386 | YTHDC1 | 91746 | -2.35 | mRNA splice site selection | RNA transcription, protein translation | CS score, function | |
| Zbtb8os | 67106 | ZBTB8O S | 339487 | -2.54 | tRNA splicing, via endonucleolytic cleavage and ligation | RNA transcription, protein translation | CS score, function | |
| Zc3h3 | 223642 | ZC3H3 | 23144 | -1.22 | mRNA polyadenylation | RNA transcription, protein translation | CS score, function | |

### Documents Cited

Rossant J, Nagy A. Nature Biotechnology, Jan., pp. 23. (1999)
Thomson JA et al. Science. 282(5391):1145. (1998)
Takahashi K, Yamanaka S. Cell. 126(4):663. (2006)
Takahashi K et al. Cell. 131(5):861. (2007)
Hussein SM et al. Nature. 471(7336):58. (2011)
Laurent LC et al. Stem Cell. 8(1):106. (2011)
Lister R et al. Nature. 471(7336):68. (2011)
Yoshida Y, Yamanaka S. Circulation. 122(1):80. (2010)
Choo AB et al. STEM CELLS. 26(6):1454. (2008)
Tan HL et al. STEM CELLS. 27(8):1792. (2009)
Ben-David U, Nudel N, Benvenisty N. Nature Communications. 4:. (2013a)
Fong CY, Peh GSL, Gauthaman K, Bongso A. Stem Cell Rev and Rep. 5(1):72. (2009)
Tang C et al. Nature Biotechnology. 29(9):829. (2011)
Blum B, Bar-Nur O, Golan-Lev T, Benvenisty N. Nature Biotechnology. 27(3):281. (2009)
Menendez S et al. Aging Cell. 11(1):41. (2012)
Chung S et al. J Neurochem. 97(5):1467. (2006)
Eiges R et al. Current Biology. 11(7):514. (2001)
Huber I et al. The FASEB Journal. 21(10):2551. (2007)
Rong Z, Fu X, Wang M, Xu Y. Journal of Biological Chemistry. 287(39):32338. (2012)
Schuldiner M, Itskovitz-Eldor J, Benvenisty N. STEM CELLS. 21(3):257. (2003)
Ben-David U et al. Cell Stem Cell. 12(2):167. (2013b)
Dabir DV et al. Developmental Cell. 25(1):81. (2013)
Tohyama S et al. Stem Cell. 12(1):127. (2013)
Ghosh Z et al. Cancer Research. 71(14):5030. (2011)
Ben-David U, Benvenisty N. Nat Protoc. 9(3):729. (2014)
Lim T-T et al. PLoS ONE. 8(7):e70543. (2013)
Halloran PJ, Fenton RG. Cancer Research. 58(17):3855. (1998)
Tomicic MT, Thust R, Kaina B. Oncogene. 21(14):2141. (2002)
Di Stasi A et al. N Engl J Med. 365(18):1673. (2011)
Santamaría D et al. Nature. 448(7155):811. (2007)
Diril MK et al. Proceedings of the National Academy of Sciences. 109(10):3826. (2012)
Scognamiglio R et al. Cell. 164(4):668. (2016)
Fernandez-Miranda G et al. Development. 138(13):2661. (2011)
Pellettieri J, Sánchez Alvarado A. Annu. Rev. Genet. 41:83. (2007)
Bianconi E et al. Ann. Hum. Biol. 40(6):463. (2013)
Wang T et al. Science. 350(6264):1096. (2015)
Morgan DO. (2007)
FUNG T, POON R. Seminars in Cell and Developmental Biology. 16(3):335. (2005)
Peters J-M. Mol Cell. 9(5):931. (2002)
Fukagawa T. Front Biosci. 13(13):2705. (2007)
Cassimeris L. Current Opinion in Cell Biology. 11(1):134. (1999)
Satyanarayana A et al. Development. 135(20):3389. (2008)
Kittler R et al. Nature. 432(7020):1036. (2004)
Harborth J et al. J. Cell. Sci. 114(Pt 24):4557. (2001)
Silk AD, Holland AJ, Cleveland DW. The Journal of Cell Biology. 184(5):677. (2009)
Oda H et al. Molecular and Cellular Biology. 29(8):2278. (2009)
Liu Q et al. Genes & Development. 14(12):1448. (2000)
Martin-McCaffrey L et al. Developmental Cell. 7(5):763. (2004)
Uren AG et al. Curr. Biol. 10(21):1319. (2000)
Leung CG et al. J Exp Med. 204(7):1603. (2007)
Cutts SM et al. Human Molecular Genetics. 8(7):1145. (1999)
Howman EV et al. Proceedings of the National Academy of Sciences. 97(3):1148. (2000)
Chauvière M, Kress C, Kress M. Biochem Biophys Res Commun. 372(4):513. (2008)
Miura K et al. Biochem Biophys Res Commun. 341(1):132. (2006)
Magnaghi P et al. Nat Chem Biol. 9(9):548. (2013)
Smith ED et al. Molecular and Cellular Biology. 24(3):1168. (2004)
Morham SG, Kluckman KD, Voulomanos N, Smithies O. Molecular and Cellular Biology. 16(12):6804. (1996)
Akimitsu N et al. Genes Cells. 8(4):393. (2003)
Jeganathan K et al. The Journal of Cell Biology. 179(2):255. (2007)
Ono T et al. Cell. 115(1):109. (2003)
Bharadwaj R, Qi W, Yu H. J. Biol. Chem. 279(13):13076. (2004)
Parisi G, Fornasari MS, Echave J. FEBS Lett. 562(1-3):1. (2004)
Harborth J, Weber K, Osborn M. J. Biol. Chem. 275(41):31979. (2000)
Krull S et al. Mol. Biol. Cell. 15(9):4261. (2004)
Topper LM et al. Cell Cycle. 1(4):282. (2002)
Zhang Y-W et al. J. Biol. Chem. 284(27):18085. (2009)
Simmons AD et al. Human Molecular Genetics. 8(12):2155. (1999)
Plate M et al. Mol Cells. 29(4):355. (2010)
Franco M et al. Proceedings of the National Academy of Sciences. 95(17):9926. (1998)
Ohno M, Shimura Y. Genes & Development. 10(8):997. (1996)
Achsel T et al. EMBO J. 18(20):5789. (1999)
Shichijo S et al. J Exp Med. 187(3):277. (1998)
Jurica MS et al. RNA. 8(4):426. (2002)
Chari A et al. Cell. 135(3):497. (2008)
Xu C et al. J. Biol. Chem. 281(23):15900. (2006)
Yeh H et al. Genomics. 23(2):443. (1994)
Kokkola T et al. FEBS Lett. 585(17):2665. (2011)
Chan CC et al. RNA. 10(2):200. (2004)
Zhou M et al. Proceedings of the National Academy of Sciences. 105(47):18139. (2008)
Takatsu H et al. J. Biol. Chem. 273(38):24693. (1998)
Chen Y et al. Cell Research. 22(2):333. (2012)
Mansfeld J et al. Nat. Cell Biol. 13(10):1234. (2011)
He F, Wang C-T, Gou L-T. Genet. Mol. Res. 8(4):1466. (2009)
Lieu A-S et al. Int J Oncol. 37(2):429. (2010)
Preuβ E et al. Human Gene Therapy. 21(8):929. (2010)
Neschadim A et al. Molecular Therapy. 20(5):1002. (2012)
Hedley D, Ogilvie L, Springer C. Nat Rev Cancer. 7(11):870. (2007)
Nawa A et al. Anticancer Agents Med Chem. 8(2):232. (2008)
Danks MK et al. Cancer Research. 67(1):22. (2007)
Shah K. Adv. Drug Deliv. Rev. 64(8):739. (2012)
Ramnaraine M et al. Cancer Research. 63(20):6847. (2003)
Denny WA. J. Biomed. Biotechnol. 2003(1):48. (2003)
Ireton GC, McDermott G, Black ME, Stoddard BL. J. Mol. Biol. 315(4):687. (2002)
Humerickhouse R et al. Cancer Research. 60(5):1189. (2000)
Wierdl M et al. Cancer Research. 61(13):5078. (2001)
Kojima A, Hackett NR, Ohwada A, Crystal RG. J. Clin. Invest. 101(8):1789. (1998)
Straathof KC et al. Blood. 105(11):4247. (2005)
Soucek L et al. Cancer Research. 62(12):3507. (2002)
Oricchio E et al. CellReports, pp. 1. (2014)
Olive M et al. J. Biol. Chem. 272(30):18586. (1997)
Urlinger S et al. Proceedings of the National Academy of Sciences. 97(14):7963. (2000)
Agha-Mohammadi S et al. J Gene Med. 6(7):817. (2004)
Gossen M et al. Science. 268(5218):1766. (1995)
Banaszynski LA et al. Cell. 126(5):995. (2006)
Maeder ML et al. Nature Methods. 10(3):243. (2013)
Pollock R, Clackson T. Current Opinion in Biotechnology. 13(5):459. (2002)
Mullick A et al. BMC Biotechnology. 6(1):43. (2006)
No D, Yao TP, Evans RM. Proceedings of the National Academy of Sciences. 93(8):3346. (1996)
Stanley SA et al. Nature Medicine. 21(1):92. (2015)
Rollins CT et al. Proceedings of the National Academy of Sciences. 97(13):7096. (2000)
Cong L et al. Science. 339(6121):819. (2013)
Hsu PD et al. Nature Biotechnology. 31(9):827. (2013)
Ran FA et al. Nat Protoc. 8(11):2281. (2013)
Gertsenstein M et al. PLoS ONE. 5(6):e11260. (2010)
Adewumi O et al. Nature Biotechnology. 25(7):803. (2007)
Guy CT, Cardiff RD, Muller WJ. Molecular and Cellular Biology. 12(3):954. (1992)
Szymczak AL et al. Nature Biotechnology. 22(5):589. (2004)
Nagy A. et al. Manipulating the Mouse Embryo. (2003)

## Claims

1. A method of controlling proliferation of an animal cell, the method comprising:
a) genetically modifying one or more endogenous genes in the animal cell selected from the group consisting of Cdk1, Top2A, Cenpa, Birc5, and Eef2, the genetic modification comprising a DNA sequence encoding a negative selectable marker that is transcriptionally linked to a DNA sequence encoding each of the one or more genes, such that the negative selectable marker and the one or more genes are co-expressed; and
b) controlling proliferation of the genetically modified animal cell with an inducer of the negative selectable marker.

2. The method of claim 1, wherein the negative selectable marker comprises herpes simplex virus-thymidine kinase, cytosine deaminase, carboxyl esterase, or iCasp9, wherein preferably the negative selectable marker is herpes simplex virus-thymidine kinase.

3. The method of claim 2, wherein the gene is Cdk1 and the negative selectable marker is herpes simplex virus-thymidine kinase.

4. The method of any one of claims 1 to 3, wherein;
the controlling proliferation of the genetically modified animal cell comprises:
i) permitting proliferation of the cell by maintaining the cell in the absence of an inducer of the negative selectable marker; or
ii) ablating or inhibiting proliferation of the cell by exposing the cell to the inducer of the negative selectable marker.

5. The method of any one of claims 1 to 4, wherein the genetic modification of the one or more genes comprises performing targeted modification of each of the one or more genes with a DNA vector comprising DNA encoding the negative selectable marker.

6. The method of any one of claims 1 to 5, wherein the genetic modification is homozygous, heterozygous, hemizygous or compound heterozygous.

7. The method of claim 2, wherein:
i) the negative selectable marker comprises herpes simplex virus-thymidine kinase and the inducer comprises ganciclovir;
ii) the negative selectable marker comprises cytosine deaminase and the inducer comprises 5-fluorocytosine;
iii) the negative selectable marker comprises carboxyl esterase and the inducer comprises irinotecan; or
iv) the negative selectable marker comprises iCasp9 and the inducer comprises AP1903.

8. The method of any one of claims 1 to 7, wherein:
a) the animal cell is a mammalian cell selected from the group consisting of a human, mouse, rat, hamster, guinea pig, cat, dog, cow, horse, deer, elk, bison, oxen, camel, llama, rabbit, pig, goat, sheep, and non-human primate cell or is an avian cell; or
b) the animal cell is or is derived from a pluripotent stem cell a multipotent cell, or a monopotent progenitor cell, or is a terminally differentiated cell.

9. A method of controlling proliferation of an animal cell population according to the method of any one of claims 1 to 8.

10. An isolated, genetically modified animal cell comprising a genetic modification of one or more endogenous genes selected from the group consisting of Cdk1, Top2A, Cenpa, Birc5, and Eef2, wherein the genetic modification comprises a DNA sequence encoding a negative selectable marker that is transcriptionally linked to a DNA sequence encoding each of the one or more genes such that the negative selectable marker and the one or more genes are co-expressed, and wherein, in the presence of an inducer of the negative selectable marker, the cell is killed or its proliferation inhibited.

11. The isolated, genetically modified animal cell of claim 10, wherein the negative selectable marker comprises herpes simplex virus-thymidine kinase, cytosine deaminase, carboxyl esterase, or iCasp9, wherein preferably the negative selectable marker is herpes simplex virus-thymidine kinase.

12. The isolated, genetically modified animal cell of claim 11, wherein the essential gene is Cdk1 and the negative selectable marker is herpes simplex virus-thymidine kinase.

13. The isolated, genetically modified animal cell of any one of claims 10 to 12, wherein the genetic modification of the one or more genes was generated with a DNA vector comprising DNA encoding the negative selectable marker.

14. The isolated, genetically modified animal cell of any one of claims 10 to 14, wherein the genetic modification is homozygous, heterozygous, hemizygous or compound heterozygous.

15. The isolated, genetically modified animal cell of claim 11, wherein:
i) the negative selectable marker comprises herpes simplex virus-thymidine kinase and the inducer comprises ganciclovir;
ii) the negative selectable marker comprises cytosine deaminase and the inducer comprises 5-fluorocytosine;
iii) the negative selectable marker comprises carboxyl esterase and the inducer comprises irinotecan; or
iv) the negative selectable marker comprises iCasp9 and the inducer comprises AP1903.

16. The isolated, genetically modified animal cell of any one of claims 10 to 15, wherein:
a) the animal cell is a mammalian cell selected from the group consisting of a human, mouse, rat, hamster, guinea pig, cat, dog, cow, horse, deer, elk, bison, oxen, camel, llama, rabbit, pig, goat, sheep, and non-human primate cell or is an avian cell; or
b) the animal cell is or is derived from a pluripotent stem cell a multipotent cell, or a monopotent progenitor cell, or is a terminally differentiated cell.

17. A DNA vector for modifying expression of an endogenous gene selected from the group consisting of Cdk1, Top2A, Cenpa, Birc5, and Eef2 in an animal cell, comprising a DNA sequence encoding a negative selectable marker that is transcriptionally linked to a DNA sequence encoding the gene,
wherein integration of the DNA sequence encoding the negative selectable marker that is transcriptionally linked to a DNA sequence encoding the gene into one or more host cells promotes death of proliferating host cells upon exposure of the host cells to an inducer of the negative selectable marker.

18. The DNA vector of claim 17, wherein the negative selectable marker comprises herpes simplex virus-thymidine kinase, cytosine deaminase, carboxyl esterase, or iCasp9, wherein preferably the negative selectable marker is herpes simplex virus-thymidine kinase.

19. The DNA vector of claim 18, wherein the gene is Cdk1 and the negative selectable marker is herpes simplex virus-thymidine kinase.

20. The DNA vector of claim 18, wherein:
i) the negative selectable marker comprises herpes simplex virus-thymidine kinase and the inducer comprises ganciclovir;
ii) the negative selectable marker comprises cytosine deaminase and the inducer comprises 5-fluorocytosine;
iii) the negative selectable marker comprises carboxyl esterase and the inducer comprises irinotecan; or
iv) the negative selectable marker comprises iCasp9 and the inducer comprises AP1903.

21. The animal cell of any one of claims 10 to 16 for use in medicine.

## Patentansprüche

1. Verfahren zur Kontrolle der Proliferation einer Tierzelle, wobei das Verfahren Folgendes umfasst:
a) gentechnisches Verändern eines oder mehrerer endogener Gene in der Tierzelle, ausgewählt aus der Gruppe bestehend aus Cdkl, Top2A, Cenpa, Bires und Eef2, wobei die gentechnische Veränderung eine einen negativen selektierbaren Marker codierende DNA-Sequenz umfasst, die mit einer jedes der ein oder mehreren Gene codierenden DNA-Sequenz transkriptionell verknüpft ist, so dass der negative selektierbare Marker und die ein oder mehreren Gene coexprimiert werden; und
b) Kontrollieren der Proliferation der gentechnisch veränderten Tierzelle mit einem Induktor des negativen selektierbaren Markers.

2. Verfahren nach Anspruch 1, wobei der negative selektierbare Marker Herpes-simplex-Virus-Thymidinkinase, Cytosin-Deaminase, Carboxylesterase oder iCasp9 umfasst, wobei es sich bei dem negativen selektierbaren Marker bevorzugt um Herpes-simplex-Virus-Thymidinkinase handelt.

3. Verfahren nach Anspruch 2, wobei es sich bei dem Gen um Cdkl und dem negativen selektierbaren Marker um Herpes-simplex-Virus-Thymidinkinase handelt.

4. Verfahren nach einem der Ansprüche 1 bis 3, wobei; das Kontrollieren der Proliferation der gentechnisch veränderten Tierzelle Folgendes umfasst:
i) Erlauben der Proliferation der Zelle durch Halten der Zelle in Abwesenheit eines Induktors des negativen selektierbaren Markers; oder
ii) Ablation oder Hemmung der Proliferation der Zelle durch In-Kontakt-Bringen der Zelle mit dem Induktor des negativen selektierbaren Markers.

5. Verfahren nach einem der Ansprüche 1 bis 4, wobei die gentechnische Veränderung der ein oder mehreren Gene Durchführen einer gezielten Veränderung jedes der ein oder mehreren Gene mit einem den negativen selektierbaren Marker codierende DNA umfassenden DNA-Vektor umfasst.

6. Verfahren nach einem der Ansprüche 1 bis 5, wobei die gentechnische Veränderung homozygot, heterozygot, hemizygot oder verbindungsheterozygot ist.

7. Verfahren nach Anspruch 2, wobei:
i) der negative selektierbare Marker Herpes-simplex-Virus-Thymidinkinase und der Induktor Ganciclovir umfasst;
ii) der negative selektierbare Marker Cytosin-Deaminase und der Induktor 5-Fluorcytosin umfasst;
iii) der negative selektierbare Marker Carboxylesterase und der Induktor Irinotecan umfasst; oder
iv) der negative selektierbare Marker iCasp9 und der Induktor AP1903 umfasst.

8. Verfahren nach einem der Ansprüche 1 bis 7, wobei:
a) es sich bei der Tierzelle um eine Säugerzelle ausgewählt aus der Gruppe bestehend aus einer Zelle von Mensch, Maus, Ratte, Hamster, Meerschweinchen, Katze, Hund, Rind, Pferd, Hirsch, Elch, Bison, Ochsen, Kamel, Lama, Kaninchen, Schwein, Ziege, Schaf und nichtmenschlichem Primaten oder um eine Vogelzelle handelt; oder
b) es sich bei der Tierzelle um eine pluripotente Stammzelle, eine multipotente Zelle oder eine monopotente Progenitorzelle oder eine davon jeweils abgeleitete Zelle oder um eine ausdifferenzierte Zelle handelt.

9. Verfahren zur Kontrolle der Proliferation einer Tierzellpopulation gemäß dem Verfahren nach einem der Ansprüche 1 bis 8.

10. Isolierte, gentechnisch veränderte Tierzelle, umfassend eine gentechnische Veränderung eines oder mehrerer endogener Gene ausgewählt aus der Gruppe bestehend aus Cdkl, Top2A, Cenpa, Bires und Eef2, wobei die gentechnische Veränderung eine einen negativen selektierbaren Marker codierende DNA-Sequenz umfasst, die mit einer jedes der ein oder mehreren Gene codierenden DNA-Sequenz transkriptionell verknüpft ist, so dass der negative selektierbare Marker und die ein oder mehreren Gene coexprimiert werden, und wobei in Gegenwart eines Induktors des negativen selektierbaren Markers die Zelle abgetötet oder ihre Proliferation gehemmt wird.

11. Isolierte, gentechnisch veränderte Tierzelle nach Anspruch 10, wobei der negative selektierbare Marker Herpes-simplex-Virus-Thymidinkinase, Cytosin-Deaminase, Carboxylesterase oder iCasp9 umfasst, wobei es sich bei dem negativen selektierbaren Marker bevorzugt um Herpes-simplex-Virus-Thymidinkinase handelt.

12. Isolierte, gentechnisch veränderte Tierzelle nach Anspruch 11, wobei es sich bei dem essentiellen Gen um Cdkl und dem negativen selektierbaren Marker um Herpes-simplex-Virus-Thymidinkinase handelt.

13. Isolierte, gentechnisch veränderte Tierzelle nach einem der Ansprüche 10 bis 12, wobei die gentechnische Veränderung der ein oder mehreren Gene mit einem den negativen selektierbaren Marker codierende DNA umfassenden DNA-Vektor erzeugt wurde.

14. Isolierte, gentechnisch veränderte Tierzelle nach einem der Ansprüche 10 bis 14, wobei die gentechnische Veränderung homozygot, heterozygot, hemizygot oder verbindungsheterozygot ist.

15. Isolierte, gentechnisch veränderte Tierzelle nach Anspruch 11, wobei:
i) der negative selektierbare Marker Herpes-simplex-Virus-Thymidinkinase und der Induktor Ganciclovir umfasst;
ii) der negative selektierbare Marker Cytosin-Deaminase und der Induktor 5-Fluorcytosin umfasst;
iii) der negative selektierbare Marker Carboxylesterase und der Induktor Irinotecan umfasst; oder
iv) der negative selektierbare Marker iCasp9 und der Induktor AP1903 umfasst.

16. Isolierte, gentechnisch veränderte Tierzelle nach einem der Ansprüche 10 bis 15, wobei:
a) es sich bei der Tierzelle um eine Säugerzelle ausgewählt aus der Gruppe bestehend aus einer Zelle von Mensch, Maus, Ratte, Hamster, Meerschweinchen, Katze, Hund, Rind, Pferd, Hirsch, Elch, Bison, Ochsen, Kamel, Lama, Kaninchen, Schwein, Ziege, Schaf und nichtmenschlichem Primaten oder um eine Vogelzelle handelt; oder
b) es sich bei der Tierzelle um eine pluripotente Stammzelle, eine multipotente Zelle oder eine monopotente Progenitorzelle oder eine davon jeweils abgeleitete Zelle oder um eine ausdifferenzierte Zelle handelt.

17. DNA-Vektor zum Verändern der Expression eines endogenen Gens ausgewählt aus der Gruppe bestehend aus Cdkl, Top2A, Cenpa, Bires und Eef2 in einer Tierzelle, umfassend eine einen negativen selektierbaren Marker codierende DNA-Sequenz, die mit einer das Gen codierenden DNA-Sequenz transkriptionell verknüpft ist,
wobei Integration der den negativen selektierbaren Marker codierenden DNA-Sequenz, die mit einer das Gen codierenden DNA-Sequenz transkriptionell verknüpft ist, in eine oder mehrere Wirtszellen den Tod proliferierender Wirtszellen nach In-Kontakt-Bringen der Wirtszellen mit einem Induktor des negativen selektierbaren Markers fördert.

18. DNA-Vektor nach Anspruch 17, wobei der negative selektierbare Marker Herpes-simplex-Virus-Thymidinkinase, Cytosin-Deaminase, Carboxylesterase oder iCasp9 umfasst, wobei es sich bei dem negativen selektierbaren Marker bevorzugt um Herpes-simplex-Virus-Thymidinkinase handelt.

19. DNA-Vektor nach Anspruch 18, wobei es sich bei dem Gen um Cdkl und dem negativen selektierbaren Marker um Herpes-simplex-Virus-Thymidinkinase handelt.

20. DNA-Vektor nach Anspruch 18, wobei:
i) der negative selektierbare Marker Herpes-simplex-Virus-Thymidinkinase und der Induktor Ganciclovir umfasst;
ii) der negative selektierbare Marker Cytosin-Deaminase und der Induktor 5-Fluorcytosin umfasst;
iii) der negative selektierbare Marker Carboxylesterase und der Induktor Irinotecan umfasst; oder
iv) der negative selektierbare Marker iCasp9 und der Induktor AP1903 umfasst.

21. Tierzelle nach einem der Ansprüche 10 bis 16 zur medizinischen Verwendung.

## Revendications

1. Procédé de maîtrise de la prolifération d'une cellule animale, le procédé comprenant :
a) la modification génétique d'un ou plusieurs gènes endogènes dans la cellule animale, choisis dans le groupe consistant en Cdkl, Top2A, Cenpa, Birc5 et Eef2, la modification génétique comprenant une séquence d'ADN codant pour un marqueur sélectionnable négatif qui est transcriptionnellement lié à une séquence d'ADN codant pour chacun du ou des gènes, de façon à coexprimer le marqueur sélectionnable négatif et le ou les gènes ; et
b) la maîtrise de la prolifération de la cellule animale génétiquement modifiée avec un inducteur du marqueur sélectionnable négatif.

2. Procédé selon la revendication 1, dans lequel le marqueur sélectionnable négatif comprend la thymidine kinase du virus herpès simplex, la cytosine désaminase, la carboxyl estérase ou l'iCasp9, le marqueur sélectionnable négatif étant de préférence la thymidine kinase du virus herpès simplex.

3. Procédé selon la revendication 2, dans lequel le gène est Cdkl et le marqueur sélectionnable négatif est la thymidine kinase du virus herpès simplex.

4. Procédé selon l'une quelconque des revendications 1 à 3, dans lequel :
la maîtrise de la prolifération de la cellule animale génétiquement modifiée comprend :
i) le fait de permettre la prolifération de la cellule en maintenant la cellule en l'absence d'inducteur du marqueur sélectionnable négatif ; ou
ii) l'ablation ou l'inhibition de la prolifération de la cellule par exposition de la cellule à l'inducteur du marqueur sélectionnable négatif.

5. Procédé selon l'une quelconque des revendications 1 à 4, dans lequel la modification génétique du ou des gènes comprend la mise en œuvre d'une modification ciblée de chacun du ou des gènes avec un vecteur à ADN comprenant un ADN codant pour le marqueur sélectionnable négatif.

6. Procédé selon l'une quelconque des revendications 1 à 5, dans lequel la modification génétique est homozygote, hétérozygote ou hémizygote composé.

7. Procédé selon la revendication 2, dans lequel :
i) le marqueur sélectionnable négatif comprend la thymidine kinase du virus herpès simplex et l'inducteur comprend le ganciclovir ;
ii) le marqueur sélectionnable négatif comprend la cytosine désaminase et l'inducteur comprend la 5-fluorocytosine ;
iii) le marqueur sélectionnable négatif comprend la carboxyl estérase et l'inducteur comprend l'irinotécan ; ou
iv) le marqueur sélectionnable négatif comprend l'iCasp9 et l'inducteur comprend l'AP1903.

8. Procédé selon l'une quelconque des revendications 1 à 7, dans lequel :
a) la cellule animale est une cellule de mammifère choisie dans le groupe consistant en une cellule d'un humain, de souris, de rat, de hamster, de cobaye, de chat, de chien, de vache, de cheval, de cerf, d'élan, de bison, de bœufs, de chameau, de lama, de lapin, de porc, de chèvre, de mouton, et de primate non humain, ou est une cellule aviaire ; ou
b) la cellule animale est ou dérive d'une cellule souche pluripotente, d'une cellule multipotente ou d'une cellule progénitrice monopotente, ou est une cellule à différenciation terminale.

9. Procédé de maîtrise de la prolifération d'une population de cellules animales, conformément au procédé selon l'une quelconque des revendications 1 à 8.

10. Cellule animale isolée génétiquement modifiée comprenant une modification génétique d'un ou plusieurs gènes endogènes choisis dans le groupe consistant en Cdkl, Top2A, Cenpa, Birc5 et Eef2, dans laquelle la modification génétique comprend une séquence d'ADN codant pour un marqueur sélectionnable négatif qui est transcriptionnellement lié à une séquence d'ADN codant pour chacun du ou des gènes, de façon à coexprimer le marqueur sélectionnable négatif et le ou les gènes, et dans laquelle, en présence d'un inducteur du marqueur sélectionnable négatif, la cellule est tuée ou sa prolifération est inhibée.

11. Cellule animale isolée génétiquement modifiée selon la revendication 10, dans laquelle le marqueur sélectionnable négatif comprend la thymidine kinase du virus de l'herpès simplex, la cytosine désaminase, la carboxyl estérase ou l'iCasp9, le marqueur sélectionnable négatif étant de préférence la thymidine kinase du virus de l'herpès simplex.

12. Cellule animale isolée génétiquement modifiée selon la revendication 11, dans laquelle le gène essentiel est Cdkl et le marqueur sélectionnable négatif est la thymidine kinase du virus de l'herpès simplex.

13. Cellule animale isolée génétiquement modifiée selon l'une quelconque des revendications 10 à 12, dans laquelle la modification génétique du ou des gènes est générée avec un vecteur à ADN comprenant un ADN codant pour le marqueur sélectionnable négatif.

14. Cellule animale isolée génétiquement modifiée selon l'une quelconque des revendications 10 à 14, dans laquelle la modification génétique est homozygote, hétérozygote, hémizygote ou hétérozygote composée.

15. Cellule animale isolée génétiquement modifiée selon la revendication 11, dans laquelle :
i) le marqueur sélectionnable négatif comprend la thymidine kinase du virus herpès simplex et l'inducteur comprend le ganciclovir ;
ii) le marqueur sélectionnable négatif comprend la cytosine désaminase et l'inducteur comprend la 5-fluorocytosine ;
iii) le marqueur sélectionnable négatif comprend la carboxyl estérase et l'inducteur comprend l'irinotécan ; ou
iv) le marqueur sélectionnable négatif comprend l'iCasp9 et l'inducteur comprend l'AP1903.

16. Cellule animale isolée génétiquement modifiée selon l'une quelconque des revendications 10 à 15, dans laquelle :
a) la cellule animale est une cellule de mammifère choisie dans le groupe consistant en une cellule d'un humain, de souris, de rat, de hamster, de cobaye, de chat, de chien, de vache, de cheval, de cerf, d'élan, de bison, de bœufs, de chameau, de lama, de lapin, de porc, de chèvre, de mouton, et de primate non humain, ou est une cellule aviaire ; ou
b) la cellule animale est ou dérive d'une cellule souche pluripotente, d'une cellule multipotente ou d'une cellule progénitrice monopotente, ou est une cellule à différenciation terminale.

17. Vecteur à ADN pour la modification de l'expression d'un gène endogène choisi dans le groupe consistant en Cdkl, Top2A, Cenpa, Birc5 et Eef2 dans une cellule animale, comprenant une séquence d'ADN codant pour un marqueur sélectionnable négatif qui est transcriptionnellement lié à une séquence d'ADN codant pour le gène,
dans lequel l'intégration de la séquence d'ADN codant pour le marqueur sélectionnable négatif qui est transcriptionnellement lié à une séquence d'ADN codant pour le gène dans une ou plusieurs cellules hôtes favorise la mort des cellules hôtes proliférantes après exposition des cellules hôtes à un inducteur du marqueur sélectionnable négatif.

18. Vecteur à ADN selon la revendication 17, dans lequel le marqueur sélectionnable négatif comprend la thymidine kinase du virus herpès simplex, la cytosine désaminase, la carboxyl estérase ou l'iCasp9, le marqueur sélectionnable négatif étant de préférence la thymidine kinase du virus herpès simplex

19. Vecteur à ADN selon la revendication 18, dans lequel le gène est Cdkl et le marqueur sélectionnable négatif est la thymidine kinase du virus herpès simplex.

20. Vecteur à ADN selon la revendication 18, dans lequel :
i) le marqueur sélectionnable négatif comprend la thymidine kinase du virus herpès simplex et l'inducteur comprend le ganciclovir ;
ii) le marqueur sélectionnable négatif comprend la cytosine désaminase et l'inducteur comprend la 5-fluorocytosine ;
iii) le marqueur sélectionnable négatif comprend la carboxyl estérase et l'inducteur comprend l'irinotécan ; ou
iv) le marqueur sélectionnable négatif comprend l'iCasp9 et l'inducteur comprend l'AP1903.

21. Cellule animale selon l'une quelconque des revendications 10 à 16 pour une utilisation en médecine.
